# EUROPEAN PATENT SPECIFICATION

(11) **EP 1 970 442 B1**
(45) Date of publication and mention of the grant of the patent: **18.01.2017**
(21) Application number: 08010039.9
(22) Date of filing: 05.05.2003
(51) Int. Cl.: C12N 9/88, C12N 15/82

(54) **TRANSGENIC HIGH TRYPTOPHAN PLANTS**
TRANSGENE TRYPTOPHANREICHE PFLANZEN
PLANTES TRYPTOPHANES TRANSGÉNIQUES

(30) Priority: 03.05.2002 US 377727 P; 05.05.2003 US 430011
(43) Date of publication of application: 17.09.2008
(62) Divisional of application: 03747662.9
(73) Proprietor: Monsanto Technology LLC, St. Louis, MO 63167 (US)
(72) Inventor: Weaver, Lisa M., O'Fallon, MO 63366 (US); Oulmassov, Tim N., Chesterfield, MO 63005 (US); Vaduva, Gabriela, Wildwood, MO 63011 (US); Liang, Jihong, Chesterfield, MO 63017 (US); Varagona, Marguerite J., Ballwin, MO 63021 (US); Venkatesh, Tyamagondlu V., St. Louis, MO 63146 (US); Manjunath, Sivalinganna, Chesterfield, MO 63017 (US)
(74) Representative: Uexküll & Stolberg

(56) References cited:
- WO-A-97/26366
- WO-A-02/090497
- US-A- 6 118 047
- US-B1- 6 271 016
- TOZAWA Y. ET AL.: "Characterization of rice anthranilate synthase alfa-subunit genes OASA1 and OASA2. Tryptophan accumulation in transgenic rice expressing a feedback-insensitive mutant of OASA1" PLANT PHYSIOLOGY, vol. 126, August 2001 (2001-08), pages 1493-1506, XP002428790
- CHO H-J ET AL: "Increasing tryptophan synthesis in a forage legume Astragalus sinicus by expressing the tobacco feedback-insensitive anthranilate synthase (ASA2) gene" PLANT PHYSIOLOGY, AMERICAN SOCIETY OF PLANT PHYSIOLOGISTS, ROCKVILLE, MD, US, vol. 123, no. 3, July 2000 (2000-07), pages 1069-1076, XP002349597 ISSN: 0032-0889
- DELLAPENNA D.: "Plant metabolic ingineering" PLANT PHYSIOLOGY, vol. 125, January 2001 (2001-01), pages 160-163, XP002428791
- G SARWAR: 'Plant Foods for Human Nutrition Influence of tryptophan supplementation of soy-based infant formulas on protein quality and on blood and brain tryptophan and brain serotonin in the rat model *', [Online] 01 January 2001, pages 275 - 284, XP055182167 Retrieved from the Internet: <URL:http://download-v2.springer.com/static /pdf/31/art%3A10.1023%2FA%3A1011121111899.p df?token2=exp=1428657006~acl=/static/pdf/31 /art%253A10.1023%252FA%253A1011121111899.pd f*~hmac=e4e39382e264511324d6c7778e955d356b4 9f321124d8d245e955b1b06aac7ba> [retrieved on 2015-04-10]
- 'The', [Online] 01 January 2001, XP055182153 Retrieved from the Internet: <URL:http://jeb.biologists.org/content/204/ 22/3867.full.pdf> [retrieved on 2015-04-10]

### SEQUENCE LISTING

<110> Monsanto Technology, LLC Weaver, Lisa Oulmassov, Tim Vaduva, Gabriela Liang, Jihong Varagona, Rita Venkatesh, Tyamagondlu
<120> Transgenic High Tryptophan Plants
<130> 51857R
<160> 143
<170> PatentIn version 3.1
<210> 1
   <211> 2190
   <212> DNA
   <213> Agrobacterium tumefaciens
<400> 1
   atggtaacga tcattcagga tgacggagcg gagacctacg agacgaaagg cggcatccag     60
   gtcagccgaa agcgccggcc caccgattat gccaacgcca tcgataatta catcgaaaag    120
   cttgattccc atcgcggcgc ggttttttcg tccaactatg aatatccggg ccgttacacc    180
   cgctgggata cggccatcgt cgatccgccg ctcggcattt cctgttttgg ccgcaagatg    240
   tggatcgaag cctataatgg ccgcggcgaa gtgctgctcg atttcattac ggaaaagctg    300
   aaggcgacac ccgatctcac cctcggcgct tcctcgaccc gccggctcga tcttaccgtc    360
   aacgaaccgg accgtgtctt caccgaagaa gaacgctcga aaatcccgac ggtcttcacc    420
   gctctcagag ccatcgtcga cctcttctat tcgagcgcgg attcggccat cggcctgttc    480
   ggtgccttcg gttacgatct cgccttccag ttcgacgcga tcaagctttc gctggcgcgt    540
   ccggaagacc agcgtgacat ggtgctgttt ctgcccgatg aaatcctcgt cgttgatcac    600
   tattccgcca aggcctggat cgaccgttac gatttcgaga aggacggcat gacgacggac    660
   ggcaaatcct ccgacattac ccccgatccc ttcaagacca ccgataccat cccgcccaag    720
   ggcgatcacc gtcccggcga atattccgag cttgtggtga aggccaagga aagcttccgc    780
   cgcggcgacc tgttcgaggt cgttcccggc cagaaattca tggagcgttg cgaaagcaat    840
   ccgtcggcga tttcccgccg cctgaaggcg atcaacccgt cgccctattc cttcttcatc    900
   aatctcggcg atcaggaata tctggtcggc gcctcgccgg aaatgttcgt gcgcgtctcc    960
   ggccgtcgca tcgagacctg cccgatatca ggcaccatca agcgcggcga cgatccgatt   1020
   gccgacagcg agcagatttt gaaactgctc aactcgaaaa aggacgaatc cgaactgacc   1080
   atgtgctcgg acgtggaccg caacgacaag agccgcgtct gcgagccggg ttcggtgaag   1140
   gtcattggcc gccgccagat cgagatgtat tcacgcctca tccacaccgt cgatcacatc   1200
   gaaggccgcc tgcgcgacga tatggacgcc tttgacggtt tcctcagcca cgcctgggcc   1260
   gtcaccgtca ccggtgcacc aaagctgtgg gccatgcgct tcatcgaagg tcatgaaaag   1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 2
   <211> 1815
   <212> DNA
   <213> Zea mays
<400> 2
   atggaatccc tagccgccac ctccgtgttc gcgccctccc gcgtcgccgt cccggcggcg 60
   cgggccctgg ttagggcggg gacggtggta ccaaccaggc ggacgagcag ccggagcgga 120
   accagcgggg tgaaatgctc tgctgccgtg acgccgcagg cgagcccagt gattagcagg 180
   agcgctgcgg cggcgaaggc ggcggaggag gacaagaggc ggttcttcga ggcggcggcg 240
   cgggggagcg ggaaggggaa cctggtgccc atgtgggagt gcatcgtgtc ggaccatctc 300
   acccccgtgc tcgcctaccg ctgcctcgtc cccgaggaca acgtcgacgc ccccagcttc 360
   ctcttcgagt ccgtcgagca ggggccccag ggcaccacca acgtcggccg ctatagcatg 420
   gtgggagccc acccagtgat ggagattgtg gccaaagacc acaaggttac gatcatggac 480
   cacgagaaga gccaagtgac agagcaggta gtggacgacc cgatgcagat cccgaggacc 540
   atgatggagg gatggcaccc acagcagatc gacgagctcc ctgaatcctt ctccggtgga 600
   tgggttgggt tcttttccta tgatacggtt aggtatgttg agaagaagaa gctaccgttc 660
   tccagtgctc ctcaggacga taggaacctt cctgatgtgc acttgggact ctatgatgat 720
   gttctagtct tcgataatgt tgagaagaaa gtatatgtta tccattgggt caatgtggac 780
   cggcatgcat ctgttgagga agcataccaa gatggcaggt cccgactaaa catgttgcta 840
   tctaaagtgc acaattccaa tgtccccaca ctctctcctg gatttgtgaa gctgcacaca 900
   cgcaagtttg gtacaccttt gaacaagtcg accatgacaa gtgatgagta taagaatgct 960
   gttctgcagg ctaaggaaca tattatggct ggggatatct tccagattgt tttaagccag 1020
   aggttcgaga gacgaacata tgccaaccca tttgaggttt atcgagcatt acggattgtg 1080
   aatcctagcc catacatggc gtatgtacag gcaagaggct gtgtattggt tgcgtctagt 1140
   cctgaaattc ttacacgagt cagtaagggg aagattatta atcgaccact tgctggaact 1200
   gttcgaaggg gcaagacaga gaaggaagat caaatgcaag agcagcaact gttaagtgat 1260
   gaaaaacagt gtgccgagca cataatgctt gtggacttgg gaaggaatga tgttggcaag 1320
   gtatccaaac caggatcagt gaaggtggag aagttgatga acattgagag atactcccat 1380
   gttatgcaca tcagctcaac ggttagtgga cagttggatg atcatctcca gagttgggat 1440
   gccttgagag ctgccttgcc cgttggaaca gtcagtggtg caccaaaggt gaaggccatg 1500
   gagttgattg ataagttgga agttacgagg cgaggaccat atagtggtgg tctaggagga 1560
   atatcgtttg atggtgacat gcaaattgca ctttctctcc gcaccatcgt attctcaaca 1620
   gcgccgagcc acaacacgat gtactcatac aaagacgcag ataggcgtcg ggagtgggtc 1680
   gctcatcttc aggctggtgc aggcattgtt gccgacagta gcccagatga cgaacaacgt 1740
   gaatgcgaga ataaggctgc tgcactagct cgggccatcg atcttgcaga gtcagctttt 1800
   gtagacaaag aatag 1815
<210> 3
   <211> 1993
   <212> DNA
   <213> Ruta graveolens
<400> 3
   aaaaaatctg tctgtttttc gtgtttggac atttcagcgg cactgggtgc catcagttga     60
   ttcgactcat ttgatttatt ttgtttgttg gccatgagtg cagcggcaac gtcgatgcaa    120
   tcccttaaat tctccaaccg tctggtccca cccagtcgcc gtctgtctcc ggttccgaac    180
   aatgtcacct gcaataacct ccccaagtct gcagctcccg tccggacagt caaatgctgc    240
   gcttcttcct ggaacagtac catcaacggc gcggccgcca cgaccaacgg tgcgtccgcc    300
   gccagtaacg gcgcatccac gaccaccact acatatgtta gtgatgcaac cagatttatc    360
   gactcttcta aaagggcaaa tctagtgcca ttataccgtt gcatattcgc ggatcatctc    420
   acgccggtgc ttgcctatag atgtttggtt caagaagacg ataaagagac tccaagtttt    480
   ttattcgaat cagtagagcc gggtcggatt tctactgttg ggaggtatag tgtggttgga    540
   gctcatcccg tgatggaagt tatagctaaa gataatatgg ttacggtgat ggatcatgag    600
   aaagggagct tagttgagga ggtggtcgat gatcccatgg agattcctag aagaatttcc    660
   gaggattgga agcctcaaat aatcgatgat cttcctgaag ctttttgcgg tggttgggtt    720
   ggtttcttct catacgatac agttcgatat gtggagaaga aaaagttacc attctcaaag    780
   gcacctcagg atgataggaa tcttgcagat atgcatctag gtctctataa cgatgttatt    840
   gtgtttgatc atgtggaaaa gaaagtatat gttattcatt gggtgaggct aaatcaacag    900
   tcttctgaag aaaaagcata tgccgagggt ctggaacact tggagagact agtatccaga    960
   gtacaggatg agaacacgcc aaggctcgcc ccaggttcca tagacttaca cactggtcat   1020
   tttggacctc cattaaaaaa gtcaaacatg acatgtgaag aatacaaaat ggctgtacta   1080
   gcggcaaaag aacatattca ggctggggat atttttcaaa tcgtactaag ccaacgtttt   1140
   gaacgtcgaa catttgctga tccatttgaa gtttataggg cactgagagt tgttaatccg   1200
   agtccctata tgacgtatat gcaggcaaga gggtgtgttc tggtagcttc aagtccagaa   1260
   attcttactc gagtaaagaa gaataagatt gtgaatcgac ctttggctgg aacagcccga   1320
   agagggagga ctactgaaga agatgagatg ttggaaacac agttgctaaa agacgcaaag   1380
   caatgtgctg agcatgttat gctggtcgat ttgggacgga atgatgttgg caaggtttca   1440
   aaatctggtt ctgtgaaagt ggaaaagctg atgaatgttg aacgatattc acatgttatg   1500
   cacataagct ctacggtcac aggtgagttg caagataatc tcagttgctg ggatgccctg   1560
   cgtgctgcac tgcctgtcgg gactgttagt ggagcaccaa aggtgaaggc aatggagtta   1620
   atcgatgaat tggaggtaaa tagacgtggc ccctacagtg gtgggtttgg cggtatctcc   1680
   ttcaccggag atatggacat tgccctggct ctaaggacca ttgttttcca aaccggtaca   1740
   cgctatgaca caatgtactc gtacaagaat gctaccaaac gccggcagtg ggtggcatac   1800
   cttcaagccg gggctggcat tgttgctgat agtgatccag acgacgagca tcgtgagtgc   1860
   cagaacaaag ccgccggact ggcccgtgcc atcgacctag ctgagtctgc ttttgtgaac   1920
   aaatcaagta gctaaagttt tggatttgga agtggagttg agtctcggat aggatttaga   1980
   gtaaaaaaag agg                                                      1993
<210> 4
   <211> 729
   <212> PRT
   <213> Agrobacterium tumefaciens
<400> 4
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Ser Ser Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
                   85                  90                  95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 6
   <211> 613
   <212> PRT
   <213> Ruta graveolens
<400> 6
   Met Ser Ala Ala Ala Thr Ser Met Gln Ser Leu Lys Phe Ser Asn Arg
   1               5                   10                  15
   Leu Val Pro Pro Ser Arg Arg Leu Ser Pro Val Pro Asn Asn Val Thr
               20                  25                  30
   Cys Asn Asn Leu Pro Lys Ser Ala Ala Pro Val Arg Thr Val Lys Cys
           35                  40                  45
   Cys Ala Ser Ser Trp Asn Ser Thr Ile Asn Gly Ala Ala Ala Thr Thr
   50 55 60
   Asn Gly Ala Ser Ala Ala Ser Asn Gly Ala Ser Thr Thr Thr Thr Thr
   65 70 75 80
   Tyr Val Ser Asp Ala Thr Arg Phe Ile Asp Ser Ser Lys Arg Ala Asn
   85 90 95
   Leu Val Pro Leu Tyr Arg Cys Ile Phe Ala Asp His Leu Thr Pro Val
   100 105 110
   Leu Ala Tyr Arg Cys Leu Val Gln Glu Asp Asp Lys Glu Thr Pro Ser
   115 120 125
   Phe Leu Phe Glu Ser Val Glu Pro Gly Arg Ile Ser Thr Val Gly Arg
   130 135 140
   Tyr Ser Val Val Gly Ala His Pro Val Met Glu Val Ile Ala Lys Asp
   145 150 155 160
   Asn Met Val Thr Val Met Asp His Glu Lys Gly Ser Leu Val Glu Glu
   165 170 175
   Val Val Asp Asp Pro Met Glu Ile Pro Arg Arg Ile Ser Glu Asp Trp
   180 185 190
   Lys Pro Gln Ile Ile Asp Asp Leu Pro Glu Ala Phe Cys Gly Gly Trp
   195 200 205
   Val Gly Phe Phe Ser Tyr Asp Thr Val Arg Tyr Val Glu Lys Lys Lys
   210 215 220
   Leu Pro Phe Ser Lys Ala Pro Gln Asp Asp Arg Asn Leu Ala Asp Met
   225 230 235 240
   His Leu Gly Leu Tyr Asn Asp Val Ile Val Phe Asp His Val Glu Lys
   245 250 255
   Lys Val Tyr Val Ile His Trp Val Arg Leu Asn Gln Gln Ser Ser Glu
   260 265 270
   Glu Lys Ala Tyr Ala Glu Gly Leu Glu His Leu Glu Arg Leu Val Ser
   275 280 285
   Arg Val Gln Asp Glu Asn Thr Pro Arg Leu Ala Pro Gly Ser Ile Asp
   290 295 300
   Leu His Thr Gly His Phe Gly Pro Pro Leu Lys Lys Ser Asn Met Thr
   305 310 315 320
   Cys Glu Glu Tyr Lys Met Ala Val Leu Ala Ala Lys Glu His Ile Gln
   325 330 335
   Ala Gly Asp Ile Phe Gln Ile Val Leu Ser Gln Arg Phe Glu Arg Arg
   340 345 350
   Thr Phe Ala Asp Pro Phe Glu Val Tyr Arg Ala Leu Arg Val Val Asn
   355 360 365
   Pro Ser Pro Tyr Met Thr Tyr Met Gln Ala Arg Gly Cys Val Leu Val
   370 375 380
   Ala Ser Ser Pro Glu Ile Leu Thr Arg Val Lys Lys Asn Lys Ile Val
   385 390 395 400
   Asn Arg Pro Leu Ala Gly Thr Ala Arg Arg Gly Arg Thr Thr Glu Glu
   405 410 415
   Asp Glu Met Leu Glu Thr Gln Leu Leu Lys Asp Ala Lys Gln Cys Ala
   420 425 430
   Glu His Val Met Leu Val Asp Leu Gly Arg Asn Asp Val Gly Lys Val
   435 440 445
   Ser Lys Ser Gly Ser Val Lys Val Glu Lys Leu Met Asn Val Glu Arg
   450 455 460
   Tyr Ser His Val Met His Ile Ser Ser Thr Val Thr Gly Glu Leu Gln
   465 470 475 480
   Asp Asn Leu Ser Cys Trp Asp Ala Leu Arg Ala Ala Leu Pro Val Gly
   485 490 495
   Thr Val Ser Gly Ala Pro Lys Val Lys Ala Met Glu Leu Ile Asp Glu
   500 505 510
   Leu Glu Val Asn Arg Arg Gly Pro Tyr Ser Gly Gly Phe Gly Gly Ile
   515 520 525
   Ser Phe Thr Gly Asp Met Asp Ile Ala Leu Ala Leu Arg Thr Ile Val
   530 535 540
   Phe Gln Thr Gly Thr Arg Tyr Asp Thr Met Tyr Ser Tyr Lys Asn Ala
   545 550 555 560
   Thr Lys Arg Arg Gln Trp Val Ala Tyr Leu Gln Ala Gly Ala Gly Ile
   565 570 575
   Val Ala Asp Ser Asp Pro Asp Asp Glu His Arg Glu Cys Gln Asn Lys
   580 585 590
   Ala Ala Gly Leu Ala Arg Ala Ile Asp Leu Ala Glu Ser Ala Phe Val
   595 600 605
   Asn Lys Ser Ser Ser
   610
<210> 7
   <211> 729
   <212> PRT
   <213> Rhizobium meliloti
<400> 7
   Met Ala Ala Val Ile Leu Glu Asp Gly Ala Glu Ser Tyr Thr Thr Lys
   1 5 10 15
   Gly Gly Ile Val Val Thr Arg Arg Arg Arg Glu Ala Ser Tyr Ser Asp
   20 25 30
   Ala Ile Ala Gly Tyr Val Asp Arg Leu Asp Glu Arg Arg Gly Ala Val
   35 40 45
   Phe Ser Ser Asn Tyr Glu Tyr Pro Gly Arg Tyr Thr Arg Trp Asp Thr
   50 55 60
   Ala Val Val Asp Pro Pro Leu Ala Ile Ser Ser Phe Gly Arg Ser Leu
   65 70 75 80
   Trp Ile Glu Ala Tyr Asn Glu Arg Gly Glu Val Leu Leu Ala Leu Ile
   85 90 95
   Ala Glu Asp Leu Lys Ser Val Ala Asp Ile Thr Leu Gly Ser Leu Ala
   100 105 110
   Ala Arg Arg Leu Asp Leu Thr Ile Asn Glu Pro Asp Arg Val Phe Thr
   115 120 125
   Glu Glu Glu Arg Ser Lys Met Pro Thr Val Phe Thr Val Leu Arg Ala
   130 135 140
   Val Thr Asn Leu Phe His Ser Glu Glu Asp Ser Asn Leu Gly Leu Tyr
   145 150 155 160
   Gly Ala Phe Gly Tyr Asp Leu Ala Phe Gln Phe Asp Ala Ile Glu Leu
   165 170 175
   Lys Leu Ser Arg Pro Asp Asp Gln Arg Asp Met Val Leu Phe Leu Pro
   180 185 190
   Asp Glu Ile Leu Val Val Asp His Tyr Ala Ala Lys Ala Trp Ile Asp
   195 200 205
   Arg Tyr Asp Phe Ala Arg Glu Asn Leu Ser Thr Glu Gly Lys Ala Ala
   210 215 220
   Asp Ile Ala Pro Glu Pro Phe Arg Ser Val Asp Ser Ile Pro Pro His
   225 230 235 240
   Gly Asp His Arg Pro Gly Glu Tyr Ala Glu Leu Val Val Lys Ala Lys
   245 250 255
   Glu Ser Phe Arg Arg Gly Asp Leu Phe Glu Val Val Pro Gly Gln Lys
   260 265 270
   Phe Tyr Glu Arg Cys Glu Ser Arg Pro Ser Glu Ile Ser Asn Arg Leu
   275 280 285
   Lys Ala Ile Asn Pro Ser Pro Tyr Ser Phe Phe Ile Asn Leu Gly Asn
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Val Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Ser His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Tyr Asp Ser Asn Pro Glu Glu Glu Glu Ala Glu Thr
   485 490 495
   Glu Leu Lys Ala Ser Ala Met Ile Ala Ala Ile Arg Asp Ala Lys Ser
               500                 505                 510
   Ala Asn Ser Ala Lys Ser Ala Arg Asp Val Ala Ala Val Gly Ala Gly
           515                 520                 525
   Val Ser Ile Leu Leu Val Asp His Glu Asp Ser Phe Val His Thr Leu
       530                 535                 540
   Ala Asn Tyr Phe Arg Gln Thr Gly Ala Ser Val Thr Thr Val Arg Thr
   545                 550                 555                 560
   Pro Val Ala Glu Glu Ile Phe Asp Arg Val Lys Pro Asp Leu Val Val
                   565                 570                 575
   Leu Ser Pro Gly Pro Gly Thr Pro Lys Asp Phe Asp Cys Lys Ala Thr
               580                 585                 590
   Ile Lys Lys Ala Arg Ala Arg Asp Leu Pro Ile Phe Gly Val Cys Leu
           595                 600                 605
   Gly Leu Gln Ala Leu Ala Glu Ala Tyr Gly Gly Asp Leu Arg Gln Leu
       610                 615                 620
   Ala Ile Pro Met His Gly Lys Pro Ser Arg Ile Arg Val Leu Glu Pro
   625                 630                 635                 640
   Gly Ile Val Phe Ser Gly Leu Gly Lys Glu Val Thr Val Gly Arg Tyr
                   645                 650                 655
   His Ser Ile Phe Ala Asp Pro Ser Asn Leu Pro Arg Glu Phe Val Ile
               660                 665                 670
   Thr Ala Glu Ser Glu Asp Gly Thr Ile Met Gly Ile Glu His Ser Lys
           675                 680                 685
   Glu Pro Val Ala Ala Val Gln Phe His Pro Glu Ser Ile Met Thr Leu
       690                 695                 700
   Gly Gly Asp Ala Gly Met Arg Met Ile Glu Asn Val Val Ala His Leu
   705                 710                 715                 720
   Ala Lys Arg Ala Lys Thr Lys Ala Ala
                   725
<210> 8
   <211> 421
   <212> PRT
   <213> Sulfolobus solfataricus
<400> 8
   Met Glu Val His Pro Ile Ser Glu Phe Ala Ser Pro Phe Glu Val Phe
   1               5                   10                  15
   Lys Cys Ile Glu Arg Asp Phe Lys Val Ala Gly Leu Leu Glu Ser Ile
               20                  25                  30
   Gly Gly Pro Gln Tyr Lys Ala Arg Tyr Ser Val Ile Ala Trp Ser Thr
           35                  40                  45
   Asn Gly Tyr Leu Lys Ile His Asp Asp Pro Val Asn Ile Leu Asn Gly
       50                  55                  60
   Tyr Leu Lys Asp Leu Lys Leu Ala Asp Ile Pro Gly Leu Phe Lys Gly
   65                  70                  75                  80
   Gly Met Ile Gly Tyr Ile Ser Tyr Asp Ala Val Arg Phe Trp Glu Lys
                   85                  90                  95
   Ile Arg Asp Leu Lys Pro Ala Ala Glu Asp Trp Pro Tyr Ala Glu Phe
               100                 105                 110
   Phe Thr Pro Asp Asn Ile Ile Ile Tyr Asp His Asn Glu Gly Lys Val
           115                 120                 125
   Tyr Val Asn Ala Asp Leu Ser Ser Val Gly Gly Cys Gly Asp Ile Gly
       130                 135                 140
   Glu Phe Lys Val Ser Phe Tyr Asp Glu Ser Leu Asn Lys Asn Ser Tyr
   145                 150                 155                 160
   Glu Arg Ile Val Ser Glu Ser Leu Glu Tyr Ile Arg Ser Gly Tyr Ile
                   165                 170                 175
   Phe Gln Val Val Leu Ser Arg Phe Tyr Arg Tyr Ile Phe Ser Gly Asp
               180                 185                 190
   Pro Leu Arg Ile Tyr Tyr Asn Leu Arg Arg Ile Asn Pro Ser Pro Tyr
           195                 200                 205
   Met Phe Tyr Leu Lys Phe Asp Glu Lys Tyr Leu Ile Gly Ser Ser Pro
       210                 215                 220
   Glu Leu Leu Phe Arg Val Gln Asp Asn Ile Val Glu Thr Tyr Pro Ile
   225                 230                 235                 240
   Ala Gly Thr Arg Pro Arg Gly Ala Asp Gln Glu Glu Asp Leu Lys Leu
                   245                 250                 255
   Glu Leu Glu Leu Met Asn Ser Glu Lys Asp Lys Ala Glu His Leu Met
               260                 265                 270
   Leu Val Asp Leu Ala Arg Asn Asp Leu Gly Lys Val Cys Val Pro Gly
   275 280 285
   Thr Val Lys Val Pro Glu Leu Met Tyr Val Glu Lys Tyr Ser His Val
   290 295 300
   Gln His Ile Val Ser Lys Val Ile Gly Thr Leu Lys Lys Lys Tyr Asn
   305 310 315 320
   Ala Leu Asn Val Leu Ser Ala Thr Phe Pro Ala Gly Thr Val Ser Gly
   325 330 335
   Arg Pro Lys Pro Met Ala Met Asn Ile Ile Glu Thr Leu Glu Glu Tyr
   340 345 350
   Lys Arg Gly Pro Tyr Ala Gly Ala Val Gly Phe Ile Ser Ala Asp Gly
   355 360 365
   Asn Ala Glu Phe Ala Ile Ala Ile Arg Thr Ala Phe Leu Asn Lys Glu
   370 375 380
   Leu Leu Arg Ile His Ala Gly Ala Gly Ile Val Tyr Asp Ser Asn Pro
   385 390 395 400
   Glu Ser Glu Tyr Phe Glu Thr Glu His Lys Leu Lys Ala Leu Lys Thr
   405 410 415
   Ala Ile Gly Val Arg
   420
<210> 9
   <211> 32
   <212> DNA
   <213> Artificial sequence
<220>
   <223> A primer.
<400> 9
   ccatcgcggc gcgttttttt cgtccaacta tg 32
<210> 10
   <211> 32
   <212> DNA
   <213> Artificial sequence
<220>
   <223> A primer.
<400> 10
   catagttgga cgaaaaaaac gcgccgcgat gg 32
<210> 11
   <211> 39
   <212> DNA
   <213> Artificial Sequence
<220>
   <223> A primer.
<400> 11
   ccatcgcggc gcgtattttt cgtccaacta tgaatatcc 39
<210> 12
   <211> 39
   <212> DNA
   <213> Artificial Sequence
<220>
   <223> A primer.
<400> 12
   ggatattcat agttggacga aaaatacgcg ccgcgatgg 39
<210> 13
   <211> 39
   <212> DNA
   <213> Artificial Sequence
<220>
   <223> A primer.
<400> 13
   ccatcgcggc gcgtggtttt cgtccaacta tgaatatcc 39
<210> 14
   <211> 39
   <212> DNA
   <213> Artificial sequence
<220>
   <223> A primer.
<400> 14
   ggatattcat agttggacga aaaccacgcg ccgcgatgg 39
<210> 15
   <211> 39
   <212> DNA
   <213> Artificial sequence
<220>
   <223> A primer.
<400> 15
   ccatcgcggc gcggttttta agtccaacta tgaatatcc 39
<210> 16
   <211> 39
   <212> DNA
   <213> Artificial Sequence
<220>
   <223> A primer.
<400> 16
   ggatattcat agttggactt aaaaaccgcg ccgcgatgg 39
<210> 17
   <211> 34
   <212> DNA
   <213> Artificial Sequence
<220>
   <223> A primer.
<400> 17
   gcgcggtttt ttcgtgcaac tatgaatatc cggg 34
<210> 18
   <211> 34
   <212> DNA
   <213> Artificial sequence
<220>
   <223> A primer.
<400> 18
   cccggatatt catagttgca cgaaaaaacc gcgc 34
<210> 19
   <211> 34
   <212> DNA
   <213> Artificial Sequence
<220>
   <223> A primer.
<400> 19
   cgcggttttt tcgttcaact atgaatatcc gggc 34
<210> 20
   <211> 34
   <212> DNA
   <213> Artificial Sequence
<220>
   <223> A primer.
<400> 20
   gcccggatat tcatagttga acgaaaaaac cgcg 34
<210> 21
   <211> 37
   <212> DNA
   <213> Artificial sequence
<220>
   <223> A primer.
<400> 21
   cggcgcggtt ttttcgatca actatgaata tccgggc 37
<210> 22
   <211> 37
   <212> DNA
   <213> Artificial sequence
<220>
   <223> A primer.
<400> 22
   gcccggatat tcatagttga tcgaaaaaac cgcgccg 37
<210> 23
   <211> 36
   <212> DNA
   <213> Artificial sequence
<220>
   <223> A primer.
<400> 23
   ggcgcggttt tttcgctcaa ctatgaatat ccgggc 36
<210> 24
   <211> 36
   <212> DNA
   <213> Artificial sequence
<220>
   <223> A primer.
<400> 24
   gcccggatat tcatagttga gcgaaaaaac cgcgcc 36
<210> 25
   <211> 39
   <212> DNA
   <213> Artificial sequence
<220>
   <223> A primer.
<400> 25
   cggcgcggtt ttttcgatga actatgaata tccgggccg 39
<210> 26
   <211> 39
   <212> DNA
   <213> Artificial sequence
<220>
   <223> A primer.
<400> 26
   cggcccggat attcatagtt catcgaaaaa accgcgccg 39
<210> 27
   <211> 34
   <212> DNA
   <213> Artificial sequence
<220>
   <223> A primer.
<400> 27
   cgcggttttt tcgaccaact atgaatatcc gggc 34
<210> 28
   <211> 34
   <212> DNA
   <213> Artificial sequence
<220>
   <223> A primer.
<400> 28
   gcccggatat tcatagttgg tcgaaaaaac cgcg 34
<210> 29
   <211> 36
   <212> DNA
   <213> Artificial Sequence
<220>
   <223> A primer.
<400> 29
   ggcgcggttt tttcggtcaa ctatgaatat ccgggc 36
<210> 30
   <211> 36
   <212> DNA
   <213> Artificial sequence
<220>
   <223> A primer.
<400> 30
   gcccggatat tcatagttga ccgaaaaaac cgcgcc 36
<210> 31
   <211> 35
   <212> DNA
   <213> Artificial Sequence
<220>
   <223> A primer.
<400> 31
   gcgcggtttt ttcgtacaac tatgaatatc cgggc 35
<210> 32
   <211> 35
   <212> DNA
   <213> Artificial sequence
<220>
   <223> A primer.
<400> 32
   gcccggatat tcatagttgt acgaaaaaac cgcgc 35
<210> 33
   <211> 36
   <212> DNA
   <213> Artificial Sequence
<220>
   <223> A primer.
<400> 33
   cggcgcggtt ttttcgtcct tctatgaata tccggg 36
<210> 34
   <211> 36
   <212> DNA
   <213> Artificial Sequence
<220>
   <223> A primer.
<400> 34
   cccggatatt catagaagga cgaaaaaacc gcgccg 36
<210> 35
   <211> 29
   <212> DNA
   <213> Artificial sequence
<220>
   <223> A primer.
<400> 35
   ctgaaggcga tcaacgcgtc gccctattc 29
<210> 36
   <211> 29
   <212> DNA
   <213> Artificial Sequence
<220>
   <223> A primer.
<400> 36
   gaatagggcg acgcgttgat cgccttcag 29
<210> 37
   <211> 31
   <212> DNA
   <213> Artificial Sequence
<220>
   <223> A primer.
<400> 37
   cctgaaggcg atcaacgggt cgccctattc c 31
<210> 38
   <211> 31
   <212> DNA
   <213> Artificial sequence
<220>
   <223> A primer.
<400> 38
   ggaatagggc gacccgttga tcgccttcag g 31
<210> 39
   <211> 33
   <212> DNA
   <213> Artificial Sequence
<220>
   <223> A primer.
<400> 39
   cgtcgcccta ttccgccttc atcaatctcg gcg 33
<210> 40
   <211> 33
   <212> DNA
   <213> Artificial Sequence
<220>
   <223> A primer.
<400> 40
   cgccgagatt gatgaaggcg gaatagggcg acg 33
<210> 41
   <211> 33
   <212> DNA
   <213> Artificial Sequence
<220>
   <223> A primer.
<400> 41
   cgtcgcccta ttcctggttc atcaatctcg gcg 33
<210> 42
   <211> 33
   <212> DNA
   <213> Artificial Sequence
<220>
   <223> A primer.
<400> 42
   cgccgagatt gatgaaccag gaatagggcg acg 33
<210> 43
   <211> 729
   <212> PRT
   <213> Rhizobium meliloti
<400> 43
   Met Ala Ala Val Ile Leu Glu Asp Gly Ala Glu Ser Tyr Thr Thr Lys
   1               5                   10                  15
   Gly Gly Ile Val Val Thr Arg Arg Arg Arg Glu Ala Ser Tyr Ser Asp
               20                  25                  30
   Ala Ile Ala Gly Tyr Val Asp Arg Leu Asp Glu Arg Arg Gly Ala Val
           35                  40                  45
   Phe Ser Ser Asn Tyr Glu Tyr Pro Gly Arg Tyr Thr Arg Trp Asp Thr
       50                  55                  60
   Ala Val Val Asp Pro Pro Leu Ala Ile Ser Ser Phe Gly Arg Ser Leu
   65                  70                  75                  80
   Trp Ile Glu Ala Tyr Asn Glu Arg Gly Glu Val Leu Leu Ala Leu Ile
   85 90 95
   Ala Glu Asp Leu Lys Ser Val Ala Asp Ile Thr Leu Gly Ser Leu Ala
   100 105 110
   Ala Arg Arg Leu Asp Leu Thr Ile Asn Glu Pro Asp Arg Val Phe Thr
   115 120 125
   Glu Glu Glu Arg Ser Lys Met Pro Thr Val Phe Thr Val Leu Arg Ala
   130 135 140
   Val Thr Asn Leu Phe His Ser Glu Glu Asp Ser Asn Leu Gly Leu Tyr
   145 150 155 160
   Gly Ala Phe Gly Tyr Asp Leu Ala Phe Gln Phe Asp Ala Ile Glu Leu
   165 170 175
   Lys Leu Ser Arg Pro Asp Asp Gln Arg Asp Met Val Leu Phe Leu Pro
   180 185 190
   Asp Glu Ile Leu Val Val Asp His Tyr Ala Ala Lys Ala Trp Ile Asp
   195 200 205
   Arg Tyr Asp Phe Ala Arg Glu Asn Leu Ser Thr Glu Gly Lys Ala Ala
   210 215 220
   Asp Ile Ala Pro Glu Pro Phe Arg Ser Val Asp Ser Ile Pro Pro His
   225 230 235 240
   Gly Asp His Arg Pro Gly Glu Tyr Ala Glu Leu Val Val Lys Ala Lys
   245 250 255
   Glu Ser Phe Arg Arg Gly Asp Leu Phe Glu Val Val Pro Gly Gln Lys
   260 265 270
   Phe Tyr Glu Arg Cys Glu Ser Arg Pro Ser Glu Ile Ser Asn Arg Leu
   275 280 285
   Lys Ala Ile Asn Pro Ser Pro Tyr Ser Phe Phe Ile Asn Leu Gly Asn
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Val Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Ser His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Tyr Asp Ser Asn Pro Glu Glu Glu Glu Ala Glu Thr
   485 490 495
   Glu Leu Lys Ala Ser Ala Met Ile Ala Ala Ile Arg Asp Ala Lys Ser
   500 505 510
   Ala Asn Ser Ala Lys Ser Ala Arg Asp Val Ala Ala Val Gly Ala Gly
   515 520 525
   Val Ser Ile Leu Leu Val Asp His Glu Asp Ser Phe Val His Thr Leu
   530 535 540
   Ala Asn Tyr Phe Arg Gln Thr Gly Ala Ser Val Thr Thr Val Arg Thr
   545 550 555 560
   Pro Val Ala Glu Glu Ile Phe Asp Arg Val Lys Pro Asp Leu Val Val
   565 570 575
   Leu Ser Pro Gly Pro Gly Thr Pro Lys Asp Phe Asp Cys Lys Ala Thr
   580 585 590
   Ile Lys Lys Ala Arg Ala Arg Asp Leu Pro Ile Phe Gly Val Cys Leu
   595 600 605
   Gly Leu Gln Ala Leu Ala Glu Ala Tyr Gly Gly Asp Leu Arg Gln Leu
   610 615 620
   Ala Ile Pro Met His Gly Lys Pro Ser Arg Ile Arg Val Leu Glu Pro
   625 630 635 640
   Gly Ile Val Phe Ser Gly Leu Gly Lys Glu Val Thr Val Gly Arg Tyr
   645 650 655
   His Ser Ile Phe Ala Asp Pro Ser Asn Leu Pro Arg Glu Phe Val Ile
   660 665 670
   Thr Ala Glu Ser Glu Asp Gly Thr Ile Met Gly Ile Glu His Ser Lys
   675 680 685
   Glu Pro Val Ala Ala Val Gln Phe His Pro Glu Ser Ile Met Thr Leu
   690 695 700
   Gly Gly Asp Ala Gly Met Arg Met Ile Glu Asn Val Val Ala His Leu
   705 710 715 720
   Ala Lys Arg Ala Lys Thr Lys Ala Ala
   725
<210> 45
   <211> 897
   <212> PRT
   <213> Arabidopsis thaliana
<400> 45
   Met Ser Ala Val Ser Ile Ser Ala Val Lys Ser Asp Phe Phe Thr Val
   1               5                   10                  15
   Glu Ala Ile Ala Val Thr His His Arg Thr Pro His Pro Pro His Phe
               20                  25                  30
   Pro Ser Leu Arg Phe Pro Leu Ser Leu Lys Ser Pro Pro Ala Thr Ser
           35                  40                  45
   Leu Asn Leu Val Ala Gly Ser Lys Leu Leu His Phe Ser Arg Arg Leu
       50                  55                  60
   Pro Ser Ile Lys Cys Ser Tyr Thr Pro Ser Leu Asp Leu Ser Glu Glu
   65                  70                  75                  80
   Gln Phe Thr Lys Phe Lys Lys Ala Ser Glu Lys Gly Asn Leu Val Pro
                   85                  90                  95
   Leu Phe Arg Cys Val Phe Ser Asp His Leu Thr Pro Ile Leu Ala Tyr
               100                 105                 110
   Arg Cys Leu Val Lys Glu Asp Asp Arg Asp Ala Pro Ser Phe Leu Phe
           115                 120                 125
   Glu Ser Val Glu Pro Gly Ser Gln Ser Ser Asn Ile Gly Arg Tyr Ser
       130                 135                 140
   Val Val Gly Ala Gln Pro Thr Ile Glu Ile Val Ala Lys Gly Asn Val
   145                 150                 155                 160
   Val Thr Val Met Asp His Gly Ala Ser Leu Arg Thr Glu Glu Glu Val
                   165                 170                 175
   Asp Asp Pro Met Met Val Pro Gln Lys Ile Met Glu Glu Trp Asn Pro
               180                 185                 190
   Gln Gly Ile Asp Glu Leu Pro Glu Ala Phe Cys Gly Gly Trp Val Gly
           195                 200                 205
   Tyr Phe Ser Tyr Asp Thr Val Arg Tyr Val Glu Lys Lys Lys Leu Pro
       210                 215                 220
   Phe Ser Asn Ala Pro Glu Asp Asp Arg Ser Leu Pro Asp Val Asn Leu
   225                 230                 235                 240
   Gly Leu Tyr Asp Asp Val Ile Val Phe Asp His Val Glu Lys Lys Ala
                   245                 250                 255
   Tyr Val Ile His Trp Val Arg Ile Asp Lys Asp Arg Ser Val Glu Glu
               260                 265                 270
   Asn Phe Arg Glu Gly Met Asn Arg Leu Glu Ser Leu Thr Ser Arg Ile
           275                 280                 285
   Gln Asp Gln Lys Pro Pro Lys Met Pro Thr Gly Phe Ile Lys Leu Arg
       290                 295                 300
   Thr Gln Leu Phe Gly Pro Lys Leu Glu Lys Ser Thr Met Thr Ser Glu
   305                 310                 315                 320
   Ala Tyr Lys Glu Ala Val Val Glu Ala Lys Glu His Ile Leu Ala Gly
                   325                 330                 335
   Asp Ile Phe Gln Ile Val Leu Ser Gln Arg Phe Glu Arg Arg Thr Phe
               340                 345                 350
   Ala Asp Pro Phe Glu Ile Tyr Arg Ala Leu Arg Ile Val Asn Pro Ser
           355                 360                 365
   Pro Tyr Met Ala Tyr Leu Gln Val Arg Gly Cys Ile Leu Val Ala Ser
       370                 375                 380
   Ser Pro Glu Ile Leu Leu Arg Ser Lys Asn Arg Lys Ile Thr Asn Arg
   385                 390                 395                 400
   Pro Leu Ala Gly Thr Val Arg Arg Gly Lys Thr Pro Lys Glu Asp Leu
                   405                 410                 415
   Met Leu Glu Lys Glu Leu Leu Ser Asp Glu Lys Gln Cys Ala Glu His
               420                 425                 430
   Ile Met Leu Val Asp Leu Gly Arg Asn Asp Val Gly Lys Val Ser Lys
           435                 440                 445
   Pro Gly Ser Val Glu Val Lys Lys Leu Lys Asp Ile Glu Trp Phe Ser
       450                 455                 460
   His Val Met His Ile Ser Ser Thr Val Val Gly Glu Leu Leu Asp His
   465                 470                 475                 480
   Leu Thr Ser Trp Asp Ala Leu Arg Ala Val Leu Pro Val Gly Thr Val
                   485                 490                 495
   Ser Gly Ala Pro Lys Val Lys Ala Met Glu Leu Ile Asp Glu Leu Glu
               500                 505                 510
   Val Thr Arg Arg Gly Pro Tyr Ser Gly Gly Phe Gly Gly Ile Ser Phe
           515                 520                 525
   Asn Gly Asp Met Asp Ile Ala Leu Ala Leu Arg Thr Met Val Phe Pro
       530                 535                 540
   Thr Asn Thr Arg Tyr Asp Thr Leu Tyr Ser Tyr Lys His Pro Gln Arg
   545                 550                 555                 560
   Arg Arg Glu Trp Ile Ala His Ile Gln Ala Gly Ala Gly Ile Val Ala
                   565                 570                 575
   Asp Ser Asn Pro Asp Asp Glu His Arg Glu Cys Glu Asn Lys Ala Ala
   580 585 590
   Ala Leu Ala Arg Ala Ile Asp Leu Ala Glu Ser Ser Phe Leu Glu Ala
   595 600 605
   Pro Glu Phe Thr Thr Ile Thr Pro His Ile Asn Asn Ile Met Ala Ala
   610 615 620
   Ser Thr Leu Tyr Lys Ser Cys Leu Leu Gln Pro Lys Ser Gly Ser Thr
   625 630 635 640
   Thr Arg Arg Leu Asn Pro Ser Leu Val Asn Pro Leu Thr Asn Pro Thr
   645 650 655
   Arg Val Ser Val Leu Gly Lys Ser Arg Arg Asp Val Phe Ala Lys Ala
   660 665 670
   Ser Ile Glu Met Ala Glu Ser Asn Ser Ile Pro Ser Val Val Val Asn
   675 680 685
   Ser Ser Lys Gln His Gly Pro Ile Ile Val Ile Asp Asn Tyr Asp Ser
   690 695 700
   Phe Thr Tyr Asn Leu Cys Gln Tyr Met Gly Glu Leu Gly Cys His Phe
   705 710 715 720
   Glu Val Tyr Arg Asn Asp Glu Leu Thr Val Glu Glu Leu Lys Lys Lys
   725 730 735
   Asn Pro Arg Gly Val Leu Ile Ser Pro Gly Pro Gly Thr Pro Gln Asp
   740 745 750
   Ser Gly Ile Ser Leu Gln Thr Val Leu Glu Leu Gly Pro Leu Val Pro
   755 760 765
   Leu Phe Gly Val Cys Met Gly Leu Gln Cys Ile Gly Glu Ala Phe Gly
   770 775 780
   Gly Lys Ile Val Arg Ser Pro Phe Gly Val Met His Gly Lys Ser Ser
   785 790 795 800
   Met Val His Tyr Asp Glu Lys Gly Glu Glu Gly Leu Phe Ser Gly Leu
   805 810 815
   Ser Asn Pro Phe Ile Val Gly Arg Tyr His Ser Leu Val Ile Glu Lys
   820 825 830
   Asp Thr Phe Pro Ser Asp Glu Leu Glu Val Thr Ala Trp Thr Glu Asp
   835 840 845
   Gly Leu Val Met Ala Ala Arg His Arg Lys Tyr Lys His Ile Gln Gly
       850                 855                 860
   Val Gln Phe His Pro Glu Ser Ile Ile Thr Thr Glu Gly Lys Thr Ile
   865                 870                 875                 880
   Val Arg Asn Phe Ile Lys Ile Val Glu Lys Lys Glu Ser Glu Lys Leu
                   885                 890                 895
   Thr
<210> 46
   <211> 252
   <212> DNA
   <213> Artificial Sequence
<220>
   <223> A truncated gene
<400> 46
   atgcaaacac aaaaaccgac tctcgaactg gaattcctgg tggaaaacgg tatcgccacc 60
   gtgcaagcgg gtgctggtgt agtccttgat tctgttccgc agtcggaagc cgacgaaacc 120
   cgtaacaaag cccgcgctgt actgcgcgct attgccaccg cgcatcatgc acaggagact 180
   ttctgatggc tgacattctg ctgctcgata atatcgactc ttttacgtac aacctggcag 240
   atcagttgcg ca 252
<210> 47
   <211> 18
   <212> DNA
   <213> Artificial Sequence
<220>
   <223> A primer.
<400> 47
   ttatgccgcc tgtcatcg 18
<210> 48
   <211> 19
   <212> DNA
   <213> Artificial Sequence
<220>
   <223> A primer.
<400> 48
   ataggcttaa tggtaaccg 19
<210> 49
   <211> 18
   <212> DNA
   <213> Artificial Sequence
<220>
   <223> A primer.
<400> 49
   ctgaacaaca gaagtacg 18
<210> 50
   <211> 18
   <212> DNA
   <213> Artificial Sequence
<220>
   <223> A primer.
<400> 50
   taaccgtgtc atcgagcg 18
<210> 51
   <211> 31
   <212> DNA
   <213> Artificial Sequence
<220>
   <223> A primer.
<400> 51
   aaaaagatct ccatggtaac gatcattcag g 31
<210> 52
   <211> 35
   <212> DNA
   <213> Artificial Sequence
<220>
   <223> A primer.
<400> 52
   aaaagaattc ttatcacgcg gccttggtct tcgcc 35
<210> 53
   <211> 19
   <212> DNA
   <213> Artificial Sequence
<220>
   <223> A primer.
<400> 53
   caaaagctgg atccccacc 19
<210> 54
   <211> 23
   <212> DNA
   <213> Artificial Sequence
<220>
   <223> A primer.
<400> 54
   cctatccgag atctctcaac tcc 23
<210> 55
   <211> 31
   <212> DNA
   <213> Artificial sequence
<220>
   <223> A primer.
<400> 55
   catcccatgg atggtaacga tcattcagga t 31
<210> 56
   <211> 31
   <212> DNA
   <213> Artificial Sequence
<220>
   <223> A primer.
<400> 56
   gatgtctaga gacactatag aatactcaag c 31
<210> 57
   <211> 719
   <212> PRT
   <213> Rhodopseudomonas palustris
<400> 57
   Met Asn Arg Thr Val Phe Ser Leu Pro Ala Thr Ser Asp Tyr Lys Thr
   1 5 10 15
   Ala Ala Gly Leu Ala Val Thr Arg Ser Ala Gln Pro Phe Ala Gly Gly
   20 25 30
   Gln Ala Leu Asp Glu Leu Ile Asp Leu Leu Asp His Arg Arg Gly Val
   35 40 45
   Met Leu Ser Ser Gly Thr Thr Val Pro Gly Arg Tyr Glu Ser Phe Asp
   50 55 60
   Leu Gly Phe Ala Asp Pro Pro Leu Ala Leu Thr Thr Arg Ala Glu Lys
   65 70 75 80
   Phe Thr Ile Glu Ala Leu Asn Pro Arg Gly Arg Val Leu Ile Ala Phe
   85 90 95
   Leu Ser Asp Lys Leu Glu Glu Pro Cys Val Val Val Glu Gln Ala Cys
   100 105 110
   Ala Thr Lys Ile Arg Gly His Ile Val Arg Gly Glu Ala Pro Val Asp
   115 120 125
   Glu Glu Gln Arg Thr Arg Arg Ala Ser Ala Ile Ser Leu Val Arg Ala
   130 135 140
   Val Ile Ala Ala Phe Ala Ser Pro Ala Asp Pro Met Leu Gly Leu Tyr
   145 150 155 160
   Gly Ala Phe Ala Tyr Asp Leu Val Phe Gln Phe Glu Asp Leu Lys Gln
   165 170 175
   Lys Arg Ala Arg Glu Ala Asp Gln Arg Asp Ile Val Leu Tyr Val Pro
   180 185 190
   Asp Arg Leu Leu Ala Tyr Asp Arg Ala Thr Gly Arg Gly Val Asp Ile
   195 200 205
   Ser Tyr Glu Phe Ala Trp Lys Gly Gln Ser Thr Ala Gly Leu Pro Asn
   210 215 220
   Glu Thr Ala Glu Ser Val Tyr Thr Gln Thr Gly Arg Gln Gly Phe Ala
   225 230 235 240
   Asp His Ala Pro Gly Asp Tyr Pro Lys Val Val Glu Lys Ala Arg Ala
   245 250 255
   Ala Phe Ala Arg Gly Asp Leu Phe Glu Ala Val Pro Gly Gln Leu Phe
   260 265 270
   Gly Glu Pro Cys Glu Arg Ser Pro Ala Glu Val Phe Lys Arg Leu Cys
   275 280 285
   Arg Ile Asn Pro Ser Pro Tyr Gly Gly Leu Leu Asn Leu Gly Asp Gly
   290 295 300
   Glu Phe Leu Val Ser Ala Ser Pro Glu Met Phe Val Arg Ser Asp Gly
   305 310 315 320
   Arg Arg Ile Glu Thr Cys Pro Ile Ser Gly Thr Ile Ala Arg Gly Val
   325 330 335
   Asp Ala Ile Ser Asp Ala Glu Gln Ile Gln Lys Leu Leu Asn Ser Glu
   340 345 350
   Lys Asp Glu Phe Glu Leu Asn Met Cys Thr Asp Val Asp Arg Asn Asp
   355 360 365
   Lys Ala Arg Val Cys Val Pro Gly Thr Ile Lys Val Leu Ala Arg Arg
   370 375 380
   Gln Ile Glu Thr Tyr Ser Lys Leu Phe His Thr Val Asp His Val Glu
   385 390 395 400
   Gly Met Leu Arg Pro Gly Phe Asp Ala Leu Asp Ala Phe Leu Thr His
   405 410 415
   Ala Trp Ala Val Thr Val Thr Gly Ala Pro Lys Leu Trp Ala Met Gln
   420 425 430
   Phe Val Glu Asp His Glu Arg Ser Pro Arg Arg Trp Tyr Ala Gly Ala
           435                 440                 445
   Phe Gly Val Val Gly Phe Asp Gly Ser Ile Asn Thr Gly Leu Thr Ile
       450                 455                 460
   Arg Thr Ile Arg Met Lys Asp Gly Leu Ala Glu Val Arg Val Gly Ala
   465                 470                 475                 480
   Thr Cys Leu Phe Asp Ser Asn Pro Val Ala Glu Asp Lys Glu Cys Gln
                   485                 490                 495
   Val Lys Ala Ala Ala Leu Phe Gln Ala Leu Arg Gly Asp Pro Ala Lys
               500                 505                 510
   Pro Leu Ser Ala Val Ala Pro Asp Ala Thr Gly Ser Gly Lys Lys Val
           515                 520                 525
   Leu Leu Val Asp His Asp Asp Ser Phe Val His Met Leu Ala Asp Tyr
       530                 535                 540
   Phe Arg Gln Val Gly Ala Gln Val Thr Val Val Arg Tyr Val His Gly
   545                 550                 555                 560
   Leu Lys Met Leu Ala Glu Asn Ser Tyr Asp Leu Leu Val Leu Ser Pro
                   565                 570                 575
   Gly Pro Gly Arg Pro Glu Asp Phe Lys Ile Lys Asp Thr Ile Asp Ala
               580                 585                 590
   Ala Leu Ala Lys Lys Leu Pro Ile Phe Gly Val Cys Leu Gly Val Gln
           595                 600                 605
   Ala Met Gly Glu Tyr Phe Gly Gly Thr Leu Gly Gln Leu Ala Gln Pro
       610                 615                 620
   Ala His Gly Arg Pro Ser Arg Ile Gln Val Arg Gly Gly Ala Leu Met
   625                 630                 635                 640
   Arg Gly Leu Pro Asn Glu Val Thr Ile Gly Arg Tyr His Ser Leu Tyr
                   645                 650                 655
   Val Asp Met Arg Asp Met Pro Lys Glu Leu Thr Val Thr Ala Ser Thr
               660                 665                 670
   Asp Asp Gly Ile Ala Met Ala Ile Glu His Lys Thr Leu Pro Val Gly
           675                 680                 685
   Gly Val Gln Phe His Pro Glu Ser Leu Met Ser Leu Gly Gly Glu Val
       690                 695                 700
   Gly Leu Arg Ile Val Glu Asn Ala Phe Arg Leu Gly Gln Ala Ala
   705 710 715
<210> 58
   <211> 729
   <212> PRT
   <213> Artificial Sequence
<220>
   <223> An A. tumefaciens mutant.
<400> 58
   Met Val Thr Ile Ile Gln Asp Asp Gly Ala Glu Thr Tyr Glu Thr Lys
   1 5 10 15
   Gly Gly Ile Gln Val Ser Arg Lys Arg Arg Pro Thr Asp Tyr Ala Asn
   20 25 30
   Ala Ile Asp Asn Tyr Ile Glu Lys Leu Asp Ser His Arg Gly Ala Phe
   35 40 45
   Phe Ser Ser Asn Tyr Glu Tyr Pro Gly Arg Tyr Thr Arg Trp Asp Thr
   50 55 60
   Ala Ile Val Asp Pro Pro Leu Gly Ile Ser Cys Phe Gly Arg Lys Met
   65 70 75 80
   Trp Ile Glu Ala Tyr Asn Gly Arg Gly Glu Val Leu Leu Asp Phe Ile
   85 90 95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 59
   <211> 729
   <212> PRT
   <213> Artificial Sequence
<220>
   <223> An A. tumefaciens mutant.
<400> 59
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Tyr
           35                  40                  45
   Phe Ser Ser Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
                   85                  90                  95
   Thr Glu Lys Leu Lys Ala Thr Pro Asp Leu Thr Leu Gly Ala Ser Ser
               100                 105                 110
   Thr Arg Arg Leu Asp Leu Thr Val Asn Glu Pro Asp Arg Val Phe Thr
           115                 120                 125
   Glu Glu Glu Arg Ser Lys Ile Pro Thr Val Phe Thr Ala Leu Arg Ala
       130                 135                 140
   Ile Val Asp Leu Phe Tyr Ser Ser Ala Asp Ser Ala Ile Gly Leu Phe
   145                 150                 155                 160
   Gly Ala Phe Gly Tyr Asp Leu Ala Phe Gln Phe Asp Ala Ile Lys Leu
                   165                 170                 175
   Ser Leu Ala Arg Pro Glu Asp Gln Arg Asp Met Val Leu Phe Leu Pro
               180                 185                 190
   Asp Glu Ile Leu Val Val Asp His Tyr Ser Ala Lys Ala Trp Ile Asp
           195                 200                 205
   Arg Tyr Asp Phe Glu Lys Asp Gly Met Thr Thr Asp Gly Lys Ser Ser
       210                 215                 220
   Asp Ile Thr Pro Asp Pro Phe Lys Thr Thr Asp Thr Ile Pro Pro Lys
   225                 230                 235                 240
   Gly Asp His Arg Pro Gly Glu Tyr Ser Glu Leu Val Val Lys Ala Lys
                   245                 250                 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
       530                 535                 540
   Ala Asn Tyr Phe Arg Gln Thr Gly Ala Thr Val Ser Thr Val Arg Ser
   545                 550                 555                 560
   Pro Val Ala Ala Asp Val Phe Asp Arg Phe Gln Pro Asp Leu Val Val
                   565                 570                 575
   Leu Ser Pro Gly Pro Gly Ser Pro Thr Asp Phe Asp Cys Lys Ala Thr
               580                 585                 590
   Ile Lys Ala Ala Arg Ala Arg Asp Leu Pro Ile Phe Gly Val Cys Leu
           595                 600                 605
   Gly Leu Gln Ala Leu Ala Glu Ala Tyr Gly Gly Glu Leu Arg Gln Leu
       610                 615                 620
   Ala Val Pro Met His Gly Lys Pro Ser Arg Ile Arg Val Leu Glu Pro
   625                 630                 635                 640
   Gly Leu Val Phe Ser Gly Leu Gly Lys Glu Val Thr Val Gly Arg Tyr
                   645                 650                 655
   His Ser Ile Phe Ala Asp Pro Ala Thr Leu Pro Arg Asp Phe Ile Ile
               660                 665                 670
   Thr Ala Glu Ser Glu Asp Gly Thr Ile Met Gly Ile Glu His Ala Lys
           675                 680                 685
   Glu Pro Val Ala Ala Val Gln Phe His Pro Glu Ser Ile Met Thr Leu
       690                 695                 700
   Gly Gln Asp Ala Gly Met Arg Met Ile Glu Asn Val Val Val His Leu
   705                 710                 715                 720
   Thr Arg Lys Ala Lys Thr Lys Ala Ala
                   725
<210> 60
   <211> 729
   <212> PRT
   <213> Artificial Sequence
<220>
   <223> An A. tumefaciens mutant.
<400> 60
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
   20 25 30
   Ala Ile Asp Asn Tyr Ile Glu Lys Leu Asp Ser His Arg Gly Ala Val
   35 40 45
   Phe Ser Phe Asn Tyr Glu Tyr Pro Gly Arg Tyr Thr Arg Trp Asp Thr
   50 55 60
   Ala Ile Val Asp Pro Pro Leu Gly Ile Ser Cys Phe Gly Arg Lys Met
   65 70 75 80
   Trp Ile Glu Ala Tyr Asn Gly Arg Gly Glu Val Leu Leu Asp Phe Ile
   85 90 95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 61
   <211> 729
   <212> PRT
   <213> Artificial Sequence
<220>
   <223> An A. tumefaciens mutant.
<400> 61
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Ser Cys Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65 70 75 80
   Trp Ile Glu Ala Tyr Asn Gly Arg Gly Glu Val Leu Leu Asp Phe Ile
   85 90 95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
               340                 345                 350
   Lys Lys Asp Glu Ser Glu Leu Thr Met Cys Ser Asp Val Asp Arg Asn
           355                 360                 365
   Asp Lys Ser Arg Val Cys Glu Pro Gly Ser Val Lys Val Ile Gly Arg
       370                 375                 380
   Arg Gln Ile Glu Met Tyr Ser Arg Leu Ile His Thr Val Asp His Ile
   385                 390                 395                 400
   Glu Gly Arg Leu Arg Asp Asp Met Asp Ala Phe Asp Gly Phe Leu Ser
                   405                 410                 415
   His Ala Trp Ala Val Thr Val Thr Gly Ala Pro Lys Leu Trp Ala Met
               420                 425                 430
   Arg Phe Ile Glu Gly His Glu Lys Ser Pro Arg Ala Trp Tyr Gly Gly
           435                 440                 445
   Ala Ile Gly Met Val Gly Phe Asn Gly Asp Met Asn Thr Gly Leu Thr
       450                 455                 460
   Leu Arg Thr Ile Arg Ile Lys Asp Gly Ile Ala Glu Val Arg Ala Gly
   465                 470                 475                 480
   Ala Thr Leu Leu Asn Asp Ser Asn Pro Gln Glu Glu Glu Ala Glu Thr
                   485                 490                 495
   Glu Leu Lys Ala Ser Ala Met Ile Ser Ala Ile Arg Asp Ala Lys Gly
               500                 505                 510
   Thr Asn Ser Ala Ala Thr Lys Arg Asp Ala Ala Lys Val Gly Thr Gly
           515                 520                 525
   Val Lys Ile Leu Leu Val Asp His Glu Asp Ser Phe Val His Thr Leu
       530                 535                 540
   Ala Asn Tyr Phe Arg Gln Thr Gly Ala Thr Val Ser Thr Val Arg Ser
   545                 550                 555                 560
   Pro Val Ala Ala Asp Val Phe Asp Arg Phe Gln Pro Asp Leu Val Val
                   565                 570                 575
   Leu Ser Pro Gly Pro Gly Ser Pro Thr Asp Phe Asp Cys Lys Ala Thr
               580                 585                 590
   Ile Lys Ala Ala Arg Ala Arg Asp Leu Pro Ile Phe Gly Val Cys Leu
           595                 600                 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 62
   <211> 729
   <212> PRT
   <213> Artificial Sequence
<220>
   <223> An A. tumefaciens mutant.
<400> 62
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Ser Ser Phe Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
                   85                  90                  95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 63
   <211> 729
   <212> PRT
   <213> Artificial Sequence
<220>
   <223> An A. tumefaciens mutant.
<400> 63
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Ser Ser Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
                   85                  90                  95
   Thr Glu Lys Leu Lys Ala Thr Pro Asp Leu Thr Leu Gly Ala Ser Ser
               100                 105                 110
   Thr Arg Arg Leu Asp Leu Thr Val Asn Glu Pro Asp Arg Val Phe Thr
           115                 120                 125
   Glu Glu Glu Arg Ser Lys Ile Pro Thr Val Phe Thr Ala Leu Arg Ala
       130                 135                 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Ala Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
       690                 695                 700
   Gly Gln Asp Ala Gly Met Arg Met Ile Glu Asn Val Val Val His Leu
   705                 710                 715                 720
   Thr Arg Lys Ala Lys Thr Lys Ala Ala
                   725
<210> 64
   <211> 729
   <212> PRT
   <213> Artificial Sequence
<220>
   <223> An A. tumefaciens mutant.
<400> 64
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Ser Ser Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
                   85                  90                  95
   Thr Glu Lys Leu Lys Ala Thr Pro Asp Leu Thr Leu Gly Ala Ser Ser
               100                 105                 110
   Thr Arg Arg Leu Asp Leu Thr Val Asn Glu Pro Asp Arg Val Phe Thr
           115                 120                 125
   Glu Glu Glu Arg Ser Lys Ile Pro Thr Val Phe Thr Ala Leu Arg Ala
       130                 135                 140
   Ile Val Asp Leu Phe Tyr Ser Ser Ala Asp Ser Ala Ile Gly Leu Phe
   145                 150                 155                 160
   Gly Ala Phe Gly Tyr Asp Leu Ala Phe Gln Phe Asp Ala Ile Lys Leu
                   165                 170                 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Gly Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
<210> 65
   <211> 729
   <212> PRT
   <213> Artificial Sequence
<220>
   <223> An A. tumefaciens mutant.
<400> 65
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Ser Ser Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
                   85                  90                  95
   Thr Glu Lys Leu Lys Ala Thr Pro Asp Leu Thr Leu Gly Ala Ser Ser
               100                 105                 110
   Thr Arg Arg Leu Asp Leu Thr Val Asn Glu Pro Asp Arg Val Phe Thr
           115                 120                 125
   Glu Glu Glu Arg Ser Lys Ile Pro Thr Val Phe Thr Ala Leu Arg Ala
       130                 135                 140
   Ile Val Asp Leu Phe Tyr Ser Ser Ala Asp Ser Ala Ile Gly Leu Phe
   145                 150                 155                 160
   Gly Ala Phe Gly Tyr Asp Leu Ala Phe Gln Phe Asp Ala Ile Lys Leu
                   165                 170                 175
   Ser Leu Ala Arg Pro Glu Asp Gln Arg Asp Met Val Leu Phe Leu Pro
               180                 185                 190
   Asp Glu Ile Leu Val Val Asp His Tyr Ser Ala Lys Ala Trp Ile Asp
           195                 200                 205
   Arg Tyr Asp Phe Glu Lys Asp Gly Met Thr Thr Asp Gly Lys Ser Ser
       210                 215                 220
   Asp Ile Thr Pro Asp Pro Phe Lys Thr Thr Asp Thr Ile Pro Pro Lys
   225                 230                 235                 240
   Gly Asp His Arg Pro Gly Glu Tyr Ser Glu Leu Val Val Lys Ala Lys
                   245                 250                 255
   Glu Ser Phe Arg Arg Gly Asp Leu Phe Glu Val Val Pro Gly Gln Lys
               260                 265                 270
   Phe Met Glu Arg Cys Glu Ser Asn Pro Ser Ala Ile Ser Arg Arg Leu
           275                 280                 285
   Lys Ala Ile Asn Pro Ser Pro Tyr Ser Trp Phe Ile Asn Leu Gly Asp
       290                 295                 300
   Gln Glu Tyr Leu Val Gly Ala Ser Pro Glu Met Phe Val Arg Val Ser
   305                 310                 315                 320
   Gly Arg Arg Ile Glu Thr Cys Pro Ile Ser Gly Thr Ile Lys Arg Gly
                   325                 330                 335
   Asp Asp Pro Ile Ala Asp Ser Glu Gln Ile Leu Lys Leu Leu Asn Ser
               340                 345                 350
   Lys Lys Asp Glu Ser Glu Leu Thr Met Cys Ser Asp Val Asp Arg Asn
           355                 360                 365
   Asp Lys Ser Arg Val Cys Glu Pro Gly Ser Val Lys Val Ile Gly Arg
       370                 375                 380
   Arg Gln Ile Glu Met Tyr Ser Arg Leu Ile His Thr Val Asp His Ile
   385                 390                 395                 400
   Glu Gly Arg Leu Arg Asp Asp Met Asp Ala Phe Asp Gly Phe Leu Ser
                   405                 410                 415
   His Ala Trp Ala Val Thr Val Thr Gly Ala Pro Lys Leu Trp Ala Met
               420                 425                 430
   Arg Phe Ile Glu Gly His Glu Lys Ser Pro Arg Ala Trp Tyr Gly Gly
           435                 440                 445
   Ala Ile Gly Met Val Gly Phe Asn Gly Asp Met Asn Thr Gly Leu Thr
       450                 455                 460
   Leu Arg Thr Ile Arg Ile Lys Asp Gly Ile Ala Glu Val Arg Ala Gly
   465                 470                 475                 480
   Ala Thr Leu Leu Asn Asp Ser Asn Pro Gln Glu Glu Glu Ala Glu Thr
                   485                 490                 495
   Glu Leu Lys Ala Ser Ala Met Ile Ser Ala Ile Arg Asp Ala Lys Gly
               500                 505                 510
   Thr Asn Ser Ala Ala Thr Lys Arg Asp Ala Ala Lys Val Gly Thr Gly
           515                 520                 525
   Val Lys Ile Leu Leu Val Asp His Glu Asp Ser Phe Val His Thr Leu
       530                 535                 540
   Ala Asn Tyr Phe Arg Gln Thr Gly Ala Thr Val Ser Thr Val Arg Ser
   545                 550                 555                 560
   Pro Val Ala Ala Asp Val Phe Asp Arg Phe Gln Pro Asp Leu Val Val
                   565                 570                 575
   Leu Ser Pro Gly Pro Gly Ser Pro Thr Asp Phe Asp Cys Lys Ala Thr
               580                 585                 590
   Ile Lys Ala Ala Arg Ala Arg Asp Leu Pro Ile Phe Gly Val Cys Leu
           595                 600                 605
   Gly Leu Gln Ala Leu Ala Glu Ala Tyr Gly Gly Glu Leu Arg Gln Leu
       610                 615                 620
   Ala Val Pro Met His Gly Lys Pro Ser Arg Ile Arg Val Leu Glu Pro
   625                 630                 635                 640
   Gly Leu Val Phe Ser Gly Leu Gly Lys Glu Val Thr Val Gly Arg Tyr
                   645                 650                 655
   His Ser Ile Phe Ala Asp Pro Ala Thr Leu Pro Arg Asp Phe Ile Ile
               660                 665                 670
   Thr Ala Glu Ser Glu Asp Gly Thr Ile Met Gly Ile Glu His Ala Lys
           675                 680                 685
   Glu Pro Val Ala Ala Val Gln Phe His Pro Glu Ser Ile Met Thr Leu
       690                 695                 700
   Gly Gln Asp Ala Gly Met Arg Met Ile Glu Asn Val Val Val His Leu
   705                 710                 715                 720
   Thr Arg Lys Ala Lys Thr Lys Ala Ala
                   725
<210> 66
   <211> 604
   <212> PRT
   <213> Artificial Sequence
<220>
   <223> An Zea mays mutant.
<210> 67
   <211> 1815
   <212> DNA
   <213> Artificial sequence
<220>
   <223> An Zea mays mutant.
<400> 67
   atggaatccc tagccgccac ctccgtgttc gcgccctccc gcgtcgccgt cccggcggcg      60
   cgggccctgg ttagggcggg gacggtggta ccaaccaggc ggacgagcag ccggagcgga     120
   accagcgggg tgaaatgctc tgctgccgtg acgccgcagg cgagcccagt gattagcagg     180
   agcgctgcgg cggcgaaggc ggcggaggag gacaagaggc ggttcttcga ggcggcggcg     240
   cgggggagcg ggaaggggaa cctggtgccc atgtgggagt gcatcgtgtc ggaccatctc     300
   acccccgtgc tcgcctaccg ctgcctcgtc cccgaggaca acgtcgacgc ccccagcttc     360
   ctcttcgagt ccgtcgagca ggggccccag ggcaccacca acgtcggccg ctatagcatg     420
   gtgggagccc acccagtgat ggagattgtg gccaaagacc acaaggttac gatcatggac     480
   cacgagaaga gccaagtgac agagcaggta gtggacgacc cgatgcagat cccgaggacc     540
   atgatggagg gatggcaccc acagcagatc gacgagctcc ctgaatcctt ctccggtgga     600
   tgggttgggt tcttttccta tgatacggtt aggtatgttg agaagaagaa gctaccgttc     660
   tccagtgctc ctcaggacga taggaacctt cctgatgtgc acttgggact ctatgatgat     720
   gttctagtct tcgataatgt tgagaagaaa gtatatgtta tccattgggt caatgtggac     780
   cggcatgcat ctgttgagga agcataccaa gatggcaggt cccgactaaa catgttgcta     840
   tctaaagtgc acaattccaa tgtccccaca ctctctcctg gatttgtgaa gctgcacaca     900
   cgcaagtttg gtacaccttt gaacaagtcg accatgacaa gtgatgagta taagaatgct     960
   gttctgcagg ctaaggaaca tattatggct ggggatatct tccagattgt tttaagccag    1020
   aggttcgaga gacgaacata tgccaaccca tttgaggttt atcgagcatt acggattgtg    1080
   aatcctagcc catacaaggc gtatgtacag gcaagaggct gtgtattggt tgcgtctagt    1140
   cctgaaattc ttacacgagt cagtaagggg aagattatta atcgaccact tgctggaact    1200
   gttcgaaggg gcaagacaga gaaggaagat caaatgcaag agcagcaact gttaagtgat    1260
   gaaaaacagt gtgccgagca cataatgctt gtggacttgg gaaggaatga tgttggcaag    1320
   gtatccaaac caggatcagt gaaggtggag aagttgatga acattgagag atactcccat    1380
   gttatgcaca tcagctcaac ggttagtgga cagttggatg atcatctcca gagttgggat    1440
   gccttgagag ctgccttgcc cgttggaaca gtcagtggtg caccaaaggt gaaggccatg    1500
   gagttgattg ataagttgga agttacgagg cgaggaccat atagtggtgg tctaggagga    1560
   atatcgtttg atggtgacat gcaaattgca ctttctctcc gcaccatcgt attctcaaca    1620
   gcgccgagcc acaacacgat gtactcatac aaagacgcag ataggcgtcg ggagtgggtc    1680
   gctcatcttc aggctggtgc aggcattgtt gccgacagta gcccagatga cgaacaacgt    1740
   gaatgcgaga ataaggctgc tgcactagct cgggccatcg atcttgcaga gtcagctttt    1800
   gtagacaaag aatag                                                     1815
<210> 68
   <211> 2204
   <212> DNA
   <213> Artificial sequence
<220>
   <223> An Zea mays mutant.
<400> 68
   atggaatccc tagccgccac ctccgtgttc gcgccctccc gcgtcgccgt cccggcggcg    60
   cgggccctgg ttagggcggg gacggtggta ccaaccaggc ggacgagcag ccggagcgga   120
   accagcgggg tgaaatgctc tgctgccgtg acgccgcagg cgagcccagt gattagcagg   180
   agcgctgcgg cggcgaaggc ggcggaggag gacaagaggc ggttcttcga ggcggcggcg   240
   cgggggagcg ggaaggggaa cctggtgccc atgtgggagt gcatcgtgtc ggaccatctc   300
   acccccgtgc tcgcctaccg ctgcctcgtc cccgaggaca acgtcgacgc ccccagcttc   360
   ctcttcgagt ccgtcgagca ggggccccag ggcaccacca acgtcggccg ctatagcatg   420
   gtgggagccc acccagtgat ggagattgtg gccaaagacc acaaggttac gatcatggac   480
   cacgagaaga gccaagtgac agagcaggta gtggacgacc cgatgcagat cccgaggacc   540
   atgatggagg gatggcaccc acagcagatc gacgagctcc ctgaatcctt ctccggtgga   600
   tgggttgggt tcttttccta tgatacggtt aggtatgttg agaagaagaa gctaccgttc   660
   tccagtgctc ctcaggacga taggaacctt cctgatgtgc acttgggact ctatgatgat   720
   gttctagtct tcgataatgt tgagaagaaa gtatatgtta tccattgggt caatgtggac   780
   cggcatgcat ctgttgagga agcataccaa gatggcaggt cccgactaaa catgttgcta   840
   tctaaagtgc acaattccaa tgtccccaca ctctctcctg gatttgtgaa gctgcacaca   900
   cgcaagtttg gtacaccttt gaacaagtcg accatgacaa gtgatgagta taagaatgct   960
   gttctgcagg ctaaggaaca tattatggct ggggatatct tccagattgt tttaagccag  1020
   aggttcgaga gacgaacata tgccaaccca tttgaggttt atcgagcatt acggattgtg  1080
   aatcctagcc catacaaggc gtatgtacag gcaagaggct gtgtattggt tgcgtctagt 1140
   cctgaaattc ttacacgagt cagtaagggg aagattatta atcgaccact tgctggaact 1200
   gttcgaaggg gcaagacaga gaaggaagat caaatgcaag agcagcaact gttaagtgat 1260
   gaaaaacagt gtgccgagca cataatgctt gtggacttgg gaaggaatga tgttggcaag 1320
   gtatccaaac caggatcagt gaaggtggag aagttgatga acattgagag atactcccat 1380
   gttatgcaca tcagctcaac ggttagtgga cagttggatg atcatctcca gagttgggat 1440
   gccttgagag ctgccttgcc cgttggaaca gtcagtggtg caccaaaggt gaaggccatg 1500
   gagttgattg ataagttgga agttacgagg cgaggaccat atagtggtgg tctaggagga 1560
   atatcgtttg atggtgacat gcaaattgca ctttctctcc gcaccatcgt attctcaaca 1620
   gcgccgagcc acaacacgat gtactcatac aaagacgcag ataggcgtcg ggagtgggtc 1680
   gctcatcttc aggctggtgc aggcattgtt gccgacagta gcccagatga cgaacaacgt 1740
   gaatgcgaga ataaggctgc tgcactagct cgggccatcg atcttgcaga gtcagctttt 1800
   gtagacaaag aatagtgtgc tatggttatc gtttagttct tgttcatgtt tcttttaccc 1860
   actttccgtt aaaaaaagat gtcattagtg ggtggagaaa agcaataaga ctgttctcta 1920
   gaattcgagc tcggtaccgg atccaattcc cgatcgttca aacatttggc aataaagttt 1980
   cttaagattg aatcctgttg ccggtcttgc gatgattatc atataatttc tgttgaatta 2040
   cgttaagcat gtaataatta acatgtaatg catgacgtta tttatgagat gggtttttat 2100
   gattagagtc ccgcaattat acatttaata cgcgatagaa aacaaaatat agcgcgcaaa 2160
   ctaggataaa ttatcgcgcg cggtgtcatc tatgttacta gatc 2204
<210> 69
   <211> 729
   <212> PRT
   <213> Artificial Sequence
<220>
   <223> An A. tumefaciens mutant.
<400> 69
   Met Val Thr Ile Ile Gln Asp Asp Gly Ala Glu Thr Tyr Glu Thr Lys
   1               5                   10                  15
   Gly Gly Ile Gln Val Ser Arg Lys Arg Arg Pro Thr Asp Tyr Ala Asn
               20                  25                  30
   Ala Ile Asp Asn Tyr Ile Glu Lys Leu Asp Ser His Arg Gly Ala Val
           35                  40                  45
   Phe Lys Ser Asn Tyr Glu Tyr Pro Gly Arg Tyr Thr Arg Trp Asp Thr
       50                  55                  60
   Ala Ile Val Asp Pro Pro Leu Gly Ile Ser Cys Phe Gly Arg Lys Met
   65                  70                  75                  80
   Trp Ile Glu Ala Tyr Asn Gly Arg Gly Glu Val Leu Leu Asp Phe Ile
   85 90 95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Phe Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Asp Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Ile Glu Gly His Glu Lys Ser Pro Arg Ala Trp Tyr Gly Gly
   435 440 445
   Ala Ile Gly Met Val Gly Phe Asn Gly Asp Met Asn Thr Gly Leu Thr
   450 455 460
   Leu Arg Thr Ile Arg Ile Lys Asp Gly Ile Ala Glu Val Arg Ala Gly
   465 470 475 480
   Ala Thr Leu Leu Asn Asp Ser Asn Pro Gln Glu Glu Glu Ala Glu Thr
   485 490 495
   Glu Leu Lys Ala Ser Ala Met Ile Ser Ala Ile Arg Asp Ala Lys Gly
   500 505 510
   Thr Asn Ser Ala Ala Thr Lys Arg Asp Ala Ala Lys Val Gly Thr Gly
   515 520 525
   Val Lys Ile Leu Leu Val Asp His Glu Asp Ser Phe Val His Thr Leu
   530 535 540
   Ala Asn Tyr Phe Arg Gln Thr Gly Ala Thr Val Ser Thr Val Arg Ser
   545 550 555 560
   Pro Val Ala Ala Asp Val Phe Asp Arg Phe Gln Pro Asp Leu Val Val
   565 570 575
   Leu Ser Pro Gly Pro Gly Ser Pro Thr Asp Phe Asp Cys Lys Ala Thr
   580 585 590
   Ile Lys Ala Ala Arg Ala Arg Asp Leu Pro Ile Phe Gly Val Cys Leu
   595 600 605
   Gly Leu Gln Ala Leu Ala Glu Ala Tyr Gly Gly Glu Leu Arg Gln Leu
   610 615 620
   Ala Val Pro Met His Gly Lys Pro Ser Arg Ile Arg Val Leu Glu Pro
   625 630 635 640
   Gly Leu Val Phe Ser Gly Leu Gly Lys Glu Val Thr Val Gly Arg Tyr
   645 650 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 70
   <211> 729
   <212> PRT
   <213> Artificial Sequence
<220>
   <223> An A. tumefaciens mutant.
<400> 70
   Met Val Thr Ile Ile Gln Asp Asp Gly Ala Glu Thr Tyr Glu Thr Lys
   1 5 10 15
   Gly Gly Ile Gln Val Ser Arg Lys Arg Arg Pro Thr Asp Tyr Ala Asn
   20 25 30
   Ala Ile Asp Asn Tyr Ile Glu Lys Leu Asp Ser His Arg Gly Ala Val
   35 40 45
   Phe Ser Ser Asn Tyr Glu Tyr Pro Gly Arg Tyr Thr Arg Trp Asp Thr
   50 55 60
   Ala Ile Val Asp Pro Pro Leu Gly Ile Ser Cys Phe Gly Arg Lys Met
   65 70 75 80
   Trp Ile Glu Ala Tyr Asn Gly Arg Gly Glu Val Leu Leu Asp Phe Ile
   85 90 95
   Thr Glu Lys Leu Lys Ala Thr Pro Asp Leu Thr Leu Gly Ala Ser Ser
   100 105 110
   Thr Arg Arg Leu Asp Leu Thr Val Asn Glu Pro Asp Arg Val Phe Thr
   115 120 125
   Glu Glu Glu Arg Ser Lys Ile Pro Thr Val Phe Thr Ala Leu Arg Ala
   130 135 140
   Ile Val Asp Leu Phe Tyr Ser Ser Ala Asp Ser Ala Ile Gly Leu Phe
   145 150 155 160
   Gly Ala Phe Gly Tyr Asp Leu Ala Phe Gln Phe Asp Ala Ile Lys Leu
   165 170 175
   Ser Leu Ala Arg Pro Glu Asp Gln Arg Asp Met Val Leu Phe Leu Pro
   180 185 190
   Asp Glu Ile Leu Val Val Asp His Tyr Ser Ala Lys Ala Trp Ile Asp
   195 200 205
   Arg Tyr Asp Phe Glu Lys Asp Gly Met Thr Thr Asp Gly Lys Ser Ser
   210 215 220
   Asp Ile Thr Pro Asp Pro Phe Lys Thr Thr Asp Thr Ile Pro Pro Lys
   225 230 235 240
   Gly Asp His Arg Pro Gly Glu Tyr Ser Glu Leu Val Val Lys Ala Lys
   245 250 255
   Glu Ser Phe Arg Arg Gly Asp Leu Phe Glu Val Val Pro Gly Gln Lys
   260 265 270
   Phe Met Glu Arg Cys Glu Ser Asn Pro Ser Ala Ile Ser Arg Arg Leu
   275 280 285
   Lys Ala Ile Asn Pro Ser Pro Tyr Ser Ala Phe Ile Asn Leu Gly Asp
   290 295 300
   Gln Glu Tyr Leu Val Gly Ala Ser Pro Glu Met Phe Val Arg Val Ser
   305 310 315 320
   Gly Arg Arg Ile Glu Thr Cys Pro Ile Ser Gly Thr Ile Lys Arg Gly
   325 330 335
   Asp Asp Pro Ile Ala Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Lys Val Ile Gly Arg
   370 375 380
   Arg Gln Ile Glu Met Tyr Ser Arg Leu Ile His Thr Val Asp His Ile
   385                 390                 395                 400
   Glu Gly Arg Leu Arg Asp Asp Met Asp Ala Phe Asp Gly Phe Leu Ser
                   405                 410                 415
   His Ala Trp Ala Val Thr Val Thr Gly Ala Pro Lys Leu Trp Ala Met
               420                 425                 430
   Arg Phe Ile Glu Gly His Glu Lys Ser Pro Arg Ala Trp Tyr Gly Gly
           435                 440                 445
   Ala Ile Gly Met Val Gly Phe Asn Gly Asp Met Asn Thr Gly Leu Thr
       450                 455                 460
   Leu Arg Thr Ile Arg Ile Lys Asp Gly Ile Ala Glu Val Arg Ala Gly
   465                 470                 475                 480
   Ala Thr Leu Leu Asn Asp Ser Asn Pro Gln Glu Glu Glu Ala Glu Thr
                   485                 490                 495
   Glu Leu Lys Ala Ser Ala Met Ile Ser Ala Ile Arg Asp Ala Lys Gly
               500                 505                 510
   Thr Asn Ser Ala Ala Thr Lys Arg Asp Ala Ala Lys Val Gly Thr Gly
           515                 520                 525
   Val Lys Ile Leu Leu Val Asp His Glu Asp Ser Phe Val His Thr Leu
       530                 535                 540
   Ala Asn Tyr Phe Arg Gln Thr Gly Ala Thr Val Ser Thr Val Arg Ser
   545                 550                 555                 560
   Pro Val Ala Ala Asp Val Phe Asp Arg Phe Gln Pro Asp Leu Val Val
                   565                 570                 575
   Leu Ser Pro Gly Pro Gly Ser Pro Thr Asp Phe Asp Cys Lys Ala Thr
               580                 585                 590
   Ile Lys Ala Ala Arg Ala Arg Asp Leu Pro Ile Phe Gly Val Cys Leu
           595                 600                 605
   Gly Leu Gln Ala Leu Ala Glu Ala Tyr Gly Gly Glu Leu Arg Gln Leu
       610                 615                 620
   Ala Val Pro Met His Gly Lys Pro Ser Arg Ile Arg Val Leu Glu Pro
   625                 630                 635                 640
   Gly Leu Val Phe Ser Gly Leu Gly Lys Glu Val Thr Val Gly Arg Tyr
                   645                 650                 655
   His Ser Ile Phe Ala Asp Pro Ala Thr Leu Pro Arg Asp Phe Ile Ile
   660 665 670
   Thr Ala Glu Ser Glu Asp Gly Thr Ile Met Gly Ile Glu His Ala Lys
   675 680 685
   Glu Pro Val Ala Ala Val Gln Phe His Pro Glu Ser Ile Met Thr Leu
   690 695 700
   Gly Gln Asp Ala Gly Met Arg Met Ile Glu Asn Val Val Val His Leu
   705 710 715 720
   Thr Arg Lys Ala Lys Thr Lys Ala Ala
   725
<210> 71
   <211> 264
   <212> DNA
   <213> Artificial Sequence
<220>
   <223> The sequence of a CTP.
<400> 71
   atggcttcct ctatgctctc ttccgctact atggttgcct ctccggctca ggccactatg 60
   gtcgctcctt tcaacggact taagtcctcc gctgccttcc cagccacccg caaggctaac 120
   aacgacatta cttccatcac aagcaacggc ggaagagtta actgcatgca ggtgtggcct 180
   ccgattggaa agaagaagtt tgagactctc tcttaccttc ctgaccttac cgattccggt 240
   ggtcgcgtca actgcatgca ggcc 264
<210> 72
   <211> 88
   <212> PRT
   <213> Artificial Sequence
<220>
   <223> The sequence of a CTP.
<400> 72
   Met Ala Ser Ser Met Leu Ser Ser Ala Thr Met Val Ala Ser Pro Ala
   1               5                   10                  15
   Gln Ala Thr Met Val Ala Pro Phe Asn Gly Leu Lys Ser Ser Ala Ala
               20                  25                  30
   Phe Pro Ala Thr Arg Lys Ala Asn Asn Asp Ile Thr Ser Ile Thr Ser
           35                  40                  45
   Asn Gly Gly Arg Val Asn Cys Met Gln Val Trp Pro Pro Ile Gly Lys
       50                  55                  60
   Lys Lys Phe Glu Thr Leu Ser Tyr Leu Pro Asp Leu Thr Asp Ser Gly
   65                  70                  75                  80
   Gly Arg Val Asn Cys Met Gln Ala
                   85
<210> 73
   <211> 264
   <212> DNA
   <213> Artificial Sequence
<220>
   <223> The sequence of a CTP.
<400> 73
   atggcttcct ctatgctctc ttccgctact atggttgcct ctccggctca ggccactatg 60
   gtcgctcctt tcaacggact taagtcctcc gctgccttcc cagccacccg caaggctaac 120
   aacgacatta cttccatcac aagcaacggc ggaagagtta actgcatgca ggtgtggcct 180
   ccgattgaaa agaagaagtt tgagactctc tcttaccttc ctgaccttac cgattccggt 240
   ggtcgcgtca actgcatgca ggcc 264
<210> 74
   <211> 88
   <212> PRT
   <213> Artificial Sequence
<220>
   <223> The sequence of a CTP.
<400> 74
   Met Ala Ser Ser Met Leu Ser Ser Ala Thr Met Val Ala Ser Pro Ala
   1 5 10 15
   Gln Ala Thr Met Val Ala Pro Phe Asn Gly Leu Lys Ser Ser Ala Ala
   20 25 30
   Phe Pro Ala Thr Arg Lys Ala Asn Asn Asp Ile Thr Ser Ile Thr Ser
   35 40 45
   Asn Gly Gly Arg Val Asn Cys Met Gln Val Trp Pro Pro Ile Glu Lys
   50 55 60
   Lys Lys Phe Glu Thr Leu Ser Tyr Leu Pro Asp Leu Thr Asp Ser Gly
   65 70 75 80
   Gly Arg Val Asn Cys Met Gln Ala
   85
<210> 75
   <211> 2190
   <212> DNA
   <213> Artificial Sequence
<220>
   <223> An optimized A. tumefaciens.
<400> 75
   atggtgacca tcattcagga tgacggtgcc gagacctacg agaccaaggg cggcatccag 60
   gtgagccgca agcgccgccc caccgattac gccaacgcca tcgataacta catcgaaaag 120
   cttgattccc atcgcggtgc cgtgttctcc tccaactacg aatacccagg ccgctacacc 180
   cgctgggata ccgccatcgt cgatccacca ctcggcattt cctgcttcgg ccgcaagatg 240
   tggatcgaag cctacaacgg ccgcggcgaa gtgctgctcg atttcattac cgaaaagctg 300
   aaggccacac ccgatctcac cctcggcgct tcctccaccc gccgcctcga tcttaccgtc 360
   aacgaaccag accgcgtctt caccgaagaa gaacgctcca aaatcccaac cgtcttcacc 420
   gctctcaggg ccatcgtcga cctcttctac tccagcgccg attccgccat cggcctgttc 480
   ggtgccttcg gttacgatct cgccttccag ttcgacgcca tcaagctttc cctggcccgc 540
   ccagaagacc agcgcgacat ggtgctgttc ctgcccgatg aaatcctcgt cgttgatcac 600
   tactccgcca aggcctggat cgaccgctac gatttcgaga aggacggcat gaccaccgac 660
   ggcaaatcct ccgacattac ccccgatccc ttcaagacca ccgataccat cccacccaag 720
   ggcgatcacc gccccggcga atactccgag cttgtggtga aggccaagga aagcttccgc 780
   cgcggcgacc tgttcgaggt cgttcccggc cagaaattca tggagcgctg cgaaagcaac 840
   ccatccgcca tttcccgccg cctgaaggcc atcaacccat ccccctactc cttcttcatc 900
   aacctcggcg atcaggaata cctggtcggc gcctccccag aaatgttcgt gcgcgtctcc 960
   ggccgccgca tcgagacctg cccaatctca ggcaccatca agcgcggcga cgatccaatt 1020
   gccgacagcg agcagatttt gaaactgctc aactccaaaa aggacgaatc cgaactgacc 1080
   atgtgctccg acgtggaccg caacgacaag agccgcgtct gcgagccagg ttccgtgaag 1140
   gtcattggcc gccgccagat cgagatgtac tcacgcctca tccacaccgt cgatcacatc 1200
   gaaggccgcc tgcgcgacga tatggacgcc ttcgacggtt tcctcagcca cgcctgggcc 1260
   gtcaccgtca ccggtgcacc aaagctgtgg gccatgcgct tcatcgaagg tcatgaaaag 1320
   agcccacgcg cctggtacgg cggtgccatc ggcatggtcg gcttcaacgg cgacatgaac 1380
   accggcctga ccctgcgcac catccgcatc aaggacggta ttgccgaagt gcgcgccggc 1440
   gccaccctgc tcaacgattc caacccacag gaagaagaag ccgaaaccga actgaaggcc 1500
   tccgccatga tctcagccat tcgcgacgca aaaggcacca actctgccgc caccaagcgc 1560
   gatgccgcca aagtcggcac cggcgtcaag atcctgctcg tcgaccacga agacagcttc 1620
   gtgcacaccc tggccaacta cttccgccag accggcgcca ccgtctccac cgtcaggtca 1680
   ccagtcgcag ccgacgtgtt cgatcgcttc cagccagacc tcgttgtcct gtcccccggt 1740
   cccggcagcc caaccgattt cgactgcaag gcaaccatca aggccgcccg cgcccgcgat 1800
   ctgccaatct tcggcgtttg cctcggtctg caggcattgg cagaagccta cggcggcgag 1860
   ctgcgccagc ttgctgtgcc catgcacggc aagccttccc gcatccgcgt gctggaaccc 1920
   ggcctcgtct tctccggtct cggcaaggaa gtcaccgtcg gtcgctacca ttccatcttc 1980
   gccgatcccg ccaccctgcc acgcgatttc atcatcaccg cagaaagcga ggacggcacc 2040
   atcatgggca tcgaacacgc caaggaacca gtggccgccg ttcagttcca cccagaatcc 2100
   atcatgaccc tcggtcagga cgccggcatg cgcatgatcg agaacgtcgt ggtgcatctg 2160
   acccgcaagg ccaagaccaa ggccgcctga 2190
<210> 76
   <211> 2160
   <212> DNA
   <213> Rhodopseudomonas palustris
<400> 76
   atgaacagga ccgttttctc gcttcccgcg accagcgact ataagaccgc cgcgggcctc 60
   gcggtgacgc gcagcgccca gccttttgcc ggcggccagg cgctcgacga gctgatcgat 120
   ctgctcgacc accgccgcgg cgtgatgctg tcgtccggca caaccgtgcc gggccgctac 180
   gagagcttcg acctcggctt tgccgatccg ccgctggcgc tcaccactag ggccgaaaaa 240
   ttcaccatcg aggcgctcaa tccgcgcggc cgggtgctga tcgcgttcct gtccgacaag 300
   cttgaagagc cctgcgtggt ggtggagcag gcctgcgcca ccaagatcag gggccacatc 360
   gtccgcggcg aggccccggt cgacgaagaa caacgcaccc gccgcgccag cgcgatctcc 420
   ctggtgcgcg cggtgattgc tgccttcgcc tcgccggccg atccgatgct cgggctgtac 480
   ggcgccttcg cctacgacct tgtgttccag ttcgaggatc tgaagcagaa gcgtgcccgc 540
   gaagccgacc agcgcgacat cgtgctgtac gtgccggatc gcctgctggc ctacgatcgc 600
   gccaccggcc gcggcgtcga catttcctac gaattcgcct ggaagggcca gtccaccgcc 660
   ggcctgccga acgagaccgc cgagagcgtc tacacccaga ccggccggca gggtttcgcc 720
   gaccacgccc cgggcgacta tcccaaggtg gtcgagaagg cccgcgcggc gttcgcccgc 780
   ggcgacctgt tcgaggcggt gccgggccag ctgttcggcg agccatgcga gcggtcgccg 840
   gccgaagtgt tcaagcggtt gtgccggatc aacccgtcgc cctatggcgg cctgctcaat 900
   ctcggcgacg gcgaattcct ggtgtcggcc tcgccggaaa tgttcgtccg ctcggacggc 960
   cgccggatcg agacctgccc gatctccggc actatcgccc gcggcgtcga tgcgatcagc 1020
   gatgctgagc agatccagaa gctcttgaac tccgagaagg acgagttcga gctgaatatg 1080
   tgcaccgacg tcgaccgcaa cgacaaggcg cgggtctgcg tgccgggcac gatcaaagtt 1140
   ctcgcgcgcc gccagatcga gacctattcg aagctgttcc acaccgtcga tcacgtcgag 1200
   ggcatgctgc gaccgggttt cgacgcgctc gacgccttcc tcacccacgc ctgggcggtc 1260
   accgtcaccg gcgcgccgaa gctgtgggcg atgcagttcg tcgaggatca cgagcgtagc 1320
   ccgcggcgct ggtatgccgg cgcgttcggc gtggtcggct tcgatggctc gatcaacacc 1380
   ggcctcacca tccgcaccat ccggatgaag gacggcctcg ccgaagttcg cgtcggcgcc 1440
   acctgcctgt tcgacagcaa tccggtcgcc gaggacaagg aatgccaggt caaggccgcg 1500
   gcactgttcc aggcgctgcg cggcgatccc gccaagccgc tgtcggcggt ggcgccggac 1560
   gccactggct cgggcaagaa ggtgctgctg gtcgaccacg acgacagctt cgtgcacatg 1620
   ctggcggact atttcaggca ggtcggcgcc caggtcaccg tggtgcgcta cgttcacggc 1680
   ctgaagatgc tggccgaaaa cagctatgat cttctggtgc tgtcgcccgg tcccggccgg 1740
   ccggaggact tcaagatcaa ggatacgatc gacgccgcgc tcgccaagaa gctgccgatc 1800
   ttcggcgtct gcctcggcgt ccaggcgatg ggcgaatatt ttggcggtac gctcggccag 1860
   ctcgcgcagc cggctcacgg ccgcccgtcg cggattcagg tgcgcggcgg cgcgctgatg 1920
   cgcggtctcc cgaacgaggt caccatcggc cgctaccact cgctctatgt cgacatgcgc 1980
   gacatgccga aggagctgac cgtcaccgcc tccaccgatg acggcatcgc gatggcgatc 2040
   gagcacaaga ccctgccggt cggcggcgtg cagttccacc ccgagtcgct gatgtcgctc 2100
   ggcggcgagg tcgggctgcg gatcgtcgaa aacgccttcc ggctcggcca ggcggcctaa 2160
<210> 77
   <211> 733
   <212> PRT
   <213> Mesorhizobium loti
<400> 77
   Met Glu Thr Ala Met Thr Met Lys Val Leu Glu Asn Gly Ala Glu Ser
   1 5 10 15
   Phe Val Thr Ala Gly Gly Ile Thr Ile Thr Arg Glu Arg His Asp Arg
   20 25 30
   Pro Tyr Ala Gly Ala Ile Asp Ala Tyr Val Asp Gly Leu Asn Ser Arg
   35 40 45
   Arg Gly Ala Val Phe Ser Ser Asn Tyr Glu Tyr Pro Gly Arg Tyr Thr
   50 55 60
   Arg Trp Asp Thr Ala Ile Ile Asp Pro Pro Leu Val Ile Ser Ala Arg
   65 70 75 80
   Gly Arg Ala Met Arg Ile Glu Ala Leu Asn Arg Arg Gly Glu Ala Leu
   85 90 95
   Leu Pro Val Ile Gly Lys Thr Leu Gly Gly Leu Ala Asp Ile Thr Ile
   100 105 110
   Ala Glu Thr Thr Lys Thr Leu Ile Arg Leu Asp Val Ala Lys Pro Gly
   115 120 125
   Arg Val Phe Thr Glu Glu Glu Arg Ser Arg Val Pro Ser Val Phe Thr
   130 135 140
   Val Leu Arg Ala Ile Thr Ala Leu Phe Lys Thr Asp Glu Asp Ala Asn
   145 150 155 160
   Leu Gly Leu Tyr Gly Ala Phe Gly Tyr Asp Leu Ser Phe Gln Phe Asp
   165 170 175
   Pro Val Asp Tyr Lys Leu Glu Arg Lys Pro Ser Gln Arg Asp Leu Val
   180 185 190
   Leu Phe Leu Pro Asp Glu Ile Leu Val Val Asp His Tyr Ser Ala Lys
   195 200 205
   Ala Trp Thr Asp Arg Tyr Asp Tyr Ser Gly Glu Gly Phe Ser Thr Glu
   210 215 220
   Gly Leu Pro Arg Asp Ala Ile Ala Glu Pro Phe Lys Thr Ala Asp Arg
   225 230 235 240
   Ile Pro Pro Arg Gly Asp His Glu Pro Gly Glu Tyr Ala Asn Leu Val
   245 250 255
   Arg Arg Ala Met Asp Ser Phe Lys Arg Gly Asp Leu Phe Glu Val Val
   260 265 270
   Pro Gly Gln Met Phe Tyr Glu Arg Cys Glu Thr Gln Pro Ser Asp Ile
   275 280 285
   Ser Arg Lys Leu Lys Ser Ile Asn Pro Ser Pro Tyr Ser Phe Phe Ile
   290 295 300
   Asn Leu Gly Glu Asn Glu Tyr Leu Ile Gly Ala Ser Pro Glu Met Phe
   305 310 315 320
   Val Arg Val Asn Gly Arg Arg Val Glu Thr Cys Pro Ile Ser Gly Thr
   325 330 335
   Ile Lys Arg Gly Asp Asp Ala Ile Ser Asp Ser Glu Gln Ile Leu Lys
   340 345 350
   Leu Leu Asn Ser Lys Lys Asp Glu Ser Glu Leu Thr Met Cys Ser Asp
   355 360 365
   Val Asp Arg Asn Asp Lys Ser Arg Val Cys Glu Pro Gly Ser Val Arg
   370 375 380
   Val Ile Gly Arg Arg Gln Ile Glu Met Tyr Ser Arg Leu Ile His Thr
   385 390 395 400
   Val Asp His Ile Glu Gly Arg Leu Arg Glu Gly Met Asp Ala Phe Asp
   405 410 415
   Ala Phe Leu Ser His Ala Trp Ala Val Thr Val Thr Gly Ala Pro Lys
   420 425 430
   Leu Trp Ala Met Arg Phe Ile Glu Gln Asn Glu Lys Ser Pro Arg Ala
   435 440 445
   Trp Tyr Gly Gly Ala Ile Gly Met Val Asn Phe Asn Gly Asp Met Asn
       450                 455                 460
   Thr Gly Leu Thr Leu Arg Thr Ile Arg Ile Lys Asp Gly Ile Ala Glu
   465                 470                 475                 480
   Val Arg Ala Gly Ala Thr Leu Leu Phe Asp Ser Ile Pro Glu Glu Glu
                   485                 490                 495
   Glu Ala Glu Thr Glu Leu Lys Ala Ser Ala Met Leu Ser Ala Ile Arg
               500                 505                 510
   Asp Ala Lys Thr Gly Asn Ser Ala Ser Thr Glu Arg Thr Thr Ala Arg
           515                 520                 525
   Val Gly Asp Gly Val Asn Ile Leu Leu Val Asp His Glu Asp Ser Phe
       530                 535                 540
   Val His Thr Leu Ala Asn Tyr Phe Arg Gln Thr Gly Ala Asn Val Ser
   545                 550                 555                 560
   Thr Val Arg Thr Pro Val Pro Asp Glu Val Phe Glu Arg Leu Lys Pro
                   565                 570                 575
   Asp Leu Val Val Leu Ser Pro Gly Pro Gly Thr Pro Lys Asp Phe Asp
               580                 585                 590
   Cys Ala Ala Thr Ile Arg Arg Ala Arg Ala Arg Asp Leu Pro Ile Phe
           595                 600                 605
   Gly Val Cys Leu Gly Leu Gln Ala Leu Ala Glu Ala Tyr Gly Gly Glu
       610                 615                 620
   Leu Arg Gln Leu His Ile Pro Met His Gly Lys Pro Ser Arg Ile Arg
   625                 630                 635                 640
   Val Ser Lys Pro Gly Ile Ile Phe Ser Gly Leu Pro Lys Glu Val Thr
                   645                 650                 655
   Val Gly Arg Tyr His Ser Ile Phe Ala Asp Pro Val Arg Leu Pro Asp
               660                 665                 670
   Asp Phe Ile Val Thr Ala Glu Thr Glu Asp Gly Ile Ile Met Ala Phe
           675                 680                 685
   Glu His Arg Lys Glu Pro Ile Ala Ala Val Gln Phe His Pro Glu Ser
       690                 695                 700
   Ile Met Thr Leu Gly His Asn Ala Gly Met Arg Ile Ile Glu Asn Ile
   705                 710                 715                 720
   Val Ala His Leu Pro Arg Lys Ala Lys Glu Lys Ala Ala
   725 730
<210> 78
   <211> 732
   <212> PRT
   <213> Azospirillum brasilense
<400> 78
   Met Tyr Pro Ala Asp Leu Leu Ala Ser Pro Asp Leu Leu Glu Pro Leu
   1 5 10 15
   Arg Phe Gln Thr Arg Gly Gly Val Thr Val Thr Arg Arg Ala Thr Ala
   20 25 30
   Leu Asp Pro Arg Thr Ala Leu Asp Pro Val Ile Asp Ala Leu Asp Arg
   35 40 45
   Arg Arg Gly Leu Leu Leu Ser Ser Gly Val Glu Ala Pro Gly Arg Tyr
   50 55 60
   Arg Arg His Ala Leu Gly Phe Thr Asp Pro Ala Val Ala Leu Thr Ala
   65 70 75 80
   Arg Gly Arg Thr Leu Arg Ile Asp Ala Leu Asn Gly Arg Gly Gln Val
   85 90 95
   Leu Leu Pro Ala Val Ala Glu Ala Leu Arg Gly Leu Glu Ala Leu Ala
   100 105 110
   Gly Leu Glu Glu Ala Pro Ser Arg Val Thr Ala Ser Ser Ala Ser Pro
   115 120 125
   Ala Pro Leu Pro Gly Glu Glu Arg Ser Arg Gln Pro Ser Val Phe Ser
   130 135 140
   Val Leu Arg Ala Val Leu Asp Leu Phe Ala Ala Pro Asp Asp Pro Leu
   145 150 155 160
   Leu Gly Leu Tyr Gly Ala Phe Ala Tyr Asp Leu Ala Phe Gln Phe Glu
   165 170 175
   Pro Ile Arg Gln Arg Leu Glu Arg Pro Asp Asp Gln Arg Asp Leu Leu
   180 185 190
   Leu Tyr Leu Pro Asp Arg Leu Val Ala Leu Asp Pro Ile Ala Gly Leu
   195 200 205
   Ala Arg Leu Val Ala Tyr Glu Phe Ile Thr Ala Ala Gly Ser Thr Glu
   210 215 220
   Gly Leu Glu Cys Gly Gly Arg Asp His Pro Tyr Arg Pro Asp Thr Asn
   225                 230                 235                 240
   Ala Glu Ala Gly Cys Asp His Ala Pro Gly Asp Tyr Gln Arg Val Val
                   245                 250                 255
   Glu Ser Ala Lys Ala Ala Phe Arg Arg Gly Asp Leu Phe Glu Val Val
               260                 265                 270
   Pro Gly Gln Thr Phe Ala Glu Pro Cys Ala Asp Ala Pro Ser Ser Val
           275                 280                 285
   Phe Arg Arg Leu Arg Ala Ala Asn Pro Ala Pro Tyr Glu Ala Phe Val
       290                 295                 300
   Asn Leu Gly Arg Gly Glu Phe Leu Val Ala Ala Ser Pro Glu Met Tyr
   305                 310                 315                 320
   Val Arg Val Ala Gly Gly Arg Val Glu Thr Cys Pro Ile Ser Gly Thr
                   325                 330                 335
   Val Ala Arg Gly Ala Asp Ala Leu Gly Asp Ala Ala Gln Val Leu Arg
               340                 345                 350
   Leu Leu Thr Ser Ala Lys Asp Ala Ala Glu Leu Thr Met Cys Thr Asp
           355                 360                 365
   Val Asp Arg Asn Asp Lys Ala Arg Val Cys Glu Pro Gly Ser Val Arg
       370                 375                 380
   Val Ile Gly Arg Arg Met Ile Glu Leu Tyr Ser Arg Leu Ile His Thr
   385                 390                 395                 400
   Val Asp His Val Glu Gly Arg Leu Arg Ser Gly Met Asp Ala Leu Asp
                   405                 410                 415
   Ala Phe Leu Thr His Ser Trp Ala Val Thr Val Thr Gly Ala Pro Lys
               420                 425                 430
   Arg Trp Ala Met Gln Phe Leu Glu Asp Thr Glu Gln Ser Pro Arg Arg
           435                 440                 445
   Trp Tyr Gly Gly Ala Phe Gly Arg Leu Gly Phe Asp Gly Gly Met Asp
       450                 455                 460
   Thr Gly Leu Thr Leu Arg Thr Ile Arg Met Ala Glu Gly Val Ala Tyr
   465                 470                 475                 480
   Val Arg Ala Gly Ala Thr Leu Leu Ser Asp Ser Asp Pro Asp Ala Glu
                   485                 490                 495
   Asp Ala Glu Cys Arg Leu Lys Ala Ala Ala Phe Arg Asp Ala Ile Arg
   500 505 510
   Gly Thr Ala Ala Gly Ala Ala Pro Thr Leu Pro Ala Ala Pro Arg Gly
   515 520 525
   Gly Glu Gly Arg Arg Val Leu Leu Val Asp His Asp Asp Ser Phe Val
   530 535 540
   His Thr Leu Ala Asp Tyr Leu Arg Gln Thr Gly Ala Ser Val Thr Thr
   545 550 555 560
   Leu Arg His Ser His Ala Arg Ala Ala Leu Ala Glu Arg Arg Pro Asp
   565 570 575
   Leu Val Val Leu Ser Pro Gly Pro Gly Arg Pro Ala Asp Phe Asp Val
   580 585 590
   Ala Gly Thr Ile Asp Ala Ala Leu Ala Leu Gly Leu Pro Val Phe Gly
   595 600 605
   Val Cys Leu Gly Leu Gln Gly Met Val Glu Arg Phe Gly Gly Ala Leu
   610 615 620
   Asp Val Leu Pro Glu Pro Val His Gly Lys Ala Thr Glu Val Arg Val
   625 630 635 640
   Leu Gly Gly Ala Leu Phe Ala Gly Leu Pro Glu Arg Leu Thr Val Gly
   645 650 655
   Arg Tyr His Ser Leu Val Ala Arg Arg Asp Arg Leu Pro Ala Asp Leu
   660 665 670
   Thr Val Thr Ala Glu Thr Ala Asp Gly Leu Val Met Ala Val Glu His
   675 680 685
   Arg Arg Leu Pro Leu Ala Ala Val Gln Phe His Pro Glu Ser Ile Leu
   690 695 700
   Ser Leu Asp Gly Gly Ala Gly Leu Ala Leu Leu Gly Asn Val Met Asp
   705 710 715 720
   Arg Leu Ala Ala Gly Ala Leu Thr Asp Ala Ala Ala
   725 730
<210> 79
   <211> 731
   <212> PRT
   <213> Brucella melitensis
<400> 79
   Met Asn Ala Lys Thr Ala Asp Ser Glu Ile Phe Gln His Glu Thr Ala
   1               5                   10                  15
   Gly Gly Ile Ile Val Glu Arg Val Arg His Leu Thr Ala Tyr Lys Gly
               20                  25                  30
   Ala Ile Glu Ser Tyr Ile Asp Val Leu Asn Glu Trp Arg Gly Ala Val
           35                  40                  45
   Phe Ser Ser Asn Tyr Glu Tyr Pro Gly Arg Tyr Thr Arg Trp Asp Thr
       50                  55                  60
   Ala Ile Val Asp Pro Pro Val Val Ile Thr Ser Arg Ala Arg Thr Met
   65                  70                  75                  80
   Arg Ile Glu Ala Leu Asn Ala Arg Gly Val Ile Leu Leu Arg Pro Ile
                   85                  90                  95
   Leu Asp Thr Val Lys Ala Leu Ser Glu Val Lys Ile Asp Gln Ser Gly
               100                 105                 110
   Glu Asn Arg Ile Asp Leu Thr Ile Val Glu Pro Val Gly Thr Phe Thr
           115                 120                 125
   Glu Glu Glu Arg Ser Arg Met Pro Ser Val Phe Thr Val Leu Arg Ala
       130                 135                 140
   Ile Val Gly Leu Phe Phe Ser Glu Glu Asp Ala Asn Leu Gly Leu Tyr
   145                 150                 155                 160
   Gly Ala Phe Gly Tyr Asp Leu Ala Phe Gln Phe Asp Pro Ile Gln Tyr
                   165                 170                 175
   Lys Leu Lys Arg Pro Asp Asp Gln Arg Asp Leu Val Leu Phe Ile Pro
               180                 185                 190
   Asp Glu Ile Phe Val Ala Asp His Tyr Ala Ala Arg Ala Trp Val Asp
           195                 200                 205
   Arg Tyr Glu Phe Arg Cys Gly Gly Ser Ser Thr His Gly Leu Asp Arg
       210                 215                 220
   Ala Thr Pro Val Val Pro Phe Lys Pro Ser Glu Arg Lys Leu Ala Arg
   225                 230                 235                 240
   Gly Asp His Asn Pro Gly Glu Tyr Ala Arg Leu Val Glu Arg Ala Lys
                   245                 250                 255
   Glu Ser Phe Lys Arg Gly Asp Leu Phe Glu Val Val Pro Gly Gln Thr
               260                 265                 270
   Phe Tyr Glu Arg Cys His Thr Ala Pro Ser Glu Ile Phe Arg Arg Leu
   275 280 285
   Lys Ser Ile Asn Pro Ser Pro Tyr Ser Phe Phe Ile Asn Leu Gly Glu
   290 295 300
   Ser Glu Tyr Leu Val Gly Ala Ser Pro Glu Met Phe Val Arg Val Asn
   305 310 315 320
   Gly Arg Arg Ile Glu Thr Cys Pro Ile Ser Gly Thr Ile Lys Arg Gly
   325 330 335
   Glu Asp Ala Ile Ser Asp Ser Glu Gln Ile Leu Lys Leu Leu Asn Ser
   340 345 350
   Lys Lys Asp Glu Ser Glu Leu Thr Met Cys Ser Asp Val Asp Arg Asn
   355 360 365
   Asp Lys Ser Arg Val Cys Glu Pro Gly Ser Val Arg Val Ile Gly Arg
   370 375 380
   Arg Gln Ile Glu Met Tyr Ser Arg Leu Ile His Thr Val Asp His Ile
   385 390 395 400
   Glu Gly Arg Leu Arg Asp Gly Met Asp Ala Phe Asp Gly Phe Leu Ser
   405 410 415
   His Ala Trp Ala Val Thr Val Thr Gly Ala Pro Lys Leu Trp Ala Met
   420 425 430
   Arg Phe Leu Glu Glu Asn Glu Arg Ser Pro Arg Ala Trp Tyr Gly Gly
   435 440 445
   Ala Ile Gly Met Met His Phe Asn Gly Asp Met Asn Thr Gly Leu Thr
   450 455 460
   Leu Arg Thr Ile Arg Ile Lys Asp Gly Val Ala Glu Ile Arg Ala Gly
   465 470 475 480
   Ala Thr Leu Leu Phe Asp Ser Asn Pro Asp Glu Glu Glu Ala Glu Thr
   485 490 495
   Glu Leu Lys Ala Ser Ala Met Ile Ala Ala Val Arg Asp Ala Gln Lys
   500 505 510
   Ser Asn Gln Ile Ala Glu Glu Ser Val Ala Ala Lys Val Gly Glu Gly
   515 520 525
   Val Ser Ile Leu Leu Val Asp His Glu Asp Ser Phe Val His Thr Leu
   530 535 540
   Ala Asn Tyr Phe Arg Gln Thr Gly Ala Lys Val Ser Thr Val Arg Ser
   545 550 555 560
   Pro Val Ala Glu Glu Ile Phe Asp Arg Val Asn Pro Asp Leu Val Val
   565 570 575
   Leu Ser Pro Gly Pro Gly Ser Pro Gln Asp Phe Asp Cys Lys Ala Thr
   580 585 590
   Ile Asp Lys Ala Arg Lys Arg Gln Leu Pro Ile Phe Gly Val Cys Leu
   595 600 605
   Gly Leu Gln Ala Leu Ala Glu Ala Tyr Gly Gly Ala Leu Arg Gln Leu
   610 615 620
   Arg Val Pro Val His Gly Lys Pro Ser Arg Ile Arg Val Ser Lys Pro
   625 630 635 640
   Glu Arg Ile Phe Ser Gly Leu Pro Glu Glu Val Thr Val Gly Arg Tyr
   645 650 655
   His Ser Ile Phe Ala Asp Pro Glu Arg Leu Pro Asp Asp Phe Leu Val
   660 665 670
   Thr Ala Glu Thr Glu Asp Gly Ile Ile Met Ala Phe Glu His Lys His
   675 680 685
   Glu Pro Val Ala Ala Val Gln Phe His Pro Glu Ser Ile Met Thr Leu
   690 695 700
   Gly His Asn Ala Gly Met Arg Met Ile Glu Asn Ile Val Thr His Leu
   705 710 715 720
   Ala Gly Lys His Lys Ala Arg Arg Thr Asn Tyr
   725 730
<210> 80
   <211> 735
   <212> PRT
   <213> Nostoc sp.
<400> 80
   Met Ile Ala Asp Ser His Ser Tyr Arg Thr Asn Gly Asn Val Arg Val
   1               5                   10                  15
   Ser Arg Ser Ile Thr Gln Val Lys Met Glu Thr Ala Leu Glu Glu Ile
               20                  25                  30
   Leu Phe Tyr Leu Asn Ser Gln Arg Gly Gly Leu Leu Thr Ser Ser Tyr
           35                  40                  45
   Glu Tyr Pro Gly Arg Tyr Lys Arg Trp Ala Ile Gly Phe Val Asn Pro
       50                  55                  60
   Pro Val Glu Leu Ser Thr Ser Gly Asn Thr Phe Thr Leu Thr Ala Leu
   65                  70                  75                  80
   Asn Glu Arg Gly Tyr Val Leu Leu Pro Val Ile Phe Glu Cys Leu Ser
                   85                  90                  95
   Lys Ser Glu Gln Leu Gln Lys Leu Thr Glu His His His Lys Ile Thr
               100                 105                 110
   Gly Leu Val Lys Ser Thr Pro Glu Phe Phe Ala Glu Glu Glu Arg Ser
           115                 120                 125
   Lys Gln Pro Ser Thr Phe Thr Val Ile Arg Glu Ile Leu His Ile Phe
       130                 135                 140
   Ser Ser Gln Glu Asp Glu His Leu Gly Leu Tyr Gly Ala Phe Gly Tyr
   145                 150                 155                 160
   Asp Leu Val Phe Gln Phe Glu Gln Ile Thr Gln Cys Leu Glu Arg Pro
                   165                 170                 175
   Gln Asp Gln Arg Asp Leu Val Leu Tyr Leu Pro Asp Glu Leu Ile Val
               180                 185                 190
   Val Asp Tyr Tyr Gln Gln Gln Ala Phe Arg Leu Glu Tyr Asp Phe Ile
           195                 200                 205
   Thr Ala His Gly Ser Thr Tyr Asp Leu Pro Arg Thr Gly Glu Ser Val
       210                 215                 220
   Asp Tyr Arg Gly Gln Cys Leu Thr Pro Pro Gln Asn Ala Asp His Lys
   225                 230                 235                 240
   Ile Gly Glu Tyr Ala Lys Leu Val Glu Phe Ala Leu Asp Tyr Phe Arg
                   245                 250                 255
   Arg Gly Asp Leu Phe Glu Val Val Pro Ser Gln Asn Phe Phe Thr Ala
               260                 265                 270
   Cys Glu Ala Pro Pro Ser Gln Leu Phe Glu Thr Leu Lys Gln Ile Asn
           275                 280                 285
   Pro Ser Pro Tyr Gly Phe Ile Phe Asn Leu Gly Gly Glu Tyr Ile Ile
       290                 295                 300
   Gly Ala Ser Pro Glu Met Phe Val Arg Val Glu Gly Arg Arg Val Glu
   305                 310                 315                 320
   Thr Cys Pro Ile Ser Gly Thr Ile Thr Arg Gly His Asp Ala Ile Asp
                   325                 330                 335
   Asp Ala Val Gln Ile Arg Gln Leu Leu Asn Ser His Lys Asp Glu Ala
   340 345 350
   Glu Leu Thr Met Cys Thr Asp Val Asp Arg Asn Asp Lys Ser Arg Ile
   355 360 365
   Cys Glu Pro Gly Ser Val Lys Val Ile Gly Arg Arg Gln Ile Glu Leu
   370 375 380
   Tyr Ser His Leu Ile His Thr Val Asp His Val Glu Gly Ile Leu Arg
   385 390 395 400
   Pro Glu Phe Asp Ala Leu Asp Ala Phe Leu Ser His Thr Trp Ala Val
   405 410 415
   Thr Val Thr Gly Ala Pro Lys Arg Ala Ala Ile Gln Phe Ile Glu Lys
   420 425 430
   Asn Glu Arg Ser Val Arg Arg Trp Tyr Gly Gly Ala Val Gly Tyr Leu
   435 440 445
   Asn Phe Asn Gly Asn Leu Asn Thr Gly Leu Ile Leu Arg Thr Ile Arg
   450 455 460
   Leu Gln Asp Ser Ile Ala Glu Val Arg Val Gly Ala Thr Leu Leu Tyr
   465 470 475 480
   Asp Ser Ile Pro Gln Ala Glu Glu Gln Glu Thr Ile Thr Lys Ala Ala
   485 490 495
   Ala Ala Phe Glu Thr Ile Arg Arg Ala Lys Gln Ile Asp Pro Gln Ile
   500 505 510
   Glu Glu Ser Ser Thr Arg Lys Leu Ser Lys Tyr Leu Pro Asp Gly Gln
   515 520 525
   Ser Gly Lys His Ile Leu Leu Ile Asp His Glu Asp Ser Phe Val His
   530 535 540
   Thr Leu Ala Asn Tyr Ile Arg Ser Thr Gly Ala Thr Val Thr Thr Leu
   545 550 555 560
   Arg His Gly Phe Ser Glu Ser Leu Phe Asp Thr Glu Arg Pro Asp Leu
   565 570 575
   Val Val Leu Ser Pro Gly Pro Gly Arg Pro Ser Glu Phe Lys Val Gln
   580 585 590
   Glu Thr Val Ala Ala Cys Val Arg Arg Gln Ile Pro Leu Phe Gly Val
   595 600 605
   Cys Leu Gly Leu Gln Gly Ile Val Glu Ala Phe Gly Gly Glu Leu Gly
   610 615 620
   Val Leu Asn Tyr Pro Gln His Gly Lys Ser Ser Arg Ile Phe Val Thr
   625 630 635 640
   Ala Pro Asp Ser Val Met Phe Gln Asp Leu Pro Glu Ser Phe Thr Val
   645 650 655
   Gly Arg Tyr His Ser Leu Phe Ala Leu Ser Gln Arg Leu Pro Lys Glu
   660 665 670
   Leu Lys Val Thr Ala Ile Ser Asp Asp Glu Val Ile Met Ala Ile Glu
   675 680 685
   His Gln Thr Leu Pro Ile Ala Ala Val Gln Phe His Pro Glu Ser Ile
   690 695 700
   Met Thr Leu Ala Gly Glu Val Gly Leu Met Met Ile Lys Asn Val Val
   705 710 715 720
   Gln Lys Tyr Thr Gln Ser Gln Gln Ser Thr Val Pro Ile Tyr Asp
   725 730 735
<210> 81
   <211> 715
   <212> PRT
   <213> Nostoc sp.
<400> 81
   Met Arg Val Ser Arg Ser Thr Thr Glu Val Lys Met Asp Thr Ala Leu
   1 5 10 15
   Asp Glu Ile Leu Phe His Leu Asn Gln Val Arg Gly Gly Leu Leu Thr
   20 25 30
   Ser Ser Tyr Glu Tyr Pro Gly Arg Tyr Lys Arg Trp Ala Ile Gly Phe
   35 40 45
   Ile Asn Pro Pro Leu Gln Leu Thr Thr Arg Glu Asn Ala Phe Thr Ile
   50 55 60
   Ser Ser Leu Asn Pro Arg Gly Gln Val Leu Leu Pro Thr Leu Phe Gln
   65 70 75 80
   His Leu Ser Ala Gln Ser Gln Leu Gln Gln Ile Ser Leu Asn His Asp
   85 90 95
   Tyr Ile Thr Gly Glu Ile Arg Pro Thr Lys Gln Leu Phe Thr Glu Glu
   100 105 110
   Gln Arg Ser Lys Gln Pro Ser Ala Phe Thr Val Ile Arg Glu Ile Leu
   115 120 125
   Gln Ile Phe Ala Ser Asp Glu Asp Glu His Leu Gly Leu Tyr Gly Ala
   130 135 140
   Phe Gly Tyr Asp Leu Val Phe Gln Phe Glu Pro Ile Pro Gln Lys Ile
   145 150 155 160
   Ala Arg Pro Ala Asp Gln Arg Asp Leu Val Leu Tyr Leu Pro Asp Glu
   165 170 175
   Leu Ile Val Val Asp Tyr Tyr Leu Gln Lys Ala Tyr Arg His Gln Tyr
   180 185 190
   Glu Phe Ala Thr Glu His Gly Asn Thr Glu His Leu Pro Arg Thr Gly
   195 200 205
   Gln Ser Ile Asp Tyr Gln Gly Lys His Leu Leu Pro Asn Gln Thr Ala
   210 215 220
   Asp His Gln Pro Gly Glu Tyr Ala Asn Leu Val Glu Gln Ala Leu Asp
   225 230 235 240
   Tyr Phe Arg Arg Gly Asp Leu Phe Glu Val Val Pro Ser Gln Asn Phe
   245 250 255
   Phe Thr Ala Cys Glu Gln Ser Pro Ser Gln Leu Phe Gln Thr Leu Arg
   260 265 270
   Gln Ile Asn Pro Ser Pro Tyr Gly Phe Leu Leu Asn Leu Gly Gly Glu
   275 280 285
   Tyr Leu Ile Gly Ala Ser Pro Glu Met Phe Val Arg Val Asp Gly Arg
   290 295 300
   Arg Val Glu Thr Cys Pro Ile Ser Gly Thr Ile Arg Arg Gly Glu Asp
   305 310 315 320
   Ala Leu Gly Asp Ala Val Gln Ile Arg Gln Leu Leu Asn Ser His Lys
   325 330 335
   Asp Glu Ala Glu Leu Thr Met Cys Thr Asp Val Asp Arg Asn Asp Lys
   340 345 350
   Ser Arg Ile Cys Glu Pro Gly Ser Val Arg Val Ile Gly Arg Arg Gln
   355 360 365
   Ile Glu Leu Tyr Ser His Leu Ile His Thr Val Asp His Val Glu Gly
   370 375 380
   Ile Leu Arg Pro Glu Phe Asp Ala Leu Asp Ala Phe Leu Ser His Thr
   385 390 395 400
   Trp Ala Val Thr Val Thr Gly Ala Pro Lys Arg Ala Ala Met Gln Phe
   405 410 415
   Ile Glu Gln His Glu Arg Ser Ala Arg Arg Trp Tyr Gly Gly Ala Val
   420 425 430
   Gly Tyr Leu Gly Phe Asn Gly Asn Leu Asn Thr Gly Leu Thr Leu Arg
   435 440 445
   Thr Ile Arg Leu Gln Asp Ser Ile Ala Glu Val Arg Val Gly Ala Thr
   450 455 460
   Val Leu Tyr Asp Ser Ile Pro Ser Ala Glu Glu Glu Glu Thr Ile Thr
   465 470 475 480
   Lys Ala Thr Ala Leu Phe Glu Thr Ile Arg Arg His Thr Thr Ala Asn
   485 490 495
   Lys Thr Gln Gly Asn Asp Ser His Arg Pro Gly Asp Ile Ala His Asn
   500 505 510
   Lys Arg Ile Leu Leu Ile Asp Tyr Glu Asp Ser Phe Val His Thr Leu
   515 520 525
   Ala Asn Tyr Ile Arg Thr Thr Gly Ala Thr Val Thr Thr Leu Arg His
   530 535 540
   Gly Phe Ala Glu Ser Tyr Phe Asp Ala Glu Arg Pro Asp Leu Val Val
   545 550 555 560
   Leu Ser Pro Gly Pro Gly Arg Pro Ser Asp Phe Arg Val Pro Gln Thr
   565 570 575
   Val Ala Ala Leu Val Gly Arg Glu Ile Pro Ile Phe Gly Val Cys Leu
   580 585 590
   Gly Leu Gln Gly Ile Val Glu Ala Phe Gly Gly Glu Leu Gly Val Leu
   595 600 605
   Asp Tyr Pro Gln His Gly Lys Pro Ala Arg Ile Ser Val Thr Ala Pro
   610 615 620
   Asp Ser Val Leu Phe Gln Asn Leu Pro Ala Ser Phe Ile Val Gly Arg
   625 630 635 640
   Tyr His Ser Leu Phe Ala Gln Pro Gln Thr Ile Pro Gly Glu Leu Lys
   645 650 655
   Val Thr Ala Ile Ser Glu Asp Asn Val Ile Met Ala Ile Glu His Gln
   660 665 670
   Thr Leu Pro Ile Ala Ala Val Gln Phe His Pro Glu Ser Ile Met Thr
   675 680 685
   Leu Ala Gly Glu Val Gly Gln Thr Ile Ile Lys Asn Val Val Gln Thr
   690 695 700
   Tyr Thr Gln Thr Leu Glu Thr Ser Ile Tyr Ser
   705 710 715
<210> 82
   <211> 719
   <212> PRT
   <213> Rhodopseudomonas palustris
<400> 82
   Met Asn Arg Thr Val Phe Ser Leu Pro Ala Thr Ser Asp Tyr Lys Thr
   1 5 10 15
   Ala Ala Gly Leu Ala Val Thr Arg Ser Ala Gln Pro Phe Ala Gly Gly
   20 25 30
   Gln Ala Leu Asp Glu Leu Ile Asp Leu Leu Asp His Arg Arg Gly Val
   35 40 45
   Met Leu Ser Ser Gly Thr Thr Val Pro Gly Arg Tyr Glu Ser Phe Asp
   50 55 60
   Leu Gly Phe Ala Asp Pro Pro Leu Ala Leu Thr Thr Arg Ala Glu Lys
   65 70 75 80
   Phe Thr Ile Glu Ala Leu Asn Pro Arg Gly Arg Val Leu Ile Ala Phe
   85 90 95
   Leu Ser Asp Lys Leu Glu Glu Pro Cys Val Val Val Glu Gln Ala Cys
   100 105 110
   Ala Thr Lys Ile Arg Gly His Ile Val Arg Gly Glu Ala Pro Val Asp
   115 120 125
   Glu Glu Gln Arg Thr Arg Arg Ala Ser Ala Ile Ser Leu Val Arg Ala
   130 135 140
   Val Ile Ala Ala Phe Ala Ser Pro Ala Asp Pro Met Leu Gly Leu Tyr
   145 150 155 160
   Gly Ala Phe Ala Tyr Asp Leu Val Phe Gln Phe Glu Asp Leu Lys Gln
   165 170 175
   Lys Arg Ala Arg Glu Ala Asp Gln Arg Asp Ile Val Leu Tyr Val Pro
   180 185 190
   Asp Arg Leu Leu Ala Tyr Asp Arg Ala Thr Gly Arg Gly Val Asp Ile
   195 200 205
   Ser Tyr Glu Phe Ala Trp Lys Gly Gln Ser Thr Ala Gly Leu Pro Asn
   210 215 220
   Glu Thr Ala Glu Ser Val Tyr Thr Gln Thr Gly Arg Gln Gly Phe Ala
   225 230 235 240
   Asp His Ala Pro Gly Asp Tyr Pro Lys Val Val Glu Lys Ala Arg Ala
   245 250 255
   Ala Phe Ala Arg Gly Asp Leu Phe Glu Ala Val Pro Gly Gln Leu Phe
   260 265 270
   Gly Glu Pro Cys Glu Arg Ser Pro Ala Glu Val Phe Lys Arg Leu Cys
   275 280 285
   Arg Ile Asn Pro Ser Pro Tyr Gly Gly Leu Leu Asn Leu Gly Asp Gly
   290 295 300
   Glu Phe Leu Val Ser Ala Ser Pro Glu Met Phe Val Arg Ser Asp Gly
   305 310 315 320
   Arg Arg Ile Glu Thr Cys Pro Ile Ser Gly Thr Ile Ala Arg Gly Val
   325 330 335
   Asp Ala Ile Ser Asp Ala Glu Gln Ile Gln Lys Leu Leu Asn Ser Glu
   340 345 350
   Lys Asp Glu Phe Glu Leu Asn Met Cys Thr Asp Val Asp Arg Asn Asp
   355 360 365
   Lys Ala Arg Val Cys Val Pro Gly Thr Ile Lys Val Leu Ala Arg Arg
   370 375 380
   Gln Ile Glu Thr Tyr Ser Lys Leu Phe His Thr Val Asp His Val Glu
   385 390 395 400
   Gly Met Leu Arg Pro Gly Phe Asp Ala Leu Asp Ala Phe Leu Thr His
   405 410 415
   Ala Trp Ala Val Thr Val Thr Gly Ala Pro Lys Leu Trp Ala Met Gln
   420 425 430
   Phe Val Glu Asp His Glu Arg Ser Pro Arg Arg Trp Tyr Ala Gly Ala
   435 440 445
   Phe Gly Val Val Gly Phe Asp Gly Ser Ile Asn Thr Gly Leu Thr Ile
   450 455 460
   Arg Thr Ile Arg Met Lys Asp Gly Leu Ala Glu Val Arg Val Gly Ala
   465 470 475 480
   Thr Cys Leu Phe Asp Ser Asn Pro Val Ala Glu Asp Lys Glu Cys Gln
   485 490 495
   Val Lys Ala Ala Ala Leu Phe Gln Ala Leu Arg Gly Asp Pro Ala Lys
   500 505 510
   Pro Leu Ser Ala Val Ala Pro Asp Ala Thr Gly Ser Gly Lys Lys Val
   515 520 525
   Leu Leu Val Asp His Asp Asp Ser Phe Val His Met Leu Ala Asp Tyr
   530 535 540
   Phe Arg Gln Val Gly Ala Gln Val Thr Val Val Arg Tyr Val His Gly
   545 550 555 560
   Leu Lys Met Leu Ala Glu Asn Ser Tyr Asp Leu Leu Val Leu Ser Pro
   565 570 575
   Gly Pro Gly Arg Pro Glu Asp Phe Lys Ile Lys Asp Thr Ile Asp Ala
   580 585 590
   Ala Leu Ala Lys Lys Leu Pro Ile Phe Gly Val Cys Leu Gly Val Gln
   595 600 605
   Ala Met Gly Glu Tyr Phe Gly Gly Thr Leu Gly Gln Leu Ala Gln Pro
   610 615 620
   Ala His Gly Arg Pro Ser Arg Ile Gln Val Arg Gly Gly Ala Leu Met
   625 630 635 640
   Arg Gly Leu Pro Asn Glu Val Thr Ile Gly Arg Tyr His Ser Leu Tyr
   645 650 655
   Val Asp Met Arg Asp Met Pro Lys Glu Leu Thr Val Thr Ala Ser Thr
   660 665 670
   Asp Asp Gly Ile Ala Met Ala Ile Glu His Lys Thr Leu Pro Val Gly
   675 680 685
   Gly Val Gln Phe His Pro Glu Ser Leu Met Ser Leu Gly Gly Glu Val
   690 695 700
   Gly Leu Arg Ile Val Glu Asn Ala Phe Arg Leu Gly Gln Ala Ala
   705 710 715
<210> 83
   <211> 2160
   <212> DNA
   <213> Rhodopseudomonas palustris
<400> 83
   atgaacagga ccgttttctc gcttcccgcg accagcgact ataagaccgc cgcgggcctc 60
   gcggtgacgc gcagcgccca gccttttgcc ggcggccagg cgctcgacga gctgatcgat 120
   ctgctcgacc accgccgcgg cgtgatgctg tcgtccggca caaccgtgcc gggccgctac 180
   gagagcttcg acctcggctt tgccgatccg ccgctggcgc tcaccactag ggccgaaaaa 240
   ttcaccatcg aggcgctcaa tccgcgcggc cgggtgctga tcgcgttcct gtccgacaag 300
   cttgaagagc cctgcgtggt ggtggagcag gcctgcgcca ccaagatcag gggccacatc 360
   gtccgcggcg aggccccggt cgacgaagaa caacgcaccc gccgcgccag cgcgatctcc 420
   ctggtgcgcg cggtgattgc tgccttcgcc tcgccggccg atccgatgct cgggctgtac 480
   ggcgccttcg cctacgacct tgtgttccag ttcgaggatc tgaagcagaa gcgtgcccgc 540
   gaagccgacc agcgcgacat cgtgctgtac gtgccggatc gcctgctggc ctacgatcgc 600
   gccaccggcc gcggcgtcga catttcctac gaattcgcct ggaagggcca gtccaccgcc 660
   ggcctgccga acgagaccgc cgagagcgtc tacacccaga ccggccggca gggtttcgcc 720
   gaccacgccc cgggcgacta tcccaaggtg gtcgagaagg cccgcgcggc gttcgcccgc 780
   ggcgacctgt tcgaggcggt gccgggccag ctgttcggcg agccatgcga gcggtcgccg 840
   gccgaagtgt tcaagcggtt gtgccggatc aacccgtcgc cctatggcgg cctgctcaat 900
   ctcggcgacg gcgaattcct ggtgtcggcc tcgccggaaa tgttcgtccg ctcggacggc 960
   cgccggatcg agacctgccc gatctccggc actatcgccc gcggcgtcga tgcgatcagc 1020
   gatgctgagc agatccagaa gctcttgaac tccgagaagg acgagttcga gctgaatatg 1080
   tgcaccgacg tcgaccgcaa cgacaaggcg cgggtctgcg tgccgggcac gatcaaagtt 1140
   ctcgcgcgcc gccagatcga gacctattcg aagctgttcc acaccgtcga tcacgtcgag 1200
   ggcatgctgc gaccgggttt cgacgcgctc gacgccttcc tcacccacgc ctgggcggtc 1260
   accgtcaccg gcgcgccgaa gctgtgggcg atgcagttcg tcgaggatca cgagcgtagc 1320
   ccgcggcgct ggtatgccgg cgcgttcggc gtggtcggct tcgatggctc gatcaacacc 1380
   ggcctcacca tccgcaccat ccggatgaag gacggcctcg ccgaagttcg cgtcggcgcc 1440
   acctgcctgt tcgacagcaa tccggtcgcc gaggacaagg aatgccaggt caaggccgcg 1500
   gcactgttcc aggcgctgcg cggcgatccc gccaagccgc tgtcggcggt ggcgccggac 1560
   gccactggct cgggcaagaa ggtgctgctg gtcgaccacg acgacagctt cgtgcacatg 1620
   ctggcggact atttcaggca ggtcggcgcc caggtcaccg tggtgcgcta cgttcacggc 1680
   ctgaagatgc tggccgaaaa cagctatgat cttctggtgc tgtcgcccgg tcccggccgg 1740
   ccggaggact tcaagatcaa ggatacgatc gacgccgcgc tcgccaagaa gctgccgatc 1800
   ttcggcgtct gcctcggcgt ccaggcgatg ggcgaatatt ttggcggtac gctcggccag 1860
   ctcgcgcagc cggctcacgg ccgcccgtcg cggattcagg tgcgcggcgg cgcgctgatg 1920
   cgcggtctcc cgaacgaggt caccatcggc cgctaccact cgctctatgt cgacatgcgc 1980
   gacatgccga aggagctgac cgtcaccgcc tccaccgatg acggcatcgc gatggcgatc 2040
   gagcacaaga ccctgccggt cggcggcgtg cagttccacc ccgagtcgct gatgtcgctc 2100
   ggcggcgagg tcgggctgcg gatcgtcgaa aacgccttcc ggctcggcca ggcggcctaa 2160
<210> 84
   <211> 2190
   <212> DNA
   <213> Artificial Sequence
<220>
   <223> An A. tumefaciens mutant.
<400> 84
   atggtaacga tcattcagga tgacggagcg gagacctacg agacgaaagg cggcatccag     60
   gtcagccgaa agcgccggcc caccgattat gccaacgcca tcgataatta catcgaaaag    120
   cttgattccc atcgcggcgc gtttttttcg tccaactatg aatatccggg ccgttacacc    180
   cgctgggata cggccatcgt cgatccgccg ctcggcattt cctgttttgg ccgcaagatg    240
   tggatcgaag cctataatgg ccgcggcgaa gtgctgctcg atttcattac ggaaaagctg    300
   aaggcgacac ccgatctcac cctcggcgct tcctcgaccc gccggctcga tcttaccgtc    360
   aacgaaccgg accgtgtctt caccgaagaa gaacgctcga aaatcccgac ggtcttcacc    420
   gctctcagag ccatcgtcga cctcttctat tcgagcgcgg attcggccat cggcctgttc    480
   ggtgccttcg gttacgatct cgccttccag ttcgacgcga tcaagctttc gctggcgcgt    540
   ccggaagacc agcgtgacat ggtgctgttt ctgcccgatg aaatcctcgt cgttgatcac    600
   tattccgcca aggcctggat cgaccgttac gatttcgaga aggacggcat gacgacggac    660
   ggcaaatcct ccgacattac ccccgatccc ttcaagacca ccgataccat cccgcccaag    720
   ggcgatcacc gtcccggcga atattccgag cttgtggtga aggccaagga aagcttccgc    780
   cgcggcgacc tgttcgaggt cgttcccggc cagaaattca tggagcgttg cgaaagcaat    840
   ccgtcggcga tttcccgccg cctgaaggcg atcaacccgt cgccctattc cttcttcatc    900
   aatctcggcg atcaggaata tctggtcggc gcctcgccgg aaatgttcgt gcgcgtctcc    960
   ggccgtcgca tcgagacctg cccgatatca ggcaccatca agcgcggcga cgatccgatt   1020
   gccgacagcg agcagatttt gaaactgctc aactcgaaaa aggacgaatc cgaactgacc   1080
   atgtgctcgg acgtggaccg caacgacaag agccgcgtct gcgagccggg ttcggtgaag   1140
   gtcattggcc gccgccagat cgagatgtat tcacgcctca tccacaccgt cgatcacatc   1200
   gaaggccgcc tgcgcgacga tatggacgcc tttgacggtt tcctcagcca cgcctgggcc   1260
   gtcaccgtca ccggtgcacc aaagctgtgg gccatgcgct tcatcgaagg tcatgaaaag   1320
   agcccgcgcg cctggtatgg cggtgcgatc ggcatggtcg gcttcaacgg cgacatgaat   1380
   accggcctga cgctgcgcac catccggatc aaggacggta ttgccgaagt gcgcgccggc   1440
   gcgaccctgc tcaatgattc caacccgcag gaagaagaag ccgaaaccga actgaaggcc   1500
   tccgccatga tatcagccat tcgtgacgca aaaggcacca actctgccgc caccaagcgt   1560
   gatgccgcca aagtcggcac cggcgtcaag atcctgctcg tcgaccacga agacagcttc   1620
   gtgcacacgc tggcgaatta tttccgccag acgggcgcga cggtctcgac cgtcagatca   1680
   ccggtcgcag ccgacgtgtt cgatcgcttc cagccggacc tcgttgtcct gtcgcccgga   1740
   cccggcagcc cgacggattt cgactgcaag gcaacgatca aggccgcccg cgcccgcgat   1800
   ctgccgatct tcggcgtttg cctcggtctg caggcattgg cagaagccta tggcggcgag   1860
   ctgcgccagc ttgctgtgcc catgcacggc aagccttcgc gcatccgcgt gctggaaccc   1920
   ggcctcgtct tctccggtct cggcaaggaa gtcacggtcg gtcgttacca ttcgatcttc   1980
   gccgatcccg ccaccctgcc gcgtgatttc atcatcaccg cagaaagcga ggacggcacg   2040
   atcatgggca tcgaacacgc caaggaaccg gtggccgccg ttcagttcca cccggaatcg   2100
   atcatgacgc tcggacagga cgcgggcatg cggatgatcg agaatgtcgt ggtgcatctg   2160
   acccgcaagg cgaagaccaa ggccgcgtga                                    2190
<210> 85
   <211> 2190
   <212> DNA
   <213> Artificial Sequence
<220>
   <223> An A. tumefaciens mutant.
<400> 85
   atggtaacga tcattcagga tgacggagcg gagacctacg agacgaaagg cggcatccag 60
   gtcagccgaa agcgccggcc caccgattat gccaacgcca tcgataatta catcgaaaag 120
   cttgattccc atcgcggcgc gtatttttcg tccaactatg aatatccggg ccgttacacc 180
   cgctgggata cggccatcgt cgatccgccg ctcggcattt cctgttttgg ccgcaagatg 240
   tggatcgaag cctataatgg ccgcggcgaa gtgctgctcg atttcattac ggaaaagctg 300
   aaggcgacac ccgatctcac cctcggcgct tcctcgaccc gccggctcga tcttaccgtc 360
   aacgaaccgg accgtgtctt caccgaagaa gaacgctcga aaatcccgac ggtcttcacc 420
   gctctcagag ccatcgtcga cctcttctat tcgagcgcgg attcggccat cggcctgttc 480
   ggtgccttcg gttacgatct cgccttccag ttcgacgcga tcaagctttc gctggcgcgt 540
   ccggaagacc agcgtgacat ggtgctgttt ctgcccgatg aaatcctcgt cgttgatcac 600
   tattccgcca aggcctggat cgaccgttac gatttcgaga aggacggcat gacgacggac 660
   ggcaaatcct ccgacattac ccccgatccc ttcaagacca ccgataccat cccgcccaag 720
   ggcgatcacc gtcccggcga atattccgag cttgtggtga aggccaagga aagcttccgc 780
   cgcggcgacc tgttcgaggt cgttcccggc cagaaattca tggagcgttg cgaaagcaat 840
   ccgtcggcga tttcccgccg cctgaaggcg atcaacccgt cgccctattc cttcttcatc 900
   aatctcggcg atcaggaata tctggtcggc gcctcgccgg aaatgttcgt gcgcgtctcc 960
   ggccgtcgca tcgagacctg cccgatatca ggcaccatca agcgcggcga cgatccgatt 1020
   gccgacagcg agcagatttt gaaactgctc aactcgaaaa aggacgaatc cgaactgacc 1080
   atgtgctcgg acgtggaccg caacgacaag agccgcgtct gcgagccggg ttcggtgaag 1140
   gtcattggcc gccgccagat cgagatgtat tcacgcctca tccacaccgt cgatcacatc 1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 86
   <211> 2190
   <212> DNA
   <213> Artificial Sequence
<220>
   <223> An A. tumefaciens mutant.
<400> 86
   atggtaacga tcattcagga tgacggagcg gagacctacg agacgaaagg cggcatccag 60
   gtcagccgaa agcgccggcc caccgattat gccaacgcca tcgataatta catcgaaaag 120
   cttgattccc atcgcggcgc ggttttttcg ttcaactatg aatatccggg ccgttacacc 180
   cgctgggata cggccatcgt cgatccgccg ctcggcattt cctgttttgg ccgcaagatg 240
   tggatcgaag cctataatgg ccgcggcgaa gtgctgctcg atttcattac ggaaaagctg 300
   aaggcgacac ccgatctcac cctcggcgct tcctcgaccc gccggctcga tcttaccgtc 360
   aacgaaccgg accgtgtctt caccgaagaa gaacgctcga aaatcccgac ggtcttcacc 420
   gctctcagag ccatcgtcga cctcttctat tcgagcgcgg attcggccat cggcctgttc 480
   ggtgccttcg gttacgatct cgccttccag ttcgacgcga tcaagctttc gctggcgcgt 540
   ccggaagacc agcgtgacat ggtgctgttt ctgcccgatg aaatcctcgt cgttgatcac 600
   tattccgcca aggcctggat cgaccgttac gatttcgaga aggacggcat gacgacggac 660
   ggcaaatcct ccgacattac ccccgatccc ttcaagacca ccgataccat cccgcccaag 720
   ggcgatcacc gtcccggcga atattccgag cttgtggtga aggccaagga aagcttccgc 780
   cgcggcgacc tgttcgaggt cgttcccggc cagaaattca tggagcgttg cgaaagcaat 840
   ccgtcggcga tttcccgccg cctgaaggcg atcaacccgt cgccctattc cttcttcatc 900
   aatctcggcg atcaggaata tctggtcggc gcctcgccgg aaatgttcgt gcgcgtctcc 960
   ggccgtcgca tcgagacctg cccgatatca ggcaccatca agcgcggcga cgatccgatt 1020
   gccgacagcg agcagatttt gaaactgctc aactcgaaaa aggacgaatc cgaactgacc 1080
   atgtgctcgg acgtggaccg caacgacaag agccgcgtct gcgagccggg ttcggtgaag 1140
   gtcattggcc gccgccagat cgagatgtat tcacgcctca tccacaccgt cgatcacatc 1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 87
   <211> 2190
   <212> DNA
   <213> Artificial sequence
<220>
   <223> An A. tumefaciens mutant.
<400> 87
   atggtaacga tcattcagga tgacggagcg gagacctacg agacgaaagg cggcatccag 60
   gtcagccgaa agcgccggcc caccgattat gccaacgcca tcgataatta catcgaaaag 120
   cttgattccc atcgcggcgc ggttttttcg tgcaactatg aatatccggg ccgttacacc 180
   cgctgggata cggccatcgt cgatccgccg ctcggcattt cctgttttgg ccgcaagatg 240
   tggatcgaag cctataatgg ccgcggcgaa gtgctgctcg atttcattac ggaaaagctg 300
   aaggcgacac ccgatctcac cctcggcgct tcctcgaccc gccggctcga tcttaccgtc 360
   aacgaaccgg accgtgtctt caccgaagaa gaacgctcga aaatcccgac ggtcttcacc 420
   gctctcagag ccatcgtcga cctcttctat tcgagcgcgg attcggccat cggcctgttc 480
   ggtgccttcg gttacgatct cgccttccag ttcgacgcga tcaagctttc gctggcgcgt 540
   ccggaagacc agcgtgacat ggtgctgttt ctgcccgatg aaatcctcgt cgttgatcac 600
   tattccgcca aggcctggat cgaccgttac gatttcgaga aggacggcat gacgacggac 660
   ggcaaatcct ccgacattac ccccgatccc ttcaagacca ccgataccat cccgcccaag 720
   ggcgatcacc gtcccggcga atattccgag cttgtggtga aggccaagga aagcttccgc 780
   cgcggcgacc tgttcgaggt cgttcccggc cagaaattca tggagcgttg cgaaagcaat 840
   ccgtcggcga tttcccgccg cctgaaggcg atcaacccgt cgccctattc cttcttcatc 900
   aatctcggcg atcaggaata tctggtcggc gcctcgccgg aaatgttcgt gcgcgtctcc 960
   ggccgtcgca tcgagacctg cccgatatca ggcaccatca agcgcggcga cgatccgatt 1020
   gccgacagcg agcagatttt gaaactgctc aactcgaaaa aggacgaatc cgaactgacc 1080
   atgtgctcgg acgtggaccg caacgacaag agccgcgtct gcgagccggg ttcggtgaag 1140
   gtcattggcc gccgccagat cgagatgtat tcacgcctca tccacaccgt cgatcacatc 1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 88
   <211> 2190
   <212> DNA
   <213> Artificial sequence
<220>
   <223> An A. tumefaciens mutant.
<400> 88
   atggtaacga tcattcagga tgacggagcg gagacctacg agacgaaagg cggcatccag      60
   gtcagccgaa agcgccggcc caccgattat gccaacgcca tcgataatta catcgaaaag     120
   cttgattccc atcgcggcgc ggttttttcg  tccttctatg aatatccggg ccgttacacc     180
   cgctgggata cggccatcgt cgatccgccg ctcggcattt cctgttttgg ccgcaagatg     240
   tggatcgaag cctataatgg ccgcggcgaa gtgctgctcg atttcattac ggaaaagctg     300
   aaggcgacac ccgatctcac cctcggcgct tcctcgaccc gccggctcga tcttaccgtc     360
   aacgaaccgg accgtgtctt caccgaagaa gaacgctcga aaatcccgac ggtcttcacc     420
   gctctcagag ccatcgtcga cctcttctat tcgagcgcgg attcggccat cggcctgttc     480
   ggtgccttcg gttacgatct cgccttccag ttcgacgcga tcaagctttc gctggcgcgt     540
   ccggaagacc agcgtgacat ggtgctgttt ctgcccgatg aaatcctcgt cgttgatcac     600
   tattccgcca aggcctggat cgaccgttac gatttcgaga aggacggcat gacgacggac     660
   ggcaaatcct ccgacattac ccccgatccc ttcaagacca ccgataccat cccgcccaag     720
   ggcgatcacc gtcccggcga atattccgag cttgtggtga aggccaagga aagcttccgc     780
   cgcggcgacc tgttcgaggt cgttcccggc cagaaattca tggagcgttg cgaaagcaat     840
   ccgtcggcga tttcccgccg cctgaaggcg atcaacccgt cgccctattc cttcttcatc     900
   aatctcggcg atcaggaata tctggtcggc gcctcgccgg aaatgttcgt gcgcgtctcc     960
   ggccgtcgca tcgagacctg cccgatatca ggcaccatca agcgcggcga cgatccgatt    1020
   gccgacagcg agcagatttt gaaactgctc aactcgaaaa aggacgaatc cgaactgacc    1080
   atgtgctcgg acgtggaccg caacgacaag agccgcgtct gcgagccggg ttcggtgaag    1140
   gtcattggcc gccgccagat cgagatgtat tcacgcctca tccacaccgt cgatcacatc    1200
   gaaggccgcc tgcgcgacga tatggacgcc tttgacggtt tcctcagcca cgcctgggcc    1260
   gtcaccgtca ccggtgcacc aaagctgtgg gccatgcgct tcatcgaagg tcatgaaaag    1320
   agcccgcgcg cctggtatgg cggtgcgatc ggcatggtcg gcttcaacgg cgacatgaat    1380
   accggcctga cgctgcgcac catccggatc aaggacggta ttgccgaagt gcgcgccggc    1440
   gcgaccctgc tcaatgattc caacccgcag gaagaagaag ccgaaaccga actgaaggcc    1500
   tccgccatga tatcagccat tcgtgacgca aaaggcacca actctgccgc caccaagcgt    1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 89
   <211> 2190
   <212> DNA
   <213> Artificial sequence
<220>
   <223> An A. tumefaciens mutant.
<400> 89
   atggtaacga tcattcagga tgacggagcg gagacctacg agacgaaagg cggcatccag     60
   gtcagccgaa agcgccggcc caccgattat gccaacgcca tcgataatta catcgaaaag    120
   cttgattccc atcgcggcgc ggttttttcg tccaactatg aatatccggg ccgttacacc    180
   cgctgggata cggccatcgt cgatccgccg ctcggcattt cctgttttgg ccgcaagatg    240
   tggatcgaag cctataatgg ccgcggcgaa gtgctgctcg atttcattac ggaaaagctg    300
   aaggcgacac ccgatctcac cctcggcgct tcctcgaccc gccggctcga tcttaccgtc    360
   aacgaaccgg accgtgtctt caccgaagaa gaacgctcga aaatcccgac ggtcttcacc    420
   gctctcagag ccatcgtcga cctcttctat tcgagcgcgg attcggccat cggcctgttc    480
   ggtgccttcg gttacgatct cgccttccag ttcgacgcga tcaagctttc gctggcgcgt    540
   ccggaagacc agcgtgacat ggtgctgttt ctgcccgatg aaatcctcgt cgttgatcac    600
   tattccgcca aggcctggat cgaccgttac gatttcgaga aggacggcat gacgacggac    660
   ggcaaatcct ccgacattac ccccgatccc ttcaagacca ccgataccat cccgcccaag    720
   ggcgatcacc gtcccggcga atattccgag cttgtggtga aggccaagga aagcttccgc    780
   cgcggcgacc tgttcgaggt cgttcccggc cagaaattca tggagcgttg cgaaagcaat    840
   ccgtcggcga tttcccgccg cctgaaggcg atcaacgcgt cgccctattc cttcttcatc    900
   aatctcggcg atcaggaata tctggtcggc gcctcgccgg aaatgttcgt gcgcgtctcc    960
   ggccgtcgca tcgagacctg cccgatatca ggcaccatca agcgcggcga cgatccgatt   1020
   gccgacagcg agcagatttt gaaactgctc aactcgaaaa aggacgaatc cgaactgacc   1080
   atgtgctcgg acgtggaccg caacgacaag agccgcgtct gcgagccggg ttcggtgaag 1140
   gtcattggcc gccgccagat cgagatgtat tcacgcctca tccacaccgt cgatcacatc 1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 90
   <211> 2190
   <212> DNA
   <213> Artificial Sequence
<220>
   <223> An A. tumefaciens mutant.
<400> 90
   atggtaacga tcattcagga tgacggagcg gagacctacg agacgaaagg cggcatccag      60
   gtcagccgaa agcgccggcc caccgattat gccaacgcca tcgataatta catcgaaaag     120
   cttgattccc atcgcggcgc ggttttttcg tccaactatg aatatccggg ccgttacacc      180
   cgctgggata cggccatcgt cgatccgccg ctcggcattt cctgttttgg ccgcaagatg     240
   tggatcgaag cctataatgg ccgcggcgaa gtgctgctcg atttcattac ggaaaagctg     300
   aaggcgacac ccgatctcac cctcggcgct tcctcgaccc gccggctcga tcttaccgtc     360
   aacgaaccgg accgtgtctt caccgaagaa gaacgctcga aaatcccgac ggtcttcacc     420
   gctctcagag ccatcgtcga cctcttctat tcgagcgcgg attcggccat cggcctgttc     480
   ggtgccttcg gttacgatct cgccttccag ttcgacgcga tcaagctttc gctggcgcgt     540
   ccggaagacc agcgtgacat ggtgctgttt ctgcccgatg aaatcctcgt cgttgatcac     600
   tattccgcca aggcctggat cgaccgttac gatttcgaga aggacggcat gacgacggac 660
   ggcaaatcct ccgacattac ccccgatccc ttcaagacca ccgataccat cccgcccaag 720
   ggcgatcacc gtcccggcga atattccgag cttgtggtga aggccaagga aagcttccgc 780
   cgcggcgacc tgttcgaggt cgttcccggc cagaaattca tggagcgttg cgaaagcaat 840
   ccgtcggcga tttcccgccg cctgaaggcg atcaacgggt cgccctattc cttcttcatc 900
   aatctcggcg atcaggaata tctggtcggc gcctcgccgg aaatgttcgt gcgcgtctcc 960
   ggccgtcgca tcgagacctg cccgatatca ggcaccatca agcgcggcga cgatccgatt 1020
   gccgacagcg agcagatttt gaaactgctc aactcgaaaa aggacgaatc cgaactgacc 1080
   atgtgctcgg acgtggaccg caacgacaag agccgcgtct gcgagccggg ttcggtgaag 1140
   gtcattggcc gccgccagat cgagatgtat tcacgcctca tccacaccgt cgatcacatc 1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 91
   <211> 2190
   <212> DNA
   <213> Artificial Sequence
<220>
   <223> An A. tumefaciens mutant.
<400> 91 acccgcaagg cgaagaccaa ggccgcgtga 2190
<210> 92
   <211> 2190
   <212> DNA
   <213> Artificial Sequence
<220>
   <223> An A. tumefaciens mutant.
<400> 92
   atggtaacga tcattcagga tgacggagcg gagacctacg agacgaaagg cggcatccag 60
   gtcagccgaa agcgccggcc caccgattat gccaacgcca tcgataatta catcgaaaag 120
   cttgattccc atcgcggcgc ggtttttaag tccaactatg aatatccggg ccgttacacc 180
   cgctgggata cggccatcgt cgatccgccg ctcggcattt cctgttttgg ccgcaagatg 240
   tggatcgaag cctataatgg ccgcggcgaa gtgctgctcg atttcattac ggaaaagctg 300
   aaggcgacac ccgatctcac cctcggcgct tcctcgaccc gccggctcga tcttaccgtc 360
   aacgaaccgg accgtgtctt caccgaagaa gaacgctcga aaatcccgac ggtcttcacc 420
   gctctcagag ccatcgtcga cctcttctat tcgagcgcgg attcggccat cggcctgttc 480
   ggtgccttcg gttacgatct cgccttccag ttcgacgcga tcaagctttc gctggcgcgt 540
   ccggaagacc agcgtgacat ggtgctgttt ctgcccgatg aaatcctcgt cgttgatcac 600
   tattccgcca aggcctggat cgaccgttac gatttcgaga aggacggcat gacgacggac 660
   ggcaaatcct ccgacattac ccccgatccc ttcaagacca ccgataccat cccgcccaag 720
   ggcgatcacc gtcccggcga atattccgag cttgtggtga aggccaagga aagcttccgc 780
   cgcggcgacc tgttcgaggt cgttcccggc cagaaattca tggagcgttg cgaaagcaat 840
   ccgtcggcga tttcccgccg cctgaaggcg atcaacccgt cgccctattc cttcttcatc 900
   aatctcggcg atcaggaata tctggtcggc gcctcgccgg aaatgttcgt gcgcgtctcc 960
   ggccgtcgca tcgagacctg cccgatatca ggcaccatca agcgcggcga cgatccgatt 1020
   gccgacagcg agcagatttt gaaactgctc aactcgaaaa aggacgaatc cgaactgacc 1080
   atgtgctcgg acgtggaccg caacgacaag agccgcgtct gcgagccggg ttcggtgaag 1140
   gtcattggcc gccgccagat cgagatgtat tcacgcctca tccacaccgt cgatcacatc 1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga   1740
   cccggcagcc cgacggattt cgactgcaag gcaacgatca aggccgcccg cgcccgcgat   1800
   ctgccgatct tcggcgtttg cctcggtctg caggcattgg cagaagccta tggcggcgag   1860
   ctgcgccagc ttgctgtgcc catgcacggc aagccttcgc gcatccgcgt gctggaaccc   1920
   ggcctcgtct tctccggtct cggcaaggaa gtcacggtcg gtcgttacca ttcgatcttc   1980
   gccgatcccg ccaccctgcc gcgtgatttc atcatcaccg cagaaagcga ggacggcacg   2040
   atcatgggca tcgaacacgc caaggaaccg gtggccgccg ttcagttcca cccggaatcg   2100
   atcatgacgc tcggacagga cgcgggcatg cggatgatcg agaatgtcgt ggtgcatctg   2160
   acccgcaagg cgaagaccaa ggccgcgtga                                    2190
<210> 93
   <211> 2190
   <212> DNA
   <213> Artificial sequence
<220>
   <223> An A. tumefaciens mutant.
<400> 93
   atggtaacga tcattcagga tgacggagcg gagacctacg agacgaaagg cggcatccag      60
   gtcagccgaa agcgccggcc caccgattat gccaacgcca tcgataatta catcgaaaag     120
   cttgattccc atcgcggcgc ggttttttcg tccaactatg aatatccggg ccgttacacc      180
   cgctgggata cggccatcgt cgatccgccg ctcggcattt cctgttttgg ccgcaagatg     240
   tggatcgaag cctataatgg ccgcggcgaa gtgctgctcg atttcattac ggaaaagctg     300
   aaggcgacac ccgatctcac cctcggcgct tcctcgaccc gccggctcga tcttaccgtc     360
   aacgaaccgg accgtgtctt caccgaagaa gaacgctcga aaatcccgac ggtcttcacc     420
   gctctcagag ccatcgtcga cctcttctat tcgagcgcgg attcggccat cggcctgttc     480
   ggtgccttcg gttacgatct cgccttccag ttcgacgcga tcaagctttc gctggcgcgt     540
   ccggaagacc agcgtgacat ggtgctgttt ctgcccgatg aaatcctcgt cgttgatcac     600
   tattccgcca aggcctggat cgaccgttac gatttcgaga aggacggcat gacgacggac     660
   ggcaaatcct ccgacattac ccccgatccc ttcaagacca ccgataccat cccgcccaag     720
   ggcgatcacc gtcccggcga atattccgag cttgtggtga aggccaagga aagcttccgc     780
   cgcggcgacc tgttcgaggt cgttcccggc cagaaattca tggagcgttg cgaaagcaat     840
   ccgtcggcga tttcccgccg cctgaaggcg atcaacccgt cgccctattc cgccttcatc     900
   aatctcggcg atcaggaata tctggtcggc gcctcgccgg aaatgttcgt gcgcgtctcc     960
   ggccgtcgca tcgagacctg cccgatatca ggcaccatca agcgcggcga cgatccgatt    1020
   gccgacagcg agcagatttt gaaactgctc aactcgaaaa aggacgaatc cgaactgacc    1080
   atgtgctcgg acgtggaccg caacgacaag agccgcgtct gcgagccggg ttcggtgaag    1140
   gtcattggcc gccgccagat cgagatgtat tcacgcctca tccacaccgt cgatcacatc    1200
   gaaggccgcc tgcgcgacga tatggacgcc tttgacggtt tcctcagcca cgcctgggcc 1260
   gtcaccgtca ccggtgcacc aaagctgtgg gccatgcgct tcatcgaagg tcatgaaaag 1320
   agcccgcgcg cctggtatgg cggtgcgatc ggcatggtcg gcttcaacgg cgacatgaat 1380
   accggcctga cgctgcgcac catccggatc aaggacggta ttgccgaagt gcgcgccggc 1440
   gcgaccctgc tcaatgattc caacccgcag gaagaagaag ccgaaaccga actgaaggcc 1500
   tccgccatga tatcagccat tcgtgacgca aaaggcacca actctgccgc caccaagcgt 1560
   gatgccgcca aagtcggcac cggcgtcaag atcctgctcg tcgaccacga agacagcttc 1620
   gtgcacacgc tggcgaatta tttccgccag acgggcgcga cggtctcgac cgtcagatca 1680
   ccggtcgcag ccgacgtgtt cgatcgcttc cagccggacc tcgttgtcct gtcgcccgga 1740
   cccggcagcc cgacggattt cgactgcaag gcaacgatca aggccgcccg cgcccgcgat 1800
   ctgccgatct tcggcgtttg cctcggtctg caggcattgg cagaagccta tggcggcgag 1860
   ctgcgccagc ttgctgtgcc catgcacggc aagccttcgc gcatccgcgt gctggaaccc 1920
   ggcctcgtct tctccggtct cggcaaggaa gtcacggtcg gtcgttacca ttcgatcttc 1980
   gccgatcccg ccaccctgcc gcgtgatttc atcatcaccg cagaaagcga ggacggcacg 2040
   atcatgggca tcgaacacgc caaggaaccg gtggccgccg ttcagttcca cccggaatcg 2100
   atcatgacgc tcggacagga cgcgggcatg cggatgatcg agaatgtcgt ggtgcatctg 2160
   acccgcaagg cgaagaccaa ggccgcgtga 2190
<210> 94
   <211> 1821
   <212> DNA
   <213> Oryza sativa
<400> 94
   atggagtcca tcgccgccgc cacgttcacg ccctcgcgcc tcgccgcccg ccccgccact     60
   ccggcggcgg cggcggcccc ggttagagcg agggcggcgg tagcggcagg agggaggagg    120
   aggacgagta ggcgcggcgg cgtgaggtgc tccgcgggga agccagaggc aagcgcggtg    180
   atcaacggga gcgcggcggc gcgggcggcg gaggaggaca ggaggcgctt cttcgaggcg    240
   gcggagcgtg ggagcgggaa gggcaacctg gtgcccatgt gggagtgcat cgtctccgac    300
   cacctcaccc ccgtgctcgc ctaccgctgc ctcgtccccg aggacaacat ggagacgccc    360
   agcttcctct tcgagtccgt cgagcagggg cccgagggca ccaccaacgt cggtcgctat    420
   agcatggtgg gagcccaccc agtgatggag gtcgtggcaa aggagcacaa ggtcacaatc    480
   atggaccacg agaagggcaa ggtgacggag caggtcgtgg atgatcctat gcagatcccc    540
   aggagcatga tggaaggatg gcacccgcag cagatcgatc agctccccga ttccttcacc    600
   ggtggatggg tcgggttctt ttcctatgat acagtccgtt atgttgaaaa gaagaagctg    660
   cccttctccg gtgctcccca ggacgatagg aaccttcctg atgttcacct tgggctttat    720
   gatgatgttc tcgtcttcga caatgtcgag aagaaagtat atgtcatcca ttgggtaaat    780
   cttgatcggc atgcaaccac cgaggatgca ttccaagatg gcaagtcccg gctgaacctg    840
   ttgctatcta aagtgcacaa ttcaaatgta cccaagcttt ctccaggatt tgtaaagtta    900
   cacactcggc agtttggtac acctttgaac aaatcaacca tgacaagtga tgagtacaag    960
   aatgctgtta tgcaggctaa ggagcatatt atggctggtg atattttcca gattgtttta   1020
   agccagaggt ttgagaggca gacatacgcc aatccatttg aagtctatcg agctttacga   1080
   attgtgaacc caagtccata catggcatat gtacaggcaa gaggctgtgt cctggtagca   1140
   tctagtccag aaattcttac tcgtgtgagg aagggtaaaa ttattaaccg tccacttgct   1200
   gggactgttc gaaggggcaa gacagagaag gaagatgaaa tgcaagagca acaactacta   1260
   agtgatgaaa aacagtgtgc tgaacatatt atgcttgtag atttgggaag gaatgatgtt   1320
   ggaaaggtct ccaaacctgg atctgtgaag gtggagaaat taatgaacat tgaacgctac   1380
   tcccatgtca tgcacatcag ttccacggtg agtggagagt tggatgatca tctccaaagt   1440
   tgggatgccc tgcgagccgc gttgcctgtt ggaacagtta gtggagcacc aaaggtgaaa   1500
   gccatggagc tgatagacga gctagaggtc acaagacgag gaccatacag tggcggcctt   1560
   ggagggatat catttgacgg ggacatgctt atcgctcttg cactccgcac cattgtgttc   1620
   tcaacagcgc caagccacaa cacgatgtac tcatacaaag acaccgagag gcgccgggag   1680
   tgggtcgctc accttcaggc tggtgctggc attgtcgctg atagcagccc agacgacgag   1740
   caacgtgaat gcgagaacaa ggcagccgct ctggctcgag ccatcgatct tgctgaatca   1800
   gctttcgtag acaaggaata g                                             1821
<210> 95
   <211> 1498
   <212> DNA
   <213> Oryza sativa
<400> 95
   gaattcaaat tttttatata gagtatttct atacatgaat ttttctaact ttttgttttt     60
   taaaaaaaat ttgtgtggtg tactgtaata ggaagagaag aaggggagga ggaaggaggg    120
   agaagaggga ggagtatatg gggagggggg gatgaactga tcgcccagcg tgatagctgg    180
   cgatcgagca cccattagaa gggcccaata aaccctggat aattgtcatt gagtggcacc    240
   tttcattgag aagacgttat taggaattgt agaagtggat aattatgcta tctgttgtat    300
   tgagtgtcac tgtcaccgat aaagctttgc tggttaatgc attgtatttc tccatcaacg    360
   cttcatgata caatggtatt tggacgtgtt tataaaataa tatacgtata atgtgggtgg    420
   cctagcggcg gccggttaca catagcagcg atcggtccga tgctagtctt cattcattca    480
   ggtatgtatt caggtatcag tgtgtgggtg atagtttttt tttttcgttt ttctagttac    540
   gatatctcat atctcatagt tgtgatctta taaacttttt catgtttatc aatataaatt    600
   tcgtgttatc tagtcgttaa aagaaccgta taatgtggca aaaaaaatgt ataatgtgtc    660
   agagtttgca cgtgtttatc ttgctgcccc gaaacgatta attcagtgat ttggcaacaa    720
   caaaatgtcg tggcggataa gcatatccgt cccaaaagga aaaaaagaaa aggaaaaata    780
   atctttagaa ataaagccct tactttttcc aagaagcaga ggtaaccgta gctggtattc    840
   cgcggctaac tcaatccctt tctctggagt cttggagcgg cacggcggct gcgcacccga    900
   cctcgcccac cacctgctcg gcgaaacgcc cggctcggcc gcgacgtgtc ccaccgcacc    960
   gcgcgcgcac ccgcgcgccc cgagcccctc gccgcctccg cgcgggcgcc gcacctattt   1020
   aaatgcggcc ccgatcccgc attctctcaa ctgcactagt ccccaccaac ggctcggtcc   1080
   agtagagttt atcccccacc tatggccagc ctcgtgctct ccctgcgcat cgcccgttcc   1140
   acgccgccgc tggggctggg cggggggcga ttccgcggcc gacgaggggc cgtcgcctgc   1200
   cgcgccgcca cgttccagca gctcgacgcc gtcggtgagt ctccgtatca aatgtggggg   1260
   ggcatgtctt ggtttgcgga ttggtgggtt gatttgaatg tgtgttctcg tggccgcagc   1320
   ggtgagggag gaggagtcca agttcaaggc gggggcggcg gagggttgca acatcctgcc   1380
   gctcaagcga tgcatcttct ccgaccacct cacgccggtg ctcgcgtacc gctgcctcgt   1440
   cagggaggac gaccgcgagg cgcccagctt cctgtttgag tccgtcgagc agggatcc     1498
<210> 96
   <211> 2073
   <212> DNA
   <213> zea mays
<400> 96
   gaattccgcc aaatcgggct atagatcaaa cgctgcactg tagggagcgt gaagccagcg 60
   gcgaatggaa tccctagccg ccacctccgt gttcgcgccc tcccgcgtcg ccgtcccggc 120
   ggcgcgggcc ctggttaggg cggggacggt ggtaccaacc aggcggacga gcagccggag 180
   cggaaccagc ggggtgaaat gctctgctgc cgtgacgccg caggcgagcc cagtgattag 240
   caggagcgct gcggcggcga aggcggcgga ggaggacaag aggcggttct tcgaggcggc 300
   ggcgcggggg agcgggaagg ggaacctggt gcccatgtgg gagtgcatca aggggaacct 360
   ggtgcccatg tgggagtgca tcgtgtcgga ccatctcacc cccgtgctcg cctaccgctg 420
   cctcgtcccc gaggacaacg tcgacgcccc cagcttcctc ttcgagtccg tcgagcaggg 480
   gccccagggc accaccaacg tcggccgcta tagcatggtg ggagcccacc cagtgatgga 540
   gattgtggcc aaagaccaca aggttacgat catggaccac gagaagagcc aagtgacaga 600
   gcaggtagtg gacgacccga tgcagatccc gaggaccatg atggagggat ggcacccaca 660
   gcagatcgac gagctccctg aatccttctc cggtggatgg gttgggttct tttcctatga 720
   tacggttagg tatgttgaga agaagaagct accgttctcc agtgctcctc aggacgatag 780
   gaaccttcct gatgtgcact tgggactcta tgatgatgtt ctagtcttcg ataatgttga 840
   gaagaaagta tatgttatcc attgggtcaa tgtggaccgg catgcatctg ttgaggaagc 900
   ataccaagat ggcaggtccc gactaaacat gttgctatct aaagtgcaca attccaatgt 960
   ccccacactc tctcctggat ttgtgaagct gcacacacgc aagtttggta cacctttgaa 1020
   caagtcgacc atgacaagtg atgagtataa gaatgctgtt ctgcaggcta aggaacatat 1080
   tatggctggg gatatcttcc agattgtttt aagccagagg ttcgagagac gaacatatgc 1140
   caacccattt gaggtttatc gagcattacg gattgtgaat cctagcccat acatggcgta 1200
   tgtacaggca agaggctgtg tattggttgc gtctagtcct gaaattctta cacgagtcag 1260
   taaggggaag attattaatc gaccacttgc tggaactgtt cgaaggggca agacagagaa 1320
   ggaagatcaa atgcaagagc agcaactgtt aagtgatgaa aaacagtgtg ccgagcacat 1380
   aatgcttgtg gacttgggaa ggaatgatgt tggcaaggta tccaaaccag gaggatcagt 1440
   gaaggtggag aagttgatta ttgagagata ctcccatgtt atgcacataa gctcaacggt 1500
   tagtggacag ttggatgatc atctccagag ttgggatgcc ttgagagctg ccttgcccgt 1560
   tggaacagtc agtggtgcac caaaggtgaa ggccatggag ttgattgata agttggaagt 1620
   tacgaggcga ggaccatata gtggtggtct aggaggaata tcgtttgatg gtgacatgca 1680
   aattgcactt tctctccgca ccatcgtatt ctcaacagcg ccgagccaca acacgatgta 1740
   ctcatacaaa gacgcagata ggcgtcggga gtgggtcgct catcttcagg ctggtgcagg 1800
   cattgttgcc gacagtagcc cagatgacga acaacgtgaa tgcgagaata aggctgctgc 1860
   actagctcgg gccatcgatc ttgcagagtc agcttttgtg aacaaagaat agtgtgctat 1920
   ggttatcgtt tagttcttgt tcatgtttct tttacccact ttccgttaaa aaaagatgtc 1980
   attagtgggt ggagaaaagc aataagactg ttctctagag aaccgaagaa atatggaaat 2040
   tgaggttatg gccggaattc ctgcagcccg ggg 2073
<210> 97
   <211> 504
   <212> DNA
   <213> Triticum aestivum
<400> 97
   cccaaacagt ggtggcttag gagggatatc atttgatggt gacatgctta tcgctcttgc 60
   tctccgcacc attgtgtttt caacagctcc aagccccaat aggatgtact catacaaaag 120
   ctcagatagg ccccgagagt gggttgctca tcttcaggct ggtgcgggca ttgttgctga 180
   tagtatccca gacgatgagc aaaaagaatt tgagaataag gcggctgccc tagctcgggc 240
   aattgatctt gcagagtcgg cttttttaga caaagaatag agtgtctatt aaattatttt 300
   ttttagttgt tcatcatttt tcacccagtt cattttggaa agttgttcat cgttttttca 360
   ccgagttcat attggggaaa aaaagcaata ccgttttgtt gtcctttgaa atgaataaat 420
   ttgagctata ataagatgta ttttgctcat cgggcaaaaa aaaaaaaaaa aatataaaaa 480
   aaaaaaaaaa aaaaaaaaaa aata 504
<210> 98
   <211> 2161
   <212> DNA
   <213> Nicotiana tabacum
<400> 98 a 2161
<210> 99
   <211> 606
   <212> PRT
   <213> Oryza sativa
<400> 99
   Met Glu Ser Ile Ala Ala Ala Thr Phe Thr Pro Ser Arg Leu Ala Ala
   1 5 10 15
   Arg Pro Ala Thr Pro Ala Ala Ala Ala Ala Pro Val Arg Ala Arg Ala
   20 25 30
   Ala Val Ala Ala Gly Gly Arg Arg Arg Thr Ser Arg Arg Gly Gly Val
   35 40 45
   Arg Cys Ser Ala Gly Lys Pro Glu Ala Ser Ala Val Ile Asn Gly Ser
   50 55 60
   Ala Ala Ala Arg Ala Ala Glu Glu Asp Arg Arg Arg Phe Phe Glu Ala
   65 70 75 80
   Ala Glu Arg Gly Ser Gly Lys Gly Asn Leu Val Pro Met Trp Glu Cys
   85 90 95
   Ile Val Ser Asp His Leu Thr Pro Val Leu Ala Tyr Arg Cys Leu Val
   100 105 110
   Pro Glu Asp Asn Met Glu Thr Pro Ser Phe Leu Phe Glu Ser Val Glu
   115 120 125
   Gln Gly Pro Glu Gly Thr Thr Asn Val Gly Arg Tyr Ser Met Val Gly
   130 135 140
   Ala His Pro Val Met Glu Val Val Ala Lys Glu His Lys Val Thr Ile
   145 150 155 160
   Met Asp His Glu Lys Gly Lys Val Thr Glu Gln Val Val Asp Asp Pro
   165 170 175
   Met Gln Ile Pro Arg Ser Met Met Glu Gly Trp His Pro Gln Gln Ile
   180 185 190
   Asp Gln Leu Pro Asp Ser Phe Thr Gly Gly Trp Val Gly Phe Phe Ser
   195 200 205
   Tyr Asp Thr Val Arg Tyr Val Glu Lys Lys Lys Leu Pro Phe Ser Gly
   210 215 220
   Ala Pro Gln Asp Asp Arg Asn Leu Pro Asp Val His Leu Gly Leu Tyr
   225 230 235 240
   Asp Asp Val Leu Val Phe Asp Asn Val Glu Lys Lys Val Tyr Val Ile
   245 250 255
   His Trp Val Asn Leu Asp Arg His Ala Thr Thr Glu Asp Ala Phe Gln
   260 265 270
   Asp Gly Lys Ser Arg Leu Asn Leu Leu Leu Ser Lys Val His Asn Ser
   275 280 285
   Asn Val Pro Lys Leu Ser Pro Gly Phe Val Lys Leu His Thr Arg Gln
   290 295 300
   Phe Gly Thr Pro Leu Asn Lys Ser Thr Met Thr Ser Asp Glu Tyr Lys
   305 310 315 320
   Asn Ala Val Met Gln Ala Lys Glu His Ile Met Ala Gly Asp Ile Phe
   325 330 335
   Gln Ile Val Leu Ser Gln Arg Phe Glu Arg Gln Thr Tyr Ala Asn Pro
   340 345 350
   Phe Glu Val Tyr Arg Ala Leu Arg Ile Val Asn Pro Ser Pro Tyr Met
   355 360 365
   Ala Tyr Val Gln Ala Arg Gly Cys Val Leu Val Ala Ser Ser Pro Glu
   370 375 380
   Ile Leu Thr Arg Val Arg Lys Gly Lys Ile Ile Asn Arg Pro Leu Ala
   385 390 395 400
   Gly Thr Val Arg Arg Gly Lys Thr Glu Lys Glu Asp Glu Met Gln Glu
   405 410 415
   Gln Gln Leu Leu Ser Asp Glu Lys Gln Cys Ala Glu His Ile Met Leu
   420 425 430
   Val Asp Leu Gly Arg Asn Asp Val Gly Lys Val Ser Lys Pro Gly Ser
   435 440 445
   Val Lys Val Glu Lys Leu Met Asn Ile Glu Arg Tyr Ser His Val Met
   450 455 460
   His Ile Ser Ser Thr Val Ser Gly Glu Leu Asp Asp His Leu Gln Ser
   465 470 475 480
   Trp Asp Ala Leu Arg Ala Ala Leu Pro Val Gly Thr Val Ser Gly Ala
   485 490 495
   Pro Lys Val Lys Ala Met Glu Leu Ile Asp Glu Leu Glu Val Thr Arg
   500 505 510
   Arg Gly Pro Tyr Ser Gly Gly Leu Gly Gly Ile Ser Phe Asp Gly Asp
   515 520 525
   Met Leu Ile Ala Leu Ala Leu Arg Thr Ile Val Phe Ser Thr Ala Pro
   530 535 540
   Ser His Asn Thr Met Tyr Ser Tyr Lys Asp Thr Glu Arg Arg Arg Glu
   545 550 555 560
   Trp Val Ala His Leu Gln Ala Gly Ala Gly Ile Val Ala Asp Ser Ser
   565 570 575
   Pro Asp Asp Glu Gln Arg Glu Cys Glu Asn Lys Ala Ala Ala Leu Ala
   580 585 590
   Arg Ala Ile Asp Leu Ala Glu Ser Ala Phe Val Asp Lys Glu
   595 600 605
<210> 100
   <211> 67
   <212> PRT
   <213> oryza sativa
<400> 100
   Met Cys Val Leu Val Ala Ala Ala Val Arg Glu Glu Glu Ser Lys Phe
   1 5 10 15
   Lys Ala Gly Ala Ala Glu Gly Cys Asn Ile Leu Pro Leu Lys Arg Cys
   20 25 30
   Ile Phe Ser Asp His Leu Thr Pro Val Leu Ala Tyr Arg Cys Leu Val
   35 40 45
   Arg Glu Asp Asp Arg Glu Ala Pro Ser Phe Leu Phe Glu Ser Val Glu
   50 55 60
   Gln Gly Ser
   65
<210> 101
   <211> 525
   <212> PRT
   <213> zea mays
<400> 101
   Met Trp Glu Cys Ile Lys Gly Asn Leu Val Pro Met Trp Glu Cys Ile
   1               5                   10                  15
   Val Ser Asp His Leu Thr Pro Val Leu Ala Tyr Arg Cys Leu Val Pro
               20                  25                  30
   Glu Asp Asn Val Asp Ala Pro Ser Phe Leu Phe Glu Ser Val Glu Gln
           35                  40                  45
   Gly Pro Gln Gly Thr Thr Asn Val Gly Arg Tyr Ser Met Val Gly Ala
       50                  55                  60
   His Pro Val Met Glu Ile Val Ala Lys Asp His Lys Val Thr Ile Met
   65                  70                  75                  80
   Asp His Glu Lys Ser Gln Val Thr Glu Gln Val Val Asp Asp Pro Met
                   85                  90                  95
   Gln Ile Pro Arg Thr Met Met Glu Gly Trp His Pro Gln Gln Ile Asp
               100                 105                 110
   Glu Leu Pro Glu Ser Phe Ser Gly Gly Trp Val Gly Phe Phe Ser Tyr
           115                 120                 125
   Asp Thr Val Arg Tyr Val Glu Lys Lys Lys Leu Pro Phe Ser Ser Ala
       130                 135                 140
   Pro Gln Asp Asp Arg Asn Leu Pro Asp Val His Leu Gly Leu Tyr Asp
   145                 150                 155                 160
   Asp Val Leu Val Phe Asp Asn Val Glu Lys Lys Val Tyr Val Ile His
                   165                 170                 175
   Trp Val Asn Val Asp Arg His Ala Ser Val Glu Glu Ala Tyr Gln Asp
               180                 185                 190
   Gly Arg Ser Arg Leu Asn Met Leu Leu Ser Lys Val His Asn Ser Asn
           195                 200                 205
   Val Pro Thr Leu Ser Pro Gly Phe Val Lys Leu His Thr Arg Lys Phe
       210                 215                 220
   Gly Thr Pro Leu Asn Lys Ser Thr Met Thr Ser Asp Glu Tyr Lys Asn
   225                 230                 235                 240
   Ala Val Leu Gln Ala Lys Glu His Ile Met Ala Gly Asp Ile Phe Gln
                   245                 250                 255
   Ile Val Leu Ser Gln Arg Phe Glu Arg Arg Thr Tyr Ala Asn Pro Phe
               260                 265                 270
   Glu Val Tyr Arg Ala Leu Arg Ile Val Asn Pro Ser Pro Tyr Met Ala
           275                 280                 285
   Tyr Val Gln Ala Arg Gly Cys Val Leu Val Ala Ser Ser Pro Glu Ile
       290                 295                 300
   Leu Thr Arg Val Ser Lys Gly Lys Ile Ile Asn Arg Pro Leu Ala Gly
   305                 310                 315                 320
   Thr Val Arg Arg Gly Lys Thr Glu Lys Glu Asp Gln Met Gln Glu Gln
                   325                 330                 335
   Gln Leu Leu Ser Asp Glu Lys Gln Cys Ala Glu His Ile Met Leu Val
               340                 345                 350
   Asp Leu Gly Arg Asn Asp Val Gly Lys Val Ser Lys Pro Gly Gly Ser
           355                 360                 365
   Val Lys Val Glu Lys Leu Ile Ile Glu Arg Tyr Ser His Val Met His
       370                 375                 380
   Ile Ser Ser Thr Val Ser Gly Gln Leu Asp Asp His Leu Gln Ser Trp
   385                 390                 395                 400
   Asp Ala Leu Arg Ala Ala Leu Pro Val Gly Thr Val Ser Gly Ala Pro
                   405                 410                 415
   Lys Val Lys Ala Met Glu Leu Ile Asp Lys Leu Glu Val Thr Arg Arg
               420                 425                 430
   Gly Pro Tyr Ser Gly Gly Leu Gly Gly Ile Ser Phe Asp Gly Asp Met
           435                 440                 445
   Gln Ile Ala Leu Ser Leu Arg Thr Ile Val Phe Ser Thr Ala Pro Ser
       450                 455                 460
   His Asn Thr Met Tyr Ser Tyr Lys Asp Ala Asp Arg Arg Arg Glu Trp
   465                 470                 475                 480
   Val Ala His Leu Gln Ala Gly Ala Gly Ile Val Ala Asp Ser Ser Pro
                   485                 490                 495
   Asp Asp Glu Gln Arg Glu Cys Glu Asn Lys Ala Ala Ala Leu Ala Arg
               500                 505                 510
   Ala Ile Asp Leu Ala Glu Ser Ala Phe Val Asn Lys Glu
           515                 520                 525
<210> 102
   <211> 92
   <212> PRT
   <213> Triticum aestivum
<400> 102
   Pro Asn Ser Gly Gly Leu Gly Gly Ile Ser Phe Asp Gly Asp Met Leu
   1               5                   10                  15
   Ile Ala Leu Ala Leu Arg Thr Ile Val Phe Ser Thr Ala Pro Ser Pro
   20 25 30
   Asn Arg Met Tyr Ser Tyr Lys Ser Ser Asp Arg Pro Arg Glu Trp Val
   35 40 45
   Ala His Leu Gln Ala Gly Ala Gly Ile Val Ala Asp Ser Ile Pro Asp
   50 55 60
   Asp Glu Gln Lys Glu Phe Glu Asn Lys Ala Ala Ala Leu Ala Arg Ala
   65 70 75 80
   Ile Asp Leu Ala Glu Ser Ala Phe Leu Asp Lys Glu
   85 90
<210> 103
   <211> 616
   <212> PRT
   <213> Nicotiana tabacum
<400> 103
   Met Gln Ser Leu Pro Ile Ser Tyr Arg Leu Phe Pro Ala Thr His Arg
   1               5                   10                  15
   Lys Val Leu Pro Phe Ala Val Ile Ser Ser Arg Ser Ser Thr Ser Ala
               20                  25                  30
   Leu Ala Leu Arg Val Arg Thr Leu Gln Cys Arg Cys Leu His Ser Ser
           35                  40                  45
   Ser Leu Val Met Asp Glu Asp Arg Phe Ile Glu Ala Ser Lys Ser Gly
       50                  55                  60
   Asn Leu Ile Pro Leu His Lys Thr Ile Phe Ser Asp His Leu Thr Pro
   65                  70                  75                  80
   Val Leu Ala Tyr Arg Cys Leu Val Lys Glu Asp Asp Arg Glu Ala Pro
                   85                  90                  95
   Ser Phe Leu Phe Glu Ser Val Glu Pro Gly Phe Arg Gly Ser Ser Val
               100                 105                 110
   Gly Arg Tyr Ser Val Val Gly Ala Gln Pro Ser Met Glu Ile Val Ala
           115                 120                 125
   Lys Glu His Asn Val Thr Ile Leu Asp His His Thr Gly Lys Leu Thr
       130                 135                 140
   Gln Lys Thr Val Gln Asp Pro Met Thr Ile Pro Arg Ser Ile Ser Glu
   145                 150                 155                 160
   Gly Trp Lys Pro Arg Leu Ile Asp Glu Leu Pro Asp Thr Phe Cys Gly
   165 170 175
   Gly Trp Val Gly Tyr Phe Ser Tyr Asp Thr Val Arg Tyr Val Glu Asn
   180 185 190
   Arg Lys Leu Pro Phe Leu Arg Ala Pro Glu Asp Asp Arg Asn Leu Ala
   195 200 205
   Asp Ile Gln Leu Gly Leu Tyr Glu Asp Val Ile Val Phe Asp His Val
   210 215 220
   Glu Lys Lys Ala His Val Ile His Trp Val Gln Leu Asp Gln Tyr Ser
   225 230 235 240
   Ser Leu Pro Glu Ala Tyr Leu Asp Gly Lys Lys Arg Leu Glu Ile Leu
   245 250 255
   Val Ser Arg Val Gln Gly Ile Glu Ser Pro Arg Leu Ser Pro Gly Ser
   260 265 270
   Val Asp Phe Cys Thr His Ala Phe Gly Pro Ser Leu Thr Lys Gly Asn
   275 280 285
   Met Thr Ser Glu Glu Tyr Lys Asn Ala Val Leu Gln Ala Lys Glu His
   290 295 300
   Ile Ala Ala Gly Asp Ile Phe Gln Ile Val Leu Ser Gln Arg Phe Glu
   305 310 315 320
   Arg Arg Thr Phe Ala Asp Pro Phe Glu Val Tyr Arg Ala Leu Arg Ile
   325 330 335
   Val Asn Pro Ser Pro Tyr Met Thr Tyr Ile Gln Ala Arg Gly Cys Ile
   340 345 350
   Leu Val Ala Ser Ser Pro Glu Ile Leu Thr Arg Val Lys Lys Arg Arg
   355 360 365
   Ile Val Asn Arg Pro Leu Ala Gly Thr Ser Arg Arg Gly Lys Thr Pro
   370 375 380
   Asp Glu Asp Val Met Leu Glu Met Gln Met Leu Lys Asp Glu Lys Gln
   385 390 395 400
   Arg Ala Glu His Ile Met Leu Val Asp Leu Gly Arg Asn Asp Val Gly
   405 410 415
   Lys Val Ser Lys Pro Gly Ser Val Asn Val Glu Lys Leu Met Ser Val
   420 425 430
   Glu Arg Tyr Ser His Val Met His Ile Ser Ser Thr Val Ser Gly Glu
   435 440 445
   Leu Leu Asp His Leu Thr Cys Trp Asp Ala Leu Arg Ala Ala Leu Pro
   450 455 460
   Val Gly Thr Val Ser Gly Ala Pro Lys Val Lys Ala Met Glu Leu Ile
   465 470 475 480
   Asp Gln Leu Glu Val Ala Arg Arg Gly Pro Tyr Ser Gly Gly Phe Gly
   485 490 495
   Gly Ile Ser Phe Ser Gly Asp Met Asp Ile Ala Leu Ala Leu Arg Thr
   500 505 510
   Met Val Phe Leu Asn Gly Ala Arg Tyr Asp Thr Met Tyr Ser Tyr Thr
   515 520 525
   Asp Ala Ser Lys Arg Gln Glu Trp Val Ala His Leu Gln Ser Gly Ala
   530 535 540
   Gly Ile Val Ala Asp Ser Asn Pro Asp Glu Glu Gln Ile Glu Cys Glu
   545 550 555 560
   Asn Lys Val Ala Gly Leu Cys Arg Ala Ile Asp Leu Ala Glu Ser Ala
   565 570 575
   Phe Val Lys Gly Arg His Lys Pro Ser Val Lys Ile Asn Gly Ser Val
   580 585 590
   Pro Asn Leu Phe Ser Arg Val Gln Arg Gln Thr Ser Val Met Ser Lys
   595 600 605
   Asp Arg Val His Glu Lys Arg Asn
   610 615
<210> 104
   <211> 1776
   <212> DNA
   <213> Gossypium hirsutum
<220>
   <221> misc_feature
   <223> Alpha subunit.
<400> 104
   atgcaggcat ccatgtcggt tacctcgacg aactccacat taccaatgcc agtgcaaagc 60
   agccttggat tctctcaccg cttccttcct tcatctcatc ggttttctca acttccgatc 120
   acccgctttt ctcccgctcc tacttcactc aaatgcaggg gctctctttc aagctttcca 180
   cttgttaatg atgaaaagaa gtttgtggag gcggccaaaa aagcaaattt agtccccctt 240
   tatcgttgca ttttctctga tcaactgact ccagtgcttg cataccggtg tttggttaaa 300
   gaagatgata gagaggctcc aagttttctc tttgagtcag tggagcctgg ttctcgggtt 360
   tctagtgttg gtcgatatag cgtggttgga gctcaaccga caatggaaat tgtggcaaaa 420
   gaaaacaaag ttatgattat ggatcatgag gcagggaatt tgactgagga ggtcgttgag 480
   gatccgatgt gtattcccaa gagaatctca gagacttgga aaccccgact tgttgaagat 540
   cttcctgatg cgttttgtgg tggatgggtt ggttatttct cctatgatac agttcgttat 600
   gtggagaaga aaaagcttcc gttcactaag gcaccacgtg atgacagaaa cctgccagat 660
   atacatctag gactctacaa cgacgtgatt gtatttgatc atgtggaaaa gaaagcatat 720
   ataattcact gggtgaggct agataaacac tcgtctgttg agaaagctta taatgaagga 780
   gttgaacacc tagagaaatt ggtagctaga gtacaagatg ttgagctacc aaagctatct 840
   ccaggctctg tagcattaca aacccatcac tttggccctt ctttaaagaa ctcaaatatg 900
   gaaaaggaag agttcaagaa agctgtactg aaagcgaaag agcatattct ggcaggggat 960
   attttccaga ttgtattaag ccaacgtttt gaacggagaa catttgctga cccctttgaa 1020
   atatatagag ctttgcgagt tgtgaatcca agtccatata tggcctactt gcaagctaga 1080
   ggaagtattc tagttgcttc aagtcccgaa attcttacca gggtaaagaa gaataagatt 1140
   gtgaataggc cattggctgg aacaacaagg agaggaaaga ctcaggctga agatgagctg 1200
   gcagaaaagc tattgctaag taatgaaaag gaatgtgcag aacacatcat gcttgttgat 1260
   ttgggtcgca atgatgttgg aaaggtctcc aaatatggtt ctgtaaaggt ggagaagctg 1320
   atgaatattg aacgatattc ccatgtgatg cacataagct ccacggttac tggtgagttg 1380
   caggatcatc tcactagttg ggatgtcctc cgtgctgcac ttcccgttgg aactgttagt 1440
   ggagcaccaa aggtgaaggc gatggagcta atcgatgagt tagaggtgtc aagacgaggt 1500
   ccttatagcg gaggatttgg gggcatttcc ttcactgggg atatggatat tgcgttggct 1560
   ctcaggacca tggtattccc tacaggtagc cgctatgaca caatgtactc atacaaaggt 1620
   tccagcagac gccaagaatg ggtagcttat cttcaagccg gcgctggtgt agttgcagac 1680
   agtgatcctg atgccgagca cctcgaatgt caaaacaaag ctgctggcct tgctcgttcc 1740
   attgacctag cggaggctgc attcgttcat aaatga 1776
<210> 105
   <211> 843
   <212> DNA
   <213> Gossypium hirsutum
<220>
   <221> misc_feature
   <223> Beta subunit.
<400> 105
   atggcagcta atattataac ccaatcatct ctgcttcagc caaaacctgc actttctgct     60
   aaaacccttc aaatcccatc tctgcatcgc ttatccggcc tccctcctcc atcaagggtt    120
   ggcttttttc tggaaaagaa aacggggatt gttggaaaag ctccattaaa atcggctgta     180
   tcggactcaa cctcgtcagt tttggagaac aagaaaaaca gcaagaatcc cattgtcgtc    240
   attgacaatt acgacagttt cacttacaat ctttgccagt atattggaga gcttggatgt    300
   tacttcgagg tttttcgaaa tgacgaatta actgtagaag acttaaaaat gaaaaaccct    360
   aggggagtgc ttatctctcc tggtccagga acaccccaag attccggaat atcactgcag    420
   actgttttgg aacttggacc tactgtacct ttgtttggtg tttgtatggg tttgcagtgc    480
   attggtgagg cttttggagg aaagatagtg cgttctccct atggtgttat gcatggcaaa    540
   agttcccctg tatattatga cgaaaagggg gaagacggtt tgttatctgg attgtcaaac    600
   cctttcaatg ctggcagata tcatagcctc gtgattgaaa aggacagttt ccctgaggaa    660
   gcacttgagg ttactgcttg gacagaagat ggactgataa tggctgctag gcacaaagtt    720
   tataagcatc tgcagggtgt tcaattccat ccagagagca tcataacctc tgaaggaaaa    780
   acaattgttc ggaatttcat caaactaatc gagagaaagg aggtggcagg atccaagaat    840
   tag                                                                  843
<210> 106
   <211> 1704
   <212> DNA
   <213> Glycine max
<220>
   <221> misc_feature
   <223> Alpha subunit.
<400> 106
   atggcgactg ttccgcaccc attatccctc gcaagtgtag gttttgctaa ccgaacctcc      60
   tccatctcca gatccactct caaatgctgc gctcaatctc cttctccttc actagttgac     120
   aacgcccaga agtttctcga agcttccaag aaggggaacg tcattcctct cttccgctgc     180
   atattttccg atcacctcac tccggtgctt gcgtaccggt gcctggttaa ggaggacgag     240
   agagatgctc cgagttttct ctttgaatcg gtcgagccag gccaaatttc tagcatcgga     300
   cggtacagtg tggttggagc acagccgtgt atggaaattg tggcgaaaga gaacgtggtt     360
   actattatgg accacgtgga agggcgcagg agtgaggaaa ttgtagagga tcctctggtg     420
   attcctcgta ggatcatgga gaagtggacg cctcaactct tagatgaact tcctgaagcg     480
   ttttgtggtg gttgggtagg gtatttctct tatgatacaa tgcgctatgt agaaaagaag     540
   aaacttccat tttctaatgc cccagtagat gacagaaacc ttcctgatgt tcatctgggc     600
   ctttatgaca gtgtgattgt gtttgatcat gttgaaaaga aagcatatgt gattcattgg     660
   gttcgggtgg atcgatattc ttcagctgag gaggccttcg aagatggaag gaaccggctg     720
   gaaactctag tatctcgggt gcatgatata attaccccaa ggctgcctac aggttcgata     780
   aagttataca ctcgtctctt tggtcctaaa ctggagatgt caaacatgac aaatgaggag     840
   tataagaggg cagtattgaa ggctaaagag cacatacggg ctggtgatat ttttcaaatt     900
   gtactaagtc aacgttttga acagagaact tttgcagacc catttgaaat ctacagagca     960
   ttgaggattg ttaatcctag tccatatatg acttatttac aggccagagg aagtattttg    1020
   gttgcttcaa gtccagaaat tcttacacgg gtgaagaaga gaaagatcac caatcggccc    1080
   cttgctggta ctgttagaag aggaaaaaca ccaaaagaag atatcatgtt ggagaaacaa    1140
   cttttgaatg atgaaaagca atgtgcagag cacgtaatgc tagttgattt ggggagaaat    1200
   gatgttggaa aggtctccaa accgggttct gttcaagttg aaaagcttat gaatattgag    1260
   cgctattccc atgttatgca catcagctca acagtcacag gggagttatt agatcactta    1320
   acaagctggg atgcattgcg tgctgcttta cctgttggta cagttagcgg agcaccgaag    1380
   gtcaaagcca tgcagttgat tgatgagttg gaagtcgcaa gaagggggcc ctatagtggg    1440
   ggatttggag gtatatcatt caatggcgat atggacatag cccttgctct gaggaccata    1500
   gttttcccta caaatgctcg ttatgacaca atgtactcct acaaggataa gaacaaacgc    1560
   agagaatggg ttgcccatct ccaggctgga gcgggaattg tggctgacag tgatcctgct    1620
   gatgaacaaa gagagtgcga gaacaaagct gcagctcttg ctcgtgccat tgatcttgca    1680
   gaatcttcat ttgttgataa ataa                                           1704
<210> 107
   <211> 837
   <212> DNA
   <213> Glycine max
<220>
   <221> misc_feature
   <223> Beta subunit.
<400> 107
   atggctgcca cattcttctc tcacttgtcg cttcttcaat ccaacaacaa cccttctctc    60
   tctcacacac cctctcgctt ccctcattct ctcaccaacc gtgtcaaacc ctccctcggt   120
   gtggtatctg tggccaaaag ggtaagtgga gtggtgccaa aggccaattt gaatgccttg   180
   gaggccaatt cgggtttccc catttcggct aagaagtcca acaacaaccc cattgttgtt   240
   attgacaact atgacagttt cacctataat ctttgccagt atatggggga gttagggttt    300
   cactttgagg tctaccgcaa tgatgagttg acagtggagg agttaagaag gaaaaatccc   360
   agaggagtgc tgatatcacc tgggccagga gaacctcaag attcaggcat atctttgcaa   420
   acggttttgg aacttggacc aactgtgcca ttgtttggtg tgtgcatggg tttgcaatgc   480
   attggagagg cttttggagg gaagattgtt cgttctcctc atggtgttat gcatggaaaa   540
   agctctatgg tttactatga tgagaaagga gaagatggat tacttgctgg actatcaaat   600
   cctttcttgg ctggtagata tcacagcctt gtaattgaaa aagagagctt tcctcatgat   660
   gaacttgagg caacagcatg gacagaagat ggtcttataa tggctgctcg tcataagaaa   720
   tataagcatc tacagggtgt tcagtttcat ccagagagca tcataacccc agaaggcaag   780
   acaattgtcc gtaattttgt caagcttatc gagaaaaggg aggctggtgg ctcttga      837
<210> 108
   <211> 591
   <212> PRT
   <213> Gossypium hirsutum
<220>
   <221> MISC_FEATURE
   <223> Alpha subunit.
<400> 108
   Met Gln Ala Ser Met Ser Val Thr Ser Thr Asn Ser Thr Leu Pro Met
   1               5                   10                  15
   Pro Val Gln Ser Ser Leu Gly Phe Ser His Arg Phe Leu Pro Ser Ser
               20                  25                  30
   His Arg Phe Ser Gln Leu Pro Ile Thr Arg Phe Ser Pro Ala Pro Thr
           35                  40                  45
   Ser Leu Lys Cys Arg Gly Ser Leu Ser Ser Phe Pro Leu Val Asn Asp
       50                  55                  60
   Glu Lys Lys Phe Val Glu Ala Ala Lys Lys Ala Asn Leu Val Pro Leu
   65                  70                  75                  80
   Tyr Arg Cys Ile Phe Ser Asp Gln Leu Thr Pro Val Leu Ala Tyr Arg
                   85                  90                  95
   Cys Leu Val Lys Glu Asp Asp Arg Glu Ala Pro Ser Phe Leu Phe Glu
               100                 105                 110
   Ser Val Glu Pro Gly Ser Arg Val Ser Ser Val Gly Arg Tyr Ser Val
           115                 120                 125
   Val Gly Ala Gln Pro Thr Met Glu Ile Val Ala Lys Glu Asn Lys Val
       130                 135                 140
   Met Ile Met Asp His Glu Ala Gly Asn Leu Thr Glu Glu Val Val Glu
   145                 150                 155                 160
   Asp Pro Met Cys Ile Pro Lys Arg Ile Ser Glu Thr Trp Lys Pro Arg
                   165                 170                 175
   Leu Val Glu Asp Leu Pro Asp Ala Phe Cys Gly Gly Trp Val Gly Tyr
               180                 185                 190
   Phe Ser Tyr Asp Thr Val Arg Tyr Val Glu Lys Lys Lys Leu Pro Phe
           195                 200                 205
   Thr Lys Ala Pro Arg Asp Asp Arg Asn Leu Pro Asp Ile His Leu Gly
       210                 215                 220
   Leu Tyr Asn Asp Val Ile Val Phe Asp His Val Glu Lys Lys Ala Tyr
   225 230 235 240
   Ile Ile His Trp Val Arg Leu Asp Lys His Ser Ser Val Glu Lys Ala
   245 250 255
   Tyr Asn Glu Gly Val Glu His Leu Glu Lys Leu Val Ala Arg Val Gln
   260 265 270
   Asp Val Glu Leu Pro Lys Leu Ser Pro Gly Ser Val Ala Leu Gln Thr
   275 280 285
   His His Phe Gly Pro Ser Leu Lys Asn Ser Asn Met Glu Lys Glu Glu
   290 295 300
   Phe Lys Lys Ala Val Leu Lys Ala Lys Glu His Ile Leu Ala Gly Asp
   305 310 315 320
   Ile Phe Gln Ile Val Leu Ser Gln Arg Phe Glu Arg Arg Thr Phe Ala
   325 330 335
   Asp Pro Phe Glu Ile Tyr Arg Ala Leu Arg Val Val Asn Pro Ser Pro
   340 345 350
   Tyr Met Ala Tyr Leu Gln Ala Arg Gly Ser Ile Leu Val Ala Ser Ser
   355 360 365
   Pro Glu Ile Leu Thr Arg Val Lys Lys Asn Lys Ile Val Asn Arg Pro
   370 375 380
   Leu Ala Gly Thr Thr Arg Arg Gly Lys Thr Gln Ala Glu Asp Glu Leu
   385 390 395 400
   Ala Glu Lys Leu Leu Leu Ser Asn Glu Lys Glu Cys Ala Glu His Ile
   405 410 415
   Met Leu Val Asp Leu Gly Arg Asn Asp Val Gly Lys Val Ser Lys Tyr
   420 425 430
   Gly Ser Val Lys Val Glu Lys Leu Met Asn Ile Glu Arg Tyr Ser His
   435 440 445
   Val Met His Ile Ser Ser Thr Val Thr Gly Glu Leu Gln Asp His Leu
   450 455 460
   Thr Ser Trp Asp Val Leu Arg Ala Ala Leu Pro Val Gly Thr Val Ser
   465 470 475 480
   Gly Ala Pro Lys Val Lys Ala Met Glu Leu Ile Asp Glu Leu Glu Val
   485 490 495
   Ser Arg Arg Gly Pro Tyr Ser Gly Gly Phe Gly Gly Ile Ser Phe Thr
   500 505 510
   Gly Asp Met Asp Ile Ala Leu Ala Leu Arg Thr Met Val Phe Pro Thr
   515 520 525
   Gly Ser Arg Tyr Asp Thr Met Tyr Ser Tyr Lys Gly Ser Ser Arg Arg
   530 535 540
   Gln Glu Trp Val Ala Tyr Leu Gln Ala Gly Ala Gly Val Val Ala Asp
   545 550 555 560
   Ser Asp Pro Asp Ala Glu His Leu Glu Cys Gln Asn Lys Ala Ala Gly
   565 570 575
   Leu Ala Arg Ser Ile Asp Leu Ala Glu Ala Ala Phe Val His Lys
   580 585 590
<210> 109
   <211> 280
   <212> PRT
   <213> Gossypium hirsutum
<220>
   <221> MISC_FEATURE
   <223> Beta subunit.
<400> 109
   Met Ala Ala Asn Ile Ile Thr Gln Ser Ser Leu Leu Gln Pro Lys Pro
   1               5                   10                  15
   Ala Leu Ser Ala Lys Thr Leu Gln Ile Pro Ser Leu His Arg Leu Ser
               20                  25                  30
   Gly Leu Pro Pro Pro Ser Arg Val Gly Phe Phe Leu Glu Lys Lys Thr
           35                  40                  45
   Gly Ile Val Gly Lys Ala Pro Leu Lys Ser Ala Val Ser Asp Ser Thr
       50                  55                  60
   Ser Ser Val Leu Glu Asn Lys Lys Asn Ser Lys Asn Pro Ile Val Val
   65                  70                  75                  80
   Ile Asp Asn Tyr Asp Ser Phe Thr Tyr Asn Leu Cys Gln Tyr Ile Gly
                   85                  90                  95
   Glu Leu Gly Cys Tyr Phe Glu Val Phe Arg Asn Asp Glu Leu Thr Val
               100                 105                 110
   Glu Asp Leu Lys Met Lys Asn Pro Arg Gly Val Leu Ile Ser Pro Gly
           115                 120                 125
   Pro Gly Thr Pro Gln Asp Ser Gly Ile Ser Leu Gln Thr Val Leu Glu
   130 135 140
   Leu Gly Pro Thr Val Pro Leu Phe Gly Val Cys Met Gly Leu Gln Cys
   145 150 155 160
   Ile Gly Glu Ala Phe Gly Gly Lys Ile Val Arg Ser Pro Tyr Gly Val
   165 170 175
   Met His Gly Lys Ser Ser Pro Val Tyr Tyr Asp Glu Lys Gly Glu Asp
   180 185 190
   Gly Leu Leu Ser Gly Leu Ser Asn Pro Phe Asn Ala Gly Arg Tyr His
   195 200 205
   Ser Leu Val Ile Glu Lys Asp Ser Phe Pro Glu Glu Ala Leu Glu Val
   210 215 220
   Thr Ala Trp Thr Glu Asp Gly Leu Ile Met Ala Ala Arg His Lys Val
   225 230 235 240
   Tyr Lys His Leu Gln Gly Val Gln Phe His Pro Glu Ser Ile Ile Thr
   245 250 255
   Ser Glu Gly Lys Thr Ile Val Arg Asn Phe Ile Lys Leu Ile Glu Arg
   260 265 270
   Lys Glu Val Ala Gly Ser Lys Asn
   275 280
<210> 110
   <211> 567
   <212> PRT
   <213> Glycine max
<220>
   <221> MISC_FEATURE
   <223> Alpha subunit.
<400> 110
   Met Ala Thr Val Pro His Pro Leu Ser Leu Ala Ser Val Gly Phe Ala
   1               5                   10                  15
   Asn Arg Thr Ser Ser Ile Ser Arg Ser Thr Leu Lys Cys Cys Ala Gln
               20                  25                  30
   Ser Pro Ser Pro Ser Leu Val Asp Asn Ala Gln Lys Phe Leu Glu Ala
           35                  40                  45
   Ser Lys Lys Gly Asn Val Ile Pro Leu Phe Arg Cys Ile Phe Ser Asp
       50                  55                  60
   His Leu Thr Pro Val Leu Ala Tyr Arg Cys Leu Val Lys Glu Asp Glu
   65 70 75 80
   Arg Asp Ala Pro Ser Phe Leu Phe Glu Ser Val Glu Pro Gly Gln Ile
   85 90 95
   Ser Ser Ile Gly Arg Tyr Ser Val Val Gly Ala Gln Pro Cys Met Glu
   100 105 110
   Ile Val Ala Lys Glu Asn Val Val Thr Ile Met Asp His Val Glu Gly
   115 120 125
   Arg Arg Ser Glu Glu Ile Val Glu Asp Pro Leu Val Ile Pro Arg Arg
   130 135 140
   Ile Met Glu Lys Trp Thr Pro Gln Leu Leu Asp Glu Leu Pro Glu Ala
   145 150 155 160
   Phe Cys Gly Gly Trp Val Gly Tyr Phe Ser Tyr Asp Thr Met Arg Tyr
   165 170 175
   Val Glu Lys Lys Lys Leu Pro Phe Ser Asn Ala Pro Val Asp Asp Arg
   180 185 190
   Asn Leu Pro Asp Val His Leu Gly Leu Tyr Asp Ser Val Ile Val Phe
   195 200 205
   Asp His Val Glu Lys Lys Ala Tyr Val Ile His Trp Val Arg Val Asp
   210 215 220
   Arg Tyr Ser Ser Ala Glu Glu Ala Phe Glu Asp Gly Arg Asn Arg Leu
   225 230 235 240
   Glu Thr Leu Val Ser Arg Val His Asp Ile Ile Thr Pro Arg Leu Pro
   245 250 255
   Thr Gly Ser Ile Lys Leu Tyr Thr Arg Leu Phe Gly Pro Lys Leu Glu
   260 265 270
   Met Ser Asn Met Thr Asn Glu Glu Tyr Lys Arg Ala Val Leu Lys Ala
   275 280 285
   Lys Glu His Ile Arg Ala Gly Asp Ile Phe Gln Ile Val Leu Ser Gln
   290 295 300
   Arg Phe Glu Gln Arg Thr Phe Ala Asp Pro Phe Glu Ile Tyr Arg Ala
   305 310 315 320
   Leu Arg Ile Val Asn Pro Ser Pro Tyr Met Thr Tyr Leu Gln Ala Arg
   325 330 335
   Gly Ser Ile Leu Val Ala Ser Ser Pro Glu Ile Leu Thr Arg Val Lys
   340 345 350
   Lys Arg Lys Ile Thr Asn Arg Pro Leu Ala Gly Thr Val Arg Arg Gly
   355 360 365
   Lys Thr Pro Lys Glu Asp Ile Met Leu Glu Lys Gln Leu Leu Asn Asp
   370 375 380
   Glu Lys Gln Cys Ala Glu His Val Met Leu Val Asp Leu Gly Arg Asn
   385 390 395 400
   Asp Val Gly Lys Val Ser Lys Pro Gly Ser Val Gln Val Glu Lys Leu
   405 410 415
   Met Asn Ile Glu Arg Tyr Ser His Val Met His Ile Ser Ser Thr Val
   420 425 430
   Thr Gly Glu Leu Leu Asp His Leu Thr Ser Trp Asp Ala Leu Arg Ala
   435 440 445
   Ala Leu Pro Val Gly Thr Val Ser Gly Ala Pro Lys Val Lys Ala Met
   450 455 460
   Gln Leu Ile Asp Glu Leu Glu Val Ala Arg Arg Gly Pro Tyr Ser Gly
   465 470 475 480
   Gly Phe Gly Gly Ile Ser Phe Asn Gly Asp Met Asp Ile Ala Leu Ala
   485 490 495
   Leu Arg Thr Ile Val Phe Pro Thr Asn Ala Arg Tyr Asp Thr Met Tyr
   500 505 510
   Ser Tyr Lys Asp Lys Asn Lys Arg Arg Glu Trp Val Ala His Leu Gln
   515 520 525
   Ala Gly Ala Gly Ile Val Ala Asp Ser Asp Pro Ala Asp Glu Gln Arg
   530 535 540
   Glu Cys Glu Asn Lys Ala Ala Ala Leu Ala Arg Ala Ile Asp Leu Ala
   545 550 555 560
   Glu Ser Ser Phe Val Asp Lys
   565
<210> 111
   <211> 278
   <212> PRT
   <213> Glycine max
<220>
   <221> MISC_FEATURE
   <223> Beta subunit.
<400> 111
   Met Ala Ala Thr Phe Phe Ser His Leu Ser Leu Leu Gln Ser Asn Asn
   1               5                   10                  15
   Asn Pro Ser Leu Ser His Thr Pro Ser Arg Phe Pro His Ser Leu Thr
               20                  25                  30
   Asn Arg Val Lys Pro Ser Leu Gly Val Val Ser Val Ala Lys Arg Val
           35                  40                  45
   Ser Gly Val Val Pro Lys Ala Asn Leu Asn Ala Leu Glu Ala Asn Ser
       50                  55                  60
   Gly Phe Pro Ile Ser Ala Lys Lys Ser Asn Asn Asn Pro Ile Val Val
   65                  70                  75                  80
   Ile Asp Asn Tyr Asp Ser Phe Thr Tyr Asn Leu Cys Gln Tyr Met Gly
                   85                  90                  95
   Glu Leu Gly Phe His Phe Glu Val Tyr Arg Asn Asp Glu Leu Thr Val
               100                 105                 110
   Glu Glu Leu Arg Arg Lys Asn Pro Arg Gly Val Leu Ile Ser Pro Gly
           115                 120                 125
   Pro Gly Glu Pro Gln Asp Ser Gly Ile Ser Leu Gln Thr Val Leu Glu
       130                 135                 140
   Leu Gly Pro Thr Val Pro Leu Phe Gly Val Cys Met Gly Leu Gln Cys
   145                 150                 155                 160
   Ile Gly Glu Ala Phe Gly Gly Lys Ile Val Arg Ser Pro His Gly Val
                   165                 170                 175
   Met His Gly Lys Ser Ser Met Val Tyr Tyr Asp Glu Lys Gly Glu Asp
               180                 185                 190
   Gly Leu Leu Ala Gly Leu Ser Asn Pro Phe Leu Ala Gly Arg Tyr His
           195                 200                 205
   Ser Leu Val Ile Glu Lys Glu Ser Phe Pro His Asp Glu Leu Glu Ala
       210                 215                 220
   Thr Ala Trp Thr Glu Asp Gly Leu Ile Met Ala Ala Arg His Lys Lys
   225                 230                 235                 240
   Tyr Lys His Leu Gln Gly Val Gln Phe His Pro Glu Ser Ile Ile Thr
                   245                 250                 255
   Pro Glu Gly Lys Thr Ile Val Arg Asn Phe Val Lys Leu Ile Glu Lys
   260 265 270
   Arg Glu Ala Gly Gly Ser
   275
<210> 112
   <211> 2210
   <212> DNA
   <213> Glycine max
<220>
   <221> 3'UTR
   <222> (1919)..(2210)
   <223>
<220>
   <221> misc_feature
   <223> Alpha subunit.
<220>
   <221> 5'UTR
   <222> (1) .. (214)
   <223>
<400> 112
   aaagaaatga cctgaagtct ctatatattc caggtaacga agaccttagc aacccaatga 60
   acgcgtcccc atgaacggca cgtgtagcgg agtactaggc cgccgttaca cgctactgct 120
   gtccgtcgtc accgtttgaa ttacccaccg ctactttgtc gtcattcttc ttcttcttcc 180
   cttttcattg accttttcct tccttcgtcc tccaatggcg actgttccgc acccattatc 240
   cctcgcaagt gtaggttttg ctaaccgaac ctcctccatc tccagatcca ctctcaaatg 300
   ctgcgctcaa tctccttctc cttcactagt tgacaacgcc cagaagtttc tcgaagcttc 360
   caagaagggg aacgtcattc ctctcttccg ctgcatattt tccgatcacc tcactccggt 420
   gcttgcgtac cggtgcctgg ttaaggagga cgagagagat gctccgagtt ttctctttga 480
   atcggtcgag ccaggccaaa tttctagcat cggacggtac agtgtggttg gagcacagcc 540
   gtgtatggaa attgtggcga aagagaacgt ggttactatt atggaccacg tggaagggcg 600
   caggagtgag gaaattgtag aggatcctct ggtgattcct cgtaggatca tggagaagtg 660
   gacgcctcaa ctcttagatg aacttcctga agcgttttgt ggtggttggg tagggtattt 720
   ctcttatgat acaatgcgct atgtagaaaa gaagaaactt ccattttcta atgccccagt 780
   agatgacaga aaccttcctg atgttcatct gggcctttat gacagtgtga ttgtgtttga 840
   tcatgttgaa aagaaagcat atgtgattca ttgggttcgg gtggatcgat attcttcagc 900
   tgaggaggcc ttcgaagatg gaaggaaccg gctggaaact ctagtatctc gggtgcatga 960
   tataattacc ccaaggctgc ctacaggttc gataaagtta tacactcgtc tctttggtcc 1020
   taaactggag atgtcaaaca tgacaaatga ggagtataag agggcagtat tgaaggctaa 1080
   agagcacata cgggctggtg atatttttca aattgtacta agtcaacgtt ttgaacagag 1140
   aacttttgca gacccatttg aaatctacag agcattgagg attgttaatc ctagtccata 1200
   tatgacttat ttacaggcca gaggaagtat tttggttgct tcaagtccag aaattcttac   1260
   acgggtgaag aagagaaaga tcaccaatcg gccccttgct ggtactgtta gaagaggaaa   1320
   aacaccaaaa gaagatatca tgttggagaa acaacttttg aatgatgaaa agcaatgtgc   1380
   agagcacgta atgctagttg atttggggag aaatgatgtt ggaaaggtct ccaaaccggg   1440
   ttctgttcaa gttgaaaagc ttatgaatat tgagcgctat tcccatgtta tgcacatcag   1500
   ctcaacagtc acaggggagt tattagatca cttaacaagc tgggatgcat tgcgtgctgc   1560
   tttacctgtt ggtacagtta gcggagcacc gaaggtcaaa gccatgcagt tgattgatga   1620
   gttggaagtc gcaagaaggg ggccctatag tgggggattt ggaggtatat cattcaatgg   1680
   cgatatggac atagcccttg ctctgaggac catagttttc cctacaaatg ctcgttatga   1740
   cacaatgtac tcctacaagg ataagaacaa acgcagagaa tgggttgccc atctccaggc   1800
   tggagcggga attgtggctg acagtgatcc tgctgatgaa caaagagagt gcgagaacaa   1860
   agctgcagct cttgctcgtg ccattgatct tgcagaatct tcatttgttg ataaataatt   1920
   tggattgatc catcatcagt gatgctcctt gataactgag gggcatcctt tttaaatggt   1980
   agagaggaag tttgtggtgt gggcagatga taggggatat gaattacgga gaatctgaaa   2040
   ctttgataat gttatgacag aagtgatgaa cataataagg tatttaatga taatgacagc   2100
   tttgtgactt tagttaagtc gtcgtttaag agacttcaat agccatttcc gtcggtccat   2160
   cttaaaccaa agaaaggtgc ctttgacgga gtttcttttg ctatcataaa              2210
<210> 113
   <211> 988
   <212> DNA
   <213> Glycine max
<220>
   <221> 3'UTR
   <222> (926) .. (988)
   <223>
<220>
   <221> misc_feature
   <223> Beta subunit.
<220>
   <221> 5'UTR
   <222> (1)..(88)
   <223>
<400> 113
   actcggctat gcatccaacg cgttgggagc tctcccatat ggtcgacctg caggcggccg      60
   cgaattcact agtgattaac atttgaacat ggctgccaca ttcttctctc acttgtcgct     120
   tcttcaatcc aacaacaacc cttctctctc tcacacaccc tctcgcttcc ctcattctct     180
   caccaaccgt gtcaaaccct ccctcggtgt ggtatctgtg gccaaaaggg taagtggagt     240
   ggtgccaaag gccaatttga atgccttgga ggccaattcg ggtttcccca tttcggctaa     300
   gaagtccaac aacaacccca ttgttgttat tgacaactat gacagtttca cctataatct     360
   ttgccagtat atgggggagt tagggtttca ctttgaggtc taccgcaatg atgagttgac     420
   agtggaggag ttaagaagga aaaatcccag aggagtgctg atatcacctg ggccaggaga     480
   acctcaagat tcaggcatat ctttgcaaac ggttttggaa cttggaccaa ctgtgccatt     540
   gtttggtgtg tgcatgggtt tgcaatgcat tggagaggct tttggaggga agattgttcg     600
   ttctcctcat ggtgttatgc atggaaaaag ctctatggtt tactatgatg agaaaggaga     660
   agatggatta cttgctggac tatcaaatcc tttcttggct ggtagatatc acagccttgt     720
   aattgaaaaa gagagctttc ctcatgatga acttgaggca acagcatgga cagaagatgg     780
   tcttataatg gctgctcgtc ataagaaata taagcatcta cagggtgttc agtttcatcc     840
   agagagcatc ataaccccag aaggcaagac aattgtccgt aattttgtca agcttatcga     900
   gaaaagggag gctggtggct cttgaaaatc gaattcccgc ggccgccatg gcggccggga     960
   gcatgcgacg tcgggccbaw kcggmgtt                                        988
<210> 114
   <211> 47
   <212> PRT
   <213> zea mays
<220>
   <221> misc_feature
   <223> The sequence of a CTP.
<400> 114
   Met Glu Ser Leu Ala Ala Thr Ser Val Phe Ala Pro Ser Arg Val Ala
   1               5                   10                  15
   Val Pro Ala Ala Arg Ala Leu Val Arg Ala Gly Thr Val Val Pro Thr
               20                  25                  30
   Arg Arg Thr Ser Ser Arg Ser Gly Thr Ser Gly Val Lys Cys Ser
           35                  40                  45
<210> 115
   <211> 32
   <212> PRT
   <213> zea mays
<220>
   <221> misc_feature
   <223> The sequence of a CTP.
<400> 115
   Met Ala Thr Ala Ser Leu Ala Leu Ser Leu Arg Leu Ala Pro Ser Ser
   1               5                   10                  15
   Arg Pro Leu Ser Leu Arg Arg Arg Gly Ala Ala Gly Val Thr Cys Arg
               20                  25                  30
<210> 116
   <211> 849
   <212> DNA
   <213> zea mays
<400> 116
   atggcctgct cccacatcgt cgccgccgcg ggggtctcct cccccgccgc agcggcggct 60
   cgttccccgg cgcattctcc cgctgccgcc ttcgcgcgcc tccggtcgac gcctcgtttc 120
   gcgagcgctg gcttgtcggt taagggaaac ggagcggcgt tcccgttggt cgccgccgcg 180
   gggccggccg cggcggcacc ggtggccgac ctggacggcc gcccggccac ggagaagcag 240
   cccatcatcg tcatcgacaa ctacgacagc ttcacataca acctctgcca gtatatgggg 300
   gagcttggat tgaacttcga agtataccgc aatgatgaac tgaccataga agatgtgaga 360
   aggaagaacc caaggggaat acttatttct ccaggacctg gtgaaccaca agattcggga 420
   atatcattgc agactgttct tgaattaggc ccaaccatcc caatttttgg agtttgcatg 480
   ggtctgcaat gcattggaga ggcatttgga ggaaagatta tccgtgctcc ttctggagtg 540
   atgcatggga aaagctctcc agtttattac gatgaggaat taggaaaggc attgttcaat 600
   ggcttgccaa acccttttac tgccgcgagg taccacagct tggtcattga gcaagaaacc 660
   ttcccacatg atgctttgga ggctactgca tggactgaag atggacttat catggctgct 720
   cgccacaaga agtacaaaca catccagggt gtccaattcc acccggagag catcatcacc 780
   cctgaaggca agaaaatcat cctcaacttt gtcagattca ttgaggaact ggagaagcag 840
   cgttcgtag 849
<210> 117
   <211> 273
   <212> PRT
   <213> Oryza sativa
<400> 117
   Met Ala Thr Ala Ala Arg Leu Leu Pro Lys Ile Gln Ser Pro Ala Ser
   1               5                   10                  15
   Pro Ala Val Ala Glu Ala Arg Arg Arg Arg Pro Ser Ser Leu Arg Leu
               20                  25                  30
   Gly Val Thr Ser Gly Pro Ala Arg Thr Leu Lys Gln Lys Leu Val Ala
           35                  40                  45
   Lys Ser Ala Val Ser Val Val Glu Gly Glu Asn Ala Phe Asp Gly Val
       50                  55                  60
   Lys Gln Asp Thr Arg Pro Ile Ile Val Ile Asp Asn Tyr Asp Ser Phe
   65                  70                  75                  80
   Thr Tyr Asn Leu Cys Gln Tyr Met Gly Glu Val Gly Ala Asn Phe Glu
                   85                  90                  95
   Val Tyr Arg Asn Asp Asp Ile Thr Val Glu Glu Ile Lys Lys Ile Ser
   100 105 110
   Pro Arg Gly Ile Leu Ile Ser Pro Gly Pro Gly Thr Pro Gln Asp Ser
   115 120 125
   Gly Ile Ser Leu Gln Thr Val Gln Asp Leu Gly Pro Ser Thr Pro Leu
   130 135 140
   Phe Gly Val Cys Met Gly Leu Gln Cys Ile Gly Glu Ala Phe Gly Gly
   145 150 155 160
   Lys Val Val Arg Ser Pro Tyr Gly Val Val His Gly Lys Gly Ser Leu
   165 170 175
   Val His Tyr Glu Glu Lys Leu Asp Gly Thr Leu Phe Ser Gly Leu Pro
   180 185 190
   Asn Pro Phe Gln Ala Gly Arg Tyr His Ser Leu Val Ile Glu Lys Asp
   195 200 205
   Ser Phe Pro His Asp Ala Leu Glu Ile Thr Ala Trp Thr Asp Asp Gly
   210 215 220
   Leu Ile Met Ala Ala Arg His Arg Lys Tyr Lys His Ile Gln Gly Val
   225 230 235 240
   Gln Phe His Pro Glu Ser Ile Ile Thr Thr Glu Gly Arg Leu Met Val
   245 250 255
   Lys Asn Phe Ile Lys Ile Ile Glu Gly Tyr Glu Ala Leu Asn Cys Leu
   260 265 270
   Pro
<210> 118
   <211> 282
   <212> PRT
   <213> zea mays
<400> 118
   Met Ala Cys Ser His Ile Val Ala Ala Ala Gly Val Ser Ser Pro Ala
   1 5 10 15
   Ala Ala Ala Ala Arg Ser Pro Ala His Ser Pro Ala Ala Ala Phe Ala
   20 25 30
   Arg Leu Arg Ser Thr Pro Arg Phe Ala Ser Ala Gly Leu Ser Val Lys
   35 40 45
   Gly Asn Gly Ala Ala Phe Pro Leu Val Ala Ala Ala Gly Pro Ala Ala
   50 55 60
   Ala Ala Pro Val Ala Asp Leu Asp Gly Arg Pro Ala Thr Glu Lys Gln
   65 70 75 80
   Pro Ile Ile Val Ile Asp Asn Tyr Asp Ser Phe Thr Tyr Asn Leu Cys
   85 90 95
   Gln Tyr Met Gly Glu Leu Gly Leu Asn Phe Glu Val Tyr Arg Asn Asp
   100 105 110
   Glu Leu Thr Ile Glu Asp Val Arg Arg Lys Asn Pro Arg Gly Ile Leu
   115 120 125
   Ile Ser Pro Gly Pro Gly Glu Pro Gln Asp Ser Gly Ile Ser Leu Gln
   130 135 140
   Thr Val Leu Glu Leu Gly Pro Thr Ile Pro Ile Phe Gly Val Cys Met
   145 150 155 160
   Gly Leu Gln Cys Ile Gly Glu Ala Phe Gly Gly Lys Ile Ile Arg Ala
   165 170 175
   Pro Ser Gly Val Met His Gly Lys Ser Ser Pro Val Tyr Tyr Asp Glu
   180 185 190
   Glu Leu Gly Lys Ala Leu Phe Asn Gly Leu Pro Asn Pro Phe Thr Ala
   195 200 205
   Ala Arg Tyr His Ser Leu Val Ile Glu Gln Glu Thr Phe Pro His Asp
   210 215 220
   Ala Leu Glu Ala Thr Ala Trp Thr Glu Asp Gly Leu Ile Met Ala Ala
   225 230 235 240
   Arg His Lys Lys Tyr Lys His Ile Gln Gly Val Gln Phe His Pro Glu
   245 250 255
   Ser Ile Ile Thr Pro Glu Gly Lys Lys Ile Ile Leu Asn Phe Val Arg
   260 265 270
   Phe Ile Glu Glu Leu Glu Lys Gln Arg Ser
   275 280
<210> 119
   <211> 822
   <212> DNA
   <213> Oryza sativa
<400> 119
   atggccaccg ccgcgcggct cctccccaag atccagtccc ccgcctcccc ggccgtcgcg 60
   gaggcgcgga ggcgccgccc ctccagtctc cgattaggag ttactagtgg acccgcaaga 120
   actctgaagc aaaagcttgt tgctaagagt gctgtttctg tggtggaagg tgaaaacgca    180
   tttgatggag taaagcaaga tactagacca atcatagtta tagataacta cgatagcttc    240
   acgtataatt tatgccagta catgggtgag gtgggagcta actttgaggt gtaccgcaat    300
   gatgatatca ccgtggaaga aattaagaag atttctccta gaggaatact catctcccct    360
   ggccctggca cacctcaaga ttcaggaata tcattgcaaa cagttcaaga tcttggacct    420
   tctacacctt tgtttggggt ttgcatgggt ttgcagtgta ttggggaggc atttggaggg    480
   aaggttgttc gttctcctta tggagttgtg catgggaaag gatcccttgt tcactatgag    540
   gagaaacttg atggaacact gttttctggt ctcccaaacc cattccaagc gggaagatac    600
   cacagccttg taattgagaa ggatagcttc ccacatgatg ccctggaaat tactgcttgg    660
   acagacgatg ggctgatcat ggctgctcgc cacaggaagt acaaacatat acagggtgtg    720
   cagttccatc cagagagcat cataacaaca gaagggaggc tcatggtcaa gaatttcatc    780
   aagattattg aaggctacga ggccttgaat tgcttaccgt ga                      822
<210> 120
   <211> 867
   <212> DNA
   <213> oryza sativa
<400> 120
   atggcgtgct cccacctggc cgccgccgcc gccgcggcct ccccggcggc cgcgcgttca     60
   ccggcggcct ccagcgccgc aaccgcgagc gccttcgcgc gcctctcggc gacgccccgg    120
   gtcgcgagcg gcgggttggc cgttaggggc cagaggggtg tagccgctgt tgtcgccgcc    180
   gccgccgggg ccgccgcggc gacgcccgtg gccgacatcg aggaacgccg ggccaccgag    240
   aagcagccca tcattgtcat cgataactac gacagcttta cctacaacct ctgccagtat    300
   atgggggagc ttggattgaa ctttgaggta tatcgcaatg atgaacttac catagaggat    360
   gtaaagagga agaacccaag aggaatactt atttctccag ggcctggtga gccacaagat    420
   tcaggtatat cattgcagac tgttctggaa cttggaccta ccatcccaat ttttggtgtc    480
   tgcatgggtc tgcagtgtat cggggaagct tttggaggaa agattatccg tgctccttct    540
   ggtgtcatgc atggaaaaag ctctccagtt cgctacgatg aggagctagg gaaggccttg    600
   ttcaatggct tgccaaaccc atttaccgct gcaagatacc atagcttggt gatcgagcag    660
   gagaccttcc cccatgacgc tctggaagcc accgcgtgga ctgaagatgg ccttatcatg    720
   gctgctcgcc acaagaagta caggcacatc cagggagtcc aattccaccc agagagcatc    780
   atcaccccag aaggcaagag gatcattctc aacttcgtga ggttcatcga ggagctggag    840
   aagcagcgtg ccggagagaa gaactag                                        867
<210> 121
   <211> 2202
   <212> DNA
   <213> Mesorhizobium loti
<400> 121
   atggagacgg caatgacgat gaaggttctg gaaaacggcg ctgaaagctt cgtgaccgcg 60
   ggtggcatca ccattacgcg tgagcgccac gaccggccct atgcgggtgc gatcgacgct 120
   tatgtcgatg gcttgaactc gcgccgcggc gcggtgtttt cctccaacta cgaatatccc 180
   ggccgctata cgcgctggga caccgccatc atcgatccgc cgctggtcat ttccgcgcgg 240
   ggccgcgcca tgcgcatcga agcgctgaac cgccgcggcg aggcgctgtt gccggtgatc 300
   ggcaaaacgc tgggcggcct tgccgacatc accatcgccg agacgacgaa gacgctcatc 360
   cgcctcgacg tcgccaagcc cggccgcgtc ttcacggagg aagagcgcag ccgcgtgccc 420
   tcggtcttca ccgtgctgcg cgccatcacc gctttgttca aaaccgacga ggacgccaat 480
   ctcggcctct atggcgcctt cggctacgac ctctccttcc agttcgaccc ggtcgactac 540
   aagctcgagc gcaagcccag ccagcgcgac ctcgtgctgt tcctgcccga cgagatcctg 600
   gtcgtcgacc actattccgc caaagcctgg accgaccgct acgactattc gggcgaagga 660
   ttttcgaccg aaggtctgcc gcgcgacgca atcgccgagc cgttcaagac cgccgaccgc 720
   atcccgccgc gcggtgacca tgagccgggc gaatacgcta atctggtgcg gcgtgccatg 780
   gactcgttca agcgcggcga cctgttcgag gtcgtgcccg gccagatgtt ctacgagcgc 840
   tgcgagacgc agccctccga catttcgcgc aagctgaaat cgatcaaccc ctcgccttat 900
   tcgttcttca tcaacctcgg cgaaaacgaa tatctgatcg gcgcctcgcc cgaaatgttc 960
   gtgcgcgtca atggccgccg cgtcgaaacc tgcccgattt cgggcaccat caaacgcggc 1020
   gacgacgcca tttccgatag cgagcagatc ctgaagctgc tcaattcgaa gaaggacgaa 1080
   tccgagctca caatgtgctc ggacgtcgac cgcaacgaca agtcgcgggt ctgcgagccc 1140
   ggctcggtgc gcgtcatcgg ccgccgccag atcgagatgt attcgcgcct catccacacc 1200
   gtcgatcaca tcgaaggccg gctgcgcgaa ggcatggacg ctttcgacgc cttcctgtcg 1260
   catgcctggg cggtcactgt caccggcgcg ccgaaactgt gggccatgcg cttcatcgag 1320
   cagaacgaga agagcccgcg cgcctggtat ggcggcgcaa tcggcatggt caacttcaac 1380
   ggcgacatga acaccggcct gacgctgcgc accatccgca tcaaggacgg cattgccgaa 1440
   gtgcgggccg gcgcgacatt gctgttcgac agcattcccg aggaagaaga agccgaaacc 1500
   gaactgaagg catccgccat gctctccgcc atccgcgacg ccaagacggg caactccgcc 1560
   agcaccgagc gcaccaccgc gcgggtcggc gacggcgtca acatcctgct cgtcgaccac 1620
   gaggactctt tcgtccacac gctggccaac tacttccgcc agaccggcgc caatgtctcg 1680
   accgtgcgca cgccggtgcc ggacgaagtg ttcgagcggc tgaagccgga ccttgtcgtg 1740
   ctctcacccg gaccgggtac gccgaaggat ttcgattgcg ccgcgaccat cagacgagcg 1800
   cgcgcccgcg acctgccgat cttcggcgtc tgcctaggcc tgcaggcgct ggccgaggcc 1860
   tatggcgggg aactgcgcca gctgcatatt cccatgcacg gcaagccctc gcgcatccgc 1920
   gtctccaagc ccggcatcat cttctccggc ctgcccaagg aagtcactgt cggccgttac 1980
   cactcgatct tcgccgatcc ggtgcgcttg cccgatgatt tcattgtcac ggcagagact 2040
   gaggacggca tcatcatggc tttcgagcac cgcaaggagc cgatcgcggc ggtgcagttc 2100
   cacccggaat cgatcatgac gctcggccac aatgccggca tgcgcatcat cgagaacatc 2160
   gtcgcccatt tgccgcgcaa ggccaaggaa aaggcagcct ga 2202
<210> 122
   <211> 2199
   <212> DNA
   <213> Azospirillum brasilense
<400> 122
   atgtaccccg ccgaccttct tgcctcgccc gacctcctcg aaccgctgcg tttccagacg     60
   cgcggcggcg tcaccgtcac gcggcgggcg acggcgctcg acccgcggac cgccctcgac    120
   ccggtgatcg acgcgctgga ccgccgccgc ggcctgctgc tgtccagcgg ggtggaggcg    180
   ccgggccgct accgccgtca cgcgctgggc ttcaccgacc ccgcggtggc gctcacggcg    240
   cgtgggcgga cgctgcgcat cgacgcgctg aacggcgggg ggcaagtgct gctgcccgcc    300
   gtcgccgagg ccctgcgtgg cctggaggcc ctggccggtc tagaggaggc gccgtcgcgg    360
   gtcactgcct cgtccgcaag cccagcaccc cttcccggag aggagcggag ccgccagccc    420
   tccgttttct cggtcctgcg ggcggtgctg gatctgtttg ccgcccccga cgacccgttg    480
   ctcgggctct acggggcctt cgcctacgac ctcgccttcc agttcgagcc gatccgccag    540
   cggttggagc ggcccgacga ccagcgcgat ctgctgctct acctgccgga ccggctcgtc    600
   gcgctggacc ccatcgcagg actcgcccgg ctcgtcgcgt atgagttcat cacggcggcg    660
   ggcagcaccg aggggctgga gtgcggcggg cgcgaccacc cctaccgtcc cgacaccaac    720
   gccgaggccg gctgcgacca cgcgcccggt gactatcagc gcgtcgtcga gagcgccaag    780
   gccgccttcc gccgcggcga cctgttcgag gtggtgcccg gccagacctt cgccgagccc    840
   tgcgccgacg cgccttcgtc ggtgttccgg cggctgcgcg ccgccaaccc ggcgccttac    900
   gaggccttcg tcaacctcgg gcggggcgag ttcctcgtcg ccgccagccc ggagatgtat    960
   gtgcgggtgg cgggcgggcg ggtggaaacc tgcccgatct ccggcaccgt ggcgcgcggg   1020
   gccgacgcgc tgggcgacgc cgcgcaggtc ctgcgcctgc tgacctcggc caaggacgcg   1080
   gcggagctga ccatgtgcac cgacgtggac cgcaacgaca aggcgcgggt gtgcgagccg   1140
   ggatccgtcc gggtgatcgg gcggcggatg atcgagctgt actcccgtct gatccacacg   1200
   gtggaccatg tggagggacg gctgcggtcc ggaatggacg cgctggacgc cttcctcacc   1260
   cacagctggg cggtgacggt gaccggcgcg cccaagcgct gggccatgca gttcctggag   1320
   gatacggagc aatcgccgcg ccgctggtac ggcggggcct tcggccggct gggcttcgac   1380
   ggcgggatgg acaccggcct gaccctgcgc accatccgca tggccgaggg cgttgcctac   1440
   gtgcgggcgg gggcgacgct gctgtccgac agcgatccgg acgcggagga cgcggagtgc   1500
   cgcctgaagg ccgccgcctt ccgcgacgcc atccgcggga cggcggcggg tgcggcgccc   1560
   acgctgccgg cggctccccg tggcggggag ggcaggcggg tgctgctggt ggatcacgac   1620
   gacagcttcg tccacacgct ggccgactat ctgcgccaga cgggcgcttc ggtgacgacg   1680
   ctgcgtcaca gtcacgcacg ggcggcgctg gcggagcgga ggccggatct ggtcgtgctg 1740
   tcccccggtc cggggcgccc ggcggatttc gacgtggcgg gcaccatcga cgcggcgctg 1800
   gcgctcggcc tgccggtgtt cggcgtctgc ctgggcctgc aagggatggt ggagcgcttc 1860
   ggcggcgcgc tggacgtgct gccggagccc gtccacggca aggcgacgga ggtccgggtg 1920
   ctgggcggcg cgctgttcgc cggcctgccg gagcggctga cggtcgggcg ctaccactct 1980
   ctggtggccc ggcgcgaccg gctgccggcg gacctcacgg tgaccgcgga gaccgccgac 2040
   ggtctggtga tggcggtcga gcaccggcgg cttccgctcg ccgccgtgca gttccacccc 2100
   gagtcgatcc tgtcgctcga cggtggggcc ggtcttgccc tgctgggcaa cgtgatggac 2160
   cggctggccg ccggcgccct gacggacgct gcggcttga 2199
<210> 123
   <211> 2196
   <212> DNA
   <213> Brucella melitensis
<400> 123
   atgaatgcga agactgcgga tagcgagata ttccagcacg aaacggcagg cggtatcatc    60
   gtcgagcggg tgcgccacct cacggcctat aagggcgcca ttgaaagcta tatcgatgtg   120
   ctgaacgaat ggcgcggtgc ggtgttctcg tccaattacg aatatccggg ccgctatacg   180
   cgatgggata ccgcaattgt cgatccgccg gtcgtcatca cgtcgcgtgc ccgcacgatg   240
   cgcatcgagg cgctgaacgc gcgcggcgtc atcctgcttc ggcccattct ggataccgtc   300
   aaggcgcttt cggaagtaaa gatcgaccag tccggcgaaa accgtatcga tctgacgatt   360
   gtggaaccgg tcggcacctt cacggaagaa gaacgctcgc gcatgccctc ggtctttacg   420
   gtgctgcgcg ccatagtcgg gcttttcttc tcggaggagg atgccaatct cggcctttat   480
   ggcgcctttg gctatgatct ggcgttccag ttcgatccca tccagtacaa gctgaagcgc   540
   ccggacgacc agcgtgacct cgtgctgttc attcccgacg aaatcttcgt cgccgaccat   600
   tatgcggcgc gcgcctgggt ggaccgttat gaatttcgct gcggcggttc gtccacgcac   660
   ggtcttgatc gcgcgacgcc ggtggtgcct ttcaagccat cggagcgcaa gcttgcgcgc   720
   ggcgatcata atccgggtga atatgccagg cttgtcgagc gcgccaagga aagcttcaag   780
   cgcggcgacc tgttcgaggt tgtgccgggc cagaccttct atgagcgctg ccacacggcg   840
   ccgtcggaga ttttccgccg gctgaagtcg atcaatcctt cgccctattc ctttttcatc   900
   aatctgggcg agagcgaata tctggtcggc gcatcgccgg aaatgtttgt gcgcgtcaat   960
   gggcggcgca tcgagacctg cccgatttcc ggcaccatca agcgcggtga agatgcaatt  1020
   tcggattctg agcagatatt gaaactgctt aattccaaga aggacgaatc cgagctgacc  1080
   atgtgttcgg atgtggaccg caacgacaag agccgcgttt gcgagccggg ttcggtgcgt  1140
   gttatcggtc gccgccagat cgagatgtat tcccgcctga tccatacggt cgatcatatc  1200
   gaaggccgcc tgcgtgacgg catggatgcg tttgacggct tcctcagcca tgcatgggct  1260
   gtgacggtga caggcgcgcc gaagctgtgg gcaatgcgct ttcttgagga aaacgaacgc 1320
   agcccgcgcg catggtatgg cggcgcgatc ggcatgatgc atttcaatgg cgatatgaat 1380
   acagggctga cgctgcgcac catccgcatc aaggatggtg tggcggaaat ccgtgcaggg 1440
   gcgacgcttc tgttcgattc caaccctgac gaggaagaag ccgagaccga attgaaggca 1500
   tcggccatga ttgcggctgt gcgggacgca cagaagagca atcagatcgc ggaagaaagt 1560
   gtggcggcaa aggtgggtga gggggtttcg atcctgctgg tcgatcacga ggattccttc 1620
   gtccatacgc ttgccaatta tttccgccag acgggcgcca aggtttccac cgtgcgttca 1680
   ccggtggcag aggagatatt cgaccgcgtc aatcccgatc tggtggtgtt atcgccggga 1740
   ccgggctcgc cgcaggattt cgattgcaag gcgaccatcg ataaggcgcg caagcgccag 1800
   cttccgattt ttggcgtctg cctcggcctt caggccctgg cggaagccta tggcggggcg 1860
   ttgcgccagc ttcgcgttcc ggtgcatggc aagccttcac gcatccgcgt atcaaagccg 1920
   gagcgcattt tctccggctt gccggaggaa gtgacggtgg ggcgttatca ttcgatcttc 1980
   gccgatcctg aacgcctgcc ggatgatttt ctcgtcacag ccgaaacgga agacgggatc 2040
   atcatggctt ttgaacataa acatgaaccg gtggcagccg ttcaattcca tcccgaatcc 2100
   atcatgacgc ttggccataa tgccggtatg cgcatgatcg agaatatcgt gacgcatctt 2160
   gcaggcaagc acaaggcgcg ccgcaccaac tattga 2196
<210> 124
   <211> 2148
   <212> DNA
   <213> Nostoc sp.
<400> 124
   gtgcgcgtat ctcgtagcac aaccgaggtg aagatggaca ctgcactaga tgaaattctc    60
   tttcacctaa atcaagtacg tggaggtttg ttaaccagta gttacgaata tccagggcga   120
   tacaaaagat gggcgattgg attcattaat cccccattac aactgacaac aagagagaac   180
   gcatttacca tctcttcact caatcctcgc ggacaggtgc tactaccaac cttgttccag   240
   catctatcag cccagtcgca actacaacaa atcagcctca atcatgacta catcacaggt   300
   gaaattcgac ccacaaaaca gttattcaca gaagaacaac ggagtaaaca accgtcagcc   360
   tttacagtca tccgcgaaat tctccagatt tttgcgagtg atgaagacga gcatttaggg   420
   ttatatggtg catttggtta cgacttagta tttcaatttg aaccaattcc ccaaaaaatt   480
   gctcgtcccg cagaccaacg ggatttagtc ctgtatctac ccgatgaact catagttgta   540
   gattactatc tacaaaaagc atatcgtcac cagtatgaat ttgccacaga acatggcaac   600
   accgagcatc ttccacggac aggccagtcc atcgactacc agggtaaaca tcttctacca   660
   aaccaaactg ctgaccatca accaggagaa tatgccaacc tagttgagca agcactcgac   720
   tacttccgcc ggggtgactt atttgaagta gttcctagtc aaaacttttt tacagcctgt   780
   gaacaatcac ccagtcaact attccagacc ttaaggcaaa ttaatcctag tccttatgga   840
   tttctgttga atttgggtgg tgaatatctc ataggtgcat caccagaaat gtttgtgcga   900
   gttgatggta ggcgagtgga aacctgtccc attagtggca ctattagacg gggagaagat 960
   gctttaggcg acgctgtaca aattcgtcag ttgcttaact cccataaaga tgaagccgag 1020
   ttaacaatgt gtactgacgt agaccgcaac gataaatcgc ggatttgtga acccggttca 1080
   gtcagggtga ttggtcgtcg ccagattgaa ctgtacagcc acctcattca tacagtagac 1140
   catgtagaag ggatactgag gccggaattt gacgctttag atgccttctt gagtcatact 1200
   tgggcagtta cagtcacagg cgcacccaaa cgagccgcca tgcagttcat cgaacagcat 1260
   gaacgcagcg cccgtcgttg gtatggggga gcagttggtt atttaggctt taatggtaac 1320
   ttgaataccg gattaacctt gcggacaatt cgtttacaag actccatcgc cgaagtgcga 1380
   gttggtgcaa cagtccttta cgactccatt ccgtcagccg aagaagagga aacaattact 1440
   aaagcgactg cattatttga gaccattcgc cgtcatacca ctgccaataa aactcaagga 1500
   aacgatagtc atcgccctgg ggatatcgcc cacaataagc gtatcctcct catcgactac 1560
   gaagattcat ttgttcacac attagccaat tacatccgca ccaccggcgc aaccgtcacc 1620
   accctacgtc atggttttgc tgaatcatat tttgatgcag aacgcccaga cttagtggta 1680
   ttgtctcccg gccctggtag acccagtgac ttccgagttc cccaaacggt tgcagccttg 1740
   gtaggtcgag aaatccccat ttttggcgtt tgtctgggat tacaaggcat agtggaagct 1800
   tttggcggag aattaggcgt gcttgattat ccccaacacg gtaaacccgc acggatttca 1860
   gtgactgcac ctgattctgt gctgtttcaa aatttaccag catccttcat cgtgggtaga 1920
   taccattcct tatttgccca accccaaact atacccggtg aactcaaagt cacagcgatt 1980
   tctgaggaca atgtaattat ggcaattgaa caccaaacac tacctatagc cgccgtccaa 2040
   tttcatccag agtcaatcat gaccctagca ggagaagttg gtcagacaat cattaaaaat 2100
   gtggtgcaga catataccca aactttagaa acatcaattt actcttag 2148
<210> 125
   <211> 2208
   <212> DNA
   <213> Nostoc sp.
<400> 125
   atgattgccg attcccacag ctacagaact aatggtaacg tgcgtgtctc tcgctccatc     60
   acacaagtta aaatggagac agctttagaa gagattcttt tctacttaaa ctctcagcgt    120
   ggtggattgc tgactagtag ctatgaatat ccaggaagat ataaaagatg ggcaattggt    180
   tttgttaatc cacctgtaga attatccaca agcggaaata cttttactct cacagcatta    240
   aatgagcgtg gctatgtact tttaccagtg atctttgagt gtttatcaaa atcagaacaa    300
   ctccagaaac tcactgaaca tcatcataaa attactggat tagttaaatc tacaccagaa    360
   ttttttgccg aagaagaacg tagtaaacaa ccttctacat ttacggttat tcgggaaata     420
   ctacatatct tctctagtca agaagacgaa catttaggat tatatggtgc gtttggttat    480
   gacttagttt tccaatttga gcaaataacc caatgcttag aacgtccaca agaccaacga    540
   gatttagttc tatatttacc cgacgaattg atagttgtag actactatca acaacaagca 600
   tttcggctag agtatgactt catcacagcg catggtagta cttatgattt gccccgcacg 660
   ggagaatctg ttgattatcg aggtcaatgt ttaacacctc ctcaaaatgc tgaccataaa 720
   ataggtgagt atgccaaact agtagaattt gcccttgatt atttccgtcg gggtgactta 780
   tttgaagtgg ttcccagtca gaattttttc acagcttgcg aagcaccacc aagccaacta 840
   tttgaaactt taaaacaaat aaatcctagt ccctatggat ttatttttaa tcttggtgga 900
   gaatacatca ttggcgcttc accagaaatg tttgtacggg tggaaggtag gcgtgtagaa 960
   acttgtccta ttagtggcac tattactaga gggcatgatg ctatagatga tgctgtgcag 1020
   attcgtcagt tactcaactc ccacaaagac gaagcagagt tgactatgtg tactgacgta 1080
   gaccgtaacg ataagtctcg catctgtgaa cccggttcag tcaaggtgat tggtcgccgg 1140
   caaattgaat tatatagcca cttaattcat acggtagacc atgtagaagg cattctccga 1200
   ccagagtttg atgctttaga tgctttcctc agtcacactt gggctgttac agtcacaggc 1260
   gcaccgaaaa gggctgctat tcaattcatc gaaaagaacg aacgcagcgt cagacgttgg 1320
   tatggtggcg cggttgggta tttgaatttt aacgggaatt taaatactgg gttaatttta 1380
   aggacaatca gattgcaaga ctcaattgct gaagtgcgag ttggtgctac tctactgtat 1440
   gactccatac cccaagcaga agaacaagaa accatcacta aagctgcggc tgcttttgaa 1500
   acgattcggc gtgctaaaca aatagaccca cagattgaag aatctagtac tagaaagtta 1560
   agcaaatatc ttcccgatgg acaatcaggt aaacacatct tactaattga ccatgaagac 1620
   tcatttgttc ataccctagc taactacatc cgttccactg gtgcaactgt caccacactg 1680
   cgtcacggct tctcagaatc cctatttgat acagaacgcc cagacttagt agtattatct 1740
   cctggccccg gtagaccgag tgaatttaaa gtacaggaaa ccgtcgccgc ctgcgtccgt 1800
   cgccaaatac ctctgtttgg tgtctgttta ggactgcaag gtattgtgga agctttcggt 1860
   ggagaattgg gagtattgaa ttatccccaa catggtaaat cttcgcggat ttttgttaca 1920
   gcacccgatt ctgtgatgtt tcaagatttg cccgaatctt ttacagtcgg gagatatcat 1980
   tcattatttg cactatcaca acgcttacca aaagaactga aggtgacagc gatttctgat 2040
   gatgaggtga ttatggcgat tgaacatcag acactaccta tcgccgccgt ccagtttcat 2100
   ccagaatcaa tcatgactct agctggagaa gttggtttaa tgatgatcaa aaatgtggtg 2160
   caaaaatata cacaaagtca acagtcaaca gttcccatct atgactaa 2208
<210> 126
   <211> 2160
   <212> DNA
   <213> Rhodopseudomonas palustris
<400> 126
   atgaacagga ccgttttctc gcttcccgcg accagcgact ataagaccgc cgcgggcctc 60
   gcggtgacgc gcagcgccca gccttttgcc ggcggccagg cgctcgacga gctgatcgat 120
   ctgctcgacc accgccgcgg cgtgatgctg tcgtccggca caaccgtgcc gggccgctac 180
   gagagcttcg acctcggctt tgccgatccg ccgctggcgc tcaccactag ggccgaaaaa 240
   ttcaccatcg aggcgctcaa tccgcgcggc cgggtgctga tcgcgttcct gtccgacaag 300
   cttgaagagc cctgcgtggt ggtggagcag gcctgcgcca ccaagatcag gggccacatc 360
   gtccgcggcg aggccccggt cgacgaagaa caacgcaccc gccgcgccag cgcgatctcc 420
   ctggtgcgcg cggtgattgc tgccttcgcc tcgccggccg atccgatgct cgggctgtac 480
   ggcgccttcg cctacgacct tgtgttccag ttcgaggatc tgaagcagaa gcgtgcccgc 540
   gaagccgacc agcgcgacat cgtgctgtac gtgccggatc gcctgctggc ctacgatcgc 600
   gccaccggcc gcggcgtcga catttcctac gaattcgcct ggaagggcca gtccaccgcc 660
   ggcctgccga acgagaccgc cgagagcgtc tacacccaga ccggccggca gggtttcgcc 720
   gaccacgccc cgggcgacta tcccaaggtg gtcgagaagg cccgcgcggc gttcgcccgc 780
   ggcgacctgt tcgaggcggt gccgggccag ctgttcggcg agccatgcga gcggtcgccg 840
   gccgaagtgt tcaagcggtt gtgccggatc aacccgtcgc cctatggcgg cctgctcaat 900
   ctcggcgacg gcgaattcct ggtgtcggcc tcgccggaaa tgttcgtccg ctcggacggc 960
   cgccggatcg agacctgccc gatctccggc actatcgccc gcggcgtcga tgcgatcagc 1020
   gatgctgagc agatccagaa gctcttgaac tccgagaagg acgagttcga gctgaatatg 1080
   tgcaccgacg tcgaccgcaa cgacaaggcg cgggtctgcg tgccgggcac gatcaaagtt 1140
   ctcgcgcgcc gccagatcga gacctattcg aagctgttcc acaccgtcga tcacgtcgag 1200
   ggcatgctgc gaccgggttt cgacgcgctc gacgccttcc tcacccacgc ctgggcggtc 1260
   accgtcaccg gcgcgccgaa gctgtgggcg atgcagttcg tcgaggatca cgagcgtagc 1320
   ccgcggcgct ggtatgccgg cgcgttcggc gtggtcggct tcgatggctc gatcaacacc 1380
   ggcctcacca tccgcaccat ccggatgaag gacggcctcg ccgaagttcg cgtcggcgcc 1440
   acctgcctgt tcgacagcaa tccggtcgcc gaggacaagg aatgccaggt caaggccgcg 1500
   gcactgttcc aggcgctgcg cggcgatccc gccaagccgc tgtcggcggt ggcgccggac 1560
   gccactggct cgggcaagaa ggtgctgctg gtcgaccacg acgacagctt cgtgcacatg 1620
   ctggcggact atttcaggca ggtcggcgcc caggtcaccg tggtgcgcta cgttcacggc 1680
   ctgaagatgc tggccgaaaa cagctatgat cttctggtgc tgtcgcccgg tcccggccgg 1740
   ccggaggact tcaagatcaa ggatacgatc gacgccgcgc tcgccaagaa gctgccgatc 1800
   ttcggcgtct gcctcggcgt ccaggcgatg ggcgaatatt ttggcggtac gctcggccag 1860
   ctcgcgcagc cggctcacgg ccgcccgtcg cggattcagg tgcgcggcgg cgcgctgatg 1920
   cgcggtctcc cgaacgaggt caccatcggc cgctaccact cgctctatgt cgacatgcgc 1980
   gacatgccga aggagctgac cgtcaccgcc tccaccgatg acggcatcgc gatggcgatc 2040
   gagcacaaga ccctgccggt cggcggcgtg cagttccacc ccgagtcgct gatgtcgctc 2100
   ggcggcgagg tcgggctgcg gatcgtcgaa aacgccttcc ggctcggcca ggcggcctaa 2160
<210> 127
   <211> 2166
   <212> DNA
   <213> Bradyrhizobium japonicum
<400> 127
   atgaacagga cagtctttgc cctcccggcc agaagcgatt acgtgacccg cggcggtctc 60
   gcgatcacgc gcgtggcgga gcagtttacc ggcggcgcga gccggctcga cgatctcgtc 120
   aacctgctcg accgccgccg cggcgtggtg ctgtcctcgg gcacgaccgt gccgggccgc 180
   tacgagagct tcgacctcgg cttctccgat ccgccgctca agctcgagac cacaggcgtc 240
   aatttcaagc tggaagccct gaacgagcgc ggccaggtgc tgatcgcctt ccttgccgat 300
   gtcctgcgcg agccctgcgt ggtgatatcc gaaaagaccg cttcgcgcct cgccggccac 360
   atcatccgcg gcgatgcccc ggtcgaggaa gaccagcgca cccggcgcgc cagcgtggtg 420
   tcgctggtgc gcgacctcgt cgccgccttc tccgccaatg acgacgggct gctcggcctg 480
   ttcggcgcct tcgcctacga tctcgtgttc cagatcgagg atctcgtgca gaagcgcgcg 540
   cgcgagagcg accagcgcga catcgtgctc tacgttcccg atcgcctgct ggcctatgac 600
   cgcgccaccg gccgcggcgt cgtgctcagc tacgacttca cgtggaaggg cagatccacc 660
   gagggcctgc cgcgcgagac cgccgacagc ccgtacatga agacaccgcg ccagggcttt 720
   gccgatcatg cgcccggcga ataccaggcc accgtcgaga ccgcgcgcgc ggcctttgcc 780
   cgcggcgatc tgttcgaggc cgtgccgggc cagctgttcg ccgagccctg cgatcgttct 840
   ccggcggaag tgttccagcg cctctgtgtc atcaacccgt cgccctacgg cgcgctgatg 900
   aatctcggcg acggcgagtt tctcgtctcc gcctcgcccg agatgttcgt gcgttcggac 960
   ggccgccgcg tcgagacctg cccgatctcg ggcaccatcg cgcgcggcac cgatgcgatc 1020
   ggcgatgccg agcagatccg ccagctcctg aattcggaga aggacgagtt cgagctcaac 1080
   atgtgcaccg acgttgatcg caacgacaag gcgcgcgtct gcgtccccgg caccatcaag 1140
   gtgctggcgc gccggcagat cgagacctac tcaaaactgt tccacaccgt cgaccacgtc 1200
   gagggcatgc tgcgtcccgg cttcgacgcg ctcgatgcct tcctcaccca tgcctgggcc 1260
   gtcaccgtga caggtgcgcc gaagctctgg gcgatgcagt tcgtcgagga tcacgagcgg 1320
   tcgccgcggc gctggtatgc gggtgcgatc ggcgcggtga atttcgacgg cagcatcaat 1380
   accggcctca ccatccgcac catccgcatg aaggatggtc tcgccgaggt gcgcgtcggc 1440
   gccacctgcc tgttcgattc cgatcccgct gccgaggacc gcgaatgcca ggtcaaggcg 1500
   gcggcgctgt tccaggcgct gcgcggcgat ccgccaaaac cgctctcgac ctttgcgccc 1560
   gatgcgaccg gaagcggcaa gcgggtgctg ctgatcgacc acgacgacag cttcgtgcac 1620
   atgctcgccg actatttccg ccaggtcggc gccagcgtca ccgtggtccg ctatgtgcat 1680
   gcgctcgaca tgctcaagca gaagaggtgg gatttgctgg tgctgtcgcc cggcccgggc 1740
   aggcccgaag atttcgggat caggaagacg atcgatgcgg cgctggagaa caagctgccg 1800
   gtgttcggcg tctgcctcgg cgtgcaggcg atcggcgaat attttggcgg cgagctcggc 1860
   cagctcacgc atcccgccca cggccggccc tcgcgggtgc aggtgcgcgg cggccgcctg   1920
   atgcgcaatt tgccgagcga gatcgtgatc ggccgctatc actcgctcta tgtcgagcgc   1980
   gacagcatgc cggaggtttt gtccgtcacc gccagcaccg aggacggcgt cgctatggcg   2040
   ctggagcaca agaccctgcc ggtcgcgggc gtgcaattcc acccggagtc gctgatgtcg   2100
   ctcggcggcg aggtggcttt gaggattgtc gagaacgcgt tccggctgga tgcgcgtgtt   2160
   gattga                                                              2166
<210> 128
   <211> 2187
   <212> DNA
   <213> Rhodospirillum rubrum
<400> 128
   atgcaaacat cgaccttcac caccgccggc ggttttacca tcagcagttg cctacgcccg      60
   cttgccgggg cggatggcgc cttcgaggcg ctgatcgacc gactcgatcg ccatcgcggc     120
   atggtgatcg cctcgaccta tgagtatccc ggccgctatc gccgccacgc cctggggttt     180
   tgcgatccgc cgctggtgct ggagggcaag gggcgcgagg cccatctgct ggcgctcaat     240
   cgccgggggc gggccctgct gccggccctg gccgttggct tggagggggc ggccgggctg     300
   gagggcctgc gccgcgaggc cgaccggctg gtcttgcgtg tcaaggccat ggcgccctgg     360
   ttccccgagg aggagcgctc gcgccaaccc tcgctgatgt cggtggtgcg cgccctcgcc     420
   gggctgttcg ccgccgaaaa cgacccgttt ttcgggcttg tcggcgcctt tggctatgac     480
   ctgggcttgg ctttcgagcg cctgccccat gcccggccgc gatcggccga tcaccgcgat     540
   ctggtgcttt acctgcccga ccgtctgctg atcgacgatc ccgaagccgg cggccttgcc     600
   gaacgccttt acgacatcac cgcggccgat ggggcaagca ccgccggctt ggcgcgggaa     660
   accgccgctt acaccgccga ccaccccgcc ggcggcgtgc cgatcgagga tgatatgccc     720
   ccgggcgctt acggggcgat cgtccgtggg ctgaaggagg ccttcgccgc cggggatctg     780
   ttcgaggcgg tgccctcgcg cgccctgcgc cggccttgcg ccgaggcgcc gagccgtttg     840
   taccggcggc tgcgcgcggc caatccggcg ccctatctgt tcctggccaa tctgggggcg     900
   ggcgaacatc tgatcggcgc ctcgcccgaa atgttcgtgc gcgtcggtgg agcgccgggg     960
   gcgcggcggg tggaaacctg cccgatctcg ggcaccatcg cccgggggcc cgacgccctg    1020
   ggcgatgccg aggccatccg caccctgctc aattcgacca aggacgaggc cgaattgacc    1080
   atgtgcaccg atgtcgaccg caacgacaag gcgcgggtct gcgtggccgg cagcgttacc    1140
   gtcatcggtc gccgccagat cgagctatat tcccggctga tccacacggt cgaccatgtg    1200
   gagggccggc tgcgccccga gcttgacgcc cttgacgcct ttctcagcca ctgctgggcg    1260
   gtgacggtga ccggagcgcc caagcgcgcc gccatggccg ccgtcgaagc cgtggagcgg    1320
   gcgccgcgcg cctggtatgg cggcgccatc ggccgcctgg gcttcgacgg taccctcgac    1380
   accggactgg tgctgcgcac catccgcctg cgtcacggcg tcgccgaggt gcgggtgggg    1440
   gcgacgctgc tccaccgttc cgatcccgag gaggaggagg ccgaaaccct gctcaaggcc    1500
   tcggccctgc tcgccctgct cgatgccacc accccggcca agccgaatgc cccgcatctt 1560
   ccgttgcgcg gccgggcgcc gcgcgtgctg gtcatcgacc acgaggacag cttcgtccat 1620
   accctggcgt cttatctgcg caatgccggg gccgagacca ccgtgctgcg ctgggacgtg 1680
   ccggcggcgg tgcgcgccgg cgtcgaggcc gatctgctgg tgctgtcgcc gggaccgggc 1740
   acgccgtcgc gcttcgccct gggggccagc ctggactggg cggtggcgcg cggcttgccg 1800
   gtcttcggcg tctgcctggg gctgcagggc atcgtcgaac aggccggggg ccgccttgcc 1860
   cggctggcgg ttcccgccca tggcatggcc tcgacgctgc ggctggtcgc ccccggggac 1920
   ccgctgttcg ccggcctgcc cacgaccatg agagtgggcc gctaccacag cctgcacgcc 1980
   gagcgcgcca gcctgcccga cagcctggag atcctggccg aaagcgacga cggggtgatc 2040
   atggcgctgc gccaccgcct gctgcccttc agcgccgtgc aattccaccc cgaaagcctg 2100
   atgaccctgg acggcggcgc cggaccccga ttgatcgcca atcttctgga aaccctaagc 2160
   gtcccgcgaa cgcgccacgc cgcctaa 2187
<210> 129
   <211> 2181
   <212> DNA
   <213> Thermobifida fusca
<400> 129
   gtggacgaca actcctacac caccagtggt ggtatcaccg tgcaccgcac ggcagtgccg     60
   tgcgatcccc gtgcgctcgc cgatctgacg gtcaacgtgg aacagcgccg aggcggcgtg    120
   ctctcctccg ggatggagta tcccggccgc tacagccgtt ggcatttggg atacgtggac    180
   ccgtgcctgg aagttgtcgc gcgcggccgc acgatcgggg cgacggccct caacgaccgc    240
   ggccgtgtcc tgctgcccgc ggtagcccgg gcgctcaccg cgcacggcgc ggtggtggag    300
   cacaccgacg acacggtcgc tgtcacggtc cctgaaccgg atcccaccga gttcttcact    360
   gaggaagagc gcagccgccg gctcagtgtt ttttctgcgc tccgcgccct cgtgggcgtg    420
   ttcttccacg cggaagaacc ccacctgggg ttgtacgggg cgttcggcta cgatctggcg    480
   ttccagttcg aaccgatcga gcaggtgctg ccgcgcgacc cggaggaccg ggacctggtg    540
   ttgcacctgc ccgacgagat catcgtccac gaccggaaac gggagatctg ccagcgctac    600
   tcctacgact tcacgctgcc ggaggagttg cgcggcccgg caggggcgac cacgcggggc    660
   ctgccacgcc acaccgagcc cacacccccg gtgaccccgg ctgccgaggt gccgccgcag    720
   ccggaacccg ggtcgtatgc gcggatcgtg gctgaggcca aggagcggtt ccgccgcggc    780
   gacttgttcg aagtggtgcc cagccaccgc ttgtacgcgc cgtgtgcgtc gcctgcgcgg    840
   ttctacgagc ggctgcggga acgcaaccct gccccgtacg agttcttcct caacctgggg    900
   gagggcgagt acctggtcgg cgcgtccccg gaaatgtttg tgcgggtcac gggccgtccc    960
   ggagaggggc agcgggtgga gacctgcccg atctccggca cgatcaagcg tggcgcggac   1020
   gcggtcggcg acgcggagaa catcaaggag ctgctgtcgt ccgcgaagga agagtcggag   1080
   ctgaccatgt gcaccgacgt ggaccgcaac gacaagtccc gggtgtgcgt gccaggcagt 1140
   gtgcgggtga tcggccgccg gcagatcgaa atgtacagtc ggctcatcca cacggtcgac 1200
   cacattgagg ggattctgcg ccccgagttg gacgctattg acgcgttcct cacccacatg 1260
   tgggcggtga ccgtgacggg ggcgccgaag acgtgggcga tgcggttcat cgaacagcat 1320
   gagagttcgc cgcgccgctg gtatgggggc gcggtcggtg tcatcaattt tgatggttcg 1380
   atgaacaccg ggttgacgtt gcggaccgcg cacatccggg acggggtggc gacggtgcgg 1440
   gcgggcgcca cactgctgta cgactctgat ccggaagctg aagagcggga gactttcctc 1500
   aaagcccgtg ccctgttgga gaccctcacc gacgagggtg aggaaacctc caaggctgcg 1560
   cctgcggtgg agcaggtggg ggcggggatg cgggtgctgc tcgtcgacca cgaagactcg 1620
   ttcgtcaaca cgctcgcgga ctacgtccgg cggcacggcg ccgaggtcac cacggtgcgc 1680
   tacgggttcg acccggccct gctcgaccag atgcgtcccg acctggtggt gctctccccg 1740
   gggccggggc tgcccgccga tttcgcgatg agcgcgctgt tgaaggagtt ggacgcgcgc 1800
   ggcctgcctg tgttcggggt gtgcctgggg ctgcaggcga tggtggagta cgcgggcggg 1860
   gagctgctca ctttggacac gccggtgcac ggtaaacccg gccgggttcg ggtcaccggg 1920
   ggcgcgctgc tggctgggct gggagaggac ggggagttca ccgcggcccg ctaccactcc 1980
   gtgtacgcga ccccggaccg ggtgaaaggg ttcgaggtga cggcggtgac ggaggacgac 2040
   ggtttccccg tggtcatggc gatcgagaat gctgaggcgc ggcggtgggc ggtccagttc 2100
   caccccgagt cgatcctcac cggccgggtg ggggagcaga tcgtggcgaa cgtgctgcgc 2160
   ttggccaggg agagcagctg a 2181
<210> 130
   <211> 721
   <212> PRT
   <213> Bradyrhizobium japonicum
<400> 130
   Met Asn Arg Thr Val Phe Ala Leu Pro Ala Arg Ser Asp Tyr Val Thr
   1               5                   10                  15
   Arg Gly Gly Leu Ala Ile Thr Arg Val Ala Glu Gln Phe Thr Gly Gly
               20                  25                  30
   Ala Ser Arg Leu Asp Asp Leu Val Asn Leu Leu Asp Arg Arg Arg Gly
           35                  40                  45
   Val Val Leu Ser Ser Gly Thr Thr Val Pro Gly Arg Tyr Glu Ser Phe
       50                  55                  60
   Asp Leu Gly Phe Ser Asp Pro Pro Leu Lys Leu Glu Thr Thr Gly Val
   65                  70                  75                  80
   Asn Phe Lys Leu Glu Ala Leu Asn Glu Arg Gly Gln Val Leu Ile Ala
                   85                  90                  95
   Phe Leu Ala Asp Val Leu Arg Glu Pro Cys Val Val Ile Ser Glu Lys
   100 105 110
   Thr Ala Ser Arg Leu Ala Gly His Ile Ile Arg Gly Asp Ala Pro Val
   115 120 125
   Glu Glu Asp Gln Arg Thr Arg Arg Ala Ser Val Val Ser Leu Val Arg
   130 135 140
   Asp Leu Val Ala Ala Phe Ser Ala Asn Asp Asp Gly Leu Leu Gly Leu
   145 150 155 160
   Phe Gly Ala Phe Ala Tyr Asp Leu Val Phe Gln Ile Glu Asp Leu Val
   165 170 175
   Gln Lys Arg Ala Arg Glu Ser Asp Gln Arg Asp Ile Val Leu Tyr Val
   180 185 190
   Pro Asp Arg Leu Leu Ala Tyr Asp Arg Ala Thr Gly Arg Gly Val Val
   195 200 205
   Leu Ser Tyr Asp Phe Thr Trp Lys Gly Arg Ser Thr Glu Gly Leu Pro
   210 215 220
   Arg Glu Thr Ala Asp Ser Pro Tyr Met Lys Thr Pro Arg Gln Gly Phe
   225 230 235 240
   Ala Asp His Ala Pro Gly Glu Tyr Gln Ala Thr Val Glu Thr Ala Arg
   245 250 255
   Ala Ala Phe Ala Arg Gly Asp Leu Phe Glu Ala Val Pro Gly Gln Leu
   260 265 270
   Phe Ala Glu Pro Cys Asp Arg Ser Pro Ala Glu Val Phe Gln Arg Leu
   275 280 285
   Cys Val Ile Asn Pro Ser Pro Tyr Gly Ala Leu Met Asn Leu Gly Asp
   290 295 300
   Gly Glu Phe Leu Val Ser Ala Ser Pro Glu Met Phe Val Arg Ser Asp
   305 310 315 320
   Gly Arg Arg Val Glu Thr Cys Pro Ile Ser Gly Thr Ile Ala Arg Gly
   325 330 335
   Thr Asp Ala Ile Gly Asp Ala Glu Gln Ile Arg Gln Leu Leu Asn Ser
   340 345 350
   Glu Lys Asp Glu Phe Glu Leu Asn Met Cys Thr Asp Val Asp Arg Asn
   355 360 365
   Asp Lys Ala Arg Val Cys Val Pro Gly Thr Ile Lys Val Leu Ala Arg
   370 375 380
   Arg Gln Ile Glu Thr Tyr Ser Lys Leu Phe His Thr Val Asp His Val
   385 390 395 400
   Glu Gly Met Leu Arg Pro Gly Phe Asp Ala Leu Asp Ala Phe Leu Thr
   405 410 415
   His Ala Trp Ala Val Thr Val Thr Gly Ala Pro Lys Leu Trp Ala Met
   420 425 430
   Gln Phe Val Glu Asp His Glu Arg Ser Pro Arg Arg Trp Tyr Ala Gly
   435 440 445
   Ala Ile Gly Ala Val Asn Phe Asp Gly Ser Ile Asn Thr Gly Leu Thr
   450 455 460
   Ile Arg Thr Ile Arg Met Lys Asp Gly Leu Ala Glu Val Arg Val Gly
   465 470 475 480
   Ala Thr Cys Leu Phe Asp Ser Asp Pro Ala Ala Glu Asp Arg Glu Cys
   485 490 495
   Gln Val Lys Ala Ala Ala Leu Phe Gln Ala Leu Arg Gly Asp Pro Pro
   500 505 510
   Lys Pro Leu Ser Thr Phe Ala Pro Asp Ala Thr Gly Ser Gly Lys Arg
   515 520 525
   Val Leu Leu Ile Asp His Asp Asp Ser Phe Val His Met Leu Ala Asp
   530 535 540
   Tyr Phe Arg Gln Val Gly Ala Ser Val Thr Val Val Arg Tyr Val His
   545 550 555 560
   Ala Leu Asp Met Leu Lys Gln Lys Arg Trp Asp Leu Leu Val Leu Ser
   565 570 575
   Pro Gly Pro Gly Arg Pro Glu Asp Phe Gly Ile Arg Lys Thr Ile Asp
   580 585 590
   Ala Ala Leu Glu Asn Lys Leu Pro Val Phe Gly Val Cys Leu Gly Val
   595 600 605
   Gln Ala Ile Gly Glu Tyr Phe Gly Gly Glu Leu Gly Gln Leu Thr His
   610 615 620
   Pro Ala His Gly Arg Pro Ser Arg Val Gln Val Arg Gly Gly Arg Leu
   625 630 635 640
   Met Arg Asn Leu Pro Ser Glu Ile Val Ile Gly Arg Tyr His Ser Leu
   645 650 655
   Tyr Val Glu Arg Asp Ser Met Pro Glu Val Leu Ser Val Thr Ala Ser
   660 665 670
   Thr Glu Asp Gly Val Ala Met Ala Leu Glu His Lys Thr Leu Pro Val
   675 680 685
   Ala Gly Val Gln Phe His Pro Glu Ser Leu Met Ser Leu Gly Gly Glu
   690 695 700
   Val Gly Leu Arg Ile Val Glu Asn Ala Phe Arg Leu Asp Ala Arg Val
   705 710 715 720
   Asp
<210> 131
   <211> 728
   <212> PRT
   <213> Rhodospirillum rubrum
<400> 131
   Met Gln Thr Ser Thr Phe Thr Thr Ala Gly Gly Phe Thr Ile Ser Ser
   1 5 10 15
   Cys Leu Arg Pro Leu Ala Gly Ala Asp Gly Ala Phe Glu Ala Leu Ile
   20 25 30
   Asp Arg Leu Asp Arg His Arg Gly Met Val Ile Ala Ser Thr Tyr Glu
   35 40 45
   Tyr Pro Gly Arg Tyr Arg Arg His Ala Leu Gly Phe Cys Asp Pro Pro
   50 55 60
   Leu Val Leu Glu Gly Lys Gly Arg Glu Ala His Leu Leu Ala Leu Asn
   65 70 75 80
   Arg Arg Gly Arg Ala Leu Leu Pro Ala Leu Ala Val Gly Leu Glu Gly
   85 90 95
   Ala Ala Gly Leu Glu Gly Leu Arg Arg Glu Ala Asp Arg Leu Val Leu
   100 105 110
   Arg Val Lys Ala Met Ala Pro Trp Phe Pro Glu Glu Glu Arg Ser Arg
   115 120 125
   Gln Pro Ser Leu Met Ser Val Val Arg Ala Leu Ala Gly Leu Phe Ala
   130 135 140
   Ala Glu Asn Asp Pro Phe Phe Gly Leu Val Gly Ala Phe Gly Tyr Asp
   145                 150                 155                 160
   Leu Gly Leu Ala Phe Glu Arg Leu Pro His Ala Arg Pro Arg Ser Ala
                   165                 170                 175
   Asp His Arg Asp Leu Val Leu Tyr Leu Pro Asp Arg Leu Leu Ile Asp
               180                 185                 190
   Asp Pro Glu Ala Gly Gly Leu Ala Glu Arg Leu Tyr Asp Ile Thr Ala
           195                 200                 205
   Ala Asp Gly Ala Ser Thr Ala Gly Leu Ala Arg Glu Thr Ala Ala Tyr
       210                 215                 220
   Thr Ala Asp His Pro Ala Gly Gly Val Pro Ile Glu Asp Asp Met Pro
   225                 230                 235                 240
   Pro Gly Ala Tyr Gly Ala Ile Val Arg Gly Leu Lys Glu Ala Phe Ala
                   245                 250                 255
   Ala Gly Asp Leu Phe Glu Ala Val Pro Ser Arg Ala Leu Arg Arg Pro
               260                 265                 270
   Cys Ala Glu Ala Pro Ser Arg Leu Tyr Arg Arg Leu Arg Ala Ala Asn
           275                 280                 285
   Pro Ala Pro Tyr Leu Phe Leu Ala Asn Leu Gly Ala Gly Glu His Leu
       290                 295                 300
   Ile Gly Ala Ser Pro Glu Met Phe Val Arg Val Gly Gly Ala Pro Gly
   305                 310                 315                 320
   Ala Arg Arg Val Glu Thr Cys Pro Ile Ser Gly Thr Ile Ala Arg Gly
                   325                 330                 335
   Pro Asp Ala Leu Gly Asp Ala Glu Ala Ile Arg Thr Leu Leu Asn Ser
               340                 345                 350
   Thr Lys Asp Glu Ala Glu Leu Thr Met Cys Thr Asp Val Asp Arg Asn
           355                 360                 365
   Asp Lys Ala Arg Val Cys Val Ala Gly Ser Val Thr Val Ile Gly Arg
       370                 375                 380
   Arg Gln Ile Glu Leu Tyr Ser Arg Leu Ile His Thr Val Asp His Val
   385                 390                 395                 400
   Glu Gly Arg Leu Arg Pro Glu Leu Asp Ala Leu Asp Ala Phe Leu Ser
                   405                 410                 415
   His Cys Trp Ala Val Thr Val Thr Gly Ala Pro Lys Arg Ala Ala Met
   420 425 430
   Ala Ala Val Glu Ala Val Glu Arg Ala Pro Arg Ala Trp Tyr Gly Gly
   435 440 445
   Ala Ile Gly Arg Leu Gly Phe Asp Gly Thr Leu Asp Thr Gly Leu Val
   450 455 460
   Leu Arg Thr Ile Arg Leu Arg His Gly Val Ala Glu Val Arg Val Gly
   465 470 475 480
   Ala Thr Leu Leu His Arg Ser Asp Pro Glu Glu Glu Glu Ala Glu Thr
   485 490 495
   Leu Leu Lys Ala Ser Ala Leu Leu Ala Leu Leu Asp Ala Thr Thr Pro
   500 505 510
   Ala Lys Pro Asn Ala Pro His Leu Pro Leu Arg Gly Arg Ala Pro Arg
   515 520 525
   Val Leu Val Ile Asp His Glu Asp Ser Phe Val His Thr Leu Ala Ser
   530 535 540
   Tyr Leu Arg Asn Ala Gly Ala Glu Thr Thr Val Leu Arg Trp Asp Val
   545 550 555 560
   Pro Ala Ala Val Arg Ala Gly Val Glu Ala Asp Leu Leu Val Leu Ser
   565 570 575
   Pro Gly Pro Gly Thr Pro Ser Arg Phe Ala Leu Gly Ala Ser Leu Asp
   580 585 590
   Trp Ala Val Ala Arg Gly Leu Pro Val Phe Gly Val Cys Leu Gly Leu
   595 600 605
   Gln Gly Ile Val Glu Gln Ala Gly Gly Arg Leu Ala Arg Leu Ala Val
   610 615 620
   Pro Ala His Gly Met Ala Ser Thr Leu Arg Leu Val Ala Pro Gly Asp
   625 630 635 640
   Pro Leu Phe Ala Gly Leu Pro Thr Thr Met Arg Val Gly Arg Tyr His
   645 650 655
   Ser Leu His Ala Glu Arg Ala Ser Leu Pro Asp Ser Leu Glu Ile Leu
   660 665 670
   Ala Glu Ser Asp Asp Gly Val Ile Met Ala Leu Arg His Arg Leu Leu
   675 680 685
   Pro Phe Ser Ala Val Gln Phe His Pro Glu Ser Leu Met Thr Leu Asp
   690 695 700
   Gly Gly Ala Gly Pro Arg Leu Ile Ala Asn Leu Leu Glu Thr Leu Ser
   705 710 715 720
   Val Pro Arg Thr Arg His Ala Ala
   725
<210> 132
   <211> 726
   <212> PRT
   <213> Thermobifida fusca
<400> 132
   Val Asp Asp Asn Ser Tyr Thr Thr Ser Gly Gly Ile Thr Val His Arg
   1 5 10 15
   Thr Ala Val Pro Cys Asp Pro Arg Ala Leu Ala Asp Leu Thr Val Asn
   20 25 30
   Val Glu Gln Arg Arg Gly Gly Val Leu Ser Ser Gly Met Glu Tyr Pro
   35 40 45
   Gly Arg Tyr Ser Arg Trp His Leu Gly Tyr Val Asp Pro Cys Leu Glu
   50 55 60
   Val Val Ala Arg Gly Arg Thr Ile Gly Ala Thr Ala Leu Asn Asp Arg
   65 70 75 80
   Gly Arg Val Leu Leu Pro Ala Val Ala Arg Ala Leu Thr Ala His Gly
   85 90 95
   Ala Val Val Glu His Thr Asp Asp Thr Val Ala Val Thr Val Pro Glu
   100 105 110
   Pro Asp Pro Thr Glu Phe Phe Thr Glu Glu Glu Arg Ser Arg Arg Leu
   115 120 125
   Ser Val Phe Ser Ala Leu Arg Ala Leu Val Gly Val Phe Phe His Ala
   130 135 140
   Glu Glu Pro His Leu Gly Leu Tyr Gly Ala Phe Gly Tyr Asp Leu Ala
   145 150 155 160
   Phe Gln Phe Glu Pro Ile Glu Gln Val Leu Pro Arg Asp Pro Glu Asp
   165 170 175
   Arg Asp Leu Val Leu His Leu Pro Asp Glu Ile Ile Val His Asp Arg
   180 185 190
   Lys Arg Glu Ile Cys Gln Arg Tyr Ser Tyr Asp Phe Thr Leu Pro Glu
   195 200 205
   Glu Leu Arg Gly Pro Ala Gly Ala Thr Thr Arg Gly Leu Pro Arg His
   210 215 220
   Thr Glu Pro Thr Pro Pro Val Thr Pro Ala Ala Glu Val Pro Pro Gln
   225 230 235 240
   Pro Glu Pro Gly Ser Tyr Ala Arg Ile Val Ala Glu Ala Lys Glu Arg
   245 250 255
   Phe Arg Arg Gly Asp Leu Phe Glu Val Val Pro Ser His Arg Leu Tyr
   260 265 270
   Ala Pro Cys Ala Ser Pro Ala Arg Phe Tyr Glu Arg Leu Arg Glu Arg
   275 280 285
   Asn Pro Ala Pro Tyr Glu Phe Phe Leu Asn Leu Gly Glu Gly Glu Tyr
   290 295 300
   Leu Val Gly Ala Ser Pro Glu Met Phe Val Arg Val Thr Gly Arg Pro
   305 310 315 320
   Gly Glu Gly Gln Arg Val Glu Thr Cys Pro Ile Ser Gly Thr Ile Lys
   325 330 335
   Arg Gly Ala Asp Ala Val Gly Asp Ala Glu Asn Ile Lys Glu Leu Leu
   340 345 350
   Ser Ser Ala Lys Glu Glu Ser Glu Leu Thr Met Cys Thr Asp Val Asp
   355 360 365
   Arg Asn Asp Lys Ser Arg Val Cys Val Pro Gly Ser Val Arg Val Ile
   370 375 380
   Gly Arg Arg Gln Ile Glu Met Tyr Ser Arg Leu Ile His Thr Val Asp
   385 390 395 400
   His Ile Glu Gly Ile Leu Arg Pro Glu Leu Asp Ala Ile Asp Ala Phe
   405 410 415
   Leu Thr His Met Trp Ala Val Thr Val Thr Gly Ala Pro Lys Thr Trp
   420 425 430
   Ala Met Arg Phe Ile Glu Gln His Glu Ser Ser Pro Arg Arg Trp Tyr
   435 440 445
   Gly Gly Ala Val Gly Val Ile Asn Phe Asp Gly Ser Met Asn Thr Gly
   450 455 460
   Leu Thr Leu Arg Thr Ala His Ile Arg Asp Gly Val Ala Thr Val Arg
   465                 470                 475                 480
   Ala Gly Ala Thr Leu Leu Tyr Asp Ser Asp Pro Glu Ala Glu Glu Arg
                   485                 490                 495
   Glu Thr Phe Leu Lys Ala Arg Ala Leu Leu Glu Thr Leu Thr Asp Glu
               500                 505                 510
   Gly Glu Glu Thr Ser Lys Ala Ala Pro Ala Val Glu Gln Val Gly Ala
           515                 520                 525
   Gly Met Arg Val Leu Leu Val Asp His Glu Asp Ser Phe Val Asn Thr
       530                 535                 540
   Leu Ala Asp Tyr Val Arg Arg His Gly Ala Glu Val Thr Thr Val Arg
   545                 550                 555                 560
   Tyr Gly Phe Asp Pro Ala Leu Leu Asp Gln Met Arg Pro Asp Leu Val
                   565                 570                 575
   Val Leu Ser Pro Gly Pro Gly Leu Pro Ala Asp Phe Ala Met Ser Ala
               580                 585                 590
   Leu Leu Lys Glu Leu Asp Ala Arg Gly Leu Pro Val Phe Gly Val Cys
           595                 600                 605
   Leu Gly Leu Gln Ala Met Val Glu Tyr Ala Gly Gly Glu Leu Leu Thr
       610                 615                 620
   Leu Asp Thr Pro Val His Gly Lys Pro Gly Arg Val Arg Val Thr Gly
   625                 630                 635                 640
   Gly Ala Leu Leu Ala Gly Leu Gly Glu Asp Gly Glu Phe Thr Ala Ala
                   645                 650                 655
   Arg Tyr His Ser Val Tyr Ala Thr Pro Asp Arg Val Lys Gly Phe Glu
               660                 665                 670
   Val Thr Ala Val Thr Glu Asp Asp Gly Phe Pro Val Val Met Ala Ile
           675                 680                 685
   Glu Asn Ala Glu Ala Arg Arg Trp Ala Val Gln Phe His Pro Glu Ser
       690                 695                 700
   Ile Leu Thr Gly Arg Val Gly Glu Gln Ile Val Ala Asn Val Leu Arg
   705                 710                 715                 720
   Leu Ala Arg Glu Ser Ser
                   725
<210> 133
   <211> 281
   <212> PRT
   <213> Sorghum bicolor
<400> 133
   Met Val Cys Ser Gln Leu Thr Ala Ala Gly Ala Ser Ser Leu Ala Ala
   1               5                   10                  15
   Ala Ala Val Arg Ser Arg Ala His Ser Pro Ala Ala Ala Phe Ala Gln
               20                  25                  30
   Leu Arg Ser Thr Pro Arg Ile Ala Ser Ala Gly Leu Ser Val Lys Gly
           35                  40                  45
   Asn Arg Ala Ala Leu Pro Leu Val Ala Ala Ala Gly Pro Ala Ala Ala
       50                  55                  60
   Ala Pro Val Ala Asp Leu Asp Gly Pro Pro Ala Thr Glu Lys Gln Pro
   65                  70                  75                  80
   Ile Ile Val Ile Asp Asn Tyr Asp Ser Phe Thr Tyr Asn Leu Cys Gln
                   85                  90                  95
   Tyr Met Gly Glu Leu Gly Leu Asn Phe Glu Val Tyr Arg Asn Asp Glu
               100                 105                 110
   Leu Thr Ile Glu Asp Val Lys Arg Lys Asn Pro Arg Gly Ile Leu Ile
           115                 120                 125
   Ser Pro Gly Pro Gly Glu Pro Gln Asp Ser Gly Ile Ser Leu Gln Ala
       130                 135                 140
   Val Leu Glu Leu Gly Pro Thr Ile Pro Ile Phe Gly Val Cys Met Gly
   145                 150                 155                 160
   Leu Gln Cys Ile Gly Glu Ala Phe Gly Gly Lys Ile Ile Arg Ala Pro
                   165                 170                 175
   Ser Gly Val Met His Gly Lys Ser Ser Pro Val Tyr Tyr Asp Glu Glu
               180                 185                 190
   Leu Gly Lys Ala Leu Phe Asn Gly Leu Pro Asn Pro Phe Thr Ala Ala
           195                 200                 205
   Arg Tyr His Ser Leu Val Ile Glu Glu Glu Thr Phe Pro His Asp Ala
       210                 215                 220
   Leu Glu Ala Thr Ala Trp Thr Glu Asp Gly Leu Ile Met Ala Ala Arg
   225                 230                 235                 240
   His Lys Lys Tyr Lys His Ile Gln Gly Val Gln Phe His Pro Glu Ser
   245 250 255
   Ile Ile Thr Pro Asp Gly Lys Lys Ile Ile Leu Asn Phe Val Arg Phe
   260 265 270
   Ile Glu Glu Leu Glu Lys Gln Arg Ser
   275 280
<210> 134
   <211> 1122
   <212> DNA
   <213> Sorghum bicolor
<400> 134
   gtcgacccac gcgtccgccg cctccccacc ctcgctccct ctcaccgccc gccaccgccg 60
   agatggtctg ctcccagctc accgccgcgg gggcctcctc cctcgccgcc gcagcggttc 120
   gttcccgggc gcattcccca gccgccgcct tcgcgcaact acggtcgacg cctcgcattg 180
   cgagcgctgg cttgtcggtt aagggaaaca gggcggctct tccgttggtc gccgccgcgg 240
   ggccggccgc ggcggcgccg gtggccgacc tggacggccc cccggccacg gagaagcagc 300
   ccatcattgt catcgataac tacgacagct tcacctacaa cctctgccag tatatggggg 360
   agcttggatt gaactttgaa gtataccgca atgatgaact gaccatagaa gatgtaaaga 420
   ggaagaaccc aagaggaata cttatttctc cagggcctgg tgaaccacaa gattcaggaa 480
   tatcattgca ggctgttctt gaattaggcc caaccatccc aatttttgga gtttgcatgg 540
   gcctgcagtg cattggggag gcatttgggg gaaagattat ccgtgctcct tctggagtga 600
   tgcatgggaa aagctctcca gtttattacg acgaggaatt aggaaaggcc ttattcaatg 660
   gcttgccaaa cccttttacc gctgcgaggt accacagctt ggtcattgag gaagaaacct 720
   tcccgcatga tgctttagag gccactgcat ggactgaaga tggacttatc atggctgctc 780
   gccacaagaa gtacaaacac atccagggtg tccaattcca cccggagagc atcatcaccc 840
   ctgacggcaa gaaaatcatc ctcaatttcg tcagattcat tgaggaactg gagaagcagc 900
   gttcctaggg aggtagatgc caccggtggc ttcatagatc agtcagaagc agagacaaag 960
   gcgcttgaag ctgcgtagta ccgggtctgg cagtggaagt tagctaggaa acagcctttt 1020
   tcctccctta attcgttgtg ctcgtggtaa tataatctgt gtggactgaa tttcgaataa 1080
   agtccagctg ttcaaataaa aaaaaaaaaa aagggcggcc gc 1122
<210> 135
   <211> 1934
   <212> DNA
   <213> Sorghum bicolor
<400> 135
   gtcgacccac gcgtccgcgt gacaccccgc gcggcacctc cgcctcccca ccctcgctcc     60
   ctctcaccgc ccgccaccgc cgagatggtc tgctcccagc tcaccgccgc gggggcctcc    120
   tccctcgccg ccgcagcggt tcgttcccgg gcgcattccc cagccgccgc cttcgcgcaa    180
   ctacggtcga cgcctcgcat tgcgagcgct ggcttgtcgg ttaagggaaa cagggcggct    240
   cttccgttgg tcgccgccgc ggggccggcc gcggcggcgc cggtggccga cctggacggc    300
   cccccggcca cggagaagca gcccatcatt gtcatcgata actacgacag cttcacctac    360
   aacctctgcc agtatatggg ggagcttgga ttgaactttg aagtataccg caatgatgaa    420
   ctgaccatag aagatgtaaa gaggaagaac ccaagaggaa tacttatttc tccagggcct    480
   ggtgaaccac aagattcagg aatatcattg caggctgttc ttgaattagg cccaaccatc    540
   ccaatttttg gagtttgcat gggcctgcag tgcattgggg aggcatttgg gggaaagatt    600
   atccgtgctc cttctggagt gatgcatggg aaaagctctc cagtttatta cgacgaggaa    660
   ttaggaaagg ccttattcaa tggcttgcca aaccctttta ccgctgcgag gtaccacagc    720
   ttggtcattg aggaagaaac cttcccgcat gatgctttag aggccactgc atggactgaa    780
   gatggactta tcatggctgc tcgccacaag aagtacaaac acatccaggg tgtccaattc    840
   cacccggaga gcatcatcac ccctgacggc aagaaaatca tcctcaattt cgtcagattc    900
   attgaggaac tggagaagca gcgttcctag ggaggtagat gccaccggtg gcttcataga    960
   tcagtcagaa gcagagacaa aggcgcttga agctgcgtag taccgggtct ggcagtggaa   1020
   gttagctagg aaacagcctt tttcctccct taattcgttg tgctcgtggt aatataatct   1080
   gtgtggactg aatttcgaat aaagtccagc tgttcaaata aaaaaaaaaa aaaagggcgg   1140
   ccgctaccct cgaggccggc cgggccggga agccgcgatg ttaaatgtgt ggtccttagc   1200
   aatcaaatct gcgtacaaga tggaatctct attattttca gcgaaaccaa gggattccgt   1260
   gagcgcatcc gagggaggaa ggtgggcgta cctttaggct tctttccgac ctccgggaag   1320
   tgcggcgagg gtttcatggc gatgttggcg gctgcaaggc cggcgcgccg gatagaggcg   1380
   ggggcagggg aactgacaag ggtttaacta cgcgaacgcg cactgcagga atggacgagc   1440
   tcgctctctc aaatcggccg aatgcgctcg tcccaccgag acgccgtctt gctccgtctc   1500
   cctgtctcgc tcacgcggac gctccacgcg agcagggcag gccgaggagg gcgacggcgc   1560
   agccgggcga cggcgcgagg agagcgatgg cgcgaagagt gcagaacgcg cggctcggcg   1620
   gcggcgcagc tgtgcggcgg cgcggttggg ccgcggtggc ggagaacggt gaacggaaga   1680
   acgaagaaga aagggaaaga agcctttgta tgacacgtgg gtcccgcagt tagaagggtt   1740
   aaatctgttg tctcatccat cacgtagtct taattttttt ttcactagtc tgtgttaatg   1800
   ctacgatctt aagaagccag cgacaataag agtagctcta ataaaaaaaa aaaaaaaaag   1860
   aaaaaaaaaa aaaaaaaaaa aaaaaaaaaa aaaacaaaaa aaaaaaaaaa aaaggcccct   1920
   agggccctcg agct                                                     1934
<210> 136
   <211> 2055
   <212> DNA
   <213> Zea mays
<400> 136
   ggccggccgg gactccactc cactccactc gcccgcgccc gccgcccgcg tgctcctccc 60
   tcgcgcccgc cccgccccgc tccgccccta tctatatcca ttcccgctcg tccccctcaa 120
   caaccctcct cacggcgcac tcgtgttcgt gtcatcccgc ccgcaggccg cagtccatgg 180
   ccaccgccag cctcgcgctc tcgctgcgcc tcgcgccgta ctcgcacccg ctgagcctcc 240
   gccgccgggg ggccgccggc gtcacctgcc gcgccaccac cgccacgttc caccagcttg 300
   acgccgtcgc ggtgagggag gaggagtcca ggttccggac ggcggcggcg gagggccgca 360
   acctgctgcc gctcacgagg tgcatcttct ccgatcacct cacgcccgtg ctcgcctacc 420
   gctgcctcgt caaggaggac gaacgcgagg cgcccagctt tctattcgag tctgtcgagc 480
   aggggtccga gggcaccaat gtggggaggt acagcgtggt cggggcgcag ccttctatgg 540
   aggtcgtggc caaggctaac catgtgacgg tcatggacca tgagatgaag tcgaggaggg 600
   agcacttcgt gcctgatccc atgaggatcc ccaggacaat catggagcag tggaacccgc 660
   agattgctga cagcctccct gatgcatttt gtggaggatg ggttggattc ttctcatatg 720
   atacagtgcg ttatgttgaa acaaagaagc ttcctttcag taaggcacca catgatgata 780
   ggaaccttcc tgatattcat ttaggcctct atagtgacgt cattgtgttt gatcatgttg 840
   aaaagaaaac acatgttatt cattgggtga ggacagactg ctatcgttct gttgatgaag 900
   catatgaaga tggaagaaat cggcttgaag ctttgttatc aagattacat tgcctcaatg 960
   tcccaacact ttcttctggt tctataaaac tcaatgttga aaactttggc ccagtaatgc 1020
   aaaaatcaac gatgtcaagc gaagaatata aaaatatcgt tgtccaagct aaagaacaca 1080
   tcttggccgg tgacattttc caagttgttt taagccagcg ttttgagaga cggacattcg 1140
   ccgacccctt tgaaatctat cgtgcattgc gcatcgtaaa tcctagtcca tatatggcct 1200
   atctacaggc acgaggttgt attctcgtgg catcgagtcc tgaaattctt acccgggtac 1260
   aaaagaggac aataatcaat cgtccgcttg ctggaaccat aagaagaggc aaaacaaaag 1320
   cagaagacaa aactttagaa caattgcttt tgagtgacga aaagcagtgt gctgaacata 1380
   ttatgctagt agatcttggc cgaaatgatg ttgggaaggt gtccaaacca ggttcagtaa 1440
   aggtagagaa attgatgaat atcgaacgat attctcatgt catgcacatc agctcaacag 1500
   taactggaga gctacgcgat gatcttacgt gttgggatgc gctacgagcc gcattgccag 1560
   ttggaaccgt tagtggcgct ccaaaggtga gagcaatgga gttgattgat cagctagaag 1620
   tgagtatgcg tgggccgtat agtggtggct ttggagggat ttcctttcgc ggcgacatgg 1680
   acattgcact ggctcttcgc actatcgtct tccccaccgc atctcggttt gataccatgt 1740
   actcgtacac agacagtaag tcccgacagg agtgggtggc tcacctccag gccggagctg 1800
   gcatagttgc tgatagcaaa ccggatgacg agcaccaaga gtgtataaac aaggctgcag 1860
   gtgttgctcg tgccattgac cttgctgaat ctacatttct tgaagactag tctagtctaa 1920
   tgaaggaaat gtatgtttaa gttctctgta caattatgga ttgtcctaga aaacaggctt 1980
   tcttaggccg aataaaaact caattgtaat aaagttaata aatggacaac tttagctaaa 2040
   aaaaaaaaaa aaaaa 2055
<210> 137
   <211> 577
   <212> PRT
   <213> Zea mays
<400> 137
   Met Ala Thr Ala Ser Leu Ala Leu Ser Leu Arg Leu Ala Pro Tyr Ser
   1 5 10 15
   His Pro Leu Ser Leu Arg Arg Arg Gly Ala Ala Gly Val Thr Cys Arg
   20 25 30
   Ala Thr Thr Ala Thr Phe His Gln Leu Asp Ala Val Ala Val Arg Glu
   35 40 45
   Glu Glu Ser Arg Phe Arg Thr Ala Ala Ala Glu Gly Arg Asn Leu Leu
   50 55 60
   Pro Leu Thr Arg Cys Ile Phe Ser Asp His Leu Thr Pro Val Leu Ala
   65 70 75 80
   Tyr Arg Cys Leu Val Lys Glu Asp Glu Arg Glu Ala Pro Ser Phe Leu
   85 90 95
   Phe Glu Ser Val Glu Gln Gly Ser Glu Gly Thr Asn Val Gly Arg Tyr
   100 105 110
   Ser Val Val Gly Ala Gln Pro Ser Met Glu Val Val Ala Lys Ala Asn
   115 120 125
   His Val Thr Val Met Asp His Glu Met Lys Ser Arg Arg Glu His Phe
   130 135 140
   Val Pro Asp Pro Met Arg Ile Pro Arg Thr Ile Met Glu Gln Trp Asn
   145 150 155 160
   Pro Gln Ile Ala Asp Ser Leu Pro Asp Ala Phe Cys Gly Gly Trp Val
   165 170 175
   Gly Phe Phe Ser Tyr Asp Thr Val Arg Tyr Val Glu Thr Lys Lys Leu
   180 185 190
   Pro Phe Ser Lys Ala Pro His Asp Asp Arg Asn Leu Pro Asp Ile His
   195 200 205
   Leu Gly Leu Tyr Ser Asp Val Ile Val Phe Asp His Val Glu Lys Lys
   210 215 220
   Thr His Val Ile His Trp Val Arg Thr Asp Cys Tyr Arg Ser Val Asp
   225 230 235 240
   Glu Ala Tyr Glu Asp Gly Arg Asn Arg Leu Glu Ala Leu Leu Ser Arg
   245 250 255
   Leu His Cys Leu Asn Val Pro Thr Leu Ser Ser Gly Ser Ile Lys Leu
   260 265 270
   Asn Val Glu Asn Phe Gly Pro Val Met Gln Lys Ser Thr Met Ser Ser
   275 280 285
   Glu Glu Tyr Lys Asn Ile Val Val Gln Ala Lys Glu His Ile Leu Ala
   290 295 300
   Gly Asp Ile Phe Gln Val Val Leu Ser Gln Arg Phe Glu Arg Arg Thr
   305 310 315 320
   Phe Ala Asp Pro Phe Glu Ile Tyr Arg Ala Leu Arg Ile Val Asn Pro
   325 330 335
   Ser Pro Tyr Met Ala Tyr Leu Gln Ala Arg Gly Cys Ile Leu Val Ala
   340 345 350
   Ser Ser Pro Glu Ile Leu Thr Arg Val Gln Lys Arg Thr Ile Ile Asn
   355 360 365
   Arg Pro Leu Ala Gly Thr Ile Arg Arg Gly Lys Thr Lys Ala Glu Asp
   370 375 380
   Lys Thr Leu Glu Gln Leu Leu Leu Ser Asp Glu Lys Gln Cys Ala Glu
   385 390 395 400
   His Ile Met Leu Val Asp Leu Gly Arg Asn Asp Val Gly Lys Val Ser
   405 410 415
   Lys Pro Gly Ser Val Lys Val Glu Lys Leu Met Asn Ile Glu Arg Tyr
   420 425 430
   Ser His Val Met His Ile Ser Ser Thr Val Thr Gly Glu Leu Arg Asp
   435 440 445
   Asp Leu Thr Cys Trp Asp Ala Leu Arg Ala Ala Leu Pro Val Gly Thr
   450 455 460
   Val Ser Gly Ala Pro Lys Val Arg Ala Met Glu Leu Ile Asp Gln Leu
   465 470 475 480
   Glu Val Ser Met Arg Gly Pro Tyr Ser Gly Gly Phe Gly Gly Ile Ser
   485 490 495
   Phe Arg Gly Asp Met Asp Ile Ala Leu Ala Leu Arg Thr Ile Val Phe
   500 505 510
   Pro Thr Ala Ser Arg Phe Asp Thr Met Tyr Ser Tyr Thr Asp Ser Lys
           515                 520                 525
   Ser Arg Gln Glu Trp Val Ala His Leu Gln Ala Gly Ala Gly Ile Val
       530                 535                 540
   Ala Asp Ser Lys Pro Asp Asp Glu His Gln Glu Cys Ile Asn Lys Ala
   545                 550                 555                 560
   Ala Gly Val Ala Arg Ala Ile Asp Leu Ala Glu Ser Thr Phe Leu Glu
                   565                 570                 575
   Asp
<210> 138
   <211> 22
   <212> DNA
   <213> Artificial
<220>
   <223> A primer.
<400> 138
   atggcagcgg taattctgga ag 22
<210> 139
   <211> 19
   <212> DNA
   <213> Artificial
<220>
   <223> A primer.
<400> 139
   tcaggctgcc ttggtcttc 19
<210> 140
   <211> 30
   <212> DNA
   <213> Artificial
<220>
   <223> A primer.
<400> 140
   actgactcca tggcagcggt aattctggaa 30
<210> 141
   <211> 20
   <212> DNA
   <213> Artificial
<220>
   <223> A primer.
<400> 141
   ctgactagtt caggctgctt 20
<210> 142
   <211> 30
   <212> DNA
   <213> Artificial
<220>
   <223> A primer.
<400> 142
   tgctgaccat ggcctgctcc cacatcgtcg 30
<210> 143
   <211> 34
   <212> DNA
   <213> Artificial
<220>
   <223> A primer.
<400> 143
   cagtgaattc ctacgaacgc tgcttctcca gttc 34

## Description

The seeds of a number of important crops, including soybean and maize do not contain sufficient quantities of several amino acids to be nutritionally complete. These amino acids include, but are not limited to: tryptophan, isoleucine, valine, arginine, lysine, methionine, and threonine. Therefore, the biosynthetic pathways for these amino acids, and/or biosynthetic pathways for metabolites that feed into those pathways, are potential targets for manipulation in order to increase the amino acid content of these plants.

Anthranilate synthase (AS, EC 4.1.3.27) catalyzes the first reaction branching from the aromatic amino acid pathway to the biosynthesis of tryptophan in plants, fungi, and bacteria.

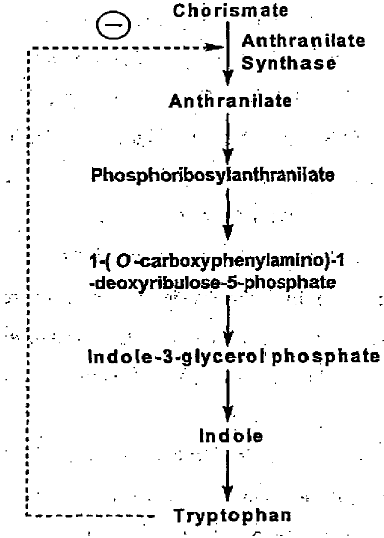

The most common form of anthranilate synthase (for example, the maize anthranilate synthase) is a heterotetrameric enzyme consisting of two subunits, the a or TrpE subunit and the β or TrpG subunit. Two α-subunits and two β-subunits assemble to form the heterotetrameric anthranilate synthases. "Monomeric" forms of AS have also been discovered that comprise a single polypeptide chain having the activities of both TrpE and TrpG subunits (for example *Rhizobium meliloti*). While monomeric anthranilate synthases comprise just one type of polypeptide, the enzymatically active form of a monomeric anthranilate synthase is typically a homodimer consisting of two such monomeric polypeptides. Both heterotetrameric and monomeric anthranilate synthases catalyze the formation of anthranilate in a reaction utilizing glutamine and chorismate. The domain found on the α-subunit (referred to herein as the "α-domain") binds chorismate and eliminates the enolpyruvate side chain, and the domain found on the β-subunit (referred to herein as the "β-domain") transfers an amino group from glutamine to the position on the chorismate phenyl ring that resides between the carboxylate and the enolpyruvate moieties.

The next reaction in the synthesis of tryptophan is the transfer of the phosphoribosyl moiety of phosphoribosyl pyrophosphate to anthranilate. The indole ring is formed in two steps involving an isomerization converting the ribose group to a ribulose followed by a cyclization reaction to yield indole glycerol phosphate. The final reaction in the pathway is catalyzed by a single enzyme that may contain either one or two subunits. The reaction accomplishes the cleavage of indole glyceraldehyde-3-phosphate and condensation of the indole group with serine (Umbarger, Ann. Rev. Biochem., 47:555 (1978)).

Metabolite flow in the tryptophan pathway in higher plants and microorganisms is apparently regulated through feedback inhibition of anthranilate synthase by tryptophan. Tryptophan may block the conformational rearrangement that is required to activate the β-domain and to create a channel for passage of ammonia toward the active site of the α-domain. Such feedback inhibition by tryptophan is believed to depress the production of tryptophan by anthranilate synthase. *See* Li J. & Last, R.L., The Arabidopsis thaliana trp5 mutant has a feedback-resistant anthranilate synthase and elevated soluble tryptophan (Plant Physiol., 110:51-59 (1996)).

Several amino acid residues have been identified as being involved in the feedback regulation of the anthranilate synthase complex from *Salmonella typhimurium.* Such information provides evidence of an amino-terminal regulatory site (J. Biol. Chem., 266:8328-8335 (1991)). *Niyogi et al.* have further characterized the anthranilate synthase from certain plants employing a molecular approach. *See,* Niyogi and Fink, Plant Cell, 4:721 (1992) and Niyogi et al., Plant Cell, 5:1011 (1993). They found that the α-subunits of the *Arabidopsis* anthranilate synthase are encoded by two closely related, nonallelic genes that are differentially regulated. One of these α-subunit genes, ASA1, is induced by wounding and bacterial pathogen infiltration, implicating its involvement in a defense response, whereas the other α-subunit gene, ASA2, is expressed at constitutive basal levels. Both predicted proteins share regions of homology with bacterial and fungal anthranilate synthase proteins, and contain conserved amino acid residues at positions that have been shown to be involved in tryptophan feedback inhibition in bacteria (Caligiuri et al., J. Biol. Chem., 266:8328 (1991)).

Amino acid analogs of tryptophan and analogs of the intermediates in the tryptophan biosynthetic pathway (*e.g*., 5-methyltryptophan, 4-methyltryptophan, 5-fluorotryptophan, 5-hydroxytryptophan, 7-azatryptophan, 3β-indoleacrylic acid, 3-methylanthranilic acid), have been shown to inhibit the growth of both prokaryotic and eukaryotic organisms. Plant cell cultures can be selected for resistance to these amino acid analogs. For example, cultured tobacco, carrot, potato, corn and *Datura innoxia* cell lines have been selected that are resistant to growth inhibition by 5-methyltryptophan (5-MT), an amino acid analog of tryptophan, due to expression of an altered anthranilate synthase.

Ranch et al., Plant Physiol., 71:136 (1983) selected for 5-MT resistance in cell cultures of *Datura innoxia*, a dicot weed, and reported that the resistant cell cultures contained increased tryptophan levels (8 to 30 times higher than the wild type level) and an anthranilate synthase with less sensitivity to tryptophan feedback inhibition. Regenerated plants were also resistant to 5-MT, contained an altered anthranilate synthase, and had greater concentrations of free tryptophan (4 to 44 times) in the leaves than did the leaves of the control plants. In contrast to the studies with *N. tabacum*, where the altered enzyme was not expressed in plants regenerated from resistant cell lines, these results indicated that the amino acid overproduction phenotype could be selected at the cellular level and expressed in whole plants regenerated from the selected cells in *Datura innoxia.*

Hibberd et al. (U.S. Patent 4,581,847) described 5-MT resistant maize cell lines that contained an anthranilate synthase that was less sensitive to feedback inhibition than wild-type anthranilate synthase. One 5-MT resistant cell line accumulated free tryptophan at levels almost twenty-fold greater than that of non-transformed cell lines.

P. C. Anderson et al. (U.S. Patent 6,118,047) disclose the use of a tryptophan-insensitive α-domain of anthranilate synthase from C28 maize in a transgene to prepare transgenic maize plants (*Zea mays*) exhibiting elevated levels of free tryptophan in the seed(s).

Although it is possible to select for 5-MT resistance in certain cell cultures and plants, this characteristic does not necessarily correlate with the overproduction of free tryptophan in whole plants. Additionally, plants regenerated from 5-MT resistant lines frequently do not express an altered form of the enzyme. Nor is it predictable that this characteristic will be stable over a period of time and will be passed along as a heritable trait.

Anthranilate synthase has also been partially purified from crude extracts of cell cultures of higher plants (Hankins et al., Plant Physiol., 57:101 (1976); Widholm, Biochim. Biophys. Acta, 320:217 (1973)). However, it was found to be very unstable. Thus, there is a need to provide plants with a source of anthranilate synthase that can increase the tryptophan content of plants.

### Summary of the Invention

The present invention in general relates to nucleic acids encoding an anthranilate synthase (AS) that can be used to generate transgenic plants. When such anthranilate synthase nucleic acids are expressed in a transgenic plant, elevated levels of tryptophan can be achieved within the cells of the plant. DNA molecules that encode a monomeric anthranilate synthase may encode a natural or genetically engineered chimeric fusion of the α- and β-domains of an anthranilate synthase. The anthranilate synthase gene from a few species (*e.g*., some bacteria and other microbes) naturally gives rise to a monomeric anthranilate synthase that constitutes a single polypeptide chain. However, most species have a heterotetramcric anthranilate synthase composed of two α and two β domains found on separate subunits. The present invention also contemplates formation of chimeric anthranilate synthase fusion proteins comprising any anthranilate synthase α-domain linked to any β-domain.

In general, the sequence identity of naturally occurring monomeric anthranilate synthases with most plant anthranilate synthases is quite low. However, such monomeric anthranilate synthases can provide high levels of tryptophan when expressed in a plant, despite a low sequence identity with the plant's endogenous anthranilate synthase enzyme. Accordingly, the present invention provides monomeric anthranilate synthases as described below that can have divergent sequences and that are capable of efficiently providing high levels of tryptophan in a plant host. For example, transgenic soybean plants containing the monomeric *Agrobacterium tumefaciens* anthranilate synthase can produce from up to about 10,000 to about 12,000 ppm tryptophan in seeds, with average trp levels ranging up to about 7,000 to about 8,000 ppm. In contrast, non-transgenic soybean plants normally have up to only about 100 to about 200 ppm tryptophan in seeds.

Accordingly, the present invention provides a transgenic soybean plant comprising a DNA molecule encoding an anthranilate synthase, wherein the DNA molecule encodes the protein of SEQ ID NO: 66 and wherein the levels of free L-tryptophan in a plant cell, plant tissue, plant part or plant are levels that are about 5 to 150 times the levels found in an untransformed plant cell, plant tissue, plant part or plant.

The present invention further provides a transgenic soybean plant comprising a DNA construct comprising a first expression cassette, wherein the first expression cassette in operable linkage comprises (i) a heterologous promoter; (ii) a DNA molecule encoding an anthranilate synthase, wherein the DNA molecule encodes the protein of SEQ ID NO: 66 or wherein the DNA molecule is one of SEQ ID NOs: 67 and 68; and (iii) a transcriptional terminator and wherein the levels of free L-tryptophan in a plant cell, plant tissue, plant part or plant are levels that are about 5 to 150 times the levels found in an untransformed plant cell, plant tissue, plant part or plant.

In a further embodiment, the present invention provides a method for altering the tryptophan content in a soybean plant comprising:
a. introducing into regenerable cells of a soybean plant a transgene comprising a DNA molecule encoding an anthranilate synthase, wherein the DNA molecule encodes the protein of SEQ ID NO: 66 or wherein the DNA molecule is one of SEQ ID NOs: 67 and 68, wherein the DNA is operably linked to a promoter functional in a plant cell, to yield transformed plant cells; and
b. regenerating a soybean plant from the transformed plant cells wherein the cells of the soybean plant express the anthranilate synthase encoded by the DNA in an amount effective to increase the tryptophan content in the soybean plant relative to the tryptophan content in a second soybean plant of the same or similar genetic background that does not include the transgene, wherein the levels of free L-tryptophan in a plant cell, plant tissue, plant part or plant are levels that are about 5 to 150 times the levels found in an untransformed plant cell, plant tissue, plant part or plant.

Further, a method for making an animal feed or a human food is provided which comprises:
a. introducing into regenerable cells of a soybean plant a transgene comprising a DNA molecule encoding an anthranilate synthase, wherein the DNA molecule encodes the protein of SEQ ID NO: 66 or wherein the DNA molecule is one of SEQ ID NOs: 67 and 68, wherein the DNA molecule is operably linked to a promoter functional in a plant cell, to yield transformed plant cells; and
b. regenerating a soybean plant from the transformed plant cells wherein the cells of the soybean plant express the anthranilate synthase encoded by the DNA molecule in an amount effective to increase the tryptophan content in the soybean plant relative to the tryptophan content in a second soybean plant of the same or similar genetic background that does not include the transgene, wherein the levels of free L-tryptophan in a plant cell, plant tissue, plant part or plant are levels that are about 5 to 150 times the levels found in an untransformed plant cell, plant tissue, plant part or plant.

According to an alternative embodiment, a part of a transgenic soybean plant as described above is provided, wherein the part comprises a DNA molecule encoding an anthranilate synthase, wherein the DNA molecule encodes the protein of SEQ ID NO: 66 or wherein the DNA molecule is one of SEQ ID NOs: 67 and 68.

The isolated DNA can encode other elements and functions. Any element or function contemplated by one of skill in the art can be included. For example, the isolated DNA can also include a promoter that can function in a plant cell that is operably linked to the DNA encoding the anthranilate synthase. The isolated DNA can further encode a plastid transit peptide. The isolated DNA can also encode a selectable marker or a reporter gene. Such a selectable marker gene can impart herbicide resistance to cells of said plant, high protein content, high oil content, high lysine content, high isoleucine content, high tocopherol content and the like. The DNA sequence can also comprise a sequence encoding one or more of the insecticidal proteins derived from *Bacillus thuringiensis.*

Further described herein are vectors comprising an isolated DNA. Such vectors can be used to express anthranilate synthase polypeptides in prokaryotic and eukaryotic cells, to transform plant cells and to generate transgenic plants.

The present invention also provides a transgenic plant comprising an isolated DNA of. Expression of these isolated DNAs in the transgenic plant can result in an elevated level of L-tryptophan, preferably free L-tryptophan, in the transgenic plant, e.g., in the seeds or other parts of the plant. The level is increased above the level of L-tryptophan in the cells of a plant that differ from the cells of the transgenic soybean plant by the absence of the DNA, *e.g.,* the corresponding untransformed cells or an untransformed plant with the same genetic background. The DNA is preferably heritable in that it is preferably transmitted through a complete normal sexual cycle of the fertile plant to its progeny and to further generations.

Transgenic plants that can have such an isolated DNA include dicotyledonous plants (dicots), for example, soybean or canola. Alternatively, the transgenic plants can be monocotyledonous plants (monocots), for example, maize, rice, wheat, barley, or sorghum.

The present invention also provides a seed of any of the transgenic plants containing any of the isolated DNAs, anthranilate synthase polypeptides, transgenes or vectors as described above.

The present invention further provides an animal feed or human food as described above. Portions of plants that can be included in the animal feed or human food include, for example, seeds, leaves, stems, roots, tubers, or fruits. Desirable portions of plants have increased levels of tryptophan provided by expression of an anthranilate synthase encoded by an isolated DNA.

The present invention further provides a method for altering, preferably increasing, the tryptophan content of a plant (dicot or a monocot) by introducing an isolated DNA into regenerable cells of the plant as described above. The DNA sequence is preferably operably linked to at least one promoter operable in the plant cells. The transformed cells are identified or selected, and then regenerated to yield a plant comprising cells that can express a functional anthranilate synthase polypeptide. In some embodiments, the DNA encoding the anthranilate synthase, or domain thereof, is a mutant DNA. The introduced DNA is preferably heritable and the plant is preferably a fertile plant. For example, the introduced DNA preferably can be passed by a complete sexual cycle to progeny plants, and can impart the high tryptophan phenotype to subsequent generations of progeny.

The anthranilate synthase-encoding DNAs, are preferably incorporated into vectors or "transgenes" that can also include DNA sequences encoding transit peptides, such as plastid transit peptides, and selectable marker or reporter genes, operably linked to one or more promoters that are functional in cells of the target plant. The promoter can be, for example, an inducible promoter, a tissue specific promoter, a strong promoter or a weak promoter. Other transcription or translation regulatory elements, e.g., enhancers or terminators, can also be functionally linked to the anthranilate synthase-encoding DNA segment.

Cells in suspension culture or as embryos, intact tissues or organs can be transformed by a wide variety of transformation techniques, for example, by microprojectile bombardment, electroporation and *Agrobacterium tumefaciens-*mediated transformation, and other procedures available to the art.

Thus, the cells of the transformed plant comprise a native anthranilate synthase gene and a transgene or other DNA segment encoding an exogenous anthranilate synthase. The expression of the exogenous anthranilate synthase in the cells of the plant can lead to increased levels of tryptophan and its secondary metabolites. In some embodiments, such expression confers tolerance to an amount of endogenous L-typtophan analogue, for example, so that at least about 10% more anthranilate synthase activity is present than in a plant cell having a wild type or tryptophan-sensitive anthranilate synthase.

The present invention also provides a method for altering the tryptophan content in a plant comprising: (a) introducing into regenerable cells of a plant a transgene comprising an isolated DNA encoding an anthranilate synthase domain as described above and a plastid transit peptide, operably linked to a promoter functional in the plant cell to yield transformed cells; and (b) regenerating a transformed plant from said transformed plant cells wherein the cells of the plant express the anthranilate synthase domain encoded by the isolated DNA in an amount effective to increase the tryptophan content in said plant relative to the tryptophan content in an untransformed plant of the same gentic background. The domain can be an anthranilate synthase α-domain. The anthranilate synthase domain can have one or more mutations, for example, mutations that reduce the sensitivity of the domain to tryptophan inhibition. Such mutations can be, for example, in the tryptophan-binding pocket. Such a domain can be, for example, an anthranilate synthase domain from *Agrobacterium tumefaciens, Anabaena* M22983, *Arabidopsis thaliana, Azospirillum brasilense, Brucella melitensis*, *Escherichia coli, Euglena gracilis, Mesorhizobium loti, Nostoc sp.* PCC7120, *Rhizobium meliloti, Ruta graveolens, Rhodopseudomonas palustris, Salmonella typhimurium, Serratia marcescens, Sulfolobus solfataricus*, soybean, rice, cotton or *Zea mays. Ruta graveolens* has its own chloroplast transport sequence that may be used with the anthranilate synthase transgene. Accordingly, one of skill in the art may not need to add a plastid transport sequence when using a *Ruta graveolens* DNA.

Further disclosed are novel isolated and purified DNA molecules comprising a DNA encoding a monomeric anthranilate synthase, or a domain thereof. Such an anthranilate synthase DNA can provide high levels of tryptophan when expressed within a plant. In some embodiments, the anthranilate synthase is substantially resistant to inhibition by free L-tryptophan or an analog thereof. Examples of novel DNA sequences include but are not limited to DNA molecules isolated from *Agrobacterium tumefaciens, Anabaena* M22983, *Arabidopsis thaliana, Azospirillum brasilense, Bradyrhizobium japonicum, Brucella melitensis, Escherichia coli, Euglena gracilis, Mesorhizobium loti, Nostoc sp.* PCC7120, *Rhizobium meliloti, Ruta graveolens, Rhodopseudomonas palustris, Rhodospirillum rubrum, Salmonella typhimurium, Serratia marcescens, Sorghum bicolor, Sulfolobus solfataricus, Thermobifida fusca,* or *Zea mays* (maize), or other such anthranilate synthases.

These DNA sequences include synthetic or naturally-occurring monomeric forms of anthranilate synthase that have the α-domain of anthranilate synthase linked to at least one other anthranilate synthase domain on a single polypeptide chain. The monomeric anthranilate synthase can, for example, be a fusion of an anthranilate synthase a or β domain. Such an anthranilate synthase α or β domain can be derived from *Agrobacterium tumefaciens, Anabaena* M22983, *Arabidopsis thaliana, Azospirillum brasilense, Bradyrhizobium japonicum, Brucella melitensis, Escherichia coli, Euglena gracilis, Mesorhizobium loti, Nostoc sp.* PCC7120, *Rhizobium meliloti, Ruta graveolens, Rhodopseudomonas palustris, Rhodospirillum rubrum, Salmonella typhimurium, Serratia marcescens, Sorghum bicolor, Sulfolobus solfataricus, Thermobifida fusca,* sorghum, soybean, rice, cotton, wheat, tobacco, or *Zea mays* (maize) or any gene encoding a subunit or domain of anthranilate synthase. Such anthranilate synthases and domains thereof are also exemplified herein by the anthranilate synthase nucleic acids isolated from *Agrobacterium tumefaciens,* (SEQ ID NOs: 1, 75, or 84-94), *Zea mays,* (SEQ ID NOs: 2, 67, 68, 96, 116, or 136), *Ruta graveolens* (SEQ ID NO: 3), *Anabaena* M22983, *Arabidopsis thaliana* (SEQ ID NO: 45), *Azospirillum brasilense* (SEQ ID NO: 122), *Brucella melitensis* (SEQ ID NO: 123), *Mesorhizobium loti* (SEQ ID NO: 121), *Nostoc sp.* PCC7120 (SEQ ID NOs: 124 or 125), *Rhizobium meliloti, Rhodopseudomonas palustris* (SEQ ID NO: 126), *Sulfolobus solfataricus*, rice (SEQ ID NOs: 94, 95, 119, or 120), wheat (SEQ ID NO: 97), tobacco (SEQ ID NO: 98), *Gossypium hirsutum* (SEQ ID NOs: 104 or 105), *Glycine max* (SEQ ID NOs: 106, 107, 112, or 113), *Bradyrhizobium janonicum* (SEQ ID NO: 127), *Rhodospirillum rubrum* (SEQ ID NO: 128), *Thermobifida fusca* (SEQ ID NO: 129) or *Sorghum bicolor* (SEQ ID NOs: 134 or 135). These nucleotide sequences encode anthranilate synthases or α-domains or β domains thereof from *Agrobacterium tumefaciens* (SEQ ID NOs: 4, 58-65, 69, or 70); *Zea mays* (SEQ ID NOs: 5, 66, 101, 118, or 137) and *Ruta graveolens* (SEQ ID NO: 6), *Anabaena* M22983, *Azospirillum brasilense* (SEQ ID NO: 78), *Brucella melitensis* (SEQ ID NO: 79), *Mesorhizobium loti* (SEQ ID NO: 77), *Nostoc sp.* PCC7120 (SEQ ID NOs: 80 or 81), *Rhizobium meliloti* (SEQ ID NOs: 7 or 43), *Rhodopseudomonas palustris* (SEQ ID NOs: 57 or 82), *Sulfolobus solfataricus* (SEQ ID NOs: 8 or 44), rice (SEQ ID NOs: 99, 100 or 117), wheat (SEQ ID NO: 102), tobacco (SEQ ID NO: 103), *Gossypium hirsutum* (SEQ ID NOs: 108 or 109), *Glycine max* (SEQ ID NOs: 110 or 111), *Bradyrhizobium japonicum* (SEQ ID NO: 130), *Rhodospirillum rubrum* (SEQ ID NO: 131), *Thermobifida fusca* (SEQ ID NO: 132) or *Sorghum bicolor* (SEQ ID NO: 133).

The isolated DNA molecule comprising a DNA sequence encoding an *Agrobacterium tumefaciens* anthranilate synthase or a domain thereof has enzymatic activity. Such a DNA molecule can encode an anthranilate synthase having SEQ ID NOs: 4, 58-65, 69, or 70, a domain or variant thereof having anthranilate synthase activity. The DNA molecule can also have a sequence comprising SEQ ID NOs: 1, 75, or 84-93, or a domain or variant thereof. Coding regions of any DNA molecule provided herein can also be optimized for expression in a selected organism, for example, a selected plant or microbe. An example of a DNA molecule having optimized codon usage for a selected plant is an *Agrobacterium tumefaciens* anthranilate synthase DNA molecule having SEQ ID NO: 75.

Further disclosed is an isolated and purified DNA molecule comprising a DNA sequence encoding a *Zea mays* anthranilate synthase domain. Such a DNA molecule can encode an anthranilate synthase domain having SEQ ID NOs: 5 or 66, or a variant or derivative thereof having anthranilate synthase activity. The DNA molecule can also have a sequence comprising SEQ ID NOs: 2, 67, or 68, or a domain or variant thereof.

Also provided is an isolated DNA molecule of at least 8 nucleotides that hybridizes to the complement of a DNA molecule comprising any one of SEQ ID NOs: 1, 75, or 84-94 under stringent conditions. Such a DNA molecule can be a probe or a primer, for example, a nucleic acid having any one of SEQ ID NOs: 9-42, 47-56, or 138-143. Alternatively, the DNA it can include up to an entire coding region for a selected anthranilate synthase, or a domain thereof. Such a DNA can also include a DNA sequence encoding a promoter operable in plant cells and/or a DNA sequence encoding a plastid transit peptide. The present invention further contemplates vectors for transformation and expression of these types of DNA molecules in plants and/or microbes.

Functional anthranilate synthase DNA sequences and functional anthranilate synthase polypeptides that exhibit 50%, preferably 60%, more preferably 70%, even more preferably 80%, most preferably 90%, *e.g.,* 95% to 99%, sequence identity to the DNA sequences and amino acid sequences are explicitly described herein. For example, 85% identity means that 85% of the amino acids are identical when the 2 sequences are aligned for maximum matching. Gaps (in either of the 2 sequences being matched) are allowed in maximizing matching; gap lengths of 5 or less are preferred with 2 or less being more preferred.

Alternatively and preferably, two polypeptide sequences are homologous, as this term is used herein; if they have an alignment score of more than 5 (in standard deviation units) using the program ALIGN with the mutation data matrix and a gap penalty of 6 or greater. *See* Dayhoff, M.O., in "Atlas of Protein Sequence and Structure", 1972, volume 5, National Biomedical Research Foundation, pp. 101-110, and Supplement 2 to this volume, pp. 1-10. The 2 sequences or parts thereof are more preferably homologous if their amino acids are greater than or equal to 50% identical when optimally aligned using the ALIGN program. Further provided are expression vectors for generating a transgenic plant with high seed levels of tryptophan comprising an isolated DNA sequence encoding a monomeric anthranilate synthase comprising an anthranilate synthase α-domain linked to an anthranilate synthase β-domain and a plastid transit peptide, operably linked to a promoter functional in a plant cell. Such a monomeric anthranilate synthase can, for example, be an *Agrobacterium tumefaciens, Rhizobium meliloti, Mesorhizobium loti, Brucella melitensis, Nostoc sp.* PCC7120, *Azospirillum brasilense, Anabaena* M22983, *Bradyrhizobium japonicum, Rhodospirillum rubrum,* or *Thermobifida fusca* anthranilate synthase. The monomeric anthranilate synthase can also be a fusion of anthranilate synthase α- and β-domains derived from *Agrobacterium tumefaciens, Anabaena* M22983, *Arabidopsis thaliana, Azospirillum brasilense, Brucella melitensis, Mesorhizobium loti, Nostoc sp.* PCC7120, *Rhizobium meliloti, Rhodopseudomonas palustris, Ruta graveolens, Sulfolobus solfataricus, Salmonella typhimurium, Serratia marcescens, Bradyrhizobium japonicum, Rhodospirillum rubrum, Thermobifida fusca, Sorghum bicolor,* soybean, rice, cotton, wheat, tobacco, *Zea mays,* or any gene encoding a subunit or domain of anthranilate synthase.

The transmission of the isolated and purified anthranilate synthase DNA providing increased levels of tryptophan can be evaluated at a molecular level, e.g., Southern or Northern blot analysis, PCR-based methodologies, the biochemical or immunological detection of anthranilate synthase, or by phenotypic analyses, *i.e*., whether cells of the transformed progeny can grow in the presence of an amount of an amino acid analog of tryptophan that inhibits the growth of untransformed plant cells.

Also disclosed is a method of producing anthranilate synthase in a prokaryotic or eukaryotic host cell, such as a yeast, insect cell, or bacterium, which can be cultured, preferably on a commercial scale. The method includes the steps of introducing a transgene comprising a DNA segment encoding an anthranilate synthase, or a domain thereof, such as a monomeric anthranilate synthase, comprising at least the α and β anthranilate synthase domains, or functional variant thereof, into a host cell and expressing anthranilate synthase in the host cell so as to yield functional anthranilate synthase or domain thereof. A transgene generally includes transcription and translation regulatory elements, *e.g.,* a promoter, functional in host cell, either of eukaryotic or prokaryotic origin. Preferably, the transgene is introduced into a prokaryotic cell, such as *Escherichia coli*, or a eukaryotic cell, such as a yeast or insect cell, that is known to be useful for production of recombinant proteins. Culturing the transformed cells can lead to enhanced production of tryptophan and its derivatives, which can be recovered from the cells or from the culture media. Accumulation of tryptophan may also lead to the increased production of secondary metabolites in microbes and plants, for example, indole containing metabolites such as simple indoles, indole conjugates; indole alkaloids, indole phytoalexins and indole glucosinalates in plants.
Anthranilate synthases insensitive to tryptophan have the potential to increase a variety of chorismate-derived metabolites, including those derived from phenylalanine due to the stimulation of phenylalanine synthesis by tryptophan via chorismate mutase. *See* Siehl, D. The biosynthesis of tryptophan, tyrosine, and phenylalanine from chorismate in Plant Amino Acids: Biochemistry and Biotechnology, ed. BK Singh, pp. 171-204. Other chorismate-derived metabolites that may increase when feedback insensitive anthranilate synthase s are present include phenylpropanoids, flavonoids, and isoflavonoids, as well as those derived from anthranilate, such as indole, indole alkaloids, and indole glucosinolates. Many of these compounds are important plant hormones, plant defense compounds, chemopreventive agents of various health conditions, and/or pharmacologically active compounds. The range of these compounds whose synthesis might be increased by expression of anthranilate synthase depends on the organism in which the anthranilate synthase is expressed. Tryptophan and other useful compounds can be synthesized in a variety of prokaryotic and eukaryotic cells or organisms, including plant cells, microbes, fungi, yeast, bacteria, insect cells, and mammalian cells.

Hence, tryptophan can be produced comprising: culturing a prokaryotic or eukaryotic host cell comprising an isolated DNA under conditions sufficient to express a monomeric anthranilate synthase encoded by the isolated DNA, wherein the monomeric anthranilate synthase comprises an anthranilate synthase a domain and an anthranilate synthase β domain, and wherein the conditions sufficient to express a monomeric anthranilate synthase comprise nutrients and precursors sufficient for the host cell to synthesize tryptophan utilizing the monomeric anthranilate synthase.

Examples of useful compounds that may be generated upon expression in a variety of host cells and/or organisms include indole acetic acid and other auxins, isoflavonoid compounds important to cardiovascular health found in soy, volatile indole compounds which act as signals to natural enemies of herbivorous insects in maize, anticarcinogens such as indole glucosinolates (indole-3-carbinol) found in the Cruciferae plant family, as well as indole alkaloids such' as ergot compounds produced by certain species of fungi. (Barnes et al., Adv Exp Med Biol., 401:87 (1996); Frey et al., Proc Natl Acad Sci., 97:14801 (2000); Muller et al., Biol Chem., 381:679 (2000); Mantegani et al., Farmaco, 54:288 (1999); Zeligs, J Med Food, 1:67 (1998); Mash et al., Ann NY Acad Sci., 844:274 (1998); Melanson et al., Proc Natl Acad Sci., 94:13345 (1997); Broadbent et al., Curr Med Chem., 5:469 (1998)).

Isolated and purified DNA molecule is least seven nucleotide bases and hybridizes under moderate, and preferably, high stringency conditions to the complement of an anthranilate synthase encoding DNA molecule. Such isolated and purified DNA molecules comprise novel DNA segments encoding anthranilate synthase or a domain or mutant thereof. The mutant DNA can encode an anthranilate synthase that is substantially resistant to inhibition by free L-tryptophan or an amino acid analog of tryptophan. Such anthranilate synthase DNA molecules can hybridize, for example, to an *Agrobacterium tumefaciens, Rhodopseudomonas palustris* or *Ruta graveolens* anthranilate synthase, or an α-domain thereof, including functional mutants thereof. When these DNA molecules encode a functional anthranilate synthase or an anthranilate synthase domain, they are termed "variants" of the primary DNA molecules encoding anthranilate synthase, anthranilate synthase domains or mutants thereof. Shorter DNA molecules or oligonucleotides can be employed as primers for amplification of target DNA sequences by PCR, or as intermediates in the synthesis of full-length genes.

A hybridization probe comprises a DNA segment of at least seven nucleotide bases, which is detectably labeled or which can bind to a detectable label, which DNA segment hybridizes under moderate or, preferably, high stringency conditions to the non-coding strand of a DNA molecule comprising a DNA segment encoding an anthranilate synthase such as a monomeric anthranilate synthase, or a domain thereof, such as the α-domain, including functional mutants thereof, that are substantially resistant to inhibition by an amino acid analog of tryptophan. Moderate and stringent hybridization conditions are well known to the art, see, for example sections 0.47-9.51 of Sambrook et al., Molecular Cloning: A Laboratory Manual, 2nd Edition (1989); *see, also,* Sambrook and Russell, Molecular Cloning: A Laboratory Manual, 3rd Edition (January 15, 2001). For example, stringent conditions are those that (1) employ low ionic strength and high temperature for washing, for example, 0.015 M NaCl/0.0015 M sodium citrate (SSC); 0.1% sodium lauryl sulfate (SDS) at 50°C, or (2) employ a denaturing agent such as formamide during hybridization, *e.g*., 50% formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C. Another example is use of 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% sodium dodecylsulfate (SDS), and 10% dextran sulfate at 42°C, with washes at 42°C in 0.2 x SSC and 0.1% SDS.

### Brief Description of the Figures

Figure 1 is a restriction map of plasmid pMON61600.
Figure 2 depicts the translated sequence of the *Agrobacterium tumefaciens* anthranilate synthase DNA sequence (upper sequence) (SEQ ID NO: 4) and the translated sequence of the anthranilate synthase DNA sequence from *Rhizobium meliloti* (lower sequence) (SEQ ID NO: 7).
Figure 3 is a restriction map of plasmid pMON34692.
Figure 4 is a restriction map of plasmid pMON34697.
Figure 5 is a restriction map of plasmid pMON34705.
Figure 6A-B depicts an anthranilate synthase amino acid sequence alignment comparing the *Agrobacterium tumefaciens* α-domain sequence (SEQ ID NO: 4) and the *Sulfolobus solfataricus* α-domain sequence (SEQ ID NO: 8).
Figure 7A-B depicts the sequences of the 34 primers (SEQ ID NOs: 9-42) used to mutate SEQ ID NO: 1. The mutated codons are underlined and the changed bases are in lower case.
Figure 8 depicts a restriction map of plasmid pMON13773.
Figure 9 depicts a restriction map of plasmid pMON58044.
Figure 10 depicts a restriction map of plasmid pMON53084.
Figure 11 depicts a restriction map of plasmid pMON58045.
Figure 12 depicts a restriction map of plasmid pMON58046.
Figure 13 depicts a restriction map of plasmid pMON38207.
Figure 14 depicts a restriction map of plasmid pMON58030.

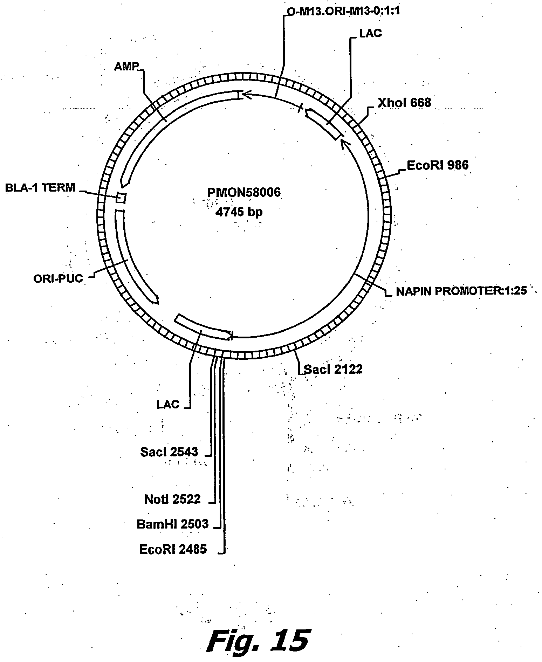

Figure 15 depicts a restriction map of plasmid pMON58006.

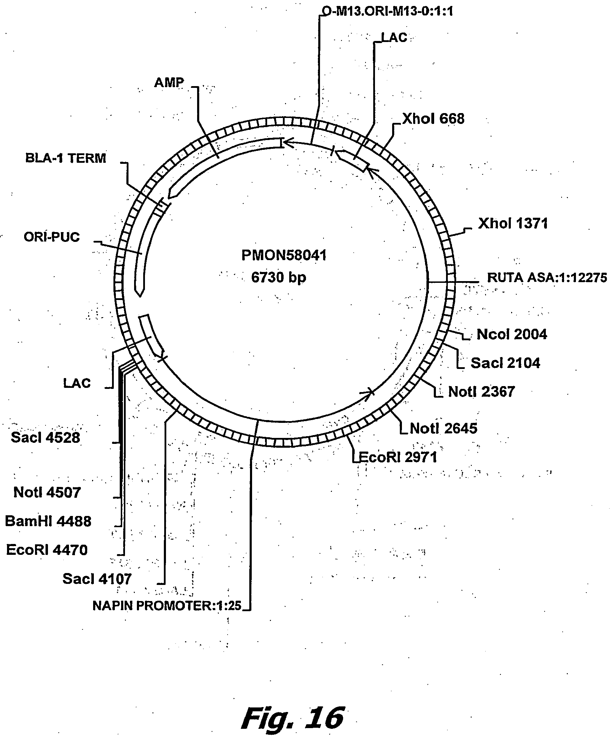

Figure 16 depicts a restriction map of plasmid pMON58041.

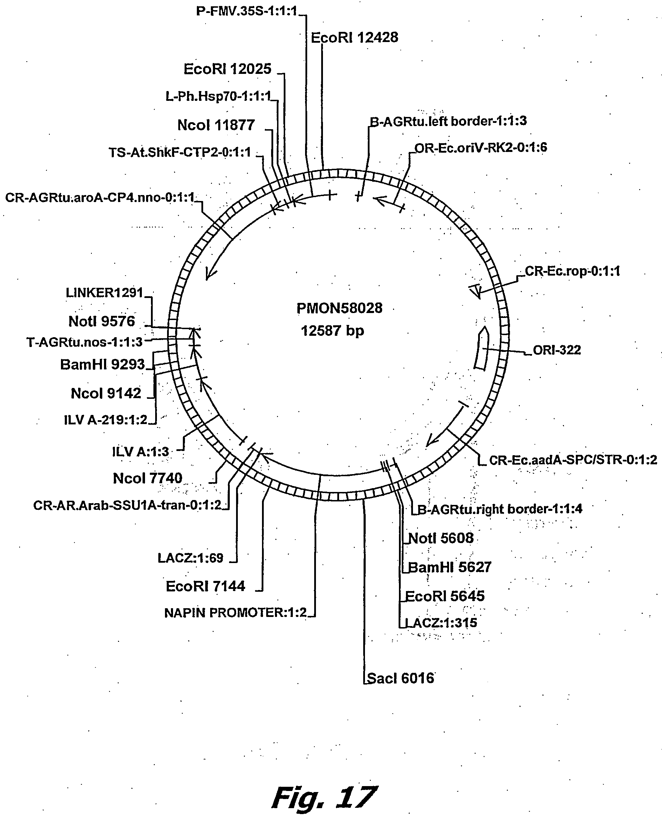

Figure 17 depicts a restriction map of plasmid pMON58028.

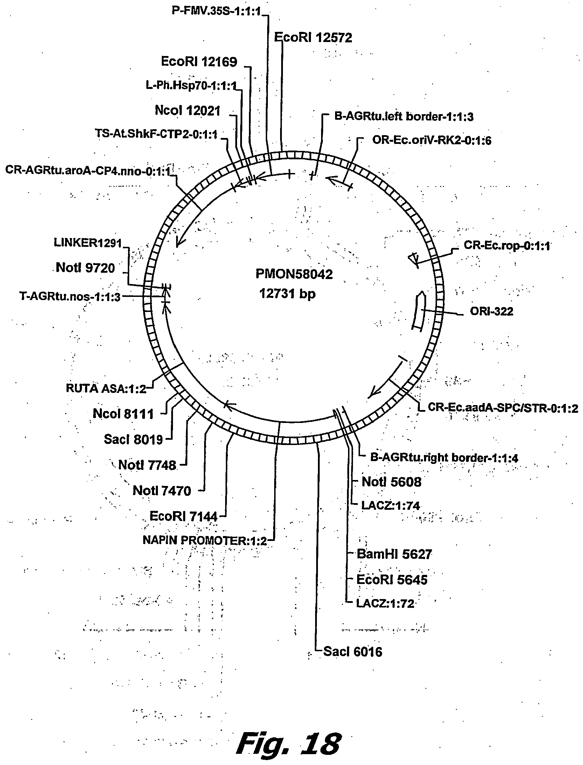

Figure 18 depicts a restriction map of plasmid pMON58042.

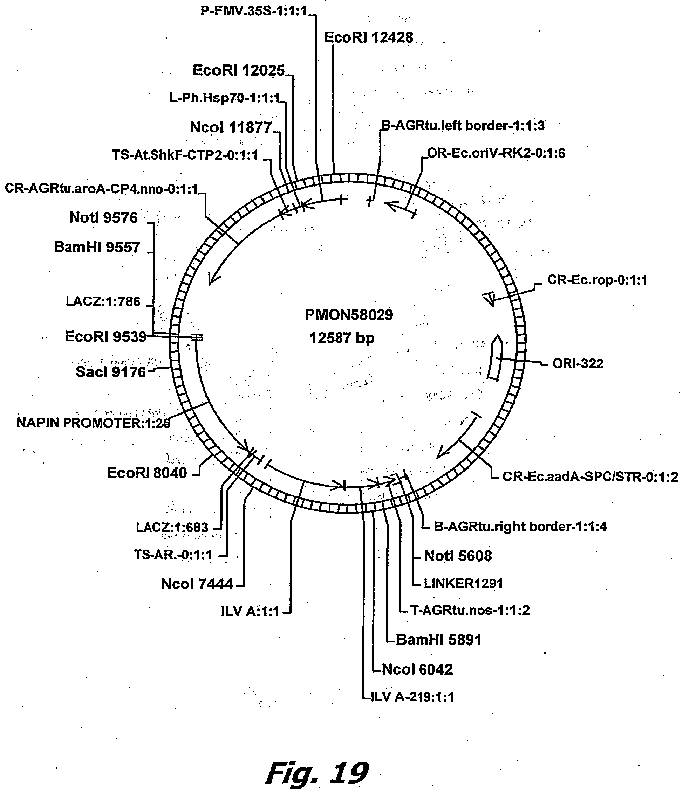

Figure 19 depicts a restriction map of plasmid pMON58029.

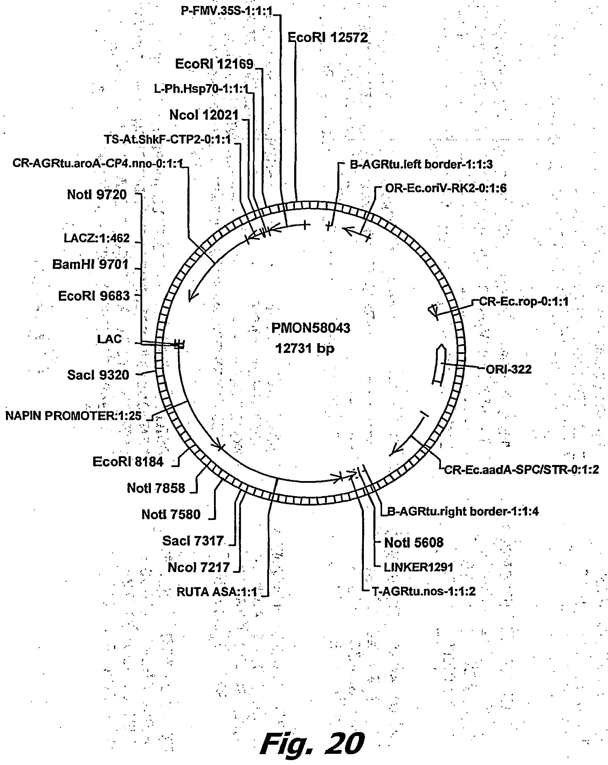

Figure 20 depicts a restriction map of plasmid pMON58043.
Figure 21A-D depicts a multiple sequence alignment of monomeric "TrpEG" anthranilate synthases having SEQ ID NOs: 4 and 43 (derived from *Agrobacterium tumefaciens* and *Rhizobium meliloti,* respectively) with the TrpE (α) and TrpG (β) domains of heterotetrameric anthranilate synthases from *Sulfolobus solfataricus* (SEQ ID NO: 44) arid *Arabidopsis thaliana* (SEQ ID NO: 45). Linker regions are underlined.
Figure 22 is a restriction map of plasmid pMON52214.
Figure 23 is a restriction map of plasmid pMON53901.
Figure 24 is a restriction map of plasmid pMON39324.
Figure 25 is a restriction map of plasmid pMON39322.
Figure 26 is a restriction map of plasmid pMON39325.
Figure 27 is a graph depicting free tryptophan levels in soybean seeds transformed with pMON39325. There were five observations from each event. NT represents non-transgenic soybean seed.
Figure 28 is a restriction map of plasmid pMON25997.
Figure 29 is a restriction map of plasmid pMON62000.
Figure 30 depicts the sequence of the truncated trpE gene of *Escherichia coli* EMG2 (K-12 wt F+) (SEQ ID NO: 46). The first 30bp and the last 150bp of this trpE nucleic acid are connected by an EcoR1 restriction site. The beginning of the trpG gene follows the trpE stop codon.

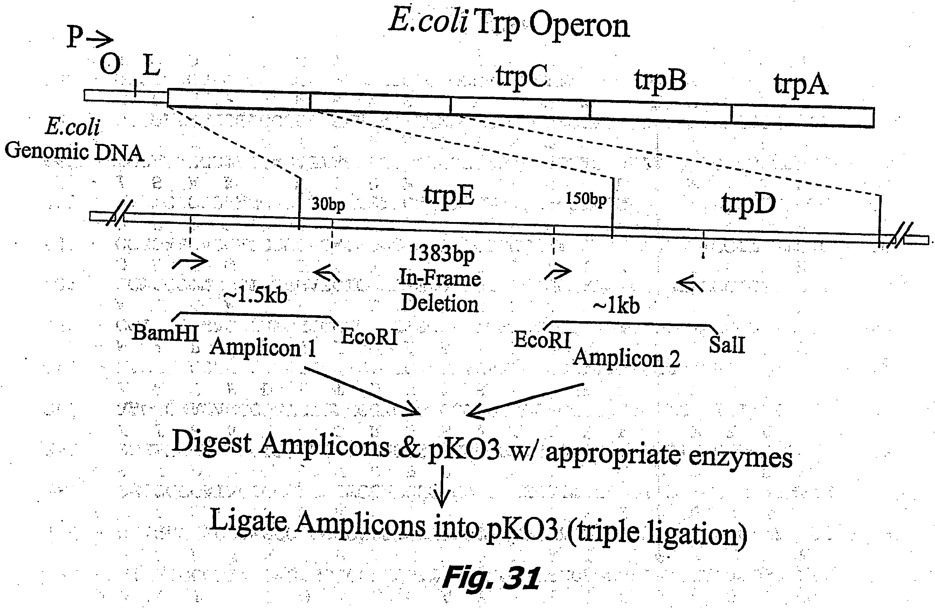

Figure 31 schematically depicts construction of the in-frame deletion in the *E. coli* trpE gene.

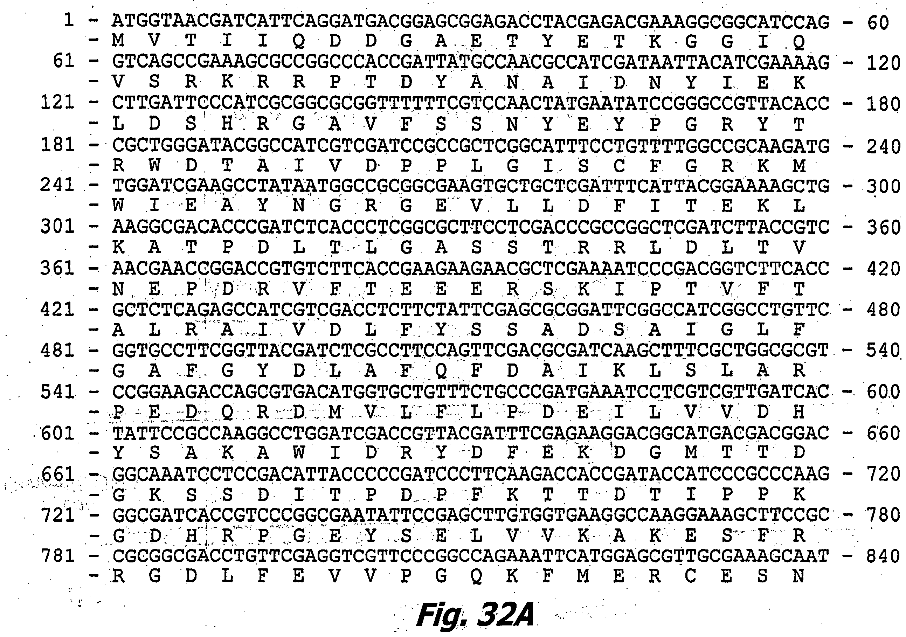

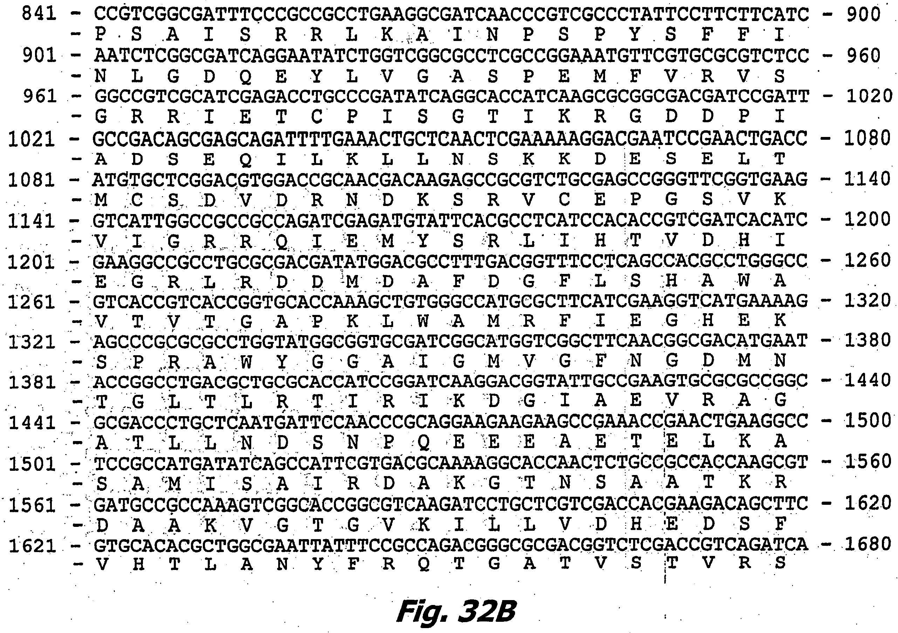

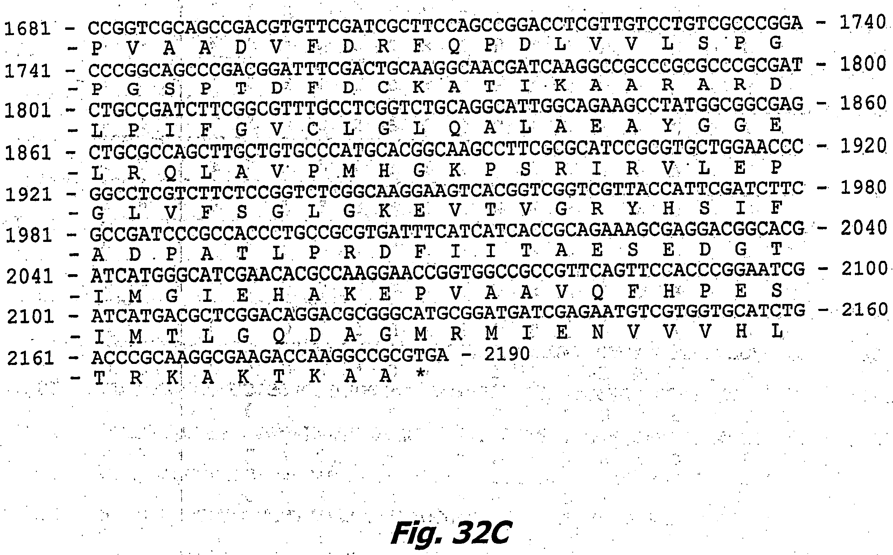

Figure 32A-C depicts the DNA (SEQ ID NO: 1) and amino acid (SEQ ID NO: 4) sequences of the α-domain of the anthranilate synthase gene isolated from *Agrobacterium tumefaciens.*

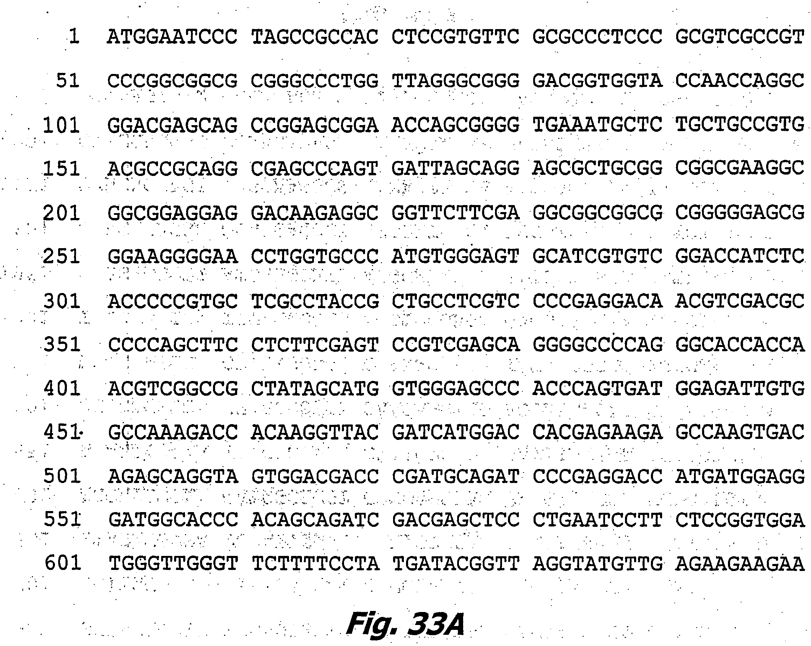

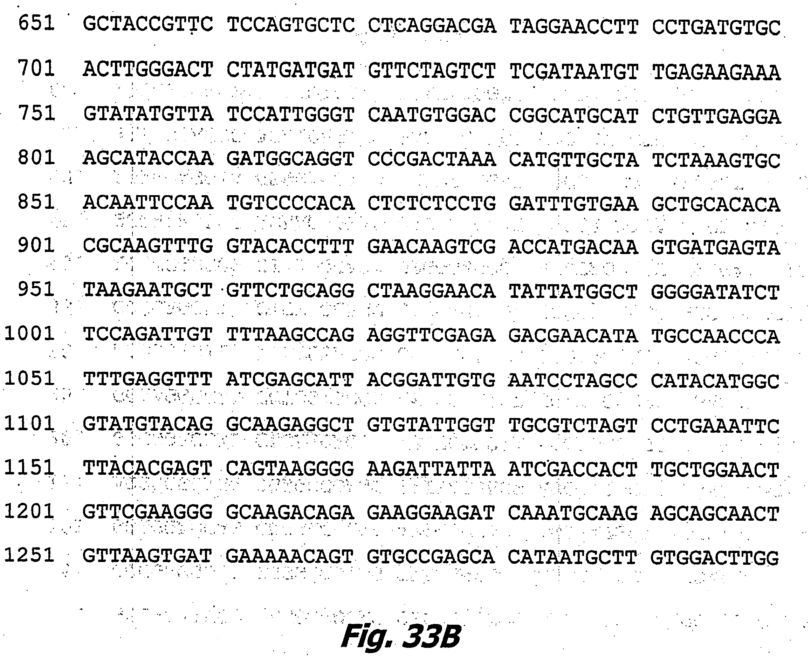

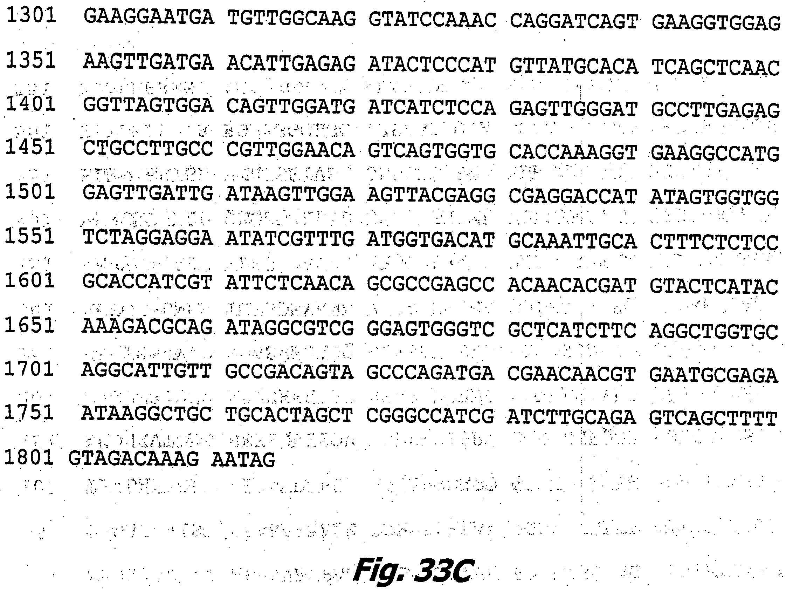

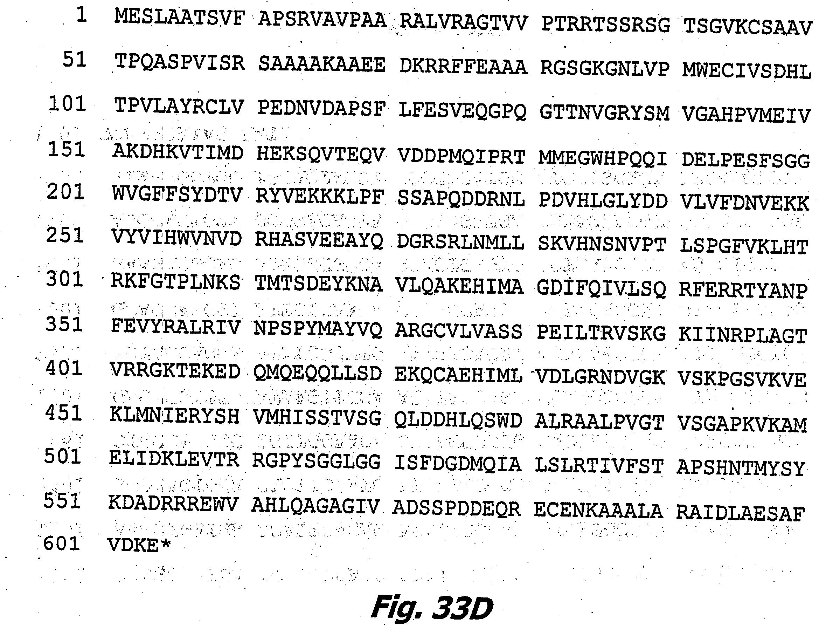

Figure 33A-C depicts the DNA (SEQ ID NO: 2) sequence of the α-domain of the anthranilate synthase gene isolated from *Zea mays.* Figure 33D depicts the amino acid (SEQ ID NO: 5) sequence of the α-domain of the anthranilate synthase gene isolated from *Zea mays.*
Figure 34 is a restriction map of plasmid pMON58120.
Figure 35A-E provides a sequence comparison of anthranilate synthase amino acid sequences from *Agrobacterium tumefaciens* (AgrTu_15889565) (SEQ ID NO: 4), *Rhizobium meliloti* (RhiMe_136328) (SEQ ID NO: 7), *Mesorhizobium loti* (MesLo_13472468) (SEQ ID NO: 77), *Azospirillum brasilense* (AzoBr_1717765) (SEQ ID NO: 78), *Brucella melitensis* (BruMe_17986732) (SEQ ID NO: 79), *Nostoc sp.* (Nostoc_17227910) (SEQ ID NO: 80), *Nostoc sp.* (Nostoc_17230725) (SEQ ID NO: 81), and *Rhodopseudomonas palustris* (RhoPa_TrpEG) (SEQ ID NO: 82).
Figure 36A-B provides an optimized nucleotide sequence for *Agrobacterium tumefaciens* anthranilate synthase (SEQ ID NO: 75).
Figure 37A-C provides an alignment of the wild type (top strand) and optimized (bottom strand) *Agrobacterium tumefaciens* anthranilate synthase nucleotide sequences (SEQ ID NOs: 1 and 75). These two sequences are 94% identical, as demonstrated by the middle strand.

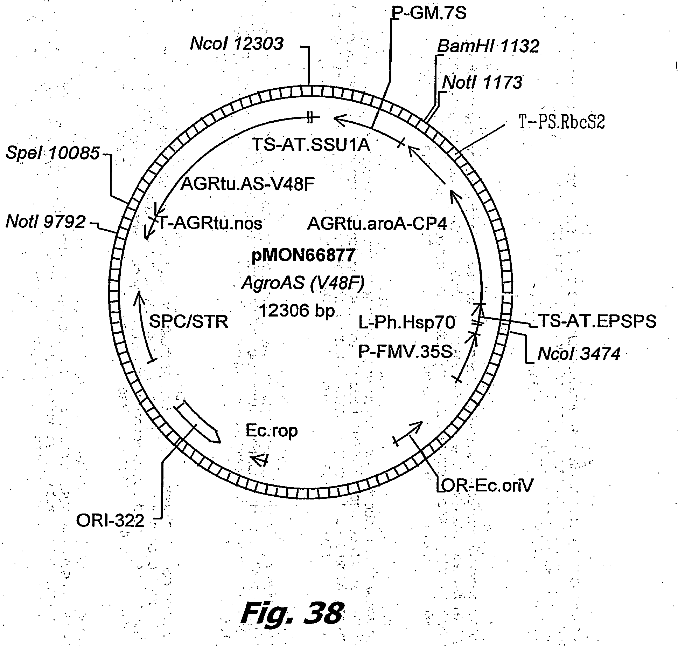

Figure 38 is a restriction map of plasmid pMON66877.

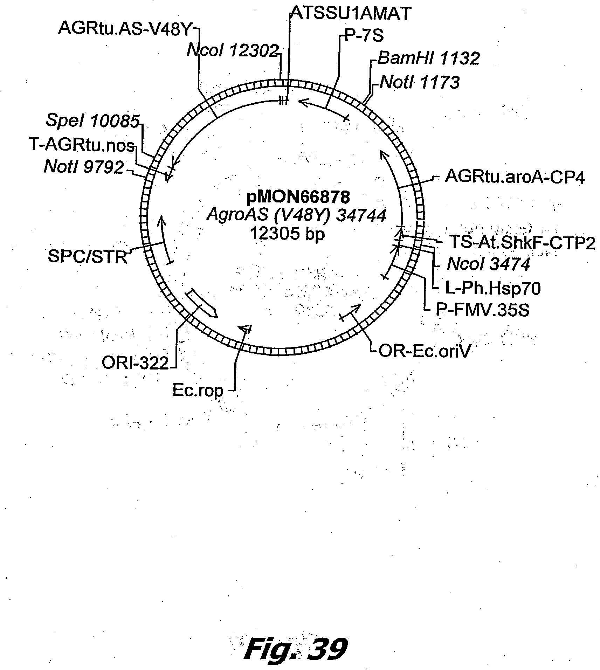

Figure 39 is a restriction map of plasmid pMON66878.

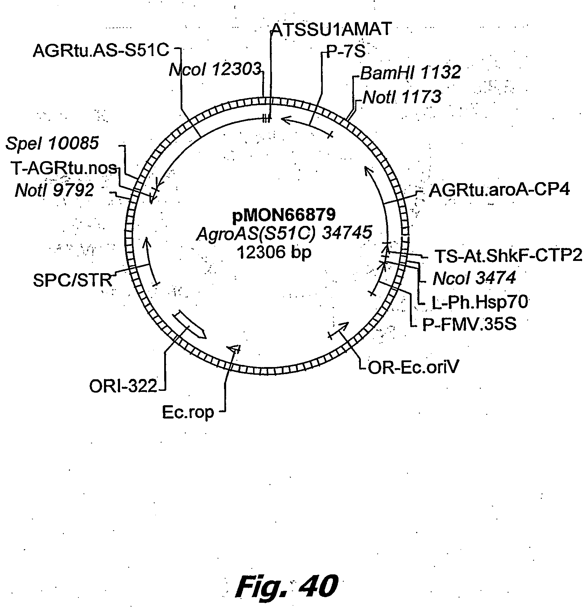

Figure 40 is a restriction map of plasmid pMON66879.
Figure 41 is a restriction map of plasmid pMON66595.
Figure 42 is a restriction map of plasmid pMON66599.
Figure 43 is a restriction map of plasmid pMON66598.
Figure 44 is a restriction map of plasmid pMON66596.
Figure 45 is a restriction map of plasmid pMON79951.
Figure 46 is a restriction map of plasmid pMON79955.
Figure 47 is a restriction map of plasmid pMON79956.
Figure 48 is a restriction map of plasmid pMON36524.
Figure 49 is a restriction map of plasmid pMON30167.

### Detailed Description of the Invention

Isolated DNAs, vectors, host cells and transgenic plants comprising an isolated nucleic acid encoding an anthranilate synthase capable of providing high levels of tryptophan upon expression within the plant are described herein. In one embodiment, the isolated nucleic acid encodes a monomeric anthranilate synthase (AS). In other embodiments, the isolated nucleic acid encodes an anthranilate synthase, or a domain thereof, that is substantially resistant to inhibition by free L-tryptophan or an amino acid analog of tryptophan. Expression of the anthranilate synthase, or domain thereof, elevates the level of tryptophan, e.g., free tryptophan in the seed, over the level present in the plant absent such expression.

Methods are also provided for producing transgenic plants having nucleic acids associated with increased anthranilate synthase activity, and producing cultured cells, plant tissues, plants, plant parts and seeds that produce high levels of tryptophan. Such transgenic plants can preferably sexually transmit the ability to produce high levels of tryptophan to their progeny. Also described are methods for producing isolated DNAs encoding mutant anthranilate synthases, and cell culture selection techniques to select for novel genotypes that overproduce tryptophan and/or are resistant to tryptophan analogs. For example, to produce soybean lines capable of producing high levels of tryptophan, transgenic soybean cells that contain at least one isolated DNA are prepared and characterized, then regenerated into plants. Some of the isolated DNAs are resistant to growth inhibition by the tryptophan analog.

### Definitions

As used herein, "altered" levels of tryptophan in a transformed plant, plant tissue, plant part or plant cell are levels which are greater or lesser than the levels found in the corresponding untransformed plant, plant tissue, plant part or plant cell.

As used herein, a "α-domain" is a portion of an enzyme or enzymatic complex that binds chorismate and eliminates the enolpyruvate side chain. Such an α-domain can be encoded by a TrpE gene. In some instances, the α-domain is a single polypeptide that functions only to bind chorismate and to eliminate the enolpyruvate side chain from chorismate. In other instances, the α-domain is part of a larger polypeptide that can carry out other enzymatic functions in addition to binding chorismate and eliminating the enolpyruvate side chain from chorismate.

The term "β-domain" refers to a portion of an enzyme or enzymatic complex that transfers an amino group from glutamine to the position on the chorismate ring that resides between the carboxylate and the enolpyruvate moieties. Such a β-domain can be encoded by a TrpG gene. In some instances, the β-domain is a single polypeptide that functions only to transfer an amino group from glutamine to the position on the chorismate ring that resides between the carboxylate and the enolpyruvate moieties. In other instances, the β-domain is part of a larger polypeptide that can carry out other enzymatic functions in addition to transferring an amino group from glutamine to the position on the chorismate ring that resides between the carboxylate and the enolpyruvate moieties.

As used herein, "an amino acid analog of tryptophan" is an amino acid that is structurally related to tryptophan and that can bind to the tryptophan-binding site in a wild type anthranilate synthase. These analogs include, but are not limited to, 6-methylanthranilate, 5-methyltryptophan, 4-methyltryptophan, 5-fluorotryptophan, 5-hydroxytryptophan, 7-azatryptophan, 3β-indoleacrylic acid, 3-methylanthranilic acid, and the like.

The phrase "consists essentially of' as used with respect to the present DNA molecules, sequences or segments is defined to mean that a major portion of the DNA molecule, sequence or segment encodes an anthranilate synthase. Unless otherwise indicated, the DNA molecule, sequence or segment generally does not encode proteins other than an anthranilate synthase.

The term "complementary to" is used herein to mean that the sequence of a nucleic acid strand could hybridize to all, or a portion, of a reference polynucleotide sequence. For illustration, the nucleotide sequence "TATAC" has 100% identity to a reference sequence 5'-TATAC-3' but is 100% complementary to a reference sequence 5'-GTATA-3'.

As used herein, an "exogenous" anthranilate synthase is an anthranilate synthase that is encoded by an isolated DNA that has been introduced into a host cell, and that is preferably not identical to any DNA sequence present in the cell in its native, untransformed state. An "endogenous" or "native" anthranilate synthase is an anthranilate synthase that is naturally present in a host cell or organism.

As used herein, "increased" or "elevated" levels of free L-tryptophan in a plant cell, plant tissue, plant part or plant are levels that are about 2 to 200 times, preferably about 5 to 150 times, and more preferably about 10-100 times, the levels found in an untransformed plant cell, plant tissue, plant part or plant, *i.e*., one where the genome has not been altered by the presence of an exogenous anthranilate synthase nucleic acid or domain thereof. For example, the levels of free L-tryptophan in a transformed plant seed are compared with those in an untransformed plant seed ("the starting material").

DNA molecules encoding an anthranilate synthase, and DNA molecules encoding a transit peptide or marker/reporter gene are "isolated" in that they were taken from their natural source and are no longer within the cell where they normally exist. Such isolated DNA molecules may have been at least partially prepared or manipulated *in vitro, e.g*., isolated from a cell in which they are normally found, purified, and amplified. Such isolated DNA molecules can also be "recombinant" in that they have been combined with exogenous DNA molecules or segments. For example, a recombinant DNA can be an isolated DNA that is operably linked to an exogenous promoter, or to a promoter that is endogenous to the host cell.

As used herein with respect to anthranilate synthase, the term "monomeric" means that two or more anthranilate synthase domains are incorporated in a functional manner into a single polypeptide chain. The monomeric anthranilate synthase may be assembled *in vivo* into a dimeric form. Monomeric anthranilate synthase nucleic acids and polypeptides can be isolated from various organisms such as *Agrobacterium tumefaciens, Anabaena* M22983, *Azospirillum brasilense, Brucella melitensis, Euglena gracilis, Mesorhizobium loti*, *Nostoc sp.* PCC7120 or *Rhizobium meliloti.* Alternatively, monomeric anthranilate synthase nucleic acids and polypeptides can be constructed from a combination of domains selected from any convenient monomeric or multimeric anthranilate synthase gene. Such organisms include, for example, *Agrobacterium tumefaciens, Anabaena* M22983, *Arabidopsis thaliana, Azospirillum brasilense, Brucella melitensis, Mesorhizobium loti, Nostoc sp.* PCC7120, *Rhizobium meliloti, Rhodopseudomonas palustris, Ruta graveolens, Sulfolobus solfataricus, Salmonella typhimurium*, *Serratia marcescens,* soybean, rice, cotton, *Zea mays,* or any gene encoding a subunit or domain of anthranilate synthase. Nucleic acids encoding the selected domains can be linked recombinantly. For example, a nucleic acid encoding the C-terminus of an α-domain can be linked to a nucleic acid encoding the N-terminus of the β-domain, or vice versa, by forming a phosphodiester bond. As an alternative, such single domain polypeptides can be linked chemically. For example, the α-domain can be linked via its C-terminus to the N-terminus of the β-domain, or vice versa, by forming a peptide bond.

As used herein, a "native" gene means a gene that has not been changed *in vitro, i.e.,* a "wild-type" gene that has not been mutated *in vitro.*

The term "plastid" refers to the class of plant cell organelles that includes amyloplasts, chloroplasts, chromoplasts, elaioplasts, eoplasts, etioplasts, leucoplasts, and proplastids. These organelles are self-replicating, and contain what is commonly referred to as a "chloroplast genome," a circular DNA molecule that ranges in size from about 120 to about 217 kb, depending upon the plant species, and which usually contains an inverted repeat region.

As used herein, "polypeptide" means a continuous chain of amino acids that are all linked together by peptide bonds, except for the N-terminal and C-terminal amino acids that have amino and carboxylate groups, respectively, and that are not linked in peptide bonds. Polypeptides can have any length and can be post-translationally modified, for example, by glycosylation or phosphorylation.

As used herein, a plant cell, plant tissue or plant that is "resistant or tolerant to inhibition by an amino acid analog of tryptophan" is a plant cell, plant tissue, or plant that retains at least about 10% more anthranilate synthase activity in the presence of an analog of L-tryptophan, than a corresponding wild type anthranilate synthase. In general, a plant cell, plant tissue, or plant that is "resistant or tolerant to inhibition by an amino acid analog of tryptophan" can grow in an amount of an amino acid analog of tryptophan that normally inhibits growth of the untransformed plant cell, plant tissue, or plant, as determined by methodologies known to the art. For example, a homozygous backcross converted inbred plant transformed with a DNA molecule that encodes an anthranilate synthase that is substantially resistant or tolerant to inhibition by an amino acid analog of tryptophan grows in an amount of an amino acid analog of tryptophan that inhibits the growth of the corresponding, *i.e*., substantially isogenic, recurrent inbred plant.

As used herein, an anthranilate synthase that is "resistant or tolerant to inhibition by tryptophan or an amino acid analog of tryptophan" is an anthranilate synthase that retains greater than about 10% more activity than a corresponding "wild-type" or native susceptible anthranilate synthase, when the tolerant/resistant and wild type anthranilate synthases are exposed to equivalent amounts of tryptophan or an amino acid analog of tryptophan. Preferably the resistant or tolerant anthranilate synthase retains greater than about 20% more activity than a corresponding "wild-type" or native susceptible anthranilate synthase.

As used herein with respect to anthranilate synthase, the phrase "a domain thereof," includes a structural or functional segment of a full-length anthranilate synthase. A structural domain includes an identifiable structure within the anthranilate synthase. An example of a structural domain includes an alpha helix, a beta sheet, an active site, a substrate or inhibitor binding site and the like. A functional domain includes a segment of an anthranilate synthase that performs an identifiable function such as a tryptophan binding pocket, an active site or a substrate or inhibitor binding site. Functional domains of anthranilate synthase include those portions of anthranilate synthase that can catalyze one step in the biosynthetic pathway of tryptophan. For example, an α-domain is a domain that can be encoded by *trp*E and that can transfer NH₃ to chorismate and form anthranilate. A β-domain can be encoded by *trp*G and can remove an amino group from glutamine to form ammonia. Hence, a functional domain includes enzymatically active fragments and domains of an anthranilate synthase. Mutant domains of anthranilate synthase are also contemplated. Wild type anthranilate synthase nucleic acids utilized to make mutant domains include, for example, any nucleic acid encoding a domain of *Agrobacterium tumefaciens, Anabaena* M22983, *Arabidopsis thaliana, Azospirillum brasilense, Brucella melitensis*, *Mesorhizobium loti, Nostoc sp.* PCC7120, *Rhizobium meliloti, Rhodopseudomonas palustris, Ruta graveolens, Sulfolobus solfataricus, Salmonella typhimurium*, *Serratia marcescens,* soybean, rice, cotton, wheat, tobacco, Zea *mays,* or any gene encoding a subunit or domain of anthranilate synthase that can comprise at least one amino acid substitution in the coding region thereof. Domains that are mutated or joined to form a monomeric anthranilate synthase having increased tryptophan biosynthetic activity, greater stability, reduced sensitivity to tryptophan or an analog thereof, and the like, are of particular interest.

The term "5' UTR" refers to the untranslated region of DNA upstream, or 5', of the coding region of a gene.

The term "3' UTR" refers to the untranslated region of DNA downstream, or 3', of the coding region of a gene.

The term "substantially homologous" refers to two sequences which are at least about 90% identical in sequence, as measured by the BestFit program described herein (Version 10; Genetics Computer Group, Inc., University of Wisconsin Biotechnology Center, Madison, WI), using default parameters.

The percent of sequence identity is preferably determined using the "Best Fit" or "Gap" program of the Sequence Analysis Software Package (Version 10; Genetics Computer Group, Inc., University of Wisconsin Biotechnology Center, Madison, WI). "Gap" utilizes the algorithm of Needleman and Wunsch (1970) to find the alignment of two sequences that maximizes the number of matches and minimizes the number of gaps.

"BestFit" performs an optimal alignment of the best segment of similarity between two sequences and inserts gaps to maximize the number of matches using the local homology algorithm of Smith and Waterman (Smith and Waterman, 1981; Smith *et al*., 1983). The percent identity is most preferably determined using the "Best Fit" program using default parameters. As used herein, the term "operatively linked" means that a promoter is connected to a coding region in such a way that the transcription of that coding region is controlled and regulated by that promoter. Means for operatively linking a promoter to a coding region are well known in the art.

### General Concepts

The present invention relates to transgenic soybean plants that produce elevated levels of free L-tryptophan as described above. The overproduction results from the introduction and expression of a nucleic acid encoding anthranilate synthase, or a domain thereof. Such anthranilate synthase nucleic acids include wild type or mutant α-domains, or monomeric forms of anthranilate synthase. A monomeric form of anthranilate synthase comprises at least two anthranilate synthase domains in a single polypeptide chain, e.g., an α-domain linked to a β-domain.

Native plant anthranilate synthases are generally quite sensitive to feedback inhibition by L-tryptophan and analogs thereof Such inhibition constitutes a key mechanism for regulating the tryptophan synthetic pathway. Therefore, an anthranilate synthase or a domain thereof that is highly active, more efficient or that is inhibited to a lesser extent by tryptophan or an analog thereof will likely produce elevated levels of tryptophan. According to the invention, the *Agrobacterium tumefaciens* anthranilate synthase is particularly useful for producing high levels of tryptophan.

To generate high levels of tryptophan in a plant or a selected host cell, the selected anthranilate synthase nucleic acid is isolated and may be manipulated *in vitro* to include regulatory signals required for gene expression in plant cells or other cell types. Because the tryptophan biosynthetic pathway in plants is reported to be present within plastids, the exogenous anthranilate synthase nucleic acids are either introduced into plastids or are modified by adding a nucleic acid segment encoding an amino-terminal plastid transit peptide. Such a plastid transit peptide can direct the anthranilate synthase gene product into plastids. In some instances the anthranilate synthase may already contain a plastid transport sequence, in which case there is no need to add one.

In order to alter the biosynthesis of tryptophan, the nucleic acid encoding an anthranilate synthase activity must be introduced into plant cells or other host cells and these transformed cells identified, either directly or indirectly. An entire anthranilate synthase or a useful portion or domain thereof can be used. The anthranilate synthase is stably incorporated into the plant cell genome. The transcriptional signals controlling expression of the anthranilate synthase must be recognized by and be functional within the plant cells or other host cells. That is, the anthranilate synthase must be transcribed into messenger RNA (mRNA), and the mRNA must be stable in the plant cell nucleus and be transported intact to the cytoplasm for translation. The anthranilate synthase mRNA must have appropriate translational signals to be recognized and properly translated by plant cell ribosomes. The polypeptide gene product must substantially escape proteolytic attack in the cytoplasm, be transported into the correct cellular compartment (e.g. a plastid) and be able to assume a three-dimensional conformation that will confer enzymatic activity. The anthranilate synthase must further be able to function in the biosynthesis of tryptophan and its derivatives; that is, it must be localized near the native plant enzymes catalyzing the flanking steps in biosynthesis (presumably in a plastid) in order to obtain the required substrates and to pass on the appropriate product.

Even if all these conditions are met, successful overproduction of tryptophan is not a predictable event. The expression of some transgenes may be negatively affected by nearby chromosomal elements. If the high level of tryptophan is achieved by mutation to reduce feedback inhibition, there may be other control mechanisms compensating for the reduced regulation at the anthranilate synthase step. There may be mechanisms that increase the rate of breakdown of the accumulated amino acids. Tryptophan and related amino acids must be also overproduced at levels that are not toxic to the plant. Finally, the introduced trait must be stable and heritable in order to permit commercial development and use.

### Isolation and Identification of DNA Coding for an Anthranilate Synthase

Nucleic acids encoding an anthranilate synthase can be identified and isolated by standard methods, for eample, as described by Sambrook et al., in "Molecular Cloning: A Laboratory Manual", 2nd Edition (1989); Sambrook and Russell, in "Molecular Cloning: A Laboratory Manual", 3rd Edition (January 15, 2001). For example, a DNA sequence encoding an anthranilate synthase or a domain thereof can be identified by screening of a DNA or cDNA library generated from nucleic acid derived from a particular cell type, cell line, primary cells, or tissue. Examples of libraries useful for identifying and isolating an anthranilate synthase include, but are not limited to, a cDNA library derived from *Agrobacterium tumefaciens strain* A348, maize inbred line B73 (Stratagene, La Jolla, California, Cat. #937005, Clontech, Palo Alto, California, Cat. # FL1032a, #FL1032b, and FL1032n), genomic library from maize inbred line Mo17 (Stratagene, Cat. #946102), genomic library from maize inbred line B73 (Clontech, Cat. # FL1032d), genomic DNA from *Anabaena* M22983 (*e.g*., Genbank Accession No. GI 152445), *Arabidopsis thaliana, Azospirillum brasilense* (*e.g.,* Genbank Accession No. GI 1174156), *Brucella melitensis* (GI 17982357), *Escherichia coli, Euglena gracilis, Mesorhizobium loti* (*e.g*., Genbank Accession No. GI 13472468), *Nostoc sp*. PCC7120 (*e.g*., Genbank Accession No. GI 17227910 or GI 17230725), *Rhizobium meliloti* (*e.g*., Genbank Accession No. GI 95177), *Ruta graveolens*, *Rhodopseudomonas palustris, Salmonella typhimurium*, *Serratia marcescens, Sulfolobus solfataricus*, soybean, rice, cotton, wheat, tobacco, *Zea mays* (maize), or other species. Moreover, anthranilate synthase nucleic acids can be isolated by nucleic acid amplification procedures using genomic DNA, mRNA or cDNA isolated from any of these species.

Screening for DNA fragments that encode all or a portion of the sequence encoding an anthranilate synthase can be accomplished by screening plaques from a genomic or cDNA library for hybridization to a probe of an anthranilate synthase gene from other organisms or by screening plaques from a cDNA expression library for binding to antibodies that specifically recognize anthranilate synthase. DNA fragments that hybridize to anthranilate synthase probes from other organisms and/or plaques carrying DNA fragments that are immunoreactive with antibodies to anthranilate synthase can be subcloned into a vector and sequenced and/or used as probes to identify other cDNA or genomic sequences encoding all or a portion of the desired anthranilate synthase gene. Preferred cDNA probes for screening a maize or plant library can be obtained from plasmid clones pDPG600 or pDPG602.

A cDNA library can be prepared, for example, by random oligo priming or oligo dT priming. Plaques containing DNA fragments can be screened with probes or antibodies specific for anthranilate synthase. DNA fragments encoding a portion of an anthranilate synthase gene can be subcloned and sequenced and used as probes to identify a genomic anthranilate synthase gene. DNA fragments encoding a portion of a bacterial or plant anthranilate synthase can be verified by determining sequence homology with other known anthranilate synthase genes or by hybridization to anthranilate synthase-specific messenger RNA. Once cDNA fragments encoding portions of the 5', middle and 3' ends of an anthranilate synthase are obtained, they can be used as probes to identify and clone a complete genomic copy of the anthranilate synthase gene from a genomic library.

Portions of the genomic copy or copies of an anthranilate synthase gene can be sequenced and the 5' end of the gene identified by standard methods including either by DNA sequence homology to other anthranilate synthase genes or by RNAase protection analysis, for example, as described by Sambrook et al., in "Molecular Cloning: A Laboratory Manual", 2nd Edition (1989); Sambrook and Russell, in "Molecular Cloning: A Laboratory Manual", 3rd Edition (January 15, 2001). The 3' and 5' ends of the target gene can also be located by computer searches of genomic sequence databases using known AS coding regions. Once portions of the 5' end of the gene are identified, complete copies of the anthranilate synthase gene can be obtained by standard methods, including cloning or polymerase chain reaction (PCR) synthesis using oligonucleotide primers complementary to the DNA sequence at the 5' end of the gene. The presence of an isolated full-length copy of the anthranilate synthase gene can be verified by hybridization, partial sequence analysis, or by expression of a maize anthranilate synthase enzyme.

Exemplary isolated include DNAs having the following nucleotide SEQ ID NO:
SEQ ID NO: 1 *Agrobacterium tumefaciens* (wild type)
SEQ ID NO: 2 *Zea mays* (wild type, alpha2)
SEQ ID NO: 3 *Ruta graveolens*
SEQ ID NO: 46 truncated TrpE gene of *E*. *coli* EMG2 (K-12 wt F+)
SEQ ID NO: 67 *Zea mays* (C28 mutant)
SEQ ID NO: 68 *Zea mays* (C28 + terminator)
SEQ ID NO: 71 Chloroplast Targeting Peptide (g)
SEQ ID NO: 73 Chloroplast Targeting Peptide (a)
SEQ ID NO: 75 *Agrobacterium tumefaciens* (optimized)
SEQ ID NO: 76 *Rhodopseudomonas palustris*
SEQ ID NO: 83 *Rhodopseudomonas palustris* (RhoPa_TrpEG)
SEQ ID NO: 84 *Agrobacterium tumefaciens* V48F mutant
SEQ ID NO: 85 *Agrobacterium tumefaciens* V48Y mutant
SEQ ID NO: 86 *Agrobacterium tumefaciens* S51F mutant
SEQ ID NO: 87 *Agrobacterium tumefaciens* S51C mutant
SEQ ID NO: 88 *Agrobacterium tumefaciens* N52F mutant
SEQ ID NO: 89 *Agrobacterium tumefaciens* P293A mutant
SEQ ID NO: 90 *Agrobacterium tumefaciens* P293G mutant
SEQ ID NO: 91 *Agrobacterium tumefaciens* F298W mutant
SEQ ID NO: 92 *Agrobacterium tumefaciens* S50K mutant
SEQ ID NO: 93 *Agrobacterium tumefaciens* F298A mutant
SEQ ID NO: 94 rice
SEQ ID NO: 95 rice isozyme
SEQ ID NO: 96 maize (U.S. Patent 6,118,047 to Anderson)
SEQ ID NO: 97 wheat
SEQ ID NO: 98 tobacco
SEQ ID NO: 104 *Gossypium hirsutum* (alpha)
SEQ ID NO: 105 *Gossypium hirsutum* (beta)
SEQ ID NO: 106 *Glycine max* (alpha)
SEQ ID NO: 107 *Glycine max* (beta)
SEQ ID NO: 112 *Glycine max* (alpha) with 5' and 3'UTRs
SEQ ID NO: 113 *Glycine max* (beta) with 5' and 3'UTRs
SEQ ID NO: 116 *Zea mays* (beta)
SEQ ID NO: 119 *Oryza sativa* (beta1)
SEQ ID NO: 120 *Oryza sativa* (beta2)
SEQ ID NO: 121 *Mesorhizobium loti*
SEQ ID NO: 122 *Azospirillum brasilense*
SEQ ID NO: 123 *Brucella melitensis*
SEQ ID NO: 124 *Nostoc sp.*
SEQ ID NO: 125 *Nostoc sp.*
SEQ ID NO: 126 *Rhodopseudomonas palustris*
SEQ ID NO: 127 *Bradyrhizobium japonicum*
SEQ ID NO: 128 *Rhodospirillum rubrum*
SEQ ID NO: 129 *Thermobifida fusca*
SEQ ID NO: 134 *Sorghum bicolor* (beta1)
SEQ ID NO: 135 *Sorghum bicolor* (beta2)
SEQ ID NO: 136 *Zea mays* (alpha1)

Certain primers are also useful, for example, primers having SEQ ID NOs: 9-42, 47-56, or 138-143.

The isolated nucleic acid encoding an anthranilate synthase have, for example, any one of the following amino acid sequences.
SEQ ID NO: 4 *Agrobacterium tumefaciens* (wild type)
SEQ ID NO: 5 *Zea mays* (wild type)
SEQ ID NO: 6 *Ruta graveolens*
SEQ ID NO: 7 *Rhizobium meliloti*
SEQ ID NO: 8 *Sulfolobus solfataricus*
SEQ ID NO: 43 *Rhizobium meliloti*
SEQ ID NO: 44 *Sulfolobus solfataricus*
SEQ ID NO: 45 *Arabidopsis thaliana*
SEQ ID NO: 57 *Rhodopseudomonas palustris*
SEQ ID NO: 58 *Agrobacterium tumefaciens* V48F mutant
SEQ ID NO: 59 *Agrobacterium tumefaciens* V48Y mutant
SEQ ID NO: 60 *Agrobacterium tumefaciens* S51F mutant
SEQ ID NO: 61 *Agrobacterium tumefaciens* S51C mutant
SEQ ID NO: 62 *Agrobacterium tumefaciens* N52F mutant
SEQ ID NO: 63 *Agrobacterium tumefaciens* P293A mutant
SEQ ID NO: 64 *Agrobacterium tumefaciens* P293G mutant
SEQ ID NO: 65 *Agrobacterium tumefaciens* F298W mutant
SEQ ID NO: 66 *Zea mays* C28 mutant
SEQ ID NO: 69 *Agrobacterium tumefaciens* S50K mutant
SEQ ID NO: 70 *Agrobacterium tumefaciens* F298A mutant
SEQ ID NO: 74 Chloroplast Targeting Peptide (a)
SEQ ID NO: 72 Chloroplast Targeting Peptide (g)
SEQ ID NO: 77 *Mesorhizobium loti* (MesLo_13472468)
SEQ ID NO: 78 *Azospirillum brasilense* (AzoBr_1717765)
SEQ ID NO: 79 *Brucella melitensis* (BraMe_17986732)
SEQ ID NO: 80 *Nostoc sp.* (Nostoc_17227910)
SEQ ID NO: 81 *Nostoc sp.* (Nostoc_17230725)
SEQ ID NO: 82 *Rhodopseudomonas palustris* RhoPa_TrpEG
SEQ ID NO: 99 rice
SEQ ID NO: 100 rice isozyme
SEQ ID NO: 101 maize (U.S. Patent 6,118,047 to Anderson)
SEQ ID NO: 102 wheat
SEQ ID NO: 103 tobacco
SEQ ID NO: 108 *Gossypium hirsutum* (alpha)
SEQ ID NO: 109 *Gossypium hirsutum* (beta)
SEQ ID NO: 110 *Glycine max* (alpha)
SEQ ID NO: 111 *Glycine max* (beta)
SEQ ID NO: 114 *Zea mays* (ASalpha2) chloroplast targeting peptide
SEQ ID NO: 115 *Zea mays* (ASalphal) chloroplast targeting peptide
SEQ ID NO: 117 *Oryza sativa* (beta)
SEQ ID NO: 118 *Zea mays* (beta)
SEQ ID NO: 130 *Bradyrhizobium japonicum*
SEQ ID NO: 131 *Rhodospirillum rubrum*
SEQ ID NO: 132 *Thermobifida fusca*
SEQ ID NO: 133 *Sorghum bicolor* (beta)
SEQ ID NO: 137 *Zea mays* (ASalphal)

Any of these nucleic acids and polypeptides can be utilized, as well as any mutant, variant or derivative thereof.

### Monomeric Anthranilate Synthases

Monomeric anthranilate synthases from plant and non-plant species are functional in plants and can provide high levels of tryptophan. Surprisingly, monomeric anthranilate synthases from non-plant species function very well in plants even though the sequences of these monomeric anthranilate synthases have low homology with most plant anthranilate synthases. For example, monomeric anthranilate synthases from species as diverse as bacteria, protists, and microbes can be used successfully. In particular, monomeric anthranilate synthases from bacterial species such as *Agrobacterium tumefaciens, Rhizobium meliloti, Mesorhizobium loti, Brucella melitensis, Nostoc sp.* PCC7120, *Azospirillum brasilense, Anabaena* M22983, *Bradyrhizobium janonicum, Rhodospirillum rubrum, and Thermobidfida fusca* are functional in plants and can provide high levels of tryptophan, despite the rather low sequence identity of these monomeric anthranilate synthases with most plant anthranilate synthases.

Transgenic plants containing, for example, the wild type monomeric *Agrobacterium tumefaciens* anthranilate synthase can produce up to about 10,000 to about 12,000 ppm, tryptophan in seeds, with average trp levels ranging up to about 7,000 to about 8,000 ppm. Non-transgenic soybean plants normally have up to only about 100 to about 200 ppm tryptophan in seeds. By comparison transgenic plants containing an added mutant *Zea mays* α domain produce somewhat lower levels of tryptophan (*e.g*., averages up to about 3000 to about 4000 ppm).

Monomeric enzymes may have certain advantages over multimeric enzymes. For example, while the present invention is not to be limited to a specific mechanism, a monomeric enzyme may provide greater stability, coordinated expression, and the like. When domains or subunits of a heterotetrameric anthranilate synthase are synthesized *in vivo*, those domains/subunits must properly assemble into a heterotetrameric form before the enzyme becomes active. Addition of a single domain of anthranilate synthase by transgenic means to a plant may not provide overproduction of the entire heterotetrameric enzyme because there may not be sufficient endogenous amounts of the non-transgenic domains to substantially increase levels of the functional tetramer. Hence, nucleic acids, vectors and enzymes encoding a monomeric anthranilate synthase can advantageously be used to overproduce all of the enzymatic functions of anthranilate synthase.

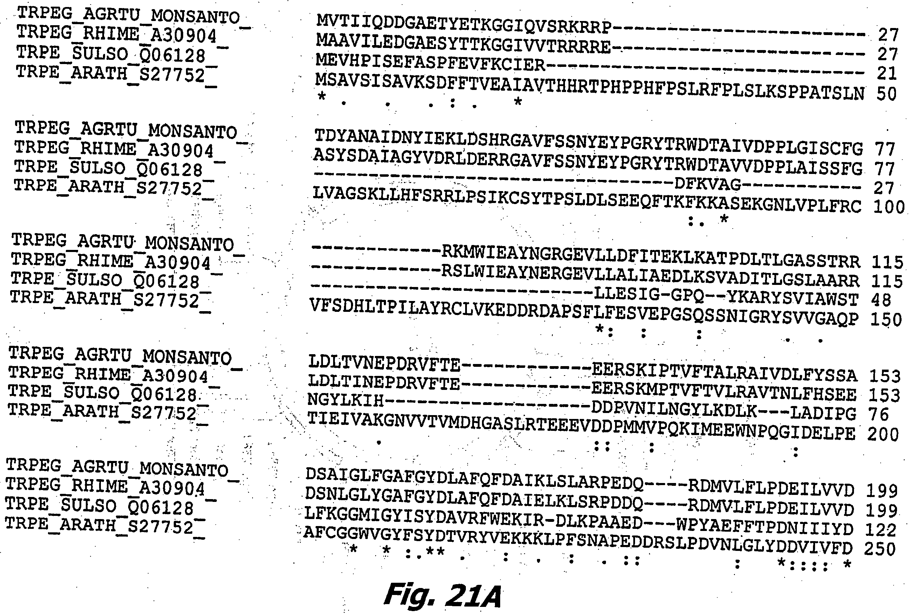

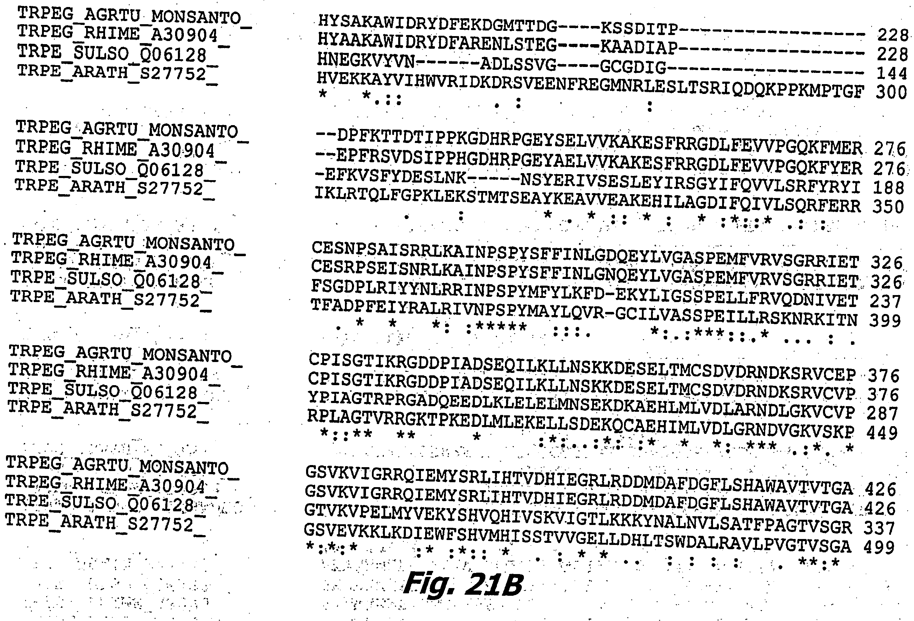

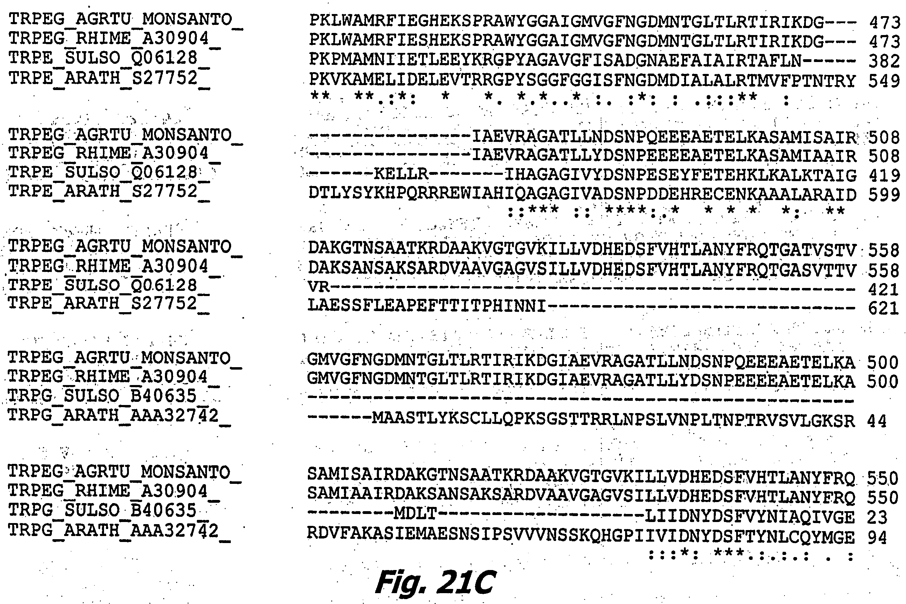

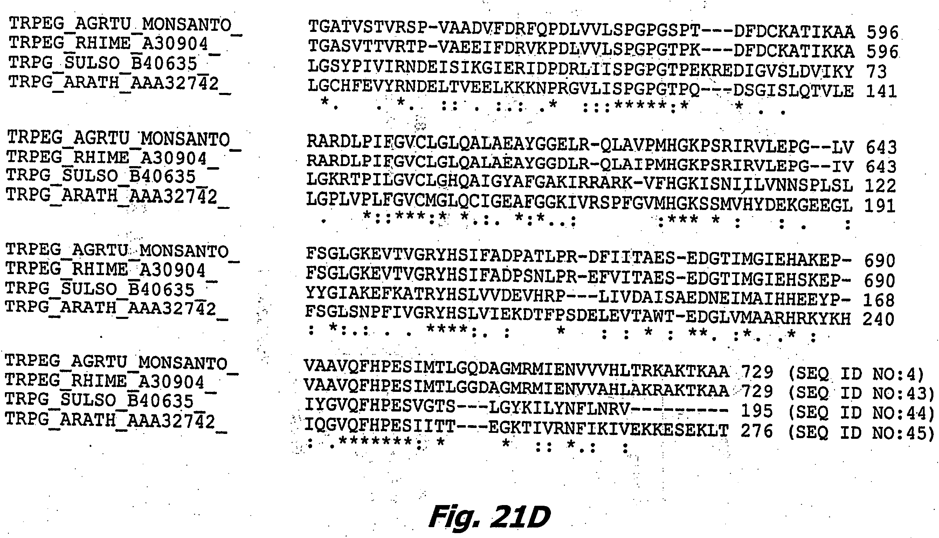

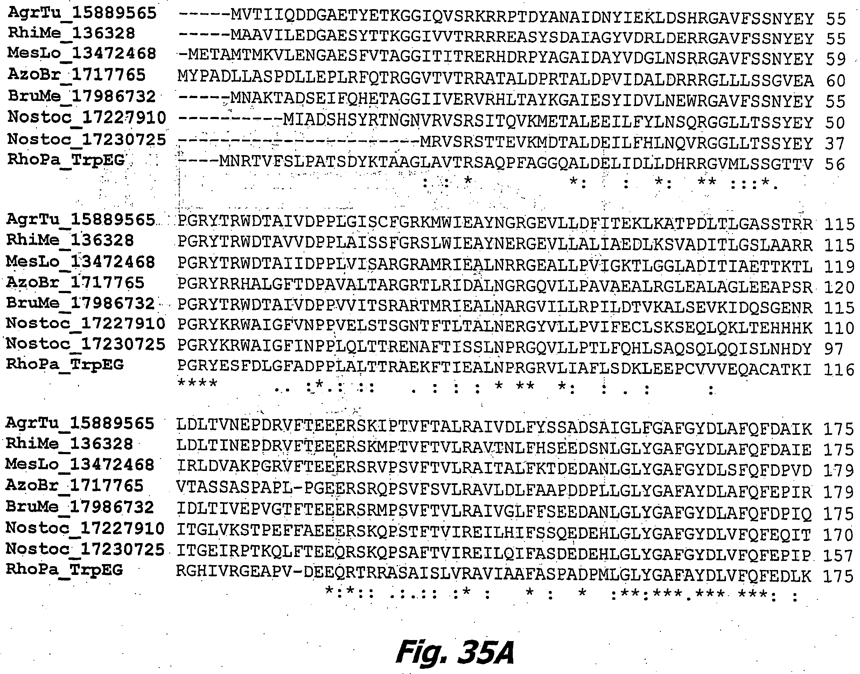

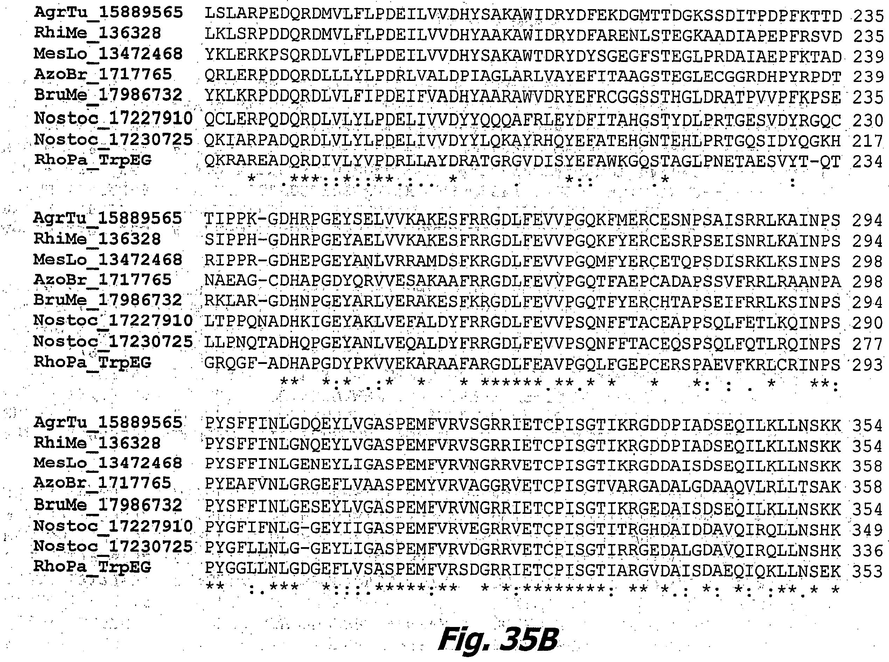

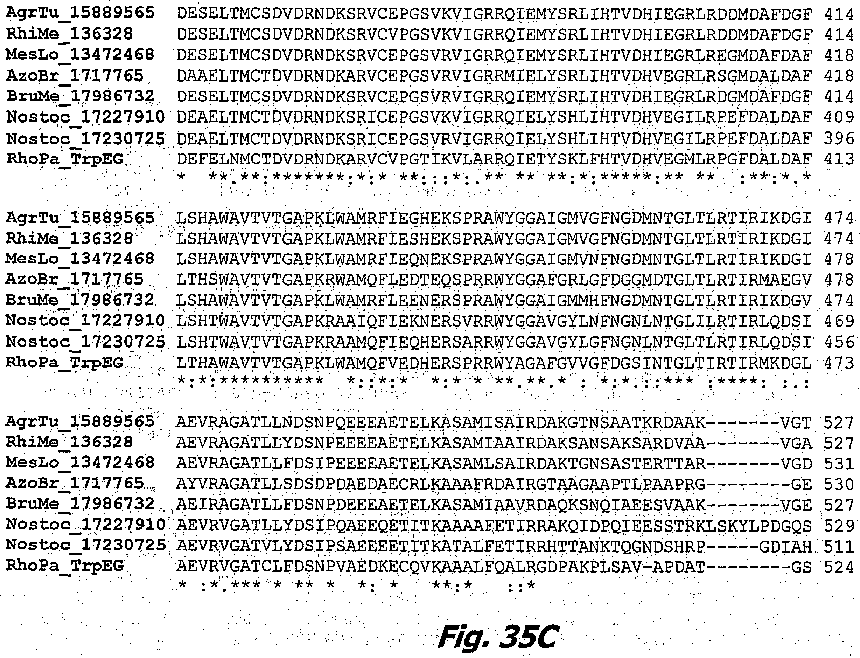

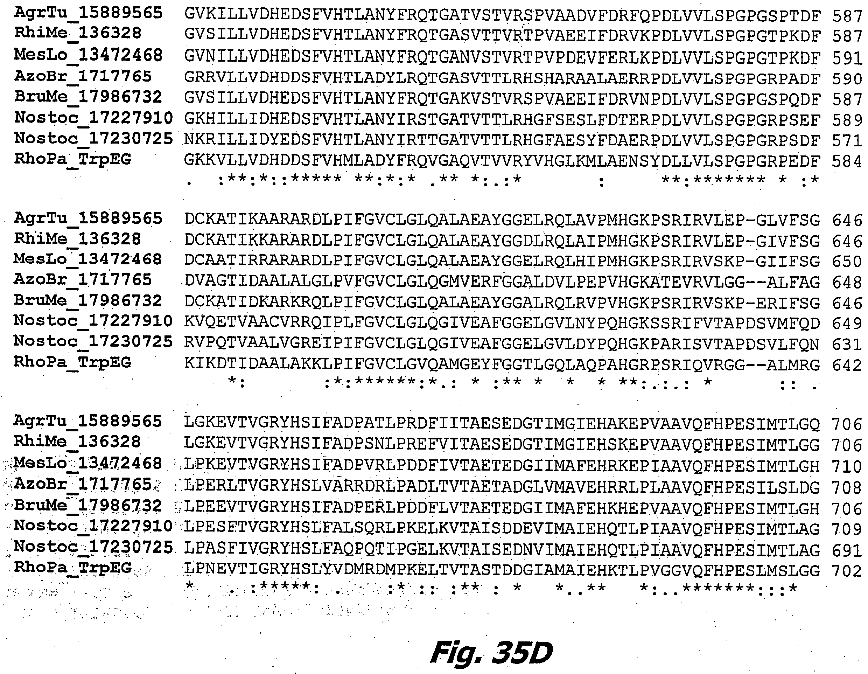

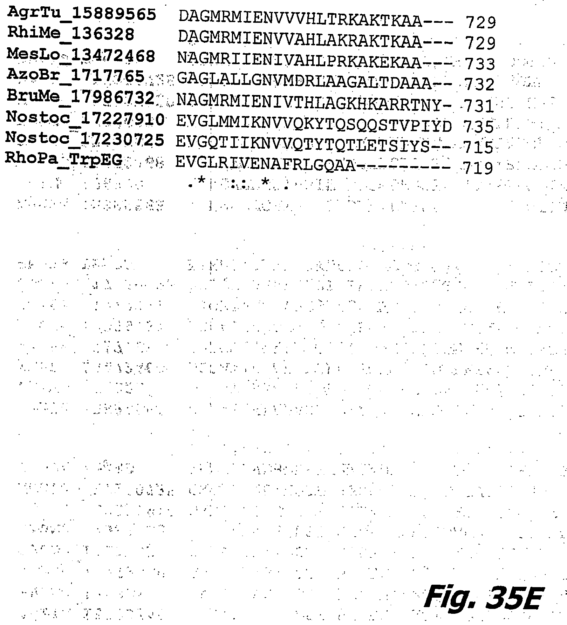

According to the present invention, anthranilate synthase domains from species that naturally produce heterotetrameric anthranilate synthases can be fused or linked to provide monomeric anthranilate synthases that can generate high tryptophan levels when expressed within a plant cell, plant tissue or seed. For example, a monomeric anthranilate synthase can be made by fusing or linking the α- and β-domains of anthranilate synthase so that the sequence of the α - β fusion generally aligns with an anthranilate synthase that is naturally monomeric. Examples of sequence alignments of monomeric and heterotetrameric anthranilate synthases are shown in Figures 21 and 35. Using such sequence alignments, the spacing and orientation of anthranilate synthase domains can be adjusted or modified to generate a monomeric anthranilate construct from heterotetrameric domains that optimally aligns with naturally monomeric anthranilate synthases. Such a fusion protein can be used to increase tryptophan levels in the tissues of a plant.

Heterotetrameric anthranilate synthases, such as the *Sulfolobus solfataricus* anthranilate synthase (*e.g*., Genbank Accession No. GI1004323), share between about 30% to about 87% sequence homology with heterotetrameric anthranilate synthases from other plant and microbial species. Monomeric anthranilate synthases, such as the *A. tumefaciencs* anthranilate synthase, have between about 83% and about 52% identity to the other monomeric enzymes such as *Rhizobium meliloti* (Genbank Accession No. GI 15966140) and *Azospirillum brasilense* (Genbank Accession No. 1717765), respectively. Bae et al., Rhizobium meliloti anthranilate synthase gene: cloning, sequence, and expression in Escherichia coli. J. Bacteriol., 171:3471-3478 (1989); De Troch et al., Isolation and characterization of the Azospirillum brasilense trpE(G) gene, encoding anthranilate synthase. Curr. Microbiol., 34:27-32 (1997).

However, the overall sequence identity shared between naturally monomeric and naturally heterotetrameric anthranilate synthases can be less than 30%. Hence, visual alignment rather than computer-generated alignment, may be needed to optimally align monomeric and heterotetrameric anthranilate synthases. Landmark structures and sequences within the anthranilate synthases can facilitate sequences alignments. For example, the motif "LLES" is part of a β-sheet of the β-sandwich that forms the tryptophan-binding pocket of anthranilate synthases. Such landmark sequences can be used to more confidently align divergent anthranilate synthase sequences, and are especially useful for determination of key residues involved in tryptophan binding.

To accomplish the fusion or linkage of anthranilate synthase domains, the C-terminus of the selected TrpE or α-domain is linked to the N-terminus of the TrpG domain or β-domain. In some cases, a linker peptide may be utilized between the domains to provide the appropriate spacing and/or flexibility. Appropriate linker sequences can be identified by sequence alignment of monomeric and heterotetrameric anthranilate synthases.

The selected β-domains can be cloned, for example, by hybridization, PCR amplification or as described in Anderson et al., U.S. Patent 6,118,047. A plastid transit peptide sequence can also be-linked to the anthranilate synthase coding region using standard methods. For example, an *Arabidopsis* small subunit (SSU) chloroplast targeting peptide (CTP, SEQ ID NOs: 71-74) may be used for this purpose. *See also,* Stark et al., Science, 25 8:287 (1992). The fused gene can then be inserted into a suitable vector for plant transformation as described herein.

### Anthranilate Synthase Mutants

Mutant anthranilate can have any type of mutation including, for example, amino acid substitutions, deletions, insertions, and/or rearrangements. Such mutants can be derivatives or variants of anthranilate synthase nucleic acids and polypeptides specifically identified herein. Alternatively, mutant anthranilate synthases can be obtained from any available species, including those not explicitly identified herein. The mutants, derivatives and variants can have identity with at least about 30% of the amino acid positions of any one of SEQ ID NOs: 4-8, 43-45, 57-66, 69-70, 77-82, 99-111, 117-118, 130-133, and 137, and have anthranilate synthase activity. In a preferred embodiment, polypeptide derivatives and variants have identity with at least about 50% of the amino acid positions of any one of SEQ ID NOs: 4-8, 43-45, 57-66, 69-70, 77-82, 99-111, 117-118, 130-133, and 137, and have anthranilate synthase activity. In a more preferred embodiment, polypeptide derivatives and variants have identity with at least about 60% of the amino acid positions of any one of SEQ ID NOs: 4-8, 43-45, 57-66, 69-70, 77-82, 99-111, 117-118, 130-133, and 137, and have anthranilate synthase activity. In a more preferred embodiment, polypeptide derivatives and variants have identity with at least about 70% of the amino acid positions of any one of SEQ ID NOs: 4-8, 43-45, 57-66, 69-70, 77-82, 99-111, 117-118, 130-133; and 137, and have anthranilate synthase activity. In an even more preferred embodiment, polypeptide derivatives and variants have identity with at least about 80% of the amino acid positions of any one of SEQ ID NOs: 4-8, 43-45, 57-66, 69-70, 77-92, 99-111, 117-118, 130-133, and 137, and have anthranilate synthase activity. In an even more preferred embodiment, polypeptide derivatives and variants have identity with at least about 90% of the amino acid positions of any one of SEQ ID NOs: 4-8, 43-45, 57-66, 69-70, 77-82, 99-111, 117-118, 130-133, and 137, and have anthranilate synthase activity. In an even more preferred embodiment, polypeptide derivatives and variants have identity with at least about 95% of the
amino acid positions of any one of SEQ ID NOs: 4-8, 43-45, 57-66, 69-70, 77-82, 99-111, 117-118, 130-133, and 137, and have anthranilate synthase activity.

In one embodiment, anthranilate synthase mutants, variants and derivatives can be identified by hybridization of any one of SEQ ID NOs: 1-3, 9-42, 46, 47-56, 67-68, 75-76, 83-98, 104-107, 112, 113, 116, 119-129, 134-136, and 138-143, or a fragment or primer thereof under moderate or, preferably, high stringency conditions to a selected source of nucleic acids. Moderate and stringent hybridization conditions are well known to the art, see, for example sections 0.47-9.51 of Sambrook et al., Molecular Cloning: A Laboratory Manual, 2nd Edition (1989); *see, also,* Sambrook and Russell, Molecular Cloning: A Laboratory Manual, 3rd Edition (January 15, 2001). For example, stringent conditions are those that (1) employ low ionic strength and high temperature for washing, for example, 0.015 M NaCl/0.0015 M sodium citrate (SSC); 0.1% sodium lauryl sulfate (SDS) at 50°C, or (2) employ a denaturing agent such as formamide during hybridization, *e.g.,* 50% formamide with 0.1 % bovine serum albumin/0.1% Ficoll/0.1% polyvinylpyrrolidone/50 mM sodium phosphate buffer at pH 6.5 with 750 mM NaCl, 75 mM sodium citrate at 42°C. Another example is use of 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 µg/ml), 0.1% sodium dodecylsulfate (SDS), and 10% dextran sulfate at 42°C, with washes at 42°C in 0.2 x SSC and 0.1% SDS.

Hybridization probes and primers are further described comprising an isolated and purified DNA segment of at least seven nucleotide bases, which can be detectably labeled or bind to a detectable label. Such a hybridization probe or primer can hybridize under moderate or high stringency conditions to either strand of a DNA molecule that encodes an anthranilate synthase. Examples of such hybridization probes and primers include any one of SEQ ID NOs: 9-42, 47-56, and 138-143.

The anthranilate synthase can be any anthranilate synthase, or a mutant or domain thereof, such as the α-domain. The anthranilate synthase can be a monomeric anthranilate synthase. Functional mutants are preferred, particularly those that can generate high levels of tryptophan in a plant, for example, those mutants that are substantially resistant to inhibition by an amino acid analog of tryptophan.

Nucleic acids encoding mutant anthranilate synthases can also be generated from any convenient species, for example, from nucleic acids encoding any domain of *Agrobacterium tumefaciens, Anabaena* M22983 (*e.g.* Genbank Accession No. GI *152445), Arabidopsis thaliana, Azospirillum brasilense* (*e.g.,* Genbank Accession No. GI 1174156), *Brucella melitensis* (*e*.*g*., Genbank Accession No. GI 17982357), *Escherichia coli, Euglena gracilis, Mesorhizobium loti* (*e*.*g*., Genbank Accession No. GI 13472468), *Nostoc sp.* PCC7120 (*e*.*g*., Genbank Accession No. GI 17227910 or GI 17230725), *Rhizobium meliloti* (*e*.*g*., Genbank Accession No. GI 95177), *Ruta graveolens, Rhodopseudomonas palustris, Salmonella typhimurium, Serratia marcescens, Sulfolobus solfataricus, Bradyrhizobium japonicum, Rhodospirillum rubrum, Thermobifida fusca, Sorghum bicolor,* soybean, rice, cotton, wheat, tobacco, *Zea mays* (maize), or any gene encoding a subunit or domain of anthranilate synthase.

Mutants having increased anthranilate synthase activity, reduced sensitivity to feedback inhibition by tryptophan or analogs thereof, and/or the ability to generate increased amounts of tryptophan in a plant are desirable. Such mutants do have a functional change in the level or type of activity they exhibit and are sometimes referred to as "derivatives" of the anthranilate synthase nucleic acids and polypeptides provided herein.

Anthranilate synthase variants as well as anthranilate synthase nucleic acids can have "silent" mutations. As used herein, a silent mutation is a mutation that changes the nucleotide sequence of the anthranilate synthase but that does not change the amino acid sequence of the encoded anthranilate synthase. A variant anthranilate synthase is encoded by a mutant nucleic acid and the variant has one or more amino acid changes that do not substantially change its activity when compared to the corresponding wild type anthranilate synthase.

DNA encoding a mutated anthranilate synthase that is resistant and/or tolerant to L-tryptophan or amino acid analogs of tryptophan can be obtained by several methods. The methods include, but are not limited to:
1. spontaneous variation and direct mutant selection in cultures;
2. direct or indirect mutagenesis procedures on tissue cultures of any cell types or tissue, seeds or plants;
3. mutation of the cloned anthranilate synthase gene by methods such as by chemical mutagenesis; site specific or site directed mutagenesis Sambrook *et al*., cited *supra),* transposon mediated mutagenesis (Berg et al., Biotechnology, 1:417 (1983)), and deletion mutagenesis (Mitra et al., Molec. Gen. Genetic., 215:294 (1989));
4. rational design of mutations in key residues; and
5. DNA shuffling to incorporate mutations of interest into various anthranilate synthase nucleic acids.

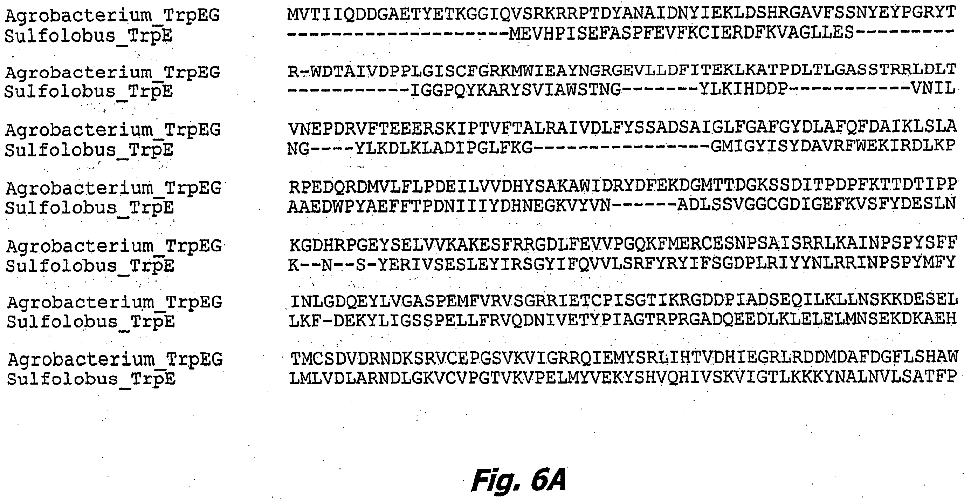

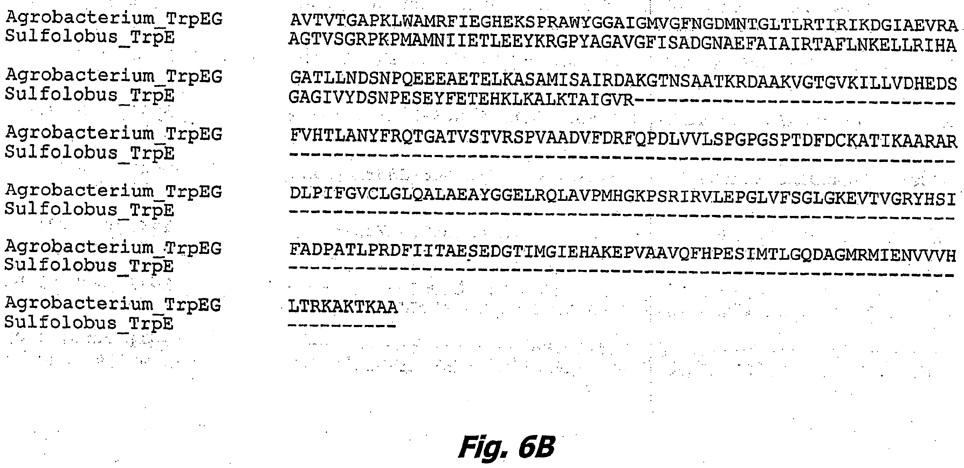

For example, protein structural information from available anthranilate synthase proteins can be used to rationally design anthranilate synthase mutants that have a high probability of having increased activity or reduced sensitivity to tryptophan or tryptophan analogs. Such protein structural information is available, for example, on the Solfulobus solfataricus anthranilate synthase (Knochel et al., Proc. Natl. Acad. Sci. (U.S.A.), 96:9479-9484 (1999)). Rational design of mutations can be accomplished by alignment of the selected anthranilate synthase amino acid sequence with the anthranilate synthase amino acid sequence from an anthranilate synthase of known structure, for example, Sulfolobus solfataricus. See Figures 6, 21, and 35. The predicted tryptophan binding and catalysis regions of the anthranilate synthase protein can be assigned by combining the knowledge of the structural information with the sequence homology. For example, residues in the tryptophan binding pocket can be identified as potential candidates for mutation to alter the resistance of the enzyme to feedback inhibition by tryptophan. Using such structural information, several Agrobacterium tumefaciens anthranilate synthase mutants were rationally designed in the site or domain involved in tryptophan binding.

Using such sequence and structural analysis, regions analogous to the monomeric *Agrobacterium tumefaciens* anthranilate synthase at approximately positions 25-60 or 200-225 or 290-300 or 370-375 were identified in the monomeric *Agrobacterium tumefaciens* anthranilate synthase as being potentially useful residues for mutation to produce active anthranilate synthases that may have less sensitivity to tryptophan feedback inhibition. More specifically, amino acids analogous to P29, E30, S31, I32, S42, V43, V48, S50, S51, N52, N204, P205, M209, F210, G221, N292, P293, F298, and A373 in the monomeric *Agrobacterium tumefaciens* anthranilate synthase are being potentially useful residues for mutation to produce active anthranilate synthases that may have less sensitivity to tryptophan feedback inhibition.

Site directed mutagenesis can be used to generate amino acid substitutions, deletions and insertions at a variety of sites. Examples of specific mutations made within the *Agrobacterium tumefaciens* anthranilate synthase coding region include the following:
at about position 48 replace Val with Phe (see *e.g.,* SEQ ID NO: 58);
at about position 48 replace Val with Tyr (see *e*.*g*., SEQ ID NO: 59);
at about position 51 replace Ser with Phe (see *e*.*g*., SEQ ID NO: 60);
at about position 51 replace Ser with Cys (see *e.g.,* SEQ ID NO: 61);
at about position 52 replace Asn with Phe (see *e*.*g*., SEQ ID NO: 62);
at about position 293 replace Pro with Ala (see *e*.*g*., SEQ ID NO: 63);
at about position 293 replace Pro with Gly (see *e*.*g*., SEQ ID NO: 64); or
at about position 298 replace Phe with Trp (see *e.g.,* SEQ ID NO: 65).

Similar mutations can be made in analogous positions of any anthranilate synthase by alignment of the amino acid sequence of the anthranilate synthase to be mutated with an *Agrobacterium tumefaciens* anthranilate synthase amino acid sequence. One example of an *Agrobacterium tumefaciens* anthranilate synthase amino acid sequence that can be used for alignment is SEQ ID NO: 4.

Useful mutants can also be identified by classical mutagenesis and genetic selection. A functional change can be detected in the activity of the enzyme encoded by the gene by exposing the enzyme to free L-tryptophan or amino acid analogs of tryptophan, or by detecting a change in the DNA molecule using restriction enzyme mapping or DNA sequence analysis.

For example, a gene encoding an anthranilate synthase substantially tolerant to 5-methyltryptophan (5-MT) can be isolated from a 5-methyltryptophan tolerant cell line. *See* U.S. Patent 4,581,847, the disclosure of which is incorporated by reference herein. Briefly, partially differentiated plant cell cultures are grown and subcultured with continuous exposures to low levels of 5-methyltryptophan. 5-methyltryptophan concentrations are then gradually increased over several subculture intervals. Cells or tissues growing in the presence of normally toxic 5-methyltryptophan levels are repeatedly subcultured in the presence of 5-methyltryptophan and characterized. Stability of the 5-methyltryptophan tolerance trait of the cultured cells may be evaluated by growing the selected cell lines in the absence of 5-methyltryptophan for various periods of time and then analyzing growth after exposing the tissue to 5-methyltryptophan. Cell lines that are tolerant by virtue of having an altered anthranilate synthase enzyme can be selected by identifying cell lines having enzyme activity in the presence of normally toxic, *i*.*e*., growth inhibitor, levels of 5-methyltryptophan.

The anthranilate synthase gene cloned from a 5-MT- or 6-methylanthramilate (6-MA)-resistant cell line can be assessed for tolerance to 5-MT, 6-MA, or other amino acid analogs of tryptophan by standard methods, as described in U.S. Patent 4,581,847, the disclosure of which is incorporated by reference herein.

Cell lines with an anthranilate synthase of reduced sensitivity to 5-methyltryptophan inhibition can be used to isolate a 5-methyltryptophan-resistant anthranilate synthase. A DNA library from a cell line tolerant to 5-methyltryptophan can be generated and DNA fragments encoding all or a portion of an anthranilate synthase gene can be identified by hybridization to a cDNA probe encoding a portion of an anthranilate synthase gene. A complete copy of the altered gene can be obtained either by cloning and ligation or by PCR synthesis using appropriate primers. The isolation of the altered gene coding for anthranilate synthase can be confirmed in transformed plant cells by determining whether the anthranilate synthase being expressed retains enzyme activity when exposed to normally toxic levels of 5-methyltryptophan. *See,* Anderson et al., U.S. Patent 6,118,047.

Coding regions of any DNA molecule provided herein can also be optimized for expression in a selected organism, for example, a selected plant or other host cell type. An example of a DNA molecule having optimized codon usage for a selected plant is an *Agrobacterium tumefaciens* anthranilate synthase DNA molecule having SEQ ID NO: 75. This optimized *Agrobacterium tumefaciens* anthranilate synthase DNA (SEQ ID NO: 75) has 94% identity with SEQ ID NO: 1.

### Transgenes and Vectors

Once a nucleic acid encoding anthranilate synthase or a domain thereof is obtained and amplified, it is operably combined with a promoter and, optionally, with other elements to form a transgene.

Most genes have regions of DNA sequence that are known as promoters and which regulate gene expression. Promoter regions are typically found in the flanking DNA sequence upstream from the coding sequence in both prokaryotic and eukaryotic cells. A promoter sequence provides for regulation of transcription of the downstream gene sequence and typically includes from about 50 to about 2,000 nucleotide base pairs. Promoter sequences also contain regulatory sequences such as enhancer sequences that can influence the level of gene expression. Some isolated promoter sequences can provide for gene expression of heterologous genes, that is, a gene different from the native or homologous gene. Promoter sequences are also known to be strong or weak or inducible. A strong promoter provides for a high level of gene expression, whereas a weak promoter provides for a very low level of gene expression. An inducible promoter is a promoter that provides for turning on and off of gene expression in response to an exogenously added agent or to an environmental or developmental stimulus. Promoters can also provide for tissue specific or developmental regulation. An isolated promoter sequence that is a strong promoter for heterologous genes is advantageous because it provides for a sufficient level of gene expression to allow for easy detection and selection of transformed cells and provides for a high level of gene expression when desired.

The promoter in a transgene invention can provide for expression of anthranilate synthase from a DNA sequence encoding anthranilate synthase. Preferably, the coding sequence is expressed so as to result in an increase in tryptophan levels within plant tissues, for example, within the seeds of the plant. In another embodiment, the coding sequence is expressed so as to result in increased tolerance of the plant cells to feedback inhibition or to growth inhibition by an amino acid analog of tryptophan or so as to result in an increase in the total tryptophan content of the cells. The promoter can also be inducible so that gene expression can be turned on or off by an exogenously added agent. For example, a bacterial promoter such as the P_{tac} promoter can be induced to varying levels of gene expression depending on the level of isothiopropylgalactoside added to the transformed bacterial cells. It may also be preferable to combine the gene with a promoter that provides tissue specific expression or developmentally regulated gene expression in plants. Many promoters useful in the practice of the invention are available to those of skill in the art.

Preferred promoters will generally include, but are not limited to, promoters that function in bacteria, bacteriophage, plastids or plant cells. Useful promoters include the CaMV 35S promoter (Odell et al., Nature, 313:810 (1985)), the CaMV 19S (Lawton et al., Plant Mol. Biol., 9:31F (1987)), *nos* (Ebert et al., Proc. Nat. Acad. Sci. (U.S.A.), 84:5745 (1987)), Adh (Walker et al., Proc. Nat. Acad. Sci. (U.S.A.), 84:6624 (1987)), sucrose synthase (Yang et al., Proc. Nat. Acad. Sci. (U.S.A.), 87:4144 (1990)), α-tubulin, napin, actin (Wang et al., Mol. Cell. Biol., 12:3399 (1992)), *cab* (Sullivan et al., Mol. Gen. Genet., 215:431.(1989)), PEPCase promoter (Hudspeth et al., Plant Mol. Biol., 12:579 (1989)), the 7S-alpha'-conglycinin promoter (Beachy et al., EMBO J, 4:3047 (1985)) or those associated with the R gene complex (Chandler et al., The Plant Cell, 1:1175 (1989)). Other useful promoters include the bacteriophage SP6, T3, and T7 promoters.

Plastid promoters can be also be used. Most plastid genes contain a promoter for the multi-subunit plastid-encoded RNA polymerase (PEP) as well as the single-subunit nuclear-encoded RNA polymerase. A consensus sequence for the nuclear-encoded polymerase (NEP) promoters and listing of specific promoter sequences for several native plastid genes can be found in Hajdukiewicz et al., EMBO J., 16:4041-4048, (1997), which is hereby in its entirety incorporated by reference.

Examples of plastid promoters that can be used include the *Zea mays* plastid RRN (ZMRRN) promoter. The ZMRRN promoter can drive expression of a gene when the *Arabidopsis thaliana* plastid RNA polymerase is present. Similar promoters that can be used in the present invention are the Glycine max plastid RRN (SOYRRN) and the Nicotiana tabacum plastid RRN (NTRRN) promoters. All three promoters can be recognized by the *Arabidopsis* plastid RNA polymerase. The general features of RRN promoters are described by Hajdukiewicz *et al.* and U.S. Patent 6,218,145.

Moreover, transcription enhancers or duplications of enhancers can be used to increase expression from a particular promoter. Examples of such enhancers include, but are not limited to, elements from the CaMV 35S promoter and octopine synthase genes (Last *et al*., U.S. Patent 5,290,924). For example, it is contemplated that vectors for use in accordance with the present invention may be constructed to include the *ocs* enhancer element. This element was first identified as a 16 bp palindromic enhancer from the octopine synthase (*ocs*) gene of *Agrobacterium* (Ellis et al., EMBO J., 6:3203 (1987)), and is present in at least 10 other promoters (Bouchez et al., EMBO J., 8:4197 (1989)). It is proposed that the use of an enhancer element, such as the *ocs* element and particularly multiple copies of the element, will act to increase the level of transcription from adjacent promoters when applied in the context of monocot transformation. Tissue-specific promoters, including but not limited to, root-cell promoters (Conkling et al., Plant Physiol., 93:1203 (1990)), and tissue-specific enhancers (Fromm et al., The Plant Cell, 1:977 (1989)) are also contemplated to be particularly useful, as are inducible promoters such as ABA- and turgor-inducible promoters, and the like.

As the DNA sequence between the transcription initiation site and the start of the coding sequence, *i*.*e*., the untranslated leader sequence, can influence gene expression, one may also wish to employ a particular leader sequence. Any leader sequence available to one of skill in the art may be employed. Preferred leader sequences direct optimum levels of expression of the attached gene, for example, by increasing or maintaining mRNA stability and/or by preventing inappropriate initiation of translation (Joshi, Nucl. Acid Res., 15:6643 (1987)). The choice of such sequences is at the discretion of those of skill in the art. Sequences that are derived from genes that are highly expressed in dicots, and in soybean in particular, are contemplated.

In some cases, extremely high expression of anthranilate synthase or a domain thereof, is not necessary. For example, using the methods of the invention such high levels of anthranilate synthase may be generated that the availability of substrate, rather than enzyme, may limit the levels of tryptophan generated. In such cases, more moderate or regulated levels of expression can be selected by one of skill in the art. Such a skilled artisan can readily modulate or regulate the levels of expression, for example, by use of a weaker promoter or by use of a developmentally regulated or tissue specific promoter.

Nucleic acids encoding the anthranilate synthase of interest can also include a plastid transit peptide (e.g. SEQ ID NOs: 72, 74, 114, or 115) to facilitate transport of the anthranilate synthase polypeptide into plastids, for example, into chloroplasts. A nucleic acid encoding the selected plastid transit peptide (e.g. SEQ ID NOs: 71 or 73) is generally linked in-frame with the coding sequence of the anthranilate synthase. However; the plastid transit peptide can be placed at either the N-terminal or C-terminal end of the anthranilate synthase.

Constructs also include the nucleic acid of interest (e.g. DNA encoding an anthranilate synthase) along with a nucleic acid sequence that acts as a transcription termination signal and that allows for the polyadenylation of the resultant mRNA. Such transcription termination signals are placed 3' or downstream of the coding region of interest. Preferred transcription termination signals contemplated include the transcription termination signal from the nopaline synthase gene of *Agrobacterium tumefaciens* (Bevan et al., Nucl. Acid Res., 11:369 (1983)), the terminator from the octopine synthase gene of *Agrobacterium tumefaciens,* and the 3' end of genes encoding protease inhibitor I or II from potato or tomato, although other transcription termination signals known to those of skill in the art are also contemplated. Regulatory elements such as Adh intron 1 (Callis et al., Genes Develop., 1:1183 (1987)), sucrose synthase intron (Vasil et al., Plant Physiol, 91:5175 (1989)) or TMV omega element (Gallie et al., The Plant Cell, 1:301 (1989)) may further be included where desired. These 3' nontranslated regulatory sequences can be obtained as described in An, Methods in Enzymology, 153:292 (1987) or are already present in plasmids available from commercial sources such as Clontech, (Palo Alto, California). The 3' nontranslated regulatory sequences can be operably linked to the 3 terminus of an anthranilate synthase gene by standard methods. Other such regulatory elements useful in the practice of the invention are known to those of skill in the art.

A DNA construct may comprise a first expression cassette, comprised of, in operable linkage, a heterologous promoter, a DNA molecule encoding an anthrantilate synthase α-domain protein and a transcriptional terminator. This DNA construct may further comprise a second expression cassette in operable linkage, comprising a heterologous promoter, a DNA molecule encoding an anthranilate synthase β-domain protein and a transcriptional terminator.

Selectable marker genes or reporter genes are also useful in the present invention. Such genes can impart a distinct phenotype to cells expressing the marker gene and thus allow such transformed cells to be distinguished from cells that do not have the marker. Selectable marker genes confer a trait that one can 'select' for by chemical means, *i.e*., through the use of a selective agent (*e.g.,* a herbicide, antibiotic, or the like). Reporter genes, or screenable genes, confer a trait that one can identify through observation or testing, *i*.*e*., by 'screening' (*e*.*g*., the R-locus trait). Of course, many examples of suitable marker genes are known to the art and can be employed in the practice of the present invention.

Possible selectable markers for use in connection with the present invention include, but are not limited to, a *neo* gene (Potrykus et al., Mol. Gen. Genet., 199:183 (1985)) which codes for neomycin resistance and can be selected for using kanamycin, G418, and the like; a *bar* gene which codes for bialaphos resistance; a gene which encodes an altered EPSP synthase protein (Hinchee et al., Biotech., 6:915 (1988)) thus conferring glyphosate resistance; a nitrilase gene such as *bxn* from *Klebsiella ozaenae* which confers resistance to bromoxynil (Stalker et al., Science, 242:419 (1988)); a mutant acetolactate synthase gene (ALS) that confers resistance to imidazolinone, sulfonylurea or other ALS-inhibiting chemicals (EP 154 204 (1985)); a methotrexate-resistant DHFR gene (Thillet et al., J. Biol. Chem., 263:12500 (1988)); a dalapon dehalogenase gene that confers resistance to the herbicide dalapon; or a mutated anthranilate synthase gene that confers resistance to 5-methyl tryptophan. Where a mutant EPSP synthase gene is employed, additional benefit may be realized through the incorporation of a suitable plastid transit peptide (CTP).

An illustrative embodiment of a selectable marker gene capable of being used in systems to select transformants is the genes that encode the enzyme phosphinothricin acetyltransferase, such as the *bar* gene *from Streptomyces hygroscopicus* or the pat gene from *Streptomyces viridochromogenes* (U.S. Patent 5,550,318, which is incorporated by reference herein). The enzyme phosphinothricin acetyl transferase (PAT) inactivates the active ingredient in the herbicide bialaphos, phosphinothricin (PPT). PPT inhibits glutamine synthetase, (Murakami et al., Mol. Gen. Genet., 205:42 (1986); Twell et al., Plant Physiol., 91:1270 (1989)) causing rapid accumulation of ammonia and cell death.

Screenable markers that may be employed include, but are not limited to, a β-glucuronidase or *uid*A gene (GUS) which encodes an enzyme for which various chromogenic substrates are known; an R-locus gene, which encodes a product that regulates the production of anthocyanin pigments (red color) in plant tissues (Dellaporta et al., in Chromosome Structure and Function, pp. 263-282 (1988)); a β-lactamase gene (Sutcliffe, Proc. Nat. Acad. Sci. (U.S.A.), 75:3737 (1978)), which encodes an enzyme for which various chromogenic substrates are known (*e*.*g*., PADAC, a chromogenic cephalosporin); *a xyl*E gene (Zukowsky et al., Proc. Nat. Acad. Sci. (U.S.A.), 80:1101 (1983)) that encodes a catechol dioxygenase that can convert chromogenic catechols; an α-amylase gene (Ikuta et al., Biotech., 8:241 (1990)); a tyrosinase gene (Katz et al., J. Gen. Microbiol., 129:2703 (1983)) that encodes an enzyme capable of oxidizing tyrosine to DOPA and dopaquinone which in turn condenses to form the easily detectable compound melanin; a β-galactosidase gene, which encodes an enzyme for which there are chromogenic substrates; a luciferase (*lux*) gene (Ow et al., Science, 234:856 (1986)), which allows for bioluminescence detection; or even an aequorin gene (Prasher et al., Biochem. Biophys. Res. Comm., 126:1259 (1985)), which may be employed in calcium-sensitive bioluminescence detection, or a green fluorescent protein gene (Niedz et al., Plant Cell Reports, 14:403 (1995)). The presence of the *lux* gene in transformed cells maybe detected using, for example, X-ray film, scintillation counting, fluorescent spectrophotometry, low-light video cameras, photon-counting cameras, or multiwell luminometry. It is also envisioned that this system may be developed for populational screening for bioluminescence, such as on tissue culture plates, or even for whole plant screening.

Additionally, transgenes may be constructed and employed to provide targeting of the gene product to an intracellular compartment within plant cells or in directing a protein to the extracellular environment. This will generally be achieved by joining a DNA sequence encoding a transit or signal peptide sequence to the coding sequence of a particular gene. The resultant transit, or signal, peptide will transport the protein to a particular intracellular, or extracellular destination, respectively, and may then be post-translationally removed. Transit or signal peptides act by facilitating the transport of proteins through intracellular membranes, *e*.*g*., vacuole, vesicle, plastid and mitochondrial membranes, whereas signal peptides direct proteins through the extracellular membrane. By facilitating transport of the protein into compartments inside or outside the cell, these sequences may increase the accumulation of gene product.

A particular example of such a use concerns the direction of an anthranilate synthase to a particular organelle, such as the plastid, rather than to the cytoplasm. This is exemplified by the use of the *Arabidopsis* SSU1A transit peptide that confers plastid-specific targeting of proteins. Alternatively, the transgene can comprise a plastid transit peptide-encoding DNA sequence or a DNA sequence encoding the the *rbcS* (RuBISCO) transit peptide operably linked between a promoter and the DNA sequence encoding an anthranilate synthase (for a review of plastid targeting peptides, see Heijne et al., Eur. J. Biochem., 180:535 (1989); Keegstra et al., Ann. Rev. Plant Physiol. Plant Mol. Biol., 40:471 (1989)). If the transgene is to be introduced into a plant cell, the transgene can also contain plant transcriptional termination and polyadenylation signals and translational signals linked to the 3' terminus of a plant anthranilate synthase gene.

An exogenous plastid transit peptide can be used which is not encoded within a native plant anthranilate synthase gene. A plastid transit peptide is typically 40 to 70 amino acids in length and functions post-translationally to direct a protein to the plastid. The transit peptide is cleaved either during or just after import into the plastid to yield the mature protein. The complete copy of a gene encoding a plant anthranilate synthase may contain a plastid transit peptide sequence. In that case, it may not be necessary to combine an exogenously obtained plastid transit peptide sequence into the transgene.

Exogenous plastid transit peptide encoding sequences can be obtained from a variety of plant nuclear genes, so long as the products of the genes are expressed as preproteins comprising an amino terminal transit peptide and transported into plastid. Examples of plant gene products known to include such transit peptide sequences include, but are not limited to, the small subunit of ribulose biphosphate carboxylase, chlorophyll a/b binding protein, plastid ribosomal proteins encoded by nuclear genes, certain heatshock proteins, amino acid biosynthetic enzymes such as acetolactate acid synthase, 3-enolpyruvylphosphoshikimate synthase, dihydrodipicolinate synthase, anthranilate synthase and the like. In some instances a plastid transport protein already may be encoded in the anthranilate synthase gene of interest, in which case there may be no need to add such plastid transit sequences. Alternatively, the DNA fragment coding for the transit peptide may be chemically synthesized either wholly or in part from the known sequences of transit peptides such as those listed above.

Regardless of the source of the DNA fragment coding for the transit peptide, it should include a translation initiation codon, for example, an ATG codon, and be expressed as an amino acid sequence that is recognized by and will function properly in plastids of the host plant. Attention should also be given to the amino acid sequence at the junction between the transit peptide and the anthranilate synthase enzyme where it is cleaved to yield the mature enzyme. Certain conserved amino acid sequences have been identified and may serve as a guideline. Precise fusion of the transit peptide coding sequence with the anthranilate synthase coding sequence may require manipulation of one or both DNA sequences to introduce, for example, a convenient restriction site. This may be accomplished by methods including site-directed mutagenesis, insertion of chemically synthesized oligonucleotide linkers, and the like.

Precise fusion of the nucleic acids encoding the plastid transport protein may not be necessary so long as the coding sequence of the plastid transport protein is in-frame with that of the anthranilate synthase. For example, additional peptidyl or amino acids can often be included without adversely affecting the expression or localization of the protein of interest.

Once obtained, the plastid transit peptide sequence can be appropriately linked to the promoter and an anthranilate synthase coding region in a transgene using standard methods. A plasmid containing a promoter functional in plant cells and having multiple cloning sites downstream can be constructed or obtained from commercial sources. The plastid transit peptide sequence can be inserted downstream from the promoter using restriction enzymes. An anthranilate synthase coding region can then be translationally fused or inserted immediately downstream from and in frame with the 3' terminus of the plastid transit peptide sequence. Hence, the plastid transit peptide is preferably linked to the amino terminus of the anthranilate synthase. Once formed, the transgene can be subcloned into other plasmids or vectors.

In addition to nuclear plant transformation, the present invention also extends to direct transformation of the plastid genome of plants. Hence, targeting of the gene product to an intracellular compartment within plant cells may also be achieved by direct delivery of a gene to the intracellular compartment. Direct transformation of plastid genome may provide additional benefits over nuclear transformation. For example, direct plastid transformation of anthranilate synthase eliminates the requirement for a plastid targeting peptide and post-translational transport and processing of the pre-protein derived from the corresponding nuclear transformants. Plastid transformation of plants has been described by P. Maliga, Current Opinion in Plant Biology, 5:164-172 (2002), P. B. Heifetz, Biochimie, 82:655-666 (2000), R.Bock., J. Mol. Biol., 312:425-438 (2001), and H. Daniell et al., Trends in Plant Science, 7:84-91 (2002), and references within.

After constructing a transgene containing an anthranilate synthase gene, the cassette can then be introduced into a plant cell. Depending on the type of plant cell, the level of gene expression, and the activity of the enzyme encoded by the gene, introduction of DNA encoding an anthranilate synthase into the plant cell can lead to the overproduction of tryptophan, confer tolerance to an amino acid analog of tryptophan, such as 5-methyltryptophan or 6-methylanthrinilate, and/or otherwise alter the tryptophan content of the plant cell.

### Transformation of Host Cells

A transgene comprising an anthranilate synthase gene can be subcloned into a known expression vector, and AS expression can be detected and/or quantitated. This method of screening is useful to identify transgenes providing for an expression of an anthranilate synthase gene, and expression of an anthranilate synthase in the plastid of a transformed plant cell.

Plasmid vectors include additional DNA sequences that provide for easy selection, amplification, and transformation of the transgene in prokaryotic and eukaryotic cells, *e.g*., pUC-derived vectors, pSK-derived vectors, pGEM-derived vectors, pSP-derived vectors, or pBS-derived vectors. The additional DNA sequences include origins of replication to provide for autonomous replication of the vector, selectable marker genes, preferably encoding antibiotic or herbicide resistance, unique multiple cloning sites providing for multiple sites to insert DNA sequences or genes encoded in the transgene, and sequences that enhance transformation of prokaryotic and eukaryotic cells.

Another vector that is useful for expression in both plant and prokaryotic cells is the binary Ti plasmid (as disclosed in Schilperoort et al., U.S. Patent 4,940,83 8) as exemplified by vector pGA582. This binary Ti plasmid vector has been previously characterized by An, cited *supra.* This binary Ti vector can be replicated in prokaryotic bacteria such as *E. coli* and *Agrobacterium.* The *Agrobacterium* plasmid vectors can also be used to transfer the transgene to plant cells. The binary Ti vectors preferably include the nopaline T DNA right and left borders to provide for efficient plant cell transformation, a selectable marker gene, unique multiple cloning sites in the T border regions, the *col*E1 replication of origin and a wide host range replicon. The binary Ti vectors carrying a transgene can be used to transform both prokaryotic and eukaryotic cells, but is preferably used to transform plant cells. See, for example, Glassman et al., U.S. Patent 5,258,300.

The expression vector can then be introduced into prokaryotic or eukaryotic cells by available methods. Methods of transformation especially effective for monocots and dicots, include, but are not limited to, microprojectile bombardment of immature embryos (U.S. Patent 5,990,390) or Type II embryogenic callus cells as described by W.J. Gordon-Kamm et al. (Plant Cell, 2:603 (1990)), M.E. Fromm et al. (Bio/Technology, 8:833 (1990)) and D.A. Walters et al. (Plant Molecular Biology, 18:189 (1992)), or by electroporation of type I embryogenic calluses described by D'Halluin et al. (The Plant Cell, 4:1495 (1992)), or by Krzyzek (U.S. Patent 5,384,253). Transformation of plant cells by vortexing with DNA-coated tungsten whiskers (Coffee et al., U.S. Patent 5,302,523) and transformation by exposure of cells to DNA-containing liposomes can also be used.

After transformation of the selected anthranilate synthase construct into a host cell, the host cell may be used for production of useful products generated by the transgenic anthranilate synthase in combination with the host cell's enzymatic machinery. Culturing the transformed cells can lead to enhanced production of tryptophan and other useful compounds, which can be recovered from the cells or from the culture media. Examples of useful compounds that may be generated upon expression in a variety of host cells and/or organisms include tryptophan, indole acetic acid and other auxins, isoflavonoid compounds important to cardiovascular health found in soy, volatile indole compounds which act as signals to natural enemies of herbivorous insects in maize, anticarcinogens such as indole glucosinolates (indole-3-carbinol) found the Cruciferae plant family, as well as indole alkaloids such as ergot compounds produced by certain species of fungi. (Barnes et al., Adv Exp Med Biol, 401:87 (1996); Frey et al., Proc Natl Acad Sci., 97:14801 (2000); Muller et al., Biol Chem., 381:679 (2000); Mantegani et al., Farmaco, 54:288 (1999); Zeligs, J Med Food, 1:67 (1998); Mash et al., Ann NY Acad Sci., 844:274 (1998); Melanson et al., Proc Natl Acad Sci., 94:13345 (1997); Broadbent et al., Curr Med Chem., 5:469 (1998)).
Accumulation of tryptophan may also lead to the increased production of secondary metabolites in microbes and plants, for example, indole containing metabolites such as simple indoles, indole conjugates, indole alkaloids, indole phytoalexins and indole glucosinalates in plants.

Anthranilate synthases insensitive to tryptophan have the potential to increase a variety of chorismate-derived metabolites, including those derived from phenylalanine due to the stimulation of phenylalanine synthesis by tryptophan via chorismate mutase. *See* Siehl, D. The biosynthesis of tryptophan, tyrosine, and phenylalanine from chorismate in "Plant Amino Acids: Biochemistry and Biotechnology", ed. BK Singh, pp 171-204. Other chorismate-derived metabolites that may increase when feedback insensitive anthranilate synthases are present include phenylpropanoids, flavonoids, and isoflavonoids, as well as those derived from anthranilate, such as indole, indole alkaloids, and indole glucosinolates. Many of these compounds are important plant hormones, plant defense compounds, chemopreventive agents of various health conditions, and/or pharmacologically active compounds.

The range of these compounds whose synthesis might be increased by expression of anthranilate synthase depends on the organism in which the anthranilate synthase is expressed. One of skill in the art can readily assess which organisms and host cells to use and/or test in order to generate the desired compounds. The present invention contemplates synthesis of tryptophan and other useful compounds in soybean plants.

### Strategy for Selection of Tryptophan Overproducer Cell Lines

Efficient selection of a desired tryptophan analog resistant, tryptophan overproducer variant using tissue culture techniques requires careful determination of selection conditions. These conditions are optimized to allow growth and accumulation of tryptophan analog resistant, tryptophan overproducer cells in the culture while inhibiting the growth of the bulk of the cell population. The situation is complicated by the fact that the vitality of individual cells in a population can be highly dependent on the vitality of neighboring cells. Conditions under which cell cultures are exposed to tryptophan analog are determined by the characteristics of the interaction of the compound with the tissue. Such factors as the degree of toxicity and the rate of inhibition should be considered. The accumulation of the compounds by cells in culture, and the persistence and stability of the compounds, both in the media and in the cells, also need to be considered along with the extent of uptake and transmission to the desired cellular compartment. Additionally, it is important to determine whether the effects of the compounds can be readily reversed by the addition of tryptophan.

The effects of the analog on culture viability and morphology is carefully evaluated. It is especially important to choose analog exposure conditions that have no impact on plant regeneration capability of cultures. Choice of analog exposure conditions is also influenced by whether the analog kills cells or simply inhibits cell divisions.

The choice of a selection protocol is dependent upon the considerations described above. The protocols briefly described below can be utilized in the selection procedure. For example, to select for cells that are resistant to growth inhibition by a tryptophan analog, finely divided cells in liquid suspension culture can be exposed to high tryptophan analog levels for brief periods of time. Surviving cells are then allowed to recover and accumulate and are then reexposed for subsequently longer periods of time. Alternatively, organized partially differentiated cell cultures are grown and subcultured with continuous exposure to initially low levels of a tryptophan analog. Concentrations are then gradually increased over several subculture intervals.

### Genes for Plant Modification

As described hereinabove, genes that function as selectable marker genes and reporter genes can be operably combined with the DNA sequence encoding the anthranilate synthase, or domain thereof, in transgenes, vectors and plants of the present invention. Additionally, other agronomical traits can be added to the transgenes, vectors and plants of the present invention. Such traits include, but are not limited to, insect resistance or tolerance; disease resistance or tolerance (viral, bacterial, fungal, nematode); stress resistance or tolerance, as exemplified by resistance or tolerance to drought, heat, chilling, freezing, excessive moisture, salt stress, oxidative stress, increased yields, food content and makeup, physical appearance, male sterility, drydown, standability, prolificacy, starch properties, oil quantity and quality, and the like. One may incorporate one or more genes conferring such traits into the plants of the present invention.

### Insect Resistance or Tolerance

*Bacillus thuringiensis* (or "Bt") bacteria include nearly 20 known subspecies of bacteria which produce endotoxin polypeptides that are toxic when ingested by a wide variety of insect species. The biology and molecular biology of the endotoxin proteins (Bt proteins) and corresponding genes (Bt genes) has been reviewed by H. R. Whitely et al., Ann. Rev. Microbiol., 40:549 (1986) and by H. Hofte et al., Microbiol. Rev., 53:242 (1989). Genes coding for a variety of Bt proteins have been cloned and sequenced. A segment of the Bt polypeptide is essential for toxicity to a variety of *Lepidoptera* pests and is contained within approximately the first 50% of the Bt polypeptide molecule. Consequently, a truncated Bt polypeptide coded by a truncated Bt gene will in many cases retain its toxicity towards a number of *Lepidoptera* insect pests. For example, the HD73 and HD1 Bt polypeptides have been shown to be toxic to the larvae of the important *Lepidoptera* insect pests of plants in the U.S.A. such as the European corn borer, cutworms and earworms. The genes coding for the HD1 and HD73 Bt polypeptides have been cloned and sequenced by M. Geiser et al., Gene, 48:109 (1986) and M. J. Adang et al., Gene, 36:289 (1985), respectively, and can be cloned from HD1 and HD73 strains obtained from culture collections (*e*.*g*. Bacillus Genetic Stock Center, Columbus, OH or USDA Bt stock collection Peoria, IL) using standard protocols. Examples of Bt genes and polypeptides are described, for example, in U.S. Patents 6,329,574; 6,303,364; 6,320,100; and 6,331,655.

DNA coding for new, previously uncharacterized Bt toxins, may be cloned from the host *Bacillus* organism using protocols that have previously been used to clone Bt genes, and new synthetic forms of Bt toxins may also be produced.

A Bt gene useful in the present invention may include a 5' DNA sequence including a sequence of DNA which will allow for the initiation of transcription and translation of a downstream located Bt sequence in a plant. The Bt gene may also comprise a 3' DNA sequence that includes a sequence derived from the 3' non-coding region of a gene that can be expressed in the plant of interest. The Bt gene would also include a DNA sequence coding for a toxic Bt polypeptide produced by *Bacillus thuringiensis* or toxic portions thereof or having substantial amino sequence homology thereto. The Bt coding sequence may include: (i) DNA sequences which code for insecticidal proteins that have substantial homology to Bt endotoxins that are active against insect pests of the plant of interest, *e*.*g*., the HD73 or HD1 Bt sequences; (ii) sequences coding for insecticidally-active segments of the Bt endotoxin polypeptide, *e*.*g*., insecticidally active HD73 or HD1 polypeptides truncated from the carboxy and/or amino termini; and/or (iii) a truncated Bt sequence fused in frame with a sequence(s) that codes for a polypeptide that provides some additional advantage such as: (a) genes that are selectable, *e*.*g*., genes that confer resistance to antibiotics or herbicides, (b) reporter genes whose products are easy to detect or assay, *e*.*g*., luciferase or beta-glucuronidase; (c) DNA sequences that code for polypeptide sequences that have some additional use in stabilizing the Bt protein against degradation or enhance the efficacy of the Bt protein against insects, *e*.*g*., protease inhibitors; and (d) sequences that help direct the Bt protein to a specific compartment inside or outside the plant cell, *e.g*., a signal sequence.

To obtain optimum synthesis of the Bt protein in the plant, it may also be appropriate to adjust the DNA sequence of the Bt gene to more resemble the genes that are efficiently expressed in the plant of interest. Since the codon usage of Bt genes may be dissimilar to that used by genes that are expressed in the plant of interest, the expression of the Bt gene in plant cells may be improved by the replacement of these codons with those that are more efficiently expressed in plants, *e.g*., are used more frequently in the plants of interest (See E. Murray et al., Nucl. Acids Res., 17:477 (1989)). Such replacement of codons may require the substitution of bases without changing the amino acid sequence of the resulting Bt polypeptide. The Bt polypeptide may be identical in sequence to the bacterial gene or segments thereof The complete Bt coding sequence, or sections thereof, containing a higher proportion of preferred codons than the original bacterial gene could be synthesized using standard chemical synthesis protocols, and introduced or assembled into the Bt gene using standard protocols, such as site-directed mutagenesis or DNA polymerization and ligation and the like.

Protease inhibitors may also provide insect resistance. For example, use of a protease inhibitor II gene, pinII, from tomato or potato may be useful. Also advantageous is the use of a pinII gene in combination with a Bt toxin gene. Other genes which encode inhibitors of the insects' digestive system, or those that encode enzymes or co-factors that facilitate the production of inhibitors, may also be useful. This group includes oryzacystatin and amylase inhibitors such as those from wheat and barley.

Genes encoding lectins may confer additional or alternative insecticide properties. (Murdock et al., Phytochemistry, 29 85 (1990); Czapla and Lang, J. Econ. Entomol., 83:2480 (1990) Lectin genes contemplated to be useful include, for example, barley and wheat germ agglutinin (WGA) and rice lectins. (Gatehouse et al., J Sci Food Agric., 35:373 (1984))

Genes controlling the production of large or small polypeptides active against insects when introduced into the insect pests such as lytic peptides, peptide hormones and toxins and venoms, may also be useful. For example, the expression of juvenile hormone esterase, directed towards specific insect pests, may also result in insecticidal activity, or perhaps cause cessation of metamorphosis. (Hammock et al., Nature, 344:458 (1990))

Transgenic plants expressing genes encoding enzymes that affect the integrity of the insect cuticle may also be useful. Such genes include those encoding, for example, chitinase, proteases, lipases and also genes for the production of nikkomycin. Genes that code for activities that affect insect molting, such those affecting the production of ecdysteroid UDP-glucosyl transferase, may also be useful.

Genes that code for enzymes that facilitate the production of compounds that reduce the nutritional quality of the plant to insect pests, may also be useful. It maybe possible, for instance, to confer insecticidal activity to a plant by altering its sterol composition. Further embodiments of the present invention concern transgenic plants with enhanced lipoxygenase activity.

The present invention also provides methods and compositions useful in altering plant secondary metabolites. One example concerns altering plants to produce DIMBOA which, it is contemplated, will confer resistance to European corn borer, rootworm and several other insect pests. *See*, *e*.*g*., U.S. Patent 6,331,880. DIMBOA is derived from indole-related compounds. The present invention provides methods for increasing the content of indole-related compounds like tryptophan within plant cells and tissues. Hence, according to the invention the methods provided herein may also increase the levels of DIMBOA, and thereby increase the reistance of plants to insects.

The introduction of genes that can regulate the production of maysin, and genes involved in the production of dhurrin in sorghum, is also contemplated to be of use in facilitating resistance to earworm and rootworm, respectively.

Further genes encoding proteins characterized as having potential insecticidal activity may also be used. Such genes include, for example, the cowpea trypsin inhibitor (CpTI; Hilder et al., Nature, 330:160 (1987)) which may be used as a rootworm deterrent; genes encoding avermectin (Avermectin and Abamectin., Campbell, W. C., Ed., 1989; Ikeda et al., J Bacteriol, 169:5615 1987) which may prove useful as a corn rootworm deterrent; ribosome inactivating protein genes; and genes that regulate plant structures. Transgenic plants including anti-insect antibody genes and genes that code for enzymes that can convert a nontoxic insecticide (pro-insecticide) applied to the outside of the plant into an insecticide inside the plant are also contemplated.

### Environmental or Stress Resistance or Tolerance

Improvement of a plant's ability to tolerate various environmental stresses can be effected through expression of genes. For example, increased resistance to freezing temperatures may be conferred through the introduction of an "antifreeze" protein such as that of the Winter Flounder (Cutler et al., J Plant Physiol., 135:351 (1989)) or synthetic gene derivatives thereof. Improved chilling tolerance may also be conferred through increased expression of glycerol-3-phosphate acetyltransferase in plastids (Wolter et al., The EMBO J., 11:4685 (1992)). Resistance to oxidative stress can be conferred by expression of superoxide dismutase (Gupta et al., Proc. Natl. Acad. Sci (U.S.A.), 90:1629 (1993)), and can be improved by glutathione reductase (Bowler et al., Ann Rev. Plant Physiol., 43:83 (1992)).

It is contemplated that the expression of genes that favorably affect plant water content, total water potential, osmotic potential, and turgor will enhance the ability of the plant to tolerate drought and will therefore be useful. It is proposed, for example, that the expression of genes encoding for the biosynthesis of osmotically-active solutes may impart protection against drought. Within this class are genes encoding for mannitol dehydrogenase (Lee and Saier, J. Bacteriol., 258, 10761 (1982)) and trehalose-6-phosphate synthase (Kaasen et al., J. Bacteriology, 174:889 (1992)).

Similarly, other metabolites may protect either enzyme function or membrane integrity (Loomis et al., J. Expt. Zoology, 252:9 (1989)), and therefore expression of genes encoding for the biosynthesis of these compounds might confer drought resistance in a manner similar to or complimentary to mannitol. Other examples of naturally occurring metabolites that are osmotically active and/or provide some direct protective effect during drought and/or desiccation include fructose, erythritol, sorbitol, dulcitol, glucosylglycerol, sucrose, stachyose, raffinose, proline, glycine, betaine, ononitol and pinitol. *See, e*.*g*., U.S. Patent 6,281,411.

Three classes of Late Embryogenic Proteins have been assigned based on structural similarities (see Dure et al., Plant Molecular Biology, 12:475 (1989)). Expression of structural genes from all three LEA groups may confer drought tolerance. Other types of proteins induced during water stress, which may be useful, include thiol proteases, aldolases and transmembrane transporters, which may confer various protective and/or repair-type functions during drought stress. *See*, *e*.*g*., PCT/CA99/00219 (Na+/H+ exchanger polypeptide genes). Genes that effect lipid biosynthesis might also be useful in conferring drought resistance.

The expression of genes involved with specific morphological traits that allow for increased water extractions from drying soil may also be useful. The expression of genes that enhance reproductive fitness during times of stress may also be useful. It is also proposed that expression of genes that minimize kernel abortion during times of stress would increase the amount of grain to be harvested and hence be of value.

Enabling plants to utilize water more efficiently, through the introduction and expression of genes, may improve the overall performance even when soil water availability is not limiting. By introducing genes that improve the ability of plants to maximize water usage across a full range of stresses relating to water availability, yield stability or consistency of yield performance may be realized.

### Disease Resistance or Tolerance

Resistance to viruses may be produced through expression of genes. For example, expression of antisense genes targeted at essential viral functions or expression of genes encoding viral coat proteins may impart resistance to the virus.

Resistance to diseases caused by bacteria and fungi may be conferred through introduction of genes. For example, genes encoding so-called "peptide antibiotics," pathogenesis related (PR) proteins, toxin resistance, and proteins affecting host-pathogen interactions such as morphological characteristics may be useful.

### Mycotoxin Reduction/Elimination

Production of mycotoxins, including aflatoxin and fumonisin, by fungi associated with plants is a significant factor in rendering grain not useful. Inhibition of the growth of these fungi may reduce the synthesis of these toxic substances and therefore reduce grain losses due to mycotoxin contamination. It may be possible to introduce genes into plants such that would inhibit synthesis of the mycotoxin without interfering with fungal growth. Further, expression of a novel gene which encodes an enzyme capable of rendering the mycotoxin nontoxic would be useful in order to achieve reduced mycotoxin contamination of grain.

### Plant Composition or Quality

The composition of the plant may be altered, for example, to improve the balance of amino acids in a variety of ways including elevating expression of native proteins, decreasing expression of those with poor composition, changing the composition of native proteins, or introducing genes encoding entirely new proteins possessing superior composition. *See*, *e*.*g*., U.S. Patent 6,160,208 (alteration of seed storage protein expression). The introduction of genes that alter the oil content of the plant may be of value. *See*, *e*.*g*., U.S. Patents 6,069,289 and 6,268,550 (ACCase gene). Genes may be introduced that enhance the nutritive value of the starch component of the plant, for example by increasing the degree of branching, resulting in improved utilization of the starch in cows by delaying its metabolism.

### Plant Agronomic Characteristics

Two of the factors determining where plants can be grown are the average daily temperature during the growing season and the length of time between frosts. Expression of genes that are involved in regulation of plant development maybe useful, *e*.*g*., the liguleless and rough sheath genes that have been identified in corn.

Genes may be introduced into corn that would improve standability and other plant growth characteristics. Expression of genes which confer stronger stalks, improved root systems, or prevent or reduce ear droppage would be of value to the farmer

### Nutrient Utilization

The ability to utilize available nutrients may be a limiting factor in growth of plants. It may be possible to alter nutrient uptake, tolerate pH extremes, mobilization through the plant, storage pools, and availability for metabolic activities by the introduction of genes. These modifications would allow a plant to more efficiently utilize available nutrients. For example, an increase in the activity of an enzyme that is normally present in the plant and involved in nutrient utilization may increase the availability of a nutrient. An example of such an enzyme would be phytase.

### Male Sterility

Male sterility is useful in the production of hybrid seed, and male sterility may be produced through expression of genes. It may be possible through the introduction of TURF-13 via transformation to separate male sterility from disease sensitivity. See Levings, Science, 250:942-947, (1990). As it may be necessary to restore male fertility for breeding purposes and for grain production, genes encoding restoration of male fertility may also be introduced.

### Selection and Characterization of Resistant Cell Lines

Selections are carried out until cells or tissue are recovered which are observed to be growing well in the presence of normally inhibitory levels of a tryptophan analog thereof. These cell "lines" are subcultured several additional times in the presence of a tryptophan analog to remove non-resistant cells and then characterized. The amount of resistance that has been obtained is determined by comparing the growth of these cell lines with the growth of unselected cells or tissue in the presence of various tryptophan analogs at various concentrations. Stability of the resistance trait of the cultured cells may be evaluated by simply growing the selected cell lines in the absence of the tryptophan analog for various periods of time and then analyzing growth after re-exposing the tissue to the analog. The resistant cell lines may also be evaluated using *in vitro* chemical studies to verify that the site of action of the analog is altered to a form that is less sensitive to inhibition by tryptophan analogs.

Transient expression of an anthranilate synthase gene can be detected and quantitated in the transformed cells. Gene expression can be quantitated by RT-PCR analysis, a quantitative Western blot using antibodies specific for the cloned anthranilate synthase or by detecting enzyme activity in the presence of tryptophan or an amino acid analog of tryptophan. The tissue and subcellular location of the cloned anthranilate synthase can be determined by immunochemical staining methods using antibodies specific for the cloned anthranilate synthase or subcellular fractionation and subsequent biochemical and/or immunological analyses. Sensitivity of the cloned anthranilate synthase to agents can also be assessed. Transgenes providing for expression of an anthranilate synthase or anthranilate synthase tolerant to inhibition by an amino acid analog of tryptophan or free L-tryptophan can then be used to transform monocot and/or dicot plant tissue cells and to regenerate transformed plants and seeds. Transformed cells can be selected by detecting the presence of a selectable marker gene or a reporter gene, for example, by detecting a selectable herbicide resistance marker. Transient expression of an anthranilate synthase gene can be detected in the transgenic embryogenic calli using antibodies specific for the cloned anthranilate synthase, or by RT-PCR analyses.

### Plant Regeneration and Production of Seed

Transformed embryogenic calli, meristematic tissue, embryos, leaf discs and the like can then be used to generate transgenic plants that exhibit stable inheritance of the transformed anthranilate synthase gene. Plant cell lines exhibiting satisfactory levels of tolerance to an amino acid analog of tryptophan are put through a plant regeneration protocol to obtain mature plants and seeds expressing the tolerance traits by methods well known in the art (for example, *see,* U.S. Patents 5,990,390 and 5,489,520; and Laursen et al., Plant Mol. Biol., 24:51 (1994)). The plant regeneration protocol allows the development of somatic embryos and the subsequent growth of roots and shoots. To determine that the tolerance trait is expressed in differentiated organs of the plant, and not solely in undifferentiated cell culture, regenerated plants can be assayed for the levels of tryptophan present in various portions of the plant relative to regenerated, non-transformed plants. Transgenic plants and seeds can be generated from transformed cells and tissues showing a change in tryptophan content or in resistance to a tryptophan analog using standard methods. It is especially preferred that the tryptophan content of the leaves or seeds is increased. A change in specific activity of the enzyme in the presence of inhibitory amounts of tryptophan or an analog thereof can be detected by measuring enzyme activity in the transformed cells as described by Widholm, Biochimica et Biophysica Acta, 279:48 (1972). A change in total tryptophan content can also be examined by standard methods as described by Jones et al., Analyst, 106:968 (1981).

Mature plants are then obtained from cell lines that are known to express the trait. If possible, the regenerated plants are self pollinated. In addition, pollen obtained from the regenerated plants is crossed to seed grown plants of agronomically important inbred lines. In some cases, pollen from plants of these inbred lines is used to pollinate regenerated plants. The trait is genetically characterized by evaluating the segregation of the trait in first and later generation progeny. The heritability and expression in plants of traits selected in tissue culture are of particular importance if the traits are to be commercially useful.

The commercial value of tryptophan overproducer soybeans, cereals and other plants is greatest if many different hybrid combinations are available for sale. The farmer typically grows more than one kind of hybrid based on such differences as maturity, standability or other agronomic traits. Additionally, hybrids adapted to one part of the country are not adapted to another part because of differences in such traits as maturity, disease, and insect resistance. Because of this, it is necessary to breed tryptophan overproduction into a large number of parental inbred lines so that many hybrid combinations can be produced.

A conversion process (backcrossing) is carried out by crossing the original overproducer line to normal elite lines and crossing the progeny back to the normal parent. The progeny from this cross will segregate such that some plants carry the gene responsible for overproduction whereas some do not. Plants carrying such genes will be crossed again to the normal parent resulting in progeny which segregate for overproduction and normal production once more. This is repeated until the original normal parent has been converted to an overproducing line, yet possesses all other important attributes as originally found in the normal parent. A separate backcrossing program is implemented for every elite line that is to be converted to tryptophan overproducer line.

Subsequent to the backcrossing, the new overproducer lines and the appropriate combinations of lines which make good commercial hybrids are evaluated for overproduction as well as a battery of important agronomic traits. Overproducer lines and hybrids are produced which are true to type of the original normal lines and hybrids. This requires evaluation under a range of environmental conditions where the lines or hybrids will generally be grown commercially. For production of high tryptophan soybeans, it may be necessary that both parents of the hybrid seed be homozygous for the high tryptophan character. Parental lines of hybrids that perform satisfactorily are increased and used for hybrid production using standard hybrid seed production practices.

The transgenic plants produced herein are expected to be useful for a variety of commercial and research purposes. Transgenic plants can be created for use in traditional agriculture to possess traits beneficial to the consumer of the grain harvested from the plant (*e*.*g*., improved nutritive content in human food or animal feed). In such uses, the plants are generally grown for the use of their grain in human or animal foods. However, other parts of the plants, including stalks, husks, vegetative parts, and the like, may also have utility, including use as part of animal silage, fermentation feed, biocatalysis, or for ornamental purposes.

Transgenic plants may also find use in the commercial manufacture of proteins or other molecules, where the molecule of interest is extracted or purified from plant parts, seeds, and the like. Cells or tissue from the plants may also be cultured, grown *in vitro,* or fermented to manufacture such molecules.

The transgenic plants may also be used in commercial breeding programs, or may be crossed or bred to plants of related crop species. Improvements encoded by the recombinant DNA may be transferred, *e.g.,* from soybean cells to cells of other species, *e.g.,* by protoplast fusion.

In one embodiment, a transgene comprised of a maize anthranilate α-domain isolated from a maize cell line tolerant to 5-MT and linked to the 35S CaMV promoter is introduced into a 5-MT sensitive monocot or dicot tissue using microprojectile bombardment. Transformed embryos or meristems are selected and used to generate transgenic plants. Transformed calli and transgenic plants can be evaluated for tolerance to 5-MT or 6-MA and for stable inheritance of the tolerance trait.

### EXAMPLE 1: Isolation and E. coli Expression of Anthranilate Synthase from

### Agrobacterium tumefaciens.

This example describes the isolation of anthranilate synthase from *Agrobacterium tumefaciens* and its expression in *E. coli.*

### Cloning of Agrobacterium tumefaciens AS

The nucleotide and amino acid sequences of the anthranilate synthase coding region from *Rhizobium meliloti* (GenBank accession number: P 15395) was used to search an *Agrobacterium tumefaciens* C58 genomic sequence database (Goodner et al. Science, 294:2323-2328 (2001)). The search consisted of tblastn using blosum62 matrix, (Altschul et al., Nucleic Acid Res., 25:3389-3402 (1997)).

The identified AS homolog in the *Agrobacterium tumefaciens* C58 genomic sequence database was cloned by PCR using genomic DNA from *Agrobacterium tumefaciens* strain C58 (ATCC No. 33970) as the template. The primary PCR reaction was carried out using the following primers:
5'-TTATGCCGCCTGTCATCG-3' (SEQ ID NO: 47); and
5'-ATAGGCTTAATGGTAACCG-3' (SEQ ID NO:48).

Gene amplification parameters were as follows: (a) denature at 95°C for 30 seconds, (b) anneal at 50°C for 30 seconds and (c) extend at 72°C for 2 minutes, using Expand high fidelity PCR (Roche Biochemicals), according to manufacturer directions.

An additional round of PCR amplification, yielding a product of approximately 2.3 Kb in length, was carried out using the amplified template from above and the following nested primers:
5'-CTGAACAACAGAAGTACG-3' (SEQ ID NO: 49); and
5'-TAACCGTGTCATCGAGCG-3' (SEQ ID NO: 50).

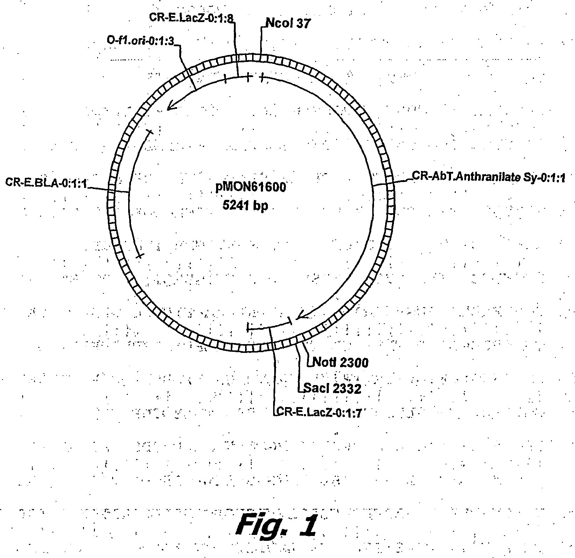

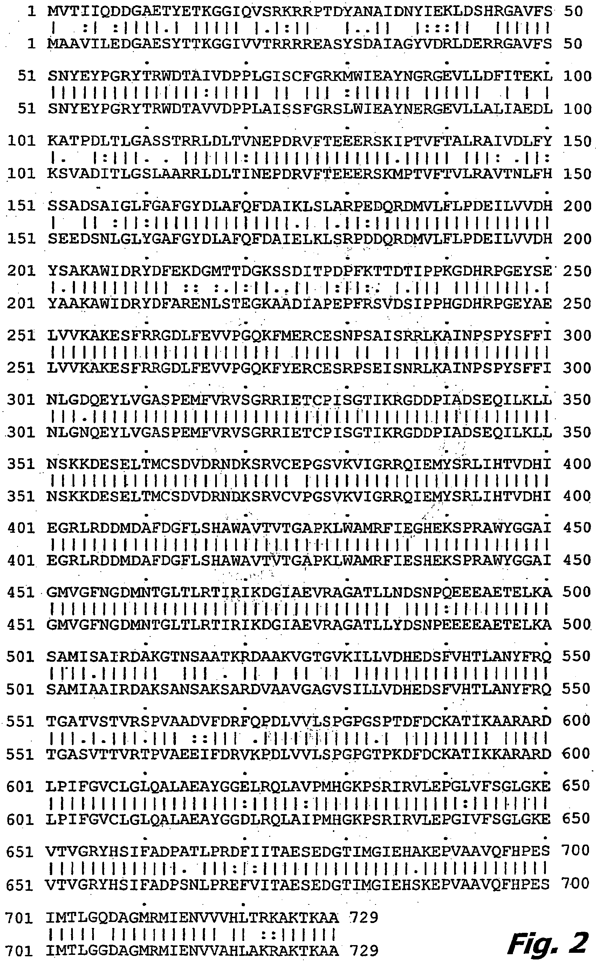

The purified PCR product was ligated into pGEM-T easy (Promega Biotech) resulting in the plasmid pMON61600 (Figure 1). pMON61600 was sequenced using standard sequencing methodology. Confirmation of the correct sequence was obtained by comparison of the sequence the *Rhizobium meliloti* anthranilate synthase sequence (Figure 2). The translated amino acid sequence from the isolated clone (SEQ ID NO: 4) shared 88% identity with the *Rhizobium meliloti* enzyme (SEQ ID NO: 7) (Figure 2).

The abbreviation "AgroAS" or *A. tumefaciens* AS is sometimes used herein to refer to *Agrobacterimn tumefaciens* anthranilate synthase.

### E. coli Expression of Agrobacterium tumefaciens AS

The following vectors were constructed to facilitate subcloning of the *Agrobacterium tumefaciens* AS gene into a suitable expression vector.

A 2215 base pair PCR fragment was generated using pMON61600 as the template and the following primers:
5'-AAAAAGATCTCCATGG TAACGATCATTCAGG-3' (SEQ ID NO: 51); and
5'-AAAAGAA TTCTTATCACGCGGCCTTGGTCTTCGCC-3' (SEQ ID NO: 52).

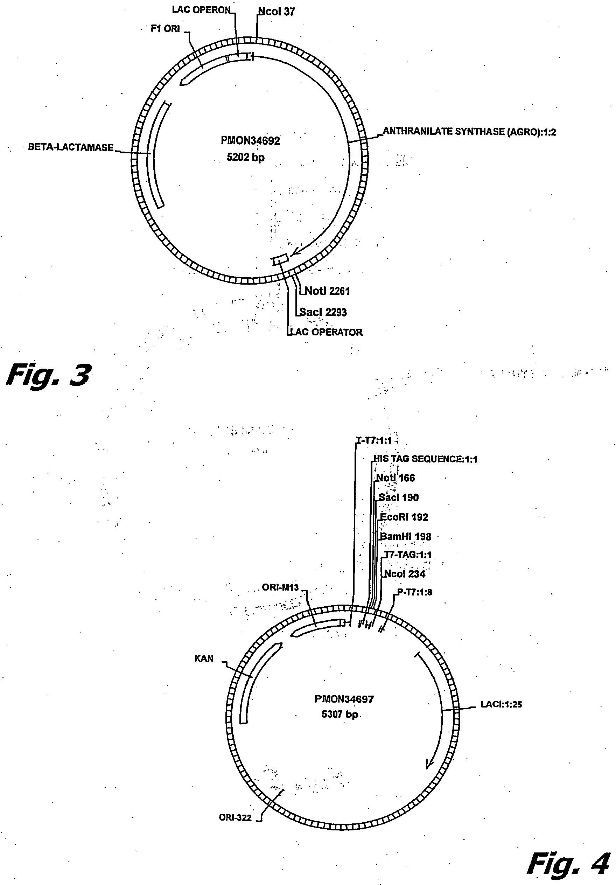

The plasmid pMON61600 was digested with restriction enzymes NcoI and RsrII. In addition, a 409bp fragment (derived by digesting the 2215 base pair PCR product with NcoI and RsrII) was then ligated into the digested pMON61600 plasmid, thereby replacing the NcoI/RsrII fragment, and resulting in a NcoI site in frame with the translation initiation codon (ATG) of *Agrobacterium tumefaciens* AS to yield plasmid pMON34692 (Figure 3).

The base T7 *E. coli* expression plasmid, pMON34697 (Figure 4), was generated by restriction digestion of pET30a (Novogen, Inc) with SphI and BamHI. The resulting 4,969 bp fragment was purified and subcloned with a 338 bp SphI and BamHI fragment from pET11d (Novogen, Inc).

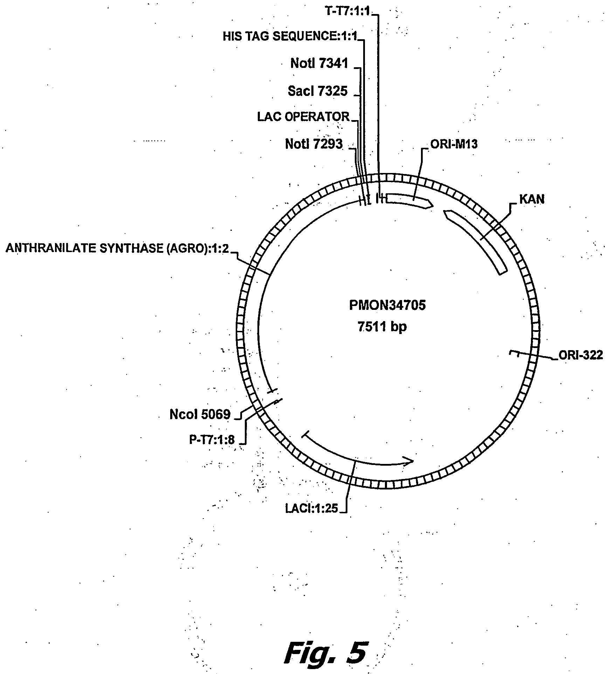

The plasmid pMON34705 (Figure 5) was generated by restriction digestion of pMON34697 with NcoI and SacI. The resulting 5,263 bp fragment was then purified and ligated with a 2,256 bp NcoI and SacI fragment from pMON34692 containing *Agrobacterium tumefaciens* AS.

The plasmid pMON34705 was transformed into *E. coli* BL21(DE3) (F-*ompT HsdS_{b}*(r_{B}⁻m_{B}⁻)*gal dcm* (DE3)) according to manufacturer's instructions (Novogen, Inc). DE3 is a host lysogen of λDE3 containing chromosomal copy of T7 RNA polymerase under control of an isopropyl-1-thio-D-galactopyranoside (IPTG) inducible *lac*UV5.

Transformed cells were selected on kanamyacin plates that had been incubated at 37°C overnight (10 hours). Single colonies were transferred to 2ml of LB (Luria Broth; per liter, 10g tryptone, 5g yeast extract, 10g NaCl, and 1g glucose (optional)) or 2X-YT broth (per liter, 16g tryptone, 10g yeast extract, 5g NaCl) and then placed in a 37°C incubator and shaken at 225rpm for 3 hours. The cells were removed and 4µL of 100mM IPTG was added to the culture and returned to the 37°C incubator for an additional 2 to 3 hours. A 1mL aliquot of the cells was removed and sonicated in sonication buffer, (5,0mM potassium phosphate (pH 7.3), 10% glycerol, 10mM 2-mercaptoethanol and 10mM MgCl₂). The resulting lysed cell extract was the source material for the standard AS assay described below. The results established that the expression system based on plasmid pMON34705 was able to produce soluble and enzymatically active *Agrobacterium tumefaciens* AS protein that accounts for approximately 50% of total soluble extracted protein.

### EXAMPLE 2: High Trp Seed Levels are Achieved by Transformation of Plants with Wild Type Agrobacterium Anthranilate Synthase.

### Expression Vector pMON58120

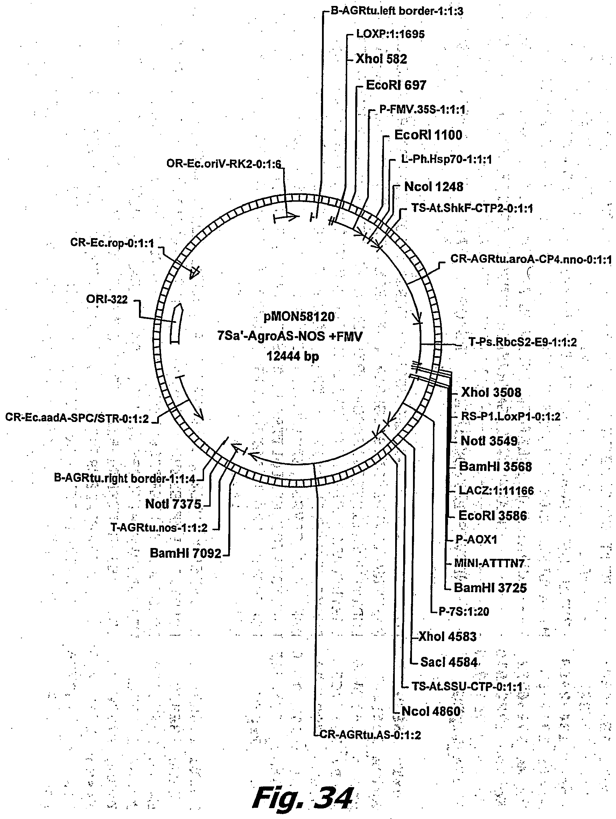

The vector pMON58120 (Figure 34) encodes a fusion between a 264 base pair *Arabidopsis* small subunit (SSU) chloroplast targeting peptide (CTP, SEQ ID NO: 71) and a 2187 base pair wild type *Agrobacterium* anthranilate synthase (AgroAS) open reading frame (SEQ ID NO: 1). *See,* Stark et al., (1992) Science, 258:287. Expression of this open reading frame is driven by the soy 7S alpha prime (7Sα') promoter.

Upon translation on cytoplasmic ribosomes, the fusion (immature protein) is imported into chloroplast where the chloroplast targeting sequence is removed. There are two cleavage sites in the CTP1. The first site is 30 base pairs upstream of the CDS start (C/M), and the other is at the initial methionine (C/M). The second cleavage site does not seem to be processed efficiently. The cleavage is predicted to yield a mature protein of about 70Kd that has AS activity as shown by enzyme activity data and tip efficacy data.

The AS gene was transformed with the synthetic CP4 gene that confers glyphosate resistance, however the CP4 gene is processed separately from the AS gene. Expression of the CP4 gene was driven by the FMV promoter, which is a 35S promoter from Figwort Mosaic Virus. Glyphosate resistance allows for selection of the transformed plants.

### Western Analysis of AS Protein

Thirty-five transformation events of pMON58120 were analyzed for AgroAS protein presence. AgroAS protein was detected with a polyclonal antibody raised in rabbits against purified His-tagged AgroAS. The His-tagged, full-length Agro-AS polypeptide was used as an antigen to generate a population of polyclonal antibodies in rabbits by CoCalico Biological, Inc. The recombinant His-tagged Agro-AS DNA was placed into a pMON 34701 (pet-30a-agroAS) expression vector. The His-AgroAS fusion protein was expressed in *E. coli* BL21 (DE3) and purified by Ni-NTA resin system (Qiagen protocol). For western analysis, primary rabbit anti-AgroAS antibodies were used at 1:5,000 dilution. Secondary, goat anti-rabbit alkaline phosphatase-conjugated antibodies were used at 1:5,000 dilution. In transgenic lines carrying 7Salpha'-Agro AS genes, western blot analysis consistently revealed the presence of a single band that specifically cross-reacted with anti-AgroAS antibodies. This band was not detected in the nontransgenic control line.

### Free Amino Acid Analysis of Soy and Arabidopsis Seed

**Amino Acid Extraction:** About 50 mg of crushed soy seed (5 mg of *Arabidopsis*) material was placed in each centrifuge vial. One milliliter of 5% trichloroacetic acid was added to each sample (100 µl for *Arabidopsis*). The samples were vortexed, and allowed to sit, with agitation, at room temperature for 15 min. They were then microcentrifuged for 15 min at 14000 rpm. Some of the supernatant was then removed, placed in a HPLC vial and sealed. Samples were kept at 4°C in the analysis queue.

**Amino Acid Analysis:** The reagents utilized for amino acid analysis included the OPA reagent (o-phthalaldehyde and 3-mercaptopropionic acid in borate buffer (Hewlett-Packard, PN 5061-3335)) where the borate buffer (0.4 N in water, pH 10.2). The analysis was performed using the Agilent 1100 series HPLC system as described in the Agilent Technical Publication, "Amino Acid Analysis Using Zorbax Eclipse-AAA Columns and the Agilent 1100 HPLC", March 17,2000. First, 0.5 µl of the sample was derivatized with 2.5 µl of OPA reagent in 10 µl of borate buffer. Second, the derivative is injected onto a Eclipse XDB-C18 5 µm, 4.6 x 150 mm column using a flow rate of 1.2 ml/min. Amino acid concentrations were measured using fluorescence: excitation at 340 nm, emission at 450 nm. Elution was with a gradient of HPLC Buffers A and B according to Table A, where HPLC Buffer A was 40 mM Na₂HPO₄, pH=7.8 and HPLC Buffer B was 9 : 9 : 2 :: Methanol: Acetonitrile : Water.

**Table A: Amino Acid Elution**

| **Time** | **0** | **20** | **21** | **26** | **27** |
|---|---|---|---|---|---|
| **% Buffer B** | 5 | 65 | 100 | 100 | 100 |

Amino acid standards were prepared from the dry chemicals, using all amino acids of interest. Proline analysis required an additional derivatization step with 9-fluorenylmethyl-chloroformate (FMOC). Amino acid standards were also sometimes purchased in concentrations ranging from 0 to 100 µg/ml. Samples were reported in µg/g of seed powder.

### Expression of Wild Type Agrobacterium Anthranilate Synthase in Arabidopsis

The vector pMON58120 was transformed into Arabidopsis plants by vacuum infiltration of the secondary influorescences, and plants were allowed to set transgenic seed. The seed was collected and screened for the presence of a selectable marker (glyphosate resistance). Glyphosate resistant plants were grown to maturity and seed from each plant, which was designated a transformation event, and analyzed for tryptophan content (Table B). Selected transformation events were also analyzed for the presence of the expressed *Agrobacterium* anthranilate synthase protein in the mature seed by Western blot analysis as shown in Table B.

**Table B: Analysis of Transformants**

| Transformation Event | **Trp (ppm)** | **Protein present** |
|---|---|---|
| 7317 | 2547 | + |
| 7315 | 2960 | + |
| 7319 | 3628 | + |
| 7313 | 3979 | + |

### Expression of Wild Type Agrobacterium Anthranilate Synthase in Soy (Glycine Max)

Thirty-three out of thirty-five soy transformation events analyzed had an increase in seed trp levels, for example, from above 500 ppm and up to 12,000 ppm. In nontransgenic soy seeds, the trp level is less than 200 ppm. All seeds that contained high amounts of trp demonstrated anthranilate synthase protein expression by western blotting. Table C presents data for nineteen soy events that contain high trp levels and also are positive for anthranilate synthase anthranilate synthase protein by western blot analysis.

**Table C: Correlation between the Presence of the Agro AS Protein and Tryptophan Levels in Nineteen Soy Transgenic Events bearing pMON58120**

| **Pedigree** | **Trp max (ppm)** | **Trp average (ppm)** | **Protein present?** |
|---|---|---|---|
| A3244 (ctr) | 306 | 96 | NO |
| GM_A20380:@. | 6444 | 2246.4 | YES |
| GM_A20532:@. | 6055 | 2556.6 | YES |
| GM_A22043:@. | 10422 | 2557.2 | YES |
| GM_A20598:@. | 8861 | 2859.9 | YES |
| GM_A20744:@. | 7121 | 3373.3 | YES |
| GM_A20381:@. | 6392 | 3572.9 | YES |
| GM_A20536:@. | 9951 | 3581.5 | YES |
| GM_A20510:@. | 8916 | 3592.7 | YES |
| GM_A20459:@. | 8043 | 3900.4 | YES |
| GM_A20337:@. | 7674 | 4088.6 | YES |
| GM_A20533:@. | 9666 | 4183.2 | YES |
| GM_A20577:@. | 6276 | 4434.1 | YES |
| GM_A20339:@. | 9028 | 4687.8 | YES |
| GM_A20386:@. | 8487 | 5285.3 | YES |
| GM_A20457:@. | 11007 | 5888.9 | YES |
| GM_A20379:@. | 7672 | 6416.1 | YES |
| GM_A20537:@. | 9163 | 6695.8 | YES |
| GM_A20534:@. | 12676 | 7618.2 | YES |
| GM_A20576:@. | 10814 | 7870.1 | YES |

### The Agro AS enzyme assay

The specific activity of anthranilate synthase was measured in eleven transformation events carrying the pMON58120 construct. Individual soybean immature seeds were analyzed using an HPLC-based end-point assay based on the method described by C. Paulsen (J. Chromatogr., 547:155-160 (1991)). Briefly, desalted extracts were generated from individual seeds in grinding buffer (100mM Tris pH7.5, 10% glycerol, 1mM EDTA, 1mM DTT) and incubated for 30 min with reaction buffer (100mM tris pH 7.5, 1 mM chorismate, 20mM glutamine, and 10mM MgCl2). Agro AS activity was measured in the presence or absence of 25mM trp. The reaction was stopped with phosphoric acid and the amount of anthranilate formed was quantified by HPLC using a fluorescence detector set at 340nm/excitation and 410 nm/emission.

The specific activity of AS in immature segregating transgenic seeds ranged from 1.5-fold up to 70-fold increase compared to a nontransgenic control, reaching as high as 6,000 pmoles/mg/min. As shown in the last column of Table D, the anthranilate synthase activity in transgenic plants is resistant to tryptophan inhibition (see Table D).

**Table D: Agro AS Enzyme Activity in Transgenic Event 20576**

| **Event** | **Seed No.** | **Specific Activity (pmoles/mg/min)** | **Specific Activity (pmoles/mg/min) (+ 25 micromolar Trp)** |
|---|---|---|---|
| Control | 3244-1 | 95.4 | 42.4 |
| Control | 3244-2 | 85.5 | 40.6 |
| 20576 | 20576-1 | 6060.2 | 4407.1 |
| 20576 | 20576-2 | 3783.8 | 1709.4 |
| 20576 | 20576-3 | 2768.3 | 2431.7 |
| 20576 | 20576-4 | 4244.08 | 2125.2 |

### EXAMPLE 3: Soybean Transformation with a Vector Containing a Maize Anthranilate Synthase α-Subunit gene.

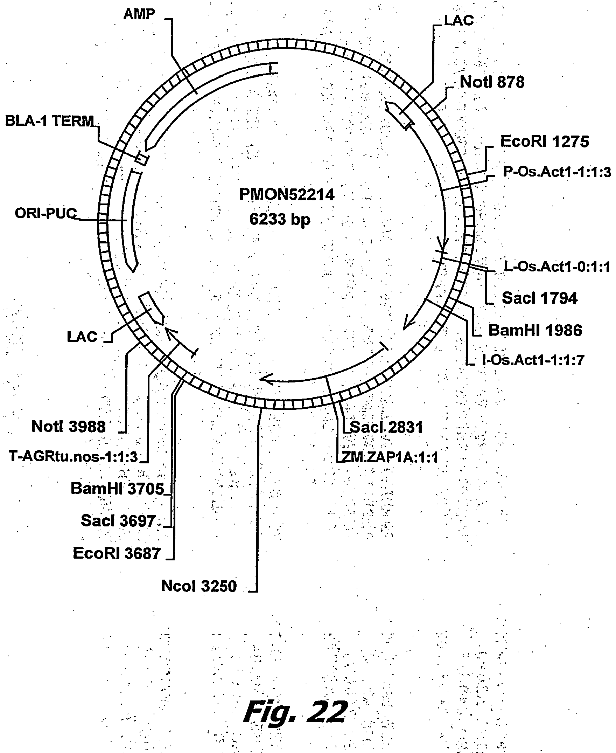

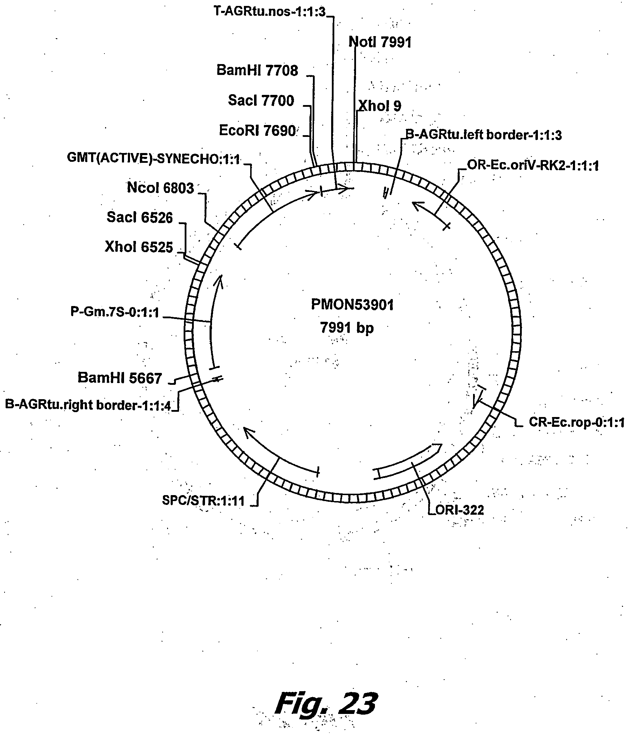

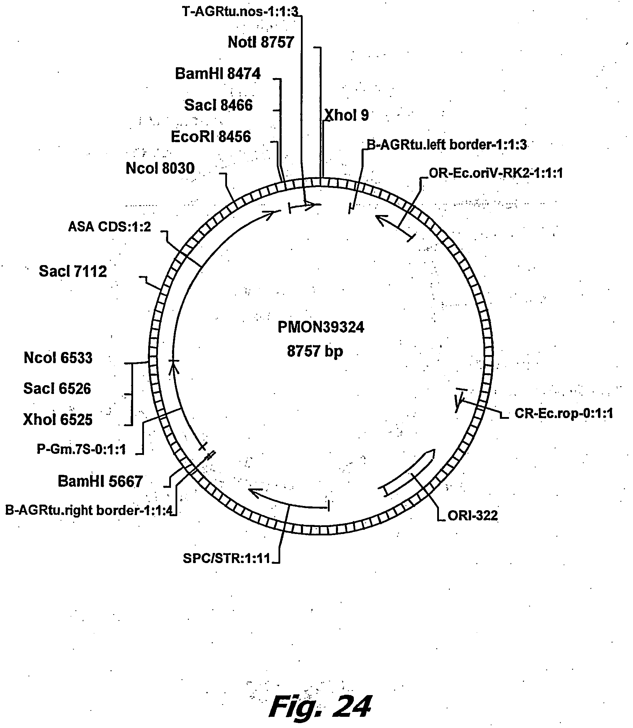

The coding sequence for a maize anthranilate synthase α-subunit was isolated from pMON52214 (Figure 22) by digesting with XbaI in combination with a partial NcoI digest (*see* Anderson *et al*., U.S. Patent 6,118,047). The resulting 1952 bp DNA fragment representing the anthranilate synthase α coding region was gel purified, and the ends were made blunt. The plasmid pMON53901 (Figure 23) was digested with BglII and EcoRI, to generate a 6.8 Kb fragment. After isolation, the ends of the 6.8 Kb fragment were made blunt and dephosphorylated. The 1952 Kb fragment containing the ASα gene was then ligated into the blunt-ended 6.8 Kb pMON53901 fragment to generate pMON39324, a maize 7S promoter-maize ASα-NOS 3' UTR expression vector (Figure 24).

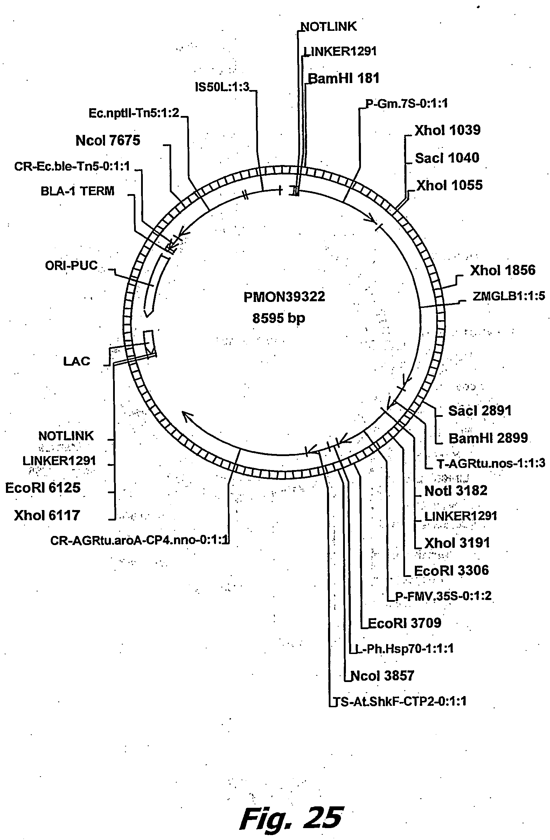

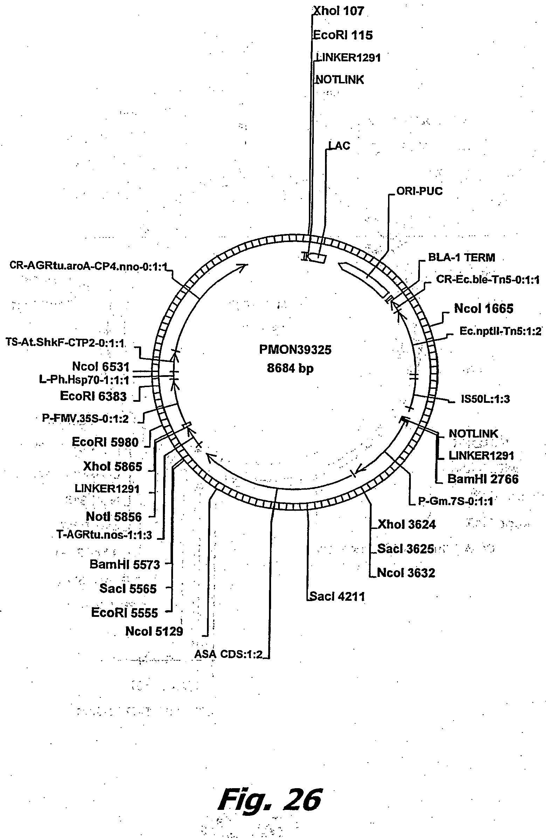

This pMON39324, a maize 7S promoter-maize ASα-NOS 3' UTR cassette, was subsequently digested with BamHI resulting in a 2.84 Kb DNA fragment, containing the 7S promoter and maize ASα coding sequence. The plasmid pMON39322 (Figure 25) was digested with BamHI resulting in a 5.88 kb DNA fragment. These two fragments were then ligated together to create pMON39325 (Figure 26), a transformation vector containing 7S promoter-maize ASα-NOS 3' UTR cassette subcloned into pMON39322.

Using similar procedures, the coding sequence for a maize anthranilate synthase α-subunit was cloned downstream from the USP promoter to generate a pMON58130 expression vector, downstream from the Arc5 promoter to generate a pMON69662 expression vector, downstream from the Lea9 promoter to generate a pMON69650 expression vector, and downstream from the Per1 promoter to generate a pMON69651 expression vector. A list with these expression vectors is presented in Table E.

**Table E: C28-Maize Anthranilate Synthase Constructs**

| **Seed Generation** | **Expression Cassette** | **Vector Name** |
|---|---|---|
| R4 | 7Sa'-maize ASα | PMON39325 |
| R2 | Napin-maize ASα | PMON58023 |
| R1 | USP-maize ASα | PMON58130 |
| R1 | Arc5-maize ASα | PMON69662 |
| R1 | Lea9-maize ASα | PMON69650 |
| R1 | Per1-maize ASα | PMON69651 |

These vectors were used for plant transformation and propagation experiments. Soybean plants were transformed with the maize AS-containing vectors using the microprojectile bombardment technology as described herein. Several transgenic soybean lines were established for each type of vector and propagated through the number of generations indicated in Table E.

For example, three homozygous lines were established that carried the 7Sα'-maize AS transgene from pMON39325. These three lines were grown in a randomized block design in two different locations. Mature seed was produced and analyzed for free amino acid content. Controls were included to establish baseline trp levels, *i.e.* the three corresponding negative isolines and the nontransgenic controls.

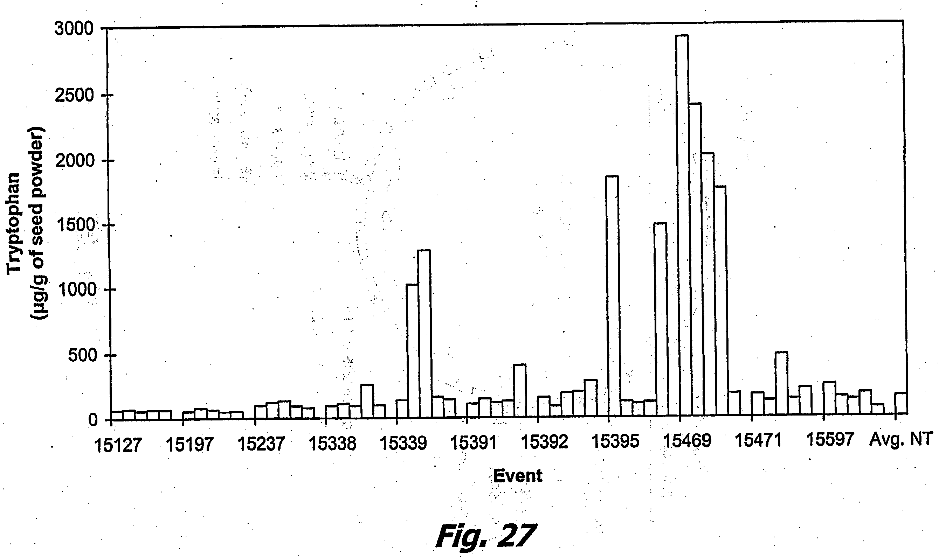

Table F provides R4 seed tryptophan in ppm for pMON39325 transformant and control lines, showing that the average non-transgenic soybeans contain about 100-200 µg tryptophan/g seed powder whereas the pMON39325 transformants contain substantially more Trp. *See also,* Figure 27.

**Table F: Trp Levels in seeds of Soybean Plants Transformed with the C28 Zea mays mutant (pMON39325)**

| **Positive isoline number** | **Average trp of Positive Isoline (ppm)** | **Standard deviation** | **Average trp of corresponding Negative isoline (ppm)** | **Standard deviation** |
|---|---|---|---|---|
| 39325-1 | 3467 | 377 | 226 | 55 |
| 35325-2 | 2623 | 307 | 164 | 20 |
| 35325-3 | 3715 | 152 | 184 | 64 |
| 35325-4 | 2833 | 165 | 202 | 146 |
| 35325-5 | 3315 | 161 | 173 | 34 |
| 35325-6 | 2394 | 318 | 144 | 22 |
| nontransgenic control-7 | | | 191 | 24 |
| nontransgenic control-8 | | | 118 | 23 |

Five other constructs, expressing the C28 maize anthranilate synthase under the control of five different promoters (Table E) were transformed into soy and transgenic plants were obtained. Each construct generated events high in trp. An example illustrating events generated by Per1-C28 maize anthranilate synthase is shown in Tables G and H.

**Table G: C28 maize AS Protein Expression Correlates with Increased Trp Levels in Three Transgenic Events bearing Per1-C28 maize AS (nMON69651)**

| **Pedigree** | **Trp average (ppm)** | **Protein present?** |
|---|---|---|
| Control | 96 | No |
| 22689 | 2375 | Yes |
| 22787 | 1707 | Yes |
| 22631 | 1116 | Yes |

Table H illustrates the enzymatic activity of C28 maize AS in R1 seeds from soybean plants transformed with the pMON69651 expression vector.

**Table H: Specific Activity of C28 maize AS in R1 Seeds of pMON69651 Transformants**

| **Event** | **Seed number** | **Specific activity (pmoles/mg/min)** | **Specific activity (pmoles/mg/min) (+ 25 micromolar tryptophan**) |
|---|---|---|---|
| Control | | 51.6 | 2.6 |
| 22689 | 22689-1 | 130.9 | 64.7 |
| | 22689-2 | 115.3 | |
| | 22689-3 | 148:5 | 61.1 |
| | 22689-4 | 149.5 | |
| | 22698-5 | 133.8 | 60.3 |

These results indicate that there is a substantial increase in tryptophan when soybean plant tissues are transformed with the C28 maize AS gene.

The high trp levels shown in Table G correlate with the presence of the AS protein and with increased specific activity (2.5 fold higher than in nontransgenic controls) for the transgenic enzyme (Table H). As shown in Table H - and as predicted by the biochemical properties of the C28 maize AS enzyme - the specific activity of transgenic events is tryptophan-resistant.

### EXAMPLE 4: Rational Design of Agrobacterium tumefacians Anthranilate Synthase tryptophan feedback insensitive mutants.

This example describes vectors containing mutant *Agrobacterium tumefaciens* anthranilate synthase enzymes that have various degrees of sensitivity or insensitivity to feedback inhibition by tryptophan or tryptophan analogs.

### Generation of Agrobacterium tumefaciens Mutant Anthranilate Synthase Genes

Using protein structural information from *Solfulobus solfataricus* anthranilate synthase as a guide (Knochel et al., Proc. Natl. Acad. Sci. (U.S.A.), 96:9479-9484 (1999)) several *Agrobacterium tumefaciens* anthranilate synthase mutants were rationally designed utilizing protein informatics to confidently assign several residues involved in tryptophan binding. This was accomplished by alignment of the *Agrobacterium tumefaciens* anthranilate synthase gene with the anthranilate synthase amino acid sequence from *Sulfolobus solfataricus* (Figure 6). The putative tryptophan binding and catalysis regions of the *Agrobacterium tumefaciens* were assigned by combining the knowledge of the structural information with the sequence homology. Residues in the binding pocket were identified as potential candidates for altering to provide resistance to feedback inhibition by tryptophan.

Based on the structural analysis of the *Sulfolobus solfataricus* anthranilate synthase enzyme, it suggested that amino acids E30, S31, I32, S42, V43, N204, P205, M209, F210, G221, and A373 were involved in tryptophan binding. Based on the pairwise alignment, N204, P205, and F210 of *Sulfolobus solfataricus* were also conserved in the monomeric *Agrobacterium tumefaciens* anthranilate synthase as residues N292, P293, and F298 respectively.

However, due to multiple insertions and deletions, the N-terminal regions of the *Sulfolobus solfataricus* and *Agrobacterium tumefaciens* enzymes were highly divergent. For this reason, it was necessary to manually assign residues at the N-terminal region of the *Agrobacterium tumefaciens* anthranilate synthase involved in tryptophan regulation (Figure 6). Structural analysis indicated that the motif "LLES" formed a β sheet in the tryptophan-binding pocket. This structure appeared to be highly conserved among the heterotetrameric enzymes. The known monomeric enzymes were then manually aligned to the *Sulfolobus solfatas-icus* sequence using the "LLES" motif as a landmark (Figure 21). Based on this protein informatics analysis, amino acid residues V48, S50, S51, and N52 in *Agrobacterium tumefaciens* AS were also likely to be involved in tryptophan binding.

With the putative tryptophan binding residues assigned in the *Agrobacterium tumefaciens* monomeric enzyme, several distinct strategies were rationalized for reducing the sensitivity of the enzyme to tryptophan inhibition. These substitutions included for example, enlarging the tryptophan-binding pocket (F298A), narrowing the binding pocket (V48F, V48Y, S51F, S51C, N52F, F298W), increasing the polarity of the binding pocket (S50K), or distorting the shape of the binding pocket by changing the protein main chain conformation (P293A, P29G).

### A. tumefaciens AS site-directed mutagenesis

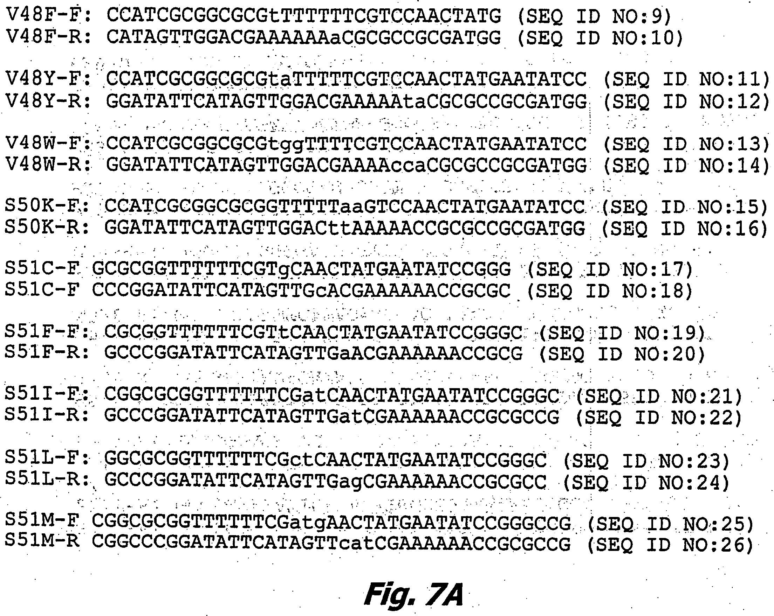

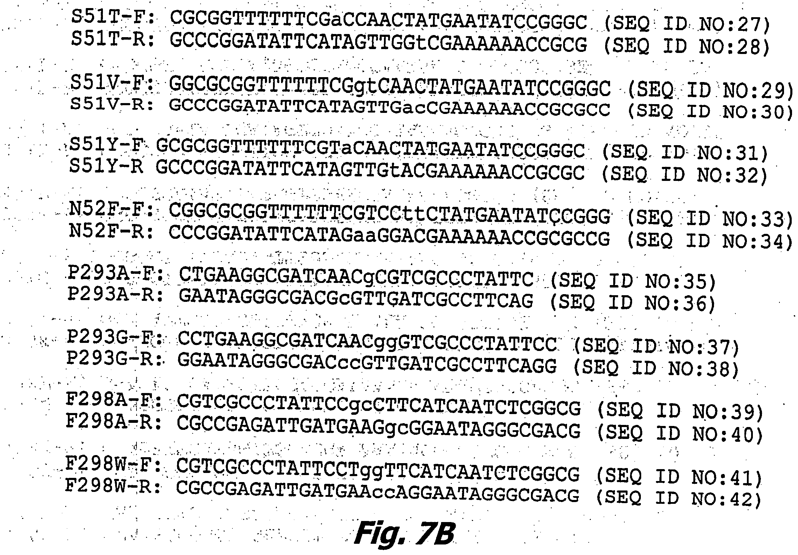

Site directed mutagenesis was used to generate ten single amino acid substitutions six sites. The mutations were introduced into the *Agrobacterium tumefaciens* AS in pMON34705 using the QuikChange^{tm} Site-Directed Mutagenesis Kit (Stratagene). The primers used for site directed mutagenesis were SEQ ID NOs: 9-42 (Figure 7; F = forward, R = reverse). Each primer sequence is specific for alteration of the nucleic acid at a specific location in the sequence and thus changing the encoded codon to code for a new amino acid. For example, S51C designates a change from serine to cysteine at amino acid position 51 in the *Agrobacterium tumefaciens* AS peptide sequence.

Following mutagenesis the sequence of the entire gene was reconfirmed and the variants expressed and purified from *E. coli* as described below for the wild type enzyme. The resultant plasmids comprising mutant *Agrobacterium tumefaciens* AS are suitably cloned into a plasmid for overproduction of protein using the T7 expression system as described in Example 1.

### Agrobacterium tumefaciens AS protein expression and purification

*Agrobacterium tumefaciens* AS wild type and mutant enzymes were expressed in *E*. *coli* as described in Example 1. The purification of all the *Agrobacterium tumefaciens* AS enzymes, including wild type and mutants therof, was performed at 4°C. The cells (approximate wet weight of 1g) were suspended in 20 ml of purification buffer (50 mM potassium phosphate, pH 7.3, 10 mM MgCl₂, 10 mM 2-mercaptoethanol, 10% glycerol) and lysed by ultrasonication (Branson sonifier Cell Disruptor, W185). Supernatant was collected after centrifugation of the homogenate at 20,000 x g for 15 min. The supernatant was subjected to ammonium sulfate fractionation (30 to 65% saturation). The precipitate was collected after centrifugation at 20,000 x g for 15 min and dissolved in 3 ml of the purification buffer and then loaded as a whole on an Econo-Pac 10DG desalting column, pre-equilibrated with the same buffer. Fractions containing the enzyme were detected by the developed assay and pooled. The pooled enzyme (4.3mls) was loaded on a 10 ml DEAE Sephacel (Pharmacia Biotech) column (1.5 x 7.5 cm) equilibrated with the same buffer. The column was washed with 30 ml of the purification buffer and the enzyme was eluted with 30 ml of 50 mM NaCl in the same buffer. Fractions containing high AS activity were pooled and precipitated by 65% ammonium sulfate saturation and isolated and desalted as above. Fractions containing the enzyme were pooled and stored at -80°C.

### Anthranilate synthase enzyme assay and kinetic analysis

The standard assay for *Agrobacterium tumefaciens* AS was performed at 25°C in an assay buffer containing 100mM potassium phosphate, pH 7.0, 10mM MgCl₂, 1mM dithiothreitol, 200µM chorismate and 10mM L-glutamine. The reaction was started by adding 30µl of enzyme to the reaction mixture and mixing. The formation of anthranilate was directly monitored by the absorbance increase at 320m for 3minutes. Initial rate of reaction was calculated as unit absorbance increase per second based on the slope of the absorbance change over the reaction time. *K*ₘ for chorismate (*K*ₘ^{Cho}) was determined in the total volume of 1 ml assay buffer containing 100mM potassium phosphate, pH 7.0, 10mM MgCl2, 1mM dithiothreitol with 10mM L-glutamine and varying the concentration of chorismate between 2.5-100µM chorismate. The *K*ₘ for glutamine (*K*ₘ^{Gln}) was determined in the total volume of 1ml assay buffer containing 100mM potassium phosphate, pH 7.0, 10mM MgCl₂, 1mM dithiothreitol with 200µM chorismate and varying the concentration of L-glutamine between 0.1-2mM L-glutamine. IC₅₀ for tryptophan (IC₅₀^{Trp}) was determined with in the total volume of 1ml assay buffer containing 100mM potassium phosphate, pH 7.0, 10mM MgCl₂, 1mM dithiothreitol, 10mM L-glutamine, 200µM chorismate and varying the concentration of L-tryptophan between 0.1-10mM L-tryptophan. Kinetic parameters and IC₅₀ of AS were calculated after fitting the data to a non-linear regression program (GraFit).

Several mutants demonstrated reduced sensitivity to tryptophan inhibition while still maintaining enzymatic activity comparable to the wild type enzyme (Table I). These results demonstrate that the extent of sensitivity to tryptophan inhibition can be decreased, for example, by mutating amino acids in the tryptophan-binding pocket of anthranilate synthase and by optimizing of the mutations demonstrating feedback insensitivity.

**Table I: Anthranilate Synthase Activity and Effect of Tryptophan on Agrobacterium tumefaciens AS Mutants**

| **Mutation** | **Codon** | ***K*ₘ^{Cho} (µM)** | ***K*ₘ^{Gln} (mM)** | ***k*_{cat}(s⁻¹)** | ***k*_{cat}/*Kₘ*^{Cho} (µM⁻¹s⁻¹)** | **IC₅₀^{Trp} (µM)** |
|---|---|---|---|---|---|---|
| WT | | 8.0 | 0.11 | 0.43 | 5.37 × 10⁻² | 5 |
| V48F | TTT | 4.5 | 0.08 | 0.24 | 5.33 × 10⁻² | 150 |
| V48Y | TAT | 4.2 | 0.10 | 0.18 | 4.28 × 10⁻² | 650 |
| S50K | AAG | 13 | 0.01 | 0.13 | 1.00 × 10⁻² | 0.1 |
| S51F | TTC | 10 | 0.06 | 0.08 | 0.80 × 10⁻² | >32,000 |
| S51C | TGC | 2.8 | 0.08 | 0.15 | 5.36 × 10⁻² | 1,500 |
| N52F | TTC | 5.5 | 0.04 | 0.21 | 3.82 × 10⁻² | 41 |
| P293A | GCG | 24 | 0.16 | 0.35 | 1.46 × 10⁻² | 14 |
| P293G | GGG | 33 | 0.07 | 0.48 | 1.45 × 10⁻² | 17 |
| F298A | GCC | 9.2 | 0.10 | 0.46 | 5.00 × 10⁻² | 5.5 |
| F298W | TGG | 18 | 0.14 | 0.44 | 2.44 × 10⁻² | 450 |

### EXAMPLE 5: Random mutagenesis of Agrobacterium tumefaciens AS to generate tryptophan feedback insensitive mutants.

In addition to the rational design approaches described in Example 4, other strategies to generate feedback insensitive mutants of anthranilate synthase include, but are not limited to, random mutageneseis. Random mutagenesis of the *Agrobacterium tumefaciens* AS, can be accomplished, for example, by chemical mutagenesis (isolated DNA or whole organism), error prone PCR, and DNA shuffling. This example describes the use of chemical mutagenesis followed by genetic selection. The genetic selection approach is also useful for selection of desirable mutants derived from other mutagenesis techniques.

### Generation of E. coli expression plasmid containing A. tumefaciens AS

The open reading frame from the *Agrobacterium tumefaciens* AS clone pMON61600 (SEQ ID NO: 1, described in Example 1) was amplified by PCR using primers that contain an Nco 1 site on the 5' end of the forward primer and an Xba1 site on the 3' end of the reverse primer:
5'-CATCCCATGGATGGTAACGATCATT CAGGAT-3' (SEQ ID NO: 55); and
5'-GATGTCTAGAGACAC TATAGAATACTCAAGC-3' (SEQ ID NO: 56).

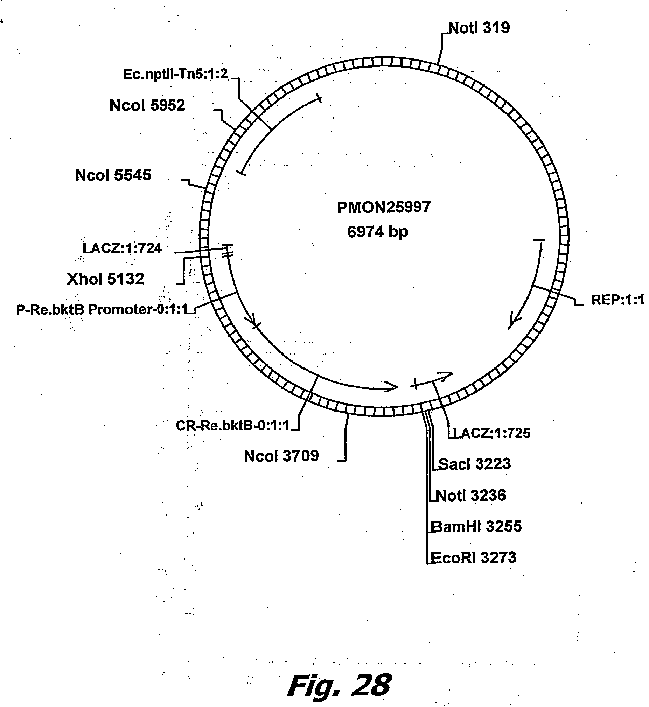

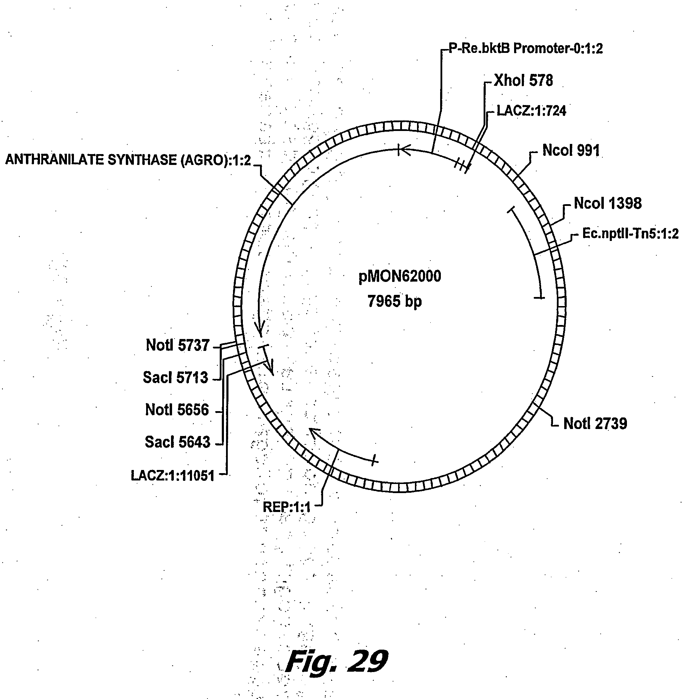

The resulting PCR product was ligated into pMON25997 (Figure 28), which had the bktB open reading frame (Slater et al., J. Bact., 180:1979-1987 (1998)) removed by digestion with BspH1 and Xba1 resulting in plasmid pMON62000 (Figure 29). pMON62000 is the base plasmid used for mutagenesis and complementation of the tryptophan auxotroph (EMG2ΔtrpE).

### Generation of an E. coli tryptophane auxotroph EMG2ΔtrpE

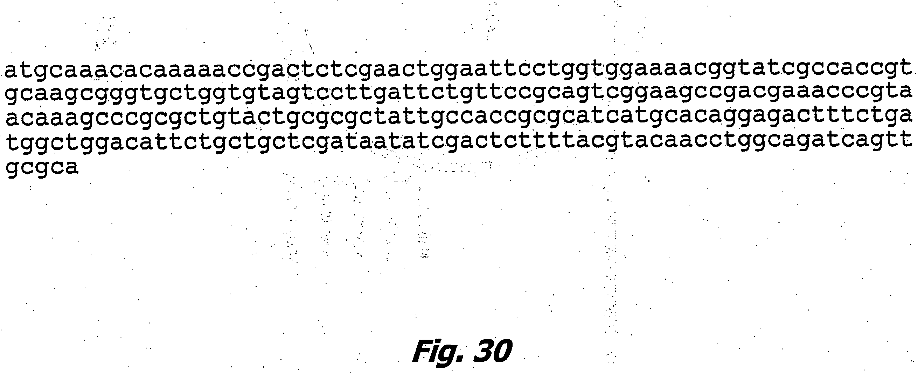

*E. coli* strain Ec-8 (EMG2ΔtrpE) was constructed using the suicide vector pKO3 to delete 1,383 base pairs from the chromosomal *trp*E gene of *E. coli* strain EMG2(K-12 wt F+) (*E. coli* Genetic Stock Center). Two amplicons from *E. coli* genomic DNA were PCR amplified. The first amplicon was approximately 1.5kb and contained the first 30bp of the *trp*E ORF at the 3' end. This amplicon contains a BamH1 site at the 5' end and an EcoR1 site at the 3' end. The second amplicon was approximately 1kb and contained the last 150 bp of the trpE ORF at the 5' end. This amplicon contains an EcoR1 site at the 5' end and a Sal1 site at the 3' end. The two amplicons were digested with the appropriate enzymes and ligated together at the EcoR1 site to create an in-frame deletion of *trp*E. Figure 30 shows the resulting sequence of the truncated gene (SEQ ID NO: 46). The *trp*E deletion amplicon was ligated into pKO3 at the BamH1 and Sal1 sites. Gene disruption was performed as described in A. J. Link et al. J. Bacteriol., 179:6228 (1997).

### Complementation of E. coli tryptophan auxotroph EMG2ΔtrpE with pMON62000

*E. coli* strain Ec-8 (EMG2ΔtrpE) was transformed with pMON62000 and plated on M9 minimal medium to determine if the deletion was complemented by the addition of pMON62000. A plasmid control (minus the *Agrobacterium tumefaciens* AS insert) and a strain control Ec-8 were also plated onto M9 minimal medium and onto M9 minimal medium with 40µg/ml tryptophan. Growth of strain Ec-8 transformed with pMON62000 was observed on M9 without tryptophan, no growth of either of the controls was observed, indicating complementation of the *trp*E deletion in strain Ec-8 by pMON62000.

### Hydroxylamine mutagenesis of pMON62000 and genetic selection of mutants

To generate mutants of anthranilate synthase, pMON62000 was mutated with the chemical mutagen hydroxylamine. The following ingredients were combined in an eppendorf tube: 20µg pMON62000 plasmid DNA and 40µl 2.5 M hydroxylamine, pH 6.0. The volume was brought to a volume of 200µl with 0.1M NaH₂PO₄, pH6.0 + 5mM EDTA, pH 6.0. The tube was incubated at 70°C. After 1.5 hours, 100µl of reaction mixture was dialyzed on a nitrocellulose filter that was floating on approximately 500ml H₂O. After 15 minutes, the DNA was concentrated using Qiagen PCR Purification Kit. After 3 hours, the remaining 100µl of the reaction mixture was removed and purified in the same manner.

*E. coli* strain Ec-8 was then transformed by electroporation with 100ng of pMON62000 that had been mutagenized for either 1.5 or 3 hours with hydroxylamine. Two transformation procedures were performed for each time point. Transformed cells were allowed to recover for 4 or 6 hours in SOC medium (20g/L Bacto-Tryptone, 5g/L Bacto Yeast Extract, 10ml/L 1M NaCl, 2.5ml/L 1M KCl, 18g glucose).

Two 245mm square bioassay plates were prepared containing M9 minimal medium, plus 2% agar, and 50µg/ml 5-methyl-DL-tryptophan (5-MT). An aliquot of 900 µl of the 1.5 hour mutagenized transformation mixture was plated onto one 50µg/ml 5-MT plate. The remaining 100 µl was plated onto the M9 control plate. The same procedure was performed for the transformation mixture containing the 3.0 hour mutagenized plasmid.

The plates were then incubated at 37°C for approx. 2.5 days. Resistant colonies were isolated from the 5-MT plates and were streaked onto LB-kanamycin (50ag/ml) plates to confirm the presence of the plasmid. All of the selected colonies grew on these plates. Individual colonies from each of the resistant clones were prepped in duplicate to isolate the plasmid. Restriction digests and PCR were performed and confirmed that all the clones contained the desired *Agrobacterium tumefaciens* AS insert.

The rescued plasmids were then transformed back into strain Ec-8. One colony from each tranformation was purified by streaking onto new LB-Kanamycin plates. To confirm resistance to 5-MT, individual purified colonies were streaked onto plates containing M9 plus 50 µg/ml 5-MT and 2% agar, and then grown at 37°C for 3 days. Resistance was confirmed for most of the clones. To determine if resistant mutants would remain resistant at an even higher concentration of 5-MT, they were plated onto M9 plus 300 µg/ml 5-MT and 2% Agar. Most clones demonstrated resistance at this high concentration also.

The plasmids from all of the resistant clones were isolated and sequenced on both strands. Some of the mutations from this experiment are diagrammed in Table J.

**Table J: A. tumefaciens trpEG Sequence Variations in 5-MT Resistant Clones.**

| **Database Clone #** | **Original Clone #** | **Determined Sequence Variations** | ***K*ₘ^{cho} (µM)** | **IC₅₀^{trp} (µM)** |
|---|---|---|---|---|
| Wt | | | 8.0 | 5.0 |
| Ec-12 | 1 | G4A Val2Ile | | |
| Ec-18 | 8 | C35T Thr12Ile | 15 | 2.5 |
| Ec-19 | 9 | C2068T Pro690Ser | 5.0 | 3.4 |
| Ec-20 | 11 | G1066A Glu356Lys & C1779T Ile593Ile | | |

As indicated by the data in Table J, several mutants had little effect on the *K*ₘ^{cho} and IC₅₀^{trp} of the mutant enzyme, indicating that these mutations are likely not the source of resistance to tryptophan feedback inhibition. For example, the mutation of C to T at nucleotide 35, which changes a threonine residue to isoleucine at amino acid position 12 (Thr12Ile), gives rise to a minor change in *K*ₘ^{cho} and IC₅₀^{trp} values. Similarly, a change of C to T at nucleotide position 2068, which changes a proline to a serine also gives rise to a minor change in *K*ₘ^{cho} and IC₅₀^{trp}values. These mutations may therefore, may be "silent" mutations that give rise to variant gene products having enzymatic properties like those of wild type.

### EXAMPLE 6: High Tryptophan Transgenic Soybean Plants.

This example sets forth preparation of transgenic soybean plants having elevated tryptophan levels resulting from transformation with tryptophan feedback insensitive mutants of anthranilate synthase from *Agrobacterium tumefaciens.*

### Vector Construction

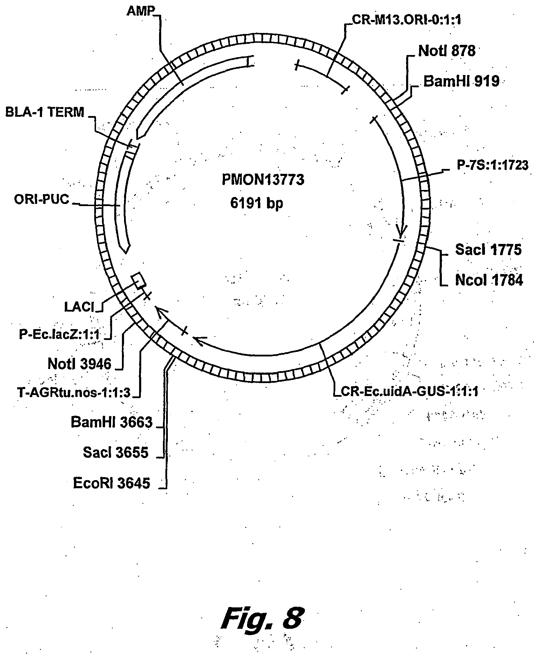

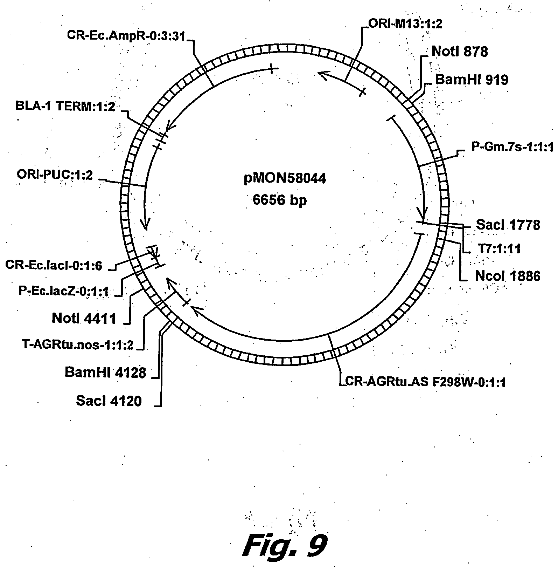

Plasmid pMON34711, which harbors the anthranilate synthase clone from *Agrobacterium tumefaciens* containing the F298W mutation described in Example 4, was digested with restriction enzyme NotI. The ends of the resulting fragment were blunted and then digested with NcoI. The plasmid pMON13773 (Figure 8) was then digested with restriction enzyme EcoRI, the ends blunted and then digested with NcoI. The resulting fragments were ligated resulting in plasmid pMON58044, which contained the AS gene under the control of the 7S promoter and NOS 3' UTR (Figure 9).

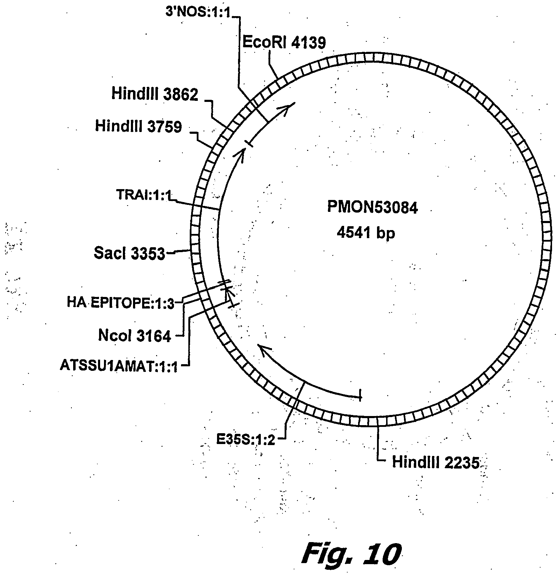

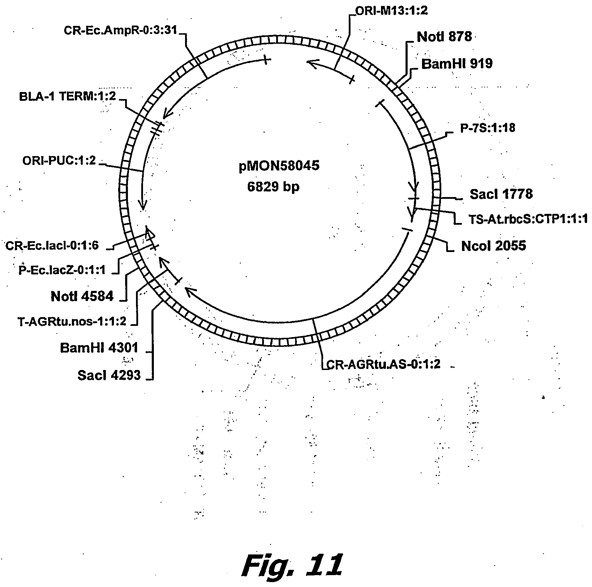

Plasmid pMON58044 was then cut with restriction enzymes BglII and NcoI and ligated with a fragment that was generated by digesting pMON53084 (Figure 10) with BglII and NcoI. The resulting fragment was named pMON58045 (Figure 11) and contained the sequence for the *Arabidopsis* SSU1A transit peptide.

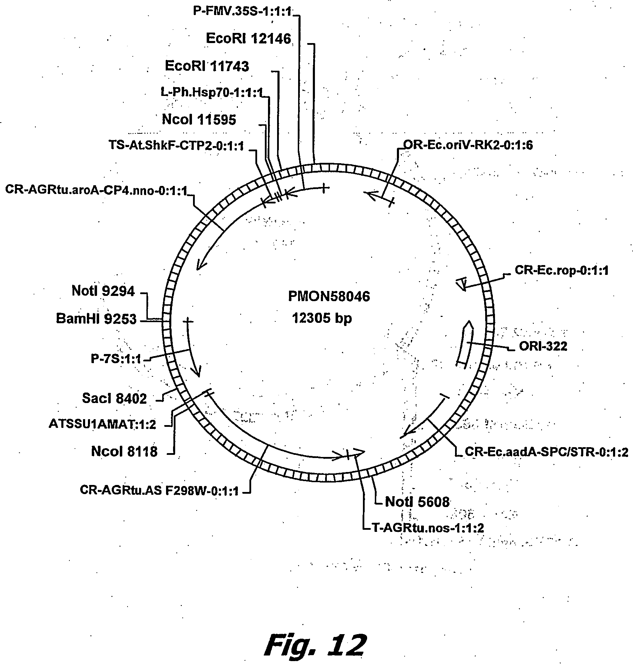

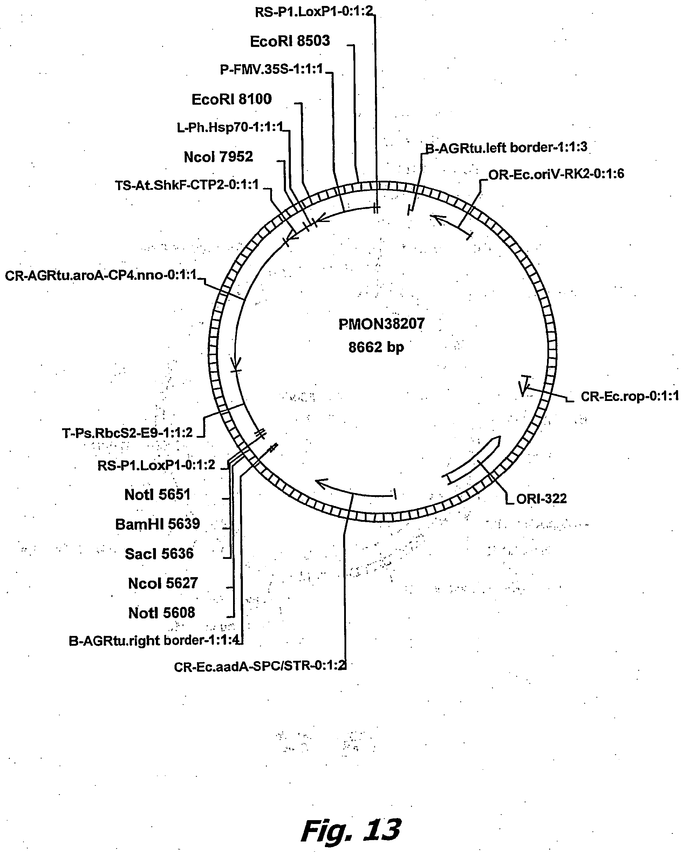

Finally, plasmid pMON58046 (Figure 12) was constructed by ligating the fragments generated by digesting pMON58045 (Figure 11) and pMON38207 (Figure 13) with restriction enzyme NotI. This resulted in the pMON58046 vector (Figure 12) that was used for soybean transformation.

### Soybean Transformation By Microprojectile Bombardment

For the particle bombardment transformation method, commercially available soybean seeds (*i*.*e*., Asgrow A3244, A4922) were germinated overnight for approximately 18-24 hours and the meristem explants were excised. The primary leaves were removed to expose the meristems and the explants were placed in targeting media with the meristems positioned perpendicular to the direction of the particle delivery.

The pMON58046 transformation vector described above was precipitated onto microscopic gold particles with CaCl₂ and spermidine and subsequently resuspended in ethanol. The suspension was coated onto a Mylar sheet that was then placed onto the electric discharge device. The particles were accelerated into the plant tissue by electric discharge at approximately 60% capacitance.

Following bombardment, the explants were placed in selection media (WPM + 0.075 mM glyphosate) (WPM = Woody Plant Medium (McCown & Lloyd, Proc. International Plant Propagation Soc., 30:421, (1981) minus BAP)) for 5-7 weeks to allow for selection and growth of transgenic shoots. Phenotype positive shoots were harvested approximately 5-7 weeks post-bombardment and placed into selective rooting media (BARM + 0.025mM glyphosate) (*see,* below for BRM recipe) for 2-3 weeks. Shoots producing roots were transferred to the greenhouse and potted in soil. Shoots that remained healthy on selection, but did hot produce roots were transferred to non-selective rooting media (BRM without glyphosate) for an additional 2 weeks. The roots from any shoots that produced roots off the selection were tested for expression of the plant selectable marker before transferring to the greenhouse and potting in soil. Plants were maintained under standard greenhouse conditions until R1 seed harvest.

The recipe used for Bean Rooting Medium (BRM) is provided below.

| **Compound** | **Quantity for 4L** |
|---|---|
| MS Salts*** | 8.6g |
| Myo-inositol(cell culture grade) | 0.40g |
| SBRM Vitamin Stock** | 8.0ml |
| L-Cysteine (10mg/ml) | 40.0ml |
| Sucrose (ultra pure) | 120g |
| Adjust pH to 5.8 | |
| Washed Agar | 32g |
| Additions after autoclaving: | |
| SBRM/TSG Hormone Stock* | 20.0ml |

| | |
|---|---|
| *SBRM/TSG Hormone Stock (per 1L of BRM): 3.0ml IAA (0.033mg/ml), 2.0ml sterile distilled water. Store stock in dark at 4°C. **SBRM Vitamin Stock (per 1L of stock): Glycine (1.0g), Nicotinic Acid (0.25g), Pyridoxine HCl (0.25g), Thiamine HCl (0.25g). ***3X MInor MS Salts (per 1L stock): H₂BO₃ (1.86g), MnSO₄ (5.07g), ZnSO₄-H₂O (2.58g), KI (0.249g), 7.5 ul NaMoO-2H₂O (1.0mg/ml), 7.5 ul CoSO₄-5H₂0 (1.0mg/ml), 7.5 ul CoCl₂-6H₂O (1.0mg/ml). | |

One ingredient at a time was added and dissolved, the volume was brought to one liter with sterile distilled water, and the solution was stored in a foil-covered bottle in the refrigerator for no longer than one month.

### Soybean Transformation Using Agrobacterium tumefaciens

For the *Agrobacterium* transformation method, commercially available soybean seeds (Asgrow A3244, A4922) were germinated overnight (approximately 10-12 hours) and the meristem explants were excised. The primary leaves may or may not have been removed to expose the meristems and the explants were placed in a wounding vessel.

*Agrobacterium* strain ABI containing the plasmid of interest was grown to log phase. Cells were harvested by centrifugation and resuspended in inoculation media containing inducers. Soybean explants and the induced *Agrobacterium* culture were mixed no later than 14 hours from the time of initiation of seed germination and wounded using sonication.

Following wounding, explants were incubated in *Agrobacterium* for a period of approximately one hour. Following this inoculation step, the *Agrobacterium* was removed by pipetting and the explants were placed in co-culture for 2-4 days. At this point, they were transferred to selection media (WPM + 0.075 mM glyphosate + antibiotics to control *Agrobacterium* overgrowth) for 5-7 weeks to allow selection and growth of transgenic shoots.

Phenotype positive shoots were harvested approximately 5-7 weeks post-bombardment and placed into selective rooting media (BRM + 0.025 mM glyphosate) for 2-3 weeks. Shoots producing roots were transferred to the greenhouse and potted in soil. Shoots that remained healthy on selection, but did not produce roots were transferred to non-selective rooting media (BRM without glyphosate) for an additional 2 weeks. The roots from any shoots that produced roots off the selection were tested for expression of the plant selectable marker glyphosate resistance before transferring to the greenhouse and potting in soil. Plants were maintained under standard greenhouse conditions until R1 seed harvest.

### Analysis of Amino Acid Content of R1 Seed

Mature R1 seed is produced and analyzed for free amino acid content using fluorescence detection as described in Agilent Technologies Technical Bulletin REV14. Five seeds are chosen for single seed analysis from each event. Soy seeds expressing AgroAS F298W, AgroAS S51F, AgroAS V48F, AgroAS V48Y or AgroAS S51C mutant proteins generate very high amounts of tryptophan. The highest levels of tryptophan have a negative impact on germination. Results are shown in Tables K, L and M.

**Table K: Protein expression in Seeds Transformed with pMON58046**

| **Pedigree** | **Trp average (ppm)** | **Protein present?** |
|---|---|---|
| Control | 96 | no |
| 228177 | 9922 | yes |
| 22891 | 12955 | yes |
| 23026 | 7968 | yes |

**Table L: AS Protein expression Correlated with pMON58123 Transformation**

| **Pedigree** | **Trp average (ppm)** | **Protein present?** |
|---|---|---|
| Control | 96 | No |
| 23562 | 88 | No |
| 23590 | 8795 | Yes |
| 23911 | 388 | No |

**Table M. Average and max trp levels in soybeans carrying one of the following Agro AS alleles: V48F (pMON66877), V48Y (pMON66878) and S51C (pMON66879).**

| **PMON number** | **Description** | **Event number** | **Average of trp (ppm)** | **Max of trp (ppm)** |
|---|---|---|---|---|
| 66877 | 7Salpha-V48F AgroAS | 26640 | 12,283 | 28,342 |
| 66877 | 7Salpha-V48F AgroAS | 26641 | 5,588 | 14,579 |
| 66877 | 7Salpha-V48F AgroAS | 26642 | 11,833 | 18,712 |
| 66878 | 7Salpha-V48Y AgroAS | 26872 | 6,015 | 11,902 |
| 66878 | 7Salpha-V48Y AgroAS | 26875 | 12,361 | 17,181 |
| 66878 | 7Salpha-V48Y AgroAS | 27010 | 13,962 | 19,323 |
| 668799 | 7Salpha-S51C AgroAS | 27105 | 12,614 | 31,827 |
| 66879 | 7Salpha-S51C AgroAS | 27300 | 16,711 | 34,263 |
| 66879 | 7Salpha-S51C AgroAS | 27568 | 10,135 | 20,237 |

### AS Enzyme Activity in R1 Seed Transformed with Agro AS

Mature R1 seed is produced and analyzed for anthranilate synthase activity. Anthranilate synthase enzymatic activity was determined in R1 soy seeds carrying the AgroAS F298W (SEQ ID NOs: 65 or 91) or the Agro AS S51F (SEQ ID NOs: 60 or 86) mutant alleles. Very high levels of tryptophan-resistant anthranilate synthase activity was observed, consistent with the high amounts of tryptophan generated by these seeds. Results are shown in Tables N and O.

**Table N: Specific activity of AS in R1 Seeds Transformed with pMON58046**

| **Event** | **Seed number** | **Specific activity (pmoles/mg/min)** | **Specific activity (pmoles/mg/min) (+ 25 micromolar Trp)** |
|---|---|---|---|
| Control | | 77.6 | |
| 23076 | 23076-1 | 100.5 | 1.04 |
| | 23076-2 | 4512.8 | |
| | 23076-3 | 9737.4 | 9290.4 |
| | 23076-4 | 136.12 | |
| | 23076-5 | 8992.5 | 9749.9 |

**Table O: Specific activity of AS in R1 Seeds Transformed with pMON58123**

| **Event** | **Seed number** | **Specific activity (pmoles/mg/min)** | **Specific activity (pmoles/mg/min) (+ 25 micromolar Trp)** |
|---|---|---|---|
| Control | | 83.7 | 32.7 |
| 23590 | 23590-1 | 891 | 692.3 |
| | 23590-2 | 466.2 | 186.5 |
| | 23590-3 | 71.7 | 38.3 |
| | 23590-4 | 320.5 | 316.2 |

### EXAMPLE 7: Preparation of Transformation Vector Comprising Ruta graveolens Anthranilate Synthase α-Subunit.

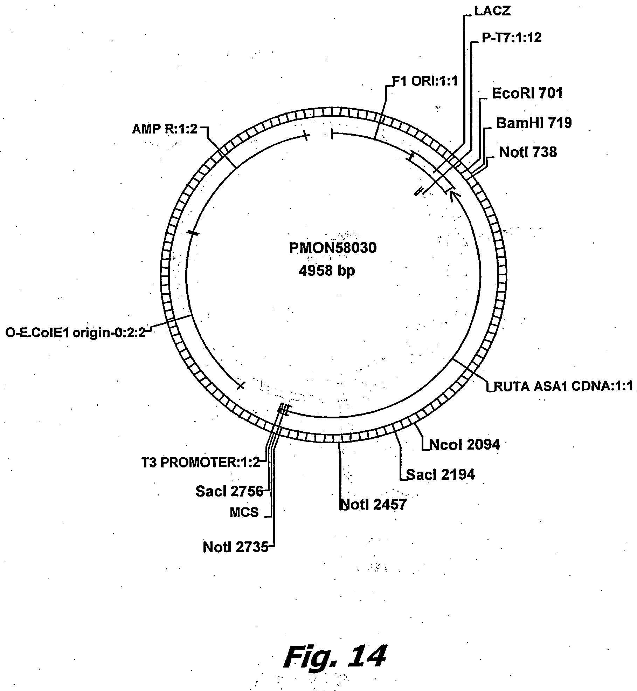

The anthranilate synthase α gene from *Ruta graveolens* (Genbank Accession No. GI 960291) provides another anthranilate synthase domain useful in the present invention (Bohlmann, J *et al*., *Plant Phys*., 111:507-514 (1996)). One isoenzyme of anthranilate synthase present in the genome of *Ruta graveolens* demonstrates less susceptibility to feedback inhibition by Tryptophan. This allele may also be useful in the present invention to elevate the levels of free L-tryptophan in transgenic plants. The vector pMON58030 (Figure 14) contains the *Ruta graveolens* anthranilate synthase α-subunit that is less sensitive to tryptophan inhibition. The *Ruta graveolens* anthranilate synthase a gene was PCR amplified from pMON58030 to provide a BamHI site at the 5' end and a BglII site at the 3' end of the *Ruta graveolens* anthranilate synthase α gene fragment by utilizing PCR primers that contained these two restriction enzyme sites:
5'-CAAAAGCTGGATCCCCACC-3' (SEQ ID NO: 53); and
5'-CCTATCCGAGATCTCTCAACTCC-3' (SEQ ID NO: 54).

The PCR fragment was purified, digested with the respective restriction enzymes, to form pMON58041, which contains the transcriptional fusion of the *Ruta graveolens* ASα to the napin promoter. The *Agrobacterium* mediated plant transformation plasmid, pMON58043, was created comprising the napin promoter, *Ruta graveolens* AS, NOS terminator, glyphosate resistance (CP4) selectable marker and borders suitable for proper chromosomal integration of the cassette as described. The resulting plant transformation vector was used to transform plants using standard plant transformation techniques as described in Examples 2, 3, and 6.

### EXAMPLE 8: Transforming multi-polypeptide anthranilate synthases into monomeric single polypeptide anthranilate synthases.

Generation of a monomeric anthranilate synthase by fusion of selected multi-subunit enzymes is desirable, for example, to maximize the catalytic efficiency, to stabilize the enzyme, to achieve coordinated expression, for example, of subunits comprising activities of TrpE and TrpG and for effective communication between the two subunits. In some instances, it may be useful to employ TrpE or α-subunits from either plant or microbial source that are deregulated with respect to feedback inhibition by standard mutagenesis techniques or by rational design as described in the foregoing Examples, *e.g*. in Example 4. In other instances, wild type TrpE or α-subunits from either plant or microbial source are employed.

The C-terminus of the selected TrpE or α-subunit is linked to the N-terminus of the TrpG subunit or β-subunit, preferably with a peptide linker. A linker can be rationally designed to provide suitable spacing and flexibility for both subunits to properly align. Alternatively a linker can be identified by sequence alignment of monomeric and heterotetrameric anthranilate synthases. Examples of sequence alignments of monomeric and heterotetrameric anthranilate synthase forms are shown in Figures 21 and 35. It is also envisioned that it may be necessary to generate monomeric anthranilate syrithases comprising heterologous subunit in order to maximize the benefits. For example, an α-subunit maybe obtained from a bacterial source, for example, *E. coli* and fused to a β-subunit from a plant source, for example, *Arabidopsis.*

The novel protein produced can be introduced into plants, for example, as described in Examples 2, 3, or 6.

### EXAMPLE 9: Identification of anthranilate synthases from genomic sequence databases.

Monomeric anthranilate synthases as well as a and domains useful in the invention can be identified by bioinformatics analysis by searching for example, genbank and/or swissprot databases using BLAST (www.ncbi.nlm.nih.gov/blast/). Useful query sequences to identify monomeric anthranilate synthase include, for example, domains of anthranilate synthase such as the α-domain (GI 1004323) or β-domain (GI 1004324) from *Sulfolobus solfataricus,* or monomeric anthranilate synthase such as *Agrobacterium tumefaciens* AS (GI 15889565). Putative monomeric anthranilate synthase will have between 50% and 100% homology with the query sequence and should minimally contain 700 amino acids. If the AS-α-domain is used to query the genomic database, in addition to identifying putative anthranilate synthase genes it is also likely to identify genes involved in PABA synthesis for example 4-amino-4-deoxychorismate (ADC) synthase. The monomeric ADC synthase genes can be easily identified away from putative monomeric AS genes based on the observation that the amidotransferase domain (β-domain) of ADC synthase resides at the N-terminus of the protein whereas the amidotransferase domain (β-domain) of AS resides at the C-terminus. Monomeric anthranilate synthases useful in the present invention identified by bioinformatics analysis include, but are not limited to, for example, *Rhizobium meliloti* (GI 95177), *Mesorhizobium loti* (GI 13472468), *Brucella melitensis* (GI 17982357), *Nostoc sp.* PCC7120 (GI 17227910, GI 17230725), *Azospirillum brasilense* (GI 1174156), *Rhodopseudomonas palustris, Anabaena* M22983 (GI 152445). Figure 21 is an example of a sequence alignment of 2 monomeric anthranilate synthases (*Agrobacterium tumefaciens* and *Rhizobium meliloti*) with 2 heterotetrameric anthranilate synthases (*Sulfolobus solfataricus* and *Arabidopsis thaliana*) useful in the present invention. Figure 35 is an example of a sequence alignment of several monomeric anthranilate synthases with the *Rhodopseudomonas palustris* heterotetrameric anthranilate synthase.

### EXAMPLE 10: Optimized Codon Usage.

This example sets forth a method of improving the expression of an anthranilate synthase gene in the seed of a plant by optimization of the codon usage.

The nucleotide sequence of the anthranilate synthase (AS) gene from wild type *Agrobacterium tumefaciens* (SEQ ID NO: 1) was inspected for the presence of underexpressed codons. To identify underexpressed codons sequences of highly expressed seed proteins from corn and soybeans were examined for relative codon frequency. The relative codon usage frequencies are shown in Table P represented in an expected value format. Expected value format can be exemplified as follows: Assume there are four codons that encode a given amino acid, and assume that they are used equally well, then each codon would be expected to account for 25% (0.25) of the frequency for that amino acid. However, due to redundancy, 0.25 was normalized to 1.0 to give a relative score for each codon as compared to other codons that encode that amino acid. For this analysis, if a codon was more prevalent that the other choices for a given amino acid, it received a number that was greater than 1.0. Correspondingly, if a codon was less prevalent, it received a number less than 1.0. For this study, a particular codon was considered underrepresented if it's relative codon usage frequency was lower than 0.5.

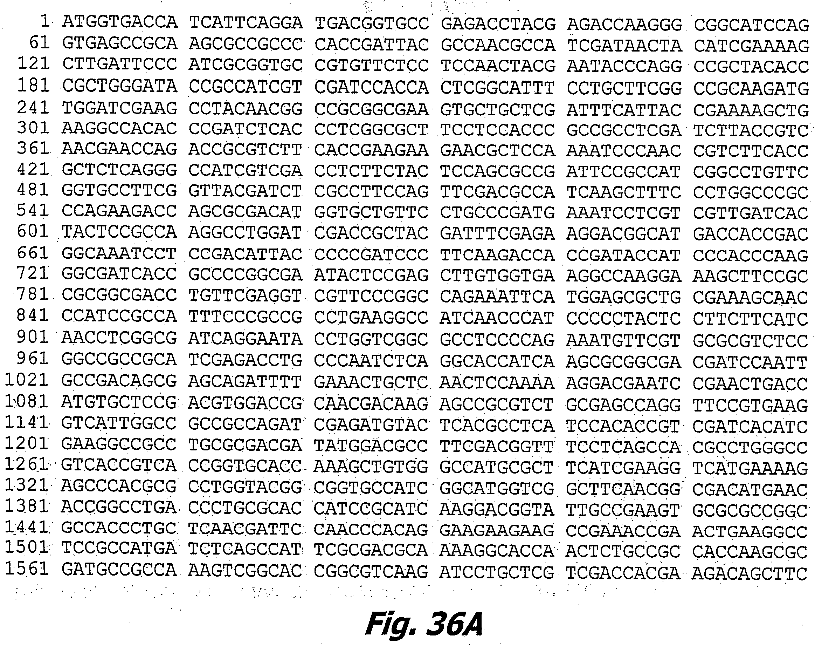

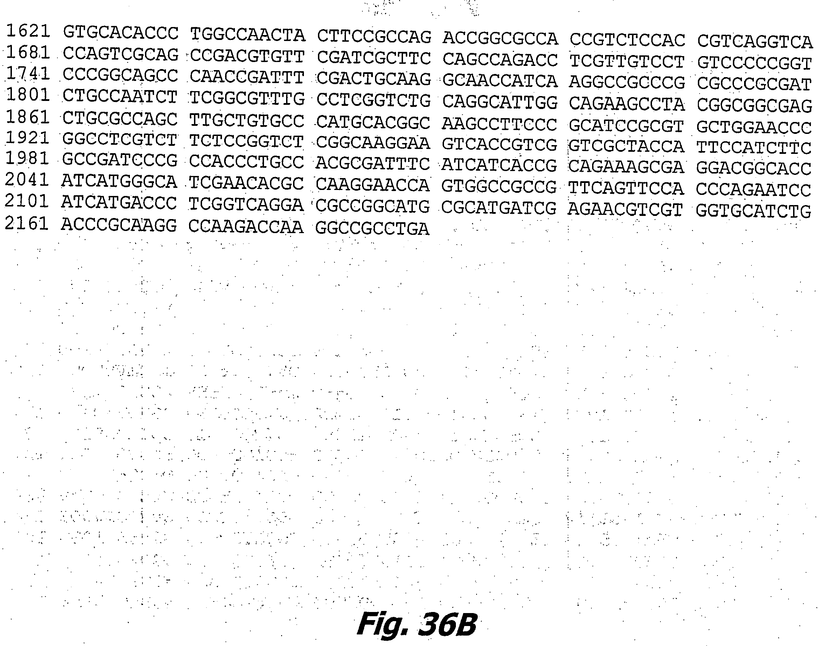

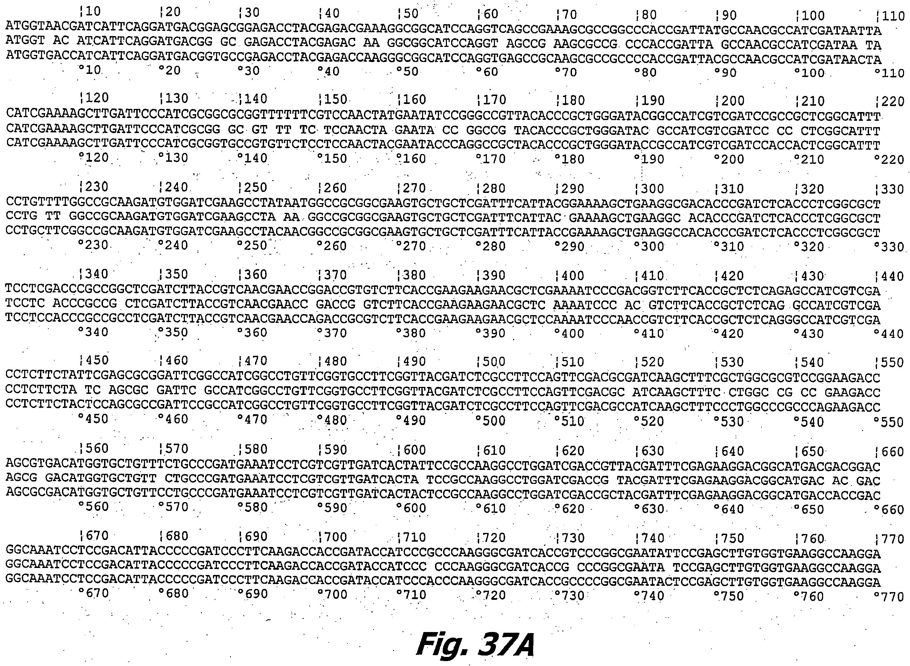

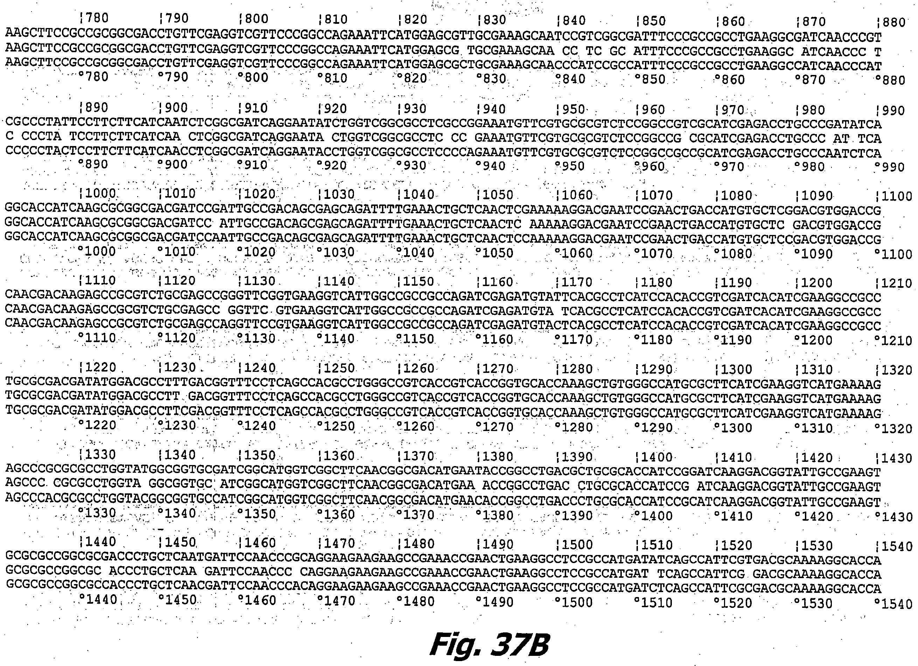

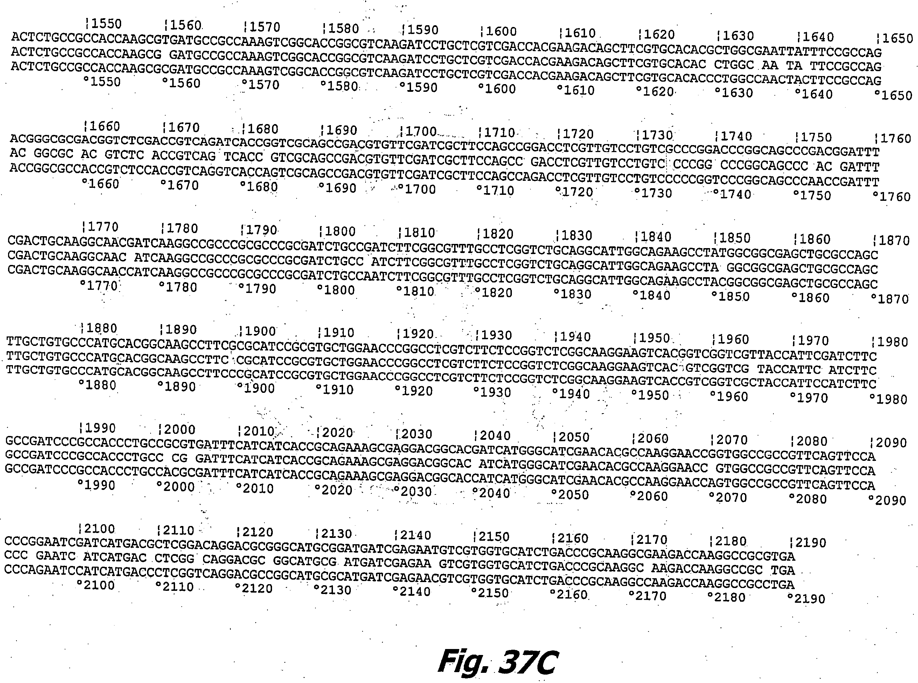

Using the results from Table P, a close examination of the wild type *Agrobacterium* AS sequence revealed that 125 codons were considered underrepresented (below the threshold of 0.5) in corn and soybeans (Table Q). These underrepresented codons were replaced by more prevalent codons as defined above. The modified nucleotide sequence is shown in Figure 36. Using bioinformatics tools, the resulting sequence was assembled and analyzed for integrity by translation and alignment of the nucleotide and protein sequences with the corresponding wild type AS sequences. While, the protein sequence was unchanged the nucleotide sequence of the optimized sequence had 94% identity with the wild type Agrobacterium AS sequence (Figure 37). The optimized nucleotide sequence was analyzed for the absence of cryptic polyadenylation signals (AATAAA, AATAAT) and cryptic introns using Lasergene EditSeq (DNASTAR, Inc., Madison, WI) and Grail2 (Oak Ridge National Laboratory, Oak Ridge, TN), respectively. No cryptic signals were found.

The modified nucleotide sequence is synthesized using techniques well known in the art or by commercial providers such as Egea Biosciencesces, Inc. (San Diego, California). The resulting nucleotide is cloned into an appropriate expression vector and tested for efficacy in corn, soybeans and *Arabidopsis* using procedures detailed in earlier examples of this specification.

**Table P: Relative codon usage frequencies in maize and soybean seed-expressed genes¹.**

| **Codon** | **AA** | **Maize Seed** | **Soy Seed** | **Codon** | **AA** | **Maize Seed** | **Soy Seed** |
|---|---|---|---|---|---|---|---|
| TTT | F | 0.4211 | 0.7348 | ATC | I | 1.7143 | 1.0563 |
| TTC | F | 1.5789 | 1.2652 | ATA | I | 0.3673 | 0.6654 |
| TTA | L | 0.4557 | 0.3875 | ATG | M | 1.0000 | 1.0000 |
| TTG | L | 0.9494 | 1.2060 | ACT | T | 0.6153 | 1.0008 |
| TCT | S | 0.9624 | 1.4851 | ACC | T | 1.2213 | 2.1020 |
| TCC | S | 1.3707 | 1.1249 | ACA | T | 0.8372 | 0.7146 |
| TCA | S | 0.9107 | 1.0044 | ACG | T | 1.3262 | 0.1826 |
| TCG | S | 0.7851 | 0.3266 | AAT | N | 0.2885 | 0.5409 |
| TAT | Y | 0.2455 | 0.6861 | AAC | N | 1.7115 | 1.4591 |
| TAC | Y | 1.7545 | 1.3139 | AAA | K | 0.5333 | 0.9030 |
| TGT | C | 0.2778 | 0.7572 | AAG | K | 1.4667 | 1.0970 |
| TGC | C | 1.7222 | 1.2428 | AGT | S | 0.2679 | 0.9714 |
| TGG | W | 1.0000 | 1.0000 | AGC | S | 1.7032 | 1.0876 |
| CTT | L | 0.7975 | 1.6298 | AGA | R | 0.3913 | 1.9459 |
| CTC | L | 1.0610 | 1.6301 | AGG | R | 2.9185 | 1.3087 |
| CTA | L | 0.8544 | 0.5905 | GTT | V | 0.5714 | 1.2381 |
| CTG | L | 1.8820 | 0.5562 | GTC | V | 1.0119 | 0.6864 |
| CCT | P | 0.6500 | 1.5822 | GTA | V | 0.3810 | 0.3472 |
| CCC | P | 0.8520 | 0.7694 | GTG | V | 2.0357 | 1.7284 |
| CCA | P | 1.2240 | 1.5838 | GCT | A | 0.9876 | 1.3583 |
| CCG | P | 1.2740 | 0.0645 | GCC | A | 1.1618 | 1.1283 |
| CAT | H | 0.8438 | 0.6066 | GCA | A | 0.8011 | 1.2898 |
| CAC | H | 1.1563 | 1.3934 | GCG | A | 1.0495 | 0.2235 |
| CAA | Q | 0.8639 | 1.2162 | GAT | D | 0.8500 | 0.9523 |
| CAG | Q | 1.1361 | 0.7838 | GAC | D | 1.1500 | 1.0477 |
| CGT | R | 0.2582 | 0.5903 | GAA | E | 0.6818 | 1.0463 |
| CGC | R | 1.0082 | 1.1159 | GAG | E | 1.3182 | 0.9537 |
| CGA | R | 0.1957 | 0.6700 | GGT | G | 1.1268 | 1.1431 |
| CGG | R | 1.2283 | 0.3692 | GGC | G | 1.8758 | 0.6577 |
| ATT | I | 0.9184 | 1.2783 | GGA | G | 0.3085 | 1.2759 |
| ATC | I | 1.7143 | 1.0563 | GGG | G | 0.6889 | 0.9233 |

| | | | | | | | |
|---|---|---|---|---|---|---|---|
| ¹ The relative codon frequencies are represented in the expected value format. This means that if there are four codons that encode a given amino acid, and they are used equally well, each codon is expected to account for 25% (0.25). Due to the redundancy, 0.25 was normalized to 1 to give a relative score for each codon as compared to all codons that encode that amino acid. In real life if a codon is more prevalent than the other choices for a given amino acid, it would get a number>1. And if it is less preferred than the other codons for the amino acid, it would get a number <1. | | | | | | | |

**Table Q. Underrepresented Agro AS codons and modifications for improved seed expression².**

| **Codon** | **Codon (wt)** | **Amino Acid** | **Modified Codon** | **Underrep in Crop²** | **Codon** | **Codon (wt)** | **Amino Acid** | **Modified Codon** | **Underrep in Crop** | **Codon** | **Codon (wt)** | **Amino Acid** | **Modified Codon** | **Underrep in Crop** |
|---|---|---|---|---|---|---|---|---|---|---|---|---|---|---|
| 2 | GTA | V | GTG | corn, soy | 177 | TCG | S | TCC | soy | 481 | GCG | A | GCC | soy |
| 3 | ACG | T | ACC | soy | 179 | GCG | A | GCC | soy | 485 | AAT | N | AAC | corn, soy |
| 9 | GGA | G | GGT | corn | 180 | CGT | R | CGC | corn | 489 | CCG | P | CCA | soy |
| 10 | GCG | A | GCC | soy | 181 | CCG | P | CCA | soy | 504 | ATA | I | ATC | corn |
| 15 | ACG | T | ACC | soy | 185 | CGT | R | CGC | corn | 508 | CGT | R | CGC | corn |
| 16 | AAA | K | AAG | com | 190 | TTT | F | TTC | corn | 520 | CGT | R | CGC | corn |
| 21 | GTC | V | GTG | soy | 201 | TAT | Y | TAC | corn | 543 | ACG | T | ACC | soy |
| 23 | CGA | R | CGC | corn | 209 | CGT | R | CGC | corn | 545 | GCG | A | GCC | soy |
| 26 | CGG | R | CGC | soy | 218 | ACG | T | ACC | soy | 546 | AAT | N | AAC | corn, soy |
| 30 | TAT | Y | TAC | corn | 219 | ACG | T | ACC | soy | 547 | TAT | Y | TAC | corn |
| 36 | AAT | N | AAC | corn, soy | 238 | CCG | P | CCA | soy | 551 | ACG | T | ACC | soy |
| 46 | GGC | G | GGT | soy | 244 | CGT | R | CGC | corn | 553 | GCG | A | GCC | soy |
| 47 | GCG | A | GCC | soy | 248 | TAT | Y | TAC | corn | 554 | ACG | T | ACC | soy |
| 48 | GTT | V | GTG | corn | 276 | CGT | R | CGC | corn | 556 | TCG | S | TCC | soy |
| 49 | TTT | F | TTC | corn | 280 | AAT | N | AAC | corn, soy | 559 | AGA | R | AGG | corn |
| 50 | TCG | S | TCC | soy | 281 | CCG | P | CCA | soy | 561 | CCG | P | CCA | soy |
| 53 | TAT | Y | TAC | corn | 282 | TCG | S | TCC | soy | 572 | CCG | P | CCA | soy |
| 55 | TAT | Y | TAC | corn | 283 | GCG | A | GCC | soy | 578 | TCG | S | TCC | soy |
| 56 | CCG | P | CCA | soy | 290 | GCG | A | GCC | soy | 580 | GGA | G | GGT | corn |
| 58 | CGT | R | CGC | corn | 293 | CCG | P | CCA | soy | 584 | CCG | P | CCA | Soy |
| 64 | ACG | T | ACC | soy | 294 | TCG | S | TCC | soy | 585 | ACG | T | ACC | Soy |
| 69 | CCG | P | CCA | soy | 296 | TAT | Y | TAC | corn | 592 | ACG | T | ACC | Soy |
| 70 | CCG | P | CCA | soy | 301 | AAT | N | AAC | corn, soy | 602 | CCG | P | CCA | Soy |
| 75 | TGT | C | TGC | corn | 307 | TAT | Y | TAC | corn | 617 | TAT | Y | TAC | Corn |
| 76 | TTT | F | TTC | corn | 312 | TCG | S | TCC | soy | 633 | TCG | S | TCC | Soy |
| 85 | TAT | Y | TAC | corn | 313 | CCG | P | CCA | soy | 652 | ACG | T | ACC | Soy |
| 86 | AAT | N | AAC | corn, soy | 322 | CGT | R | CGC | corn | 655 | CGT | R | CGC | Com |
| 97 | ACG | T | ACC | soy | 328 | CCG | P | CCA | soy | 658 | TCG | S | TCC | Soy |
| 102 | GCG | A | GCC | soy | 329 | ATA | I | ATC | corn | 667 | CCG | P | CCA | Soy |
| 112 | TCG | S | TCC | soy | 339 | CCG | P | CCA | soy | 668 | CGT | R | CGC | Corn |
| 115 | CGG | R | CGC | soy | 352 | TCG | S | TCC | soy | 680 | ACG | T | ACC | Soy |
| 123 | CCG | P | CCA | soy | 363 | TCG | S | TCC | soy | 690 | CCG | P | CCA | Soy |
| 125 | CGT | R | CGC | corn | 376 | CCG | P | CCA | soy | 698 | CCG | P | CCA | Soy |
| 133 | TCG | S | TCC | soy | 378 | TCG | S | TCC | soy | 700 | TCG | S | TCC | Soy |
| 136 | CCG | P | CCA | soy | 390 | TAT | Y | TAC | corn | 703 | ACG | T | ACC | Soy |
| 137 | ACG | T | ACC | soy | 411 | TTT | F | TTC | corn | 705 | GGA | G | GGT | Corn |
| 143 | AGA | R | AGG | corn | 442 | CCG | P | CCA | soy | 708 | GCG | A | GCC | Soy |
| 150 | TAT | Y | TAC | corn | 446 | TAT | Y | TAC | corn | 711 | CGG | R | CGC | Soy |
| 151 | TCG | S | TCC | soy | 449 | GCG | A | GCC | soy | 715 | AAT | N | AAC | corn, soy |
| 153 | GCG | A | GCC | soy | 460 | AAT | N | AAC | corn, soy | 724 | GCG | A | GCC | Soy |
| 155 | TCG | S | TCC | soy | 464 | ACG | T | ACC | soy | 729 | GCG | A | GCC | Soy |
| 173 | GCG | A | GCC | soy | 469 | CGG | R | CGC | soy | | | | | |

| | | | | | | | | | | | | | | |
|---|---|---|---|---|---|---|---|---|---|---|---|---|---|---|
| ² The columns titled "Underrep in Crop" indicate in which crop (maize or soybean) a particular codon is underrepresented. | | | | | | | | | | | | | | |

### EXAMPLE 11: Preparation of a Transformation Vector Comprising Monomeric Rhizobium meliloti Anthranilate Synthase.

### Gene Cloning of Rhizobium meliloti

A stab culture of *Rhizobium meliloti* 1021 obtained from ATCC was used to streak a YM media (10 g mannitol, 0.5 g K₂BPO₄, 0.2 g MgSO₄-7H₂O, 1.0 g yeast extract, 0.2 g NaCl,, 88 mg FeCl₃-6H₂O, 15 g agar per 1 L) plate. This plate was grown for two days at 30°C. A single colony was used to inoculate 1 liter of YM media. This culture was grown overnight at 30°C. The cell pellet was spun down at 5,000 X g for 10 minute and frozen at -20°C. The Qiagen Genomic-tip DNA kit (Qiagen Inc., Valencia, California) was used to extract genomic DNA according to the August 1999 Qiagen Genomic DNA Handbook (p. 42).

A PCR reaction was used to amplify the gene. The primers used were Rhizo F2: ATGGCAGCGGTAATTCTGGAAG (SEQ ID NO: 138) and Rhizo R8: TCAGGCTGCCTTGGTCTTC (SEQ ID NO: 139). The resulting PCR fragment was cloned into the pGEM (Promega Corp., Madison, WI) vector.

Finally, the PCR product in pGEM was amplified using PCR with the following primers: Rhizo NcoI ACTGACTCCATGGCAGCGGTAATTCTGGAA (SEQ ID NO: 140) and RhizoSpeI: CTGACTAGTTCAGGCTGCTT (SEQ ID NO: 141) and the product was cloned into TOPO 2.1 PCR vector (Invitrogen Corp., Grand Island, NY).

### Vector Construction

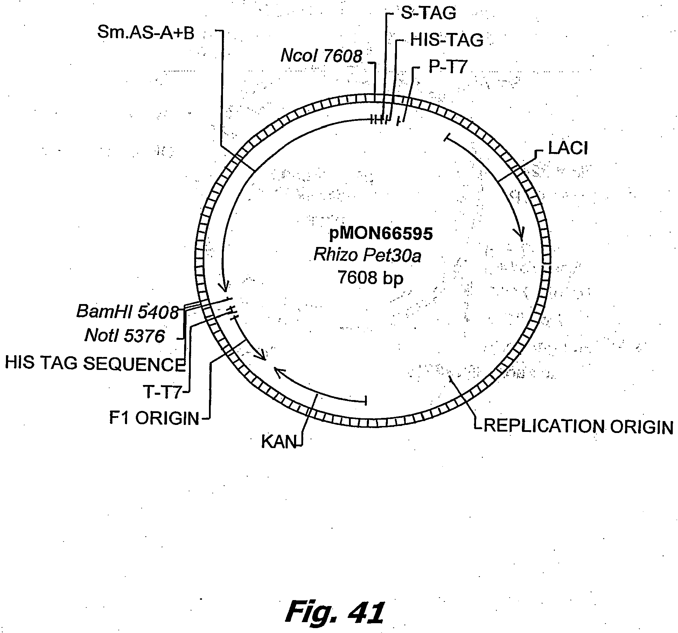

The vector containing the *Rhizobium* gene in the TOPO 2.1 vector was digested with SpeI and a klenow reaction was performed to blunt the site. The DNA was PCR purified (Qiagen PCR purification kit and MinElute Handbook, 2001) and then digested with NcoI. This fragment was cloned into pET30a at the EcoRV and NcoI site creating pMON66595 (Figure 41).

The *Anabidopsis* transformation vector was created in several steps by first digesting pMON13773 (Figure 8) with NcoI/EcoRI to generate a backbone piece. pMON66595 was digested with NcoI and EcoRI and the larger portion of the *Rhizobium* AS gene (approximately 2000 base pairs) was removed. The two pieces were then ligated together. A positive clone was digested with EcoRI and treated with calf intestinal phosphatase (CIP). The second fragment of the *Rhizobium* gene was removed by digesting pMON66595 with EcoRI and keeping the approximately 200 base pair piece. The two fragments were ligated together and the resultant clones were sequenced to check for correct orientation of the small *Rhizobium* fragment.

One of the above clones, having correct sequence and orientation, was digested with BglII and NcoI. Then pMON58046 (Figure 12) was digested with BglII and NcoI and the CTP2 transit peptide fragment removed. These fragments were ligated together to complete the cassette.

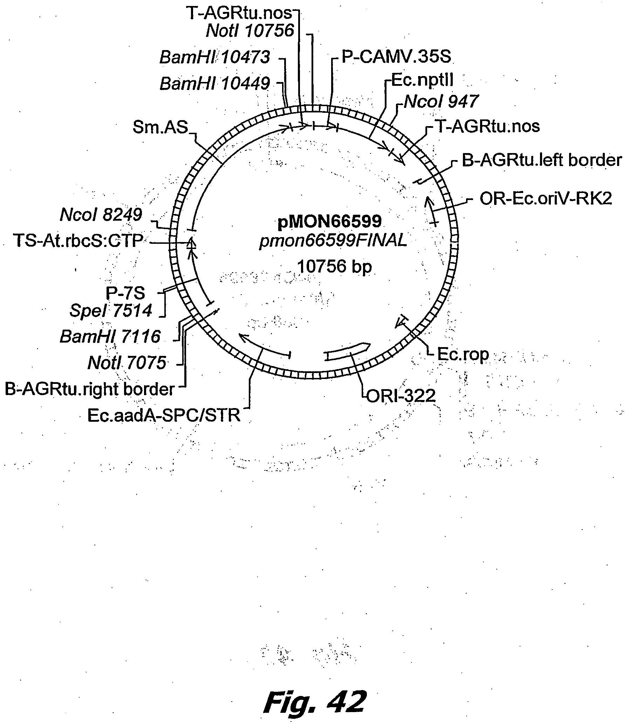

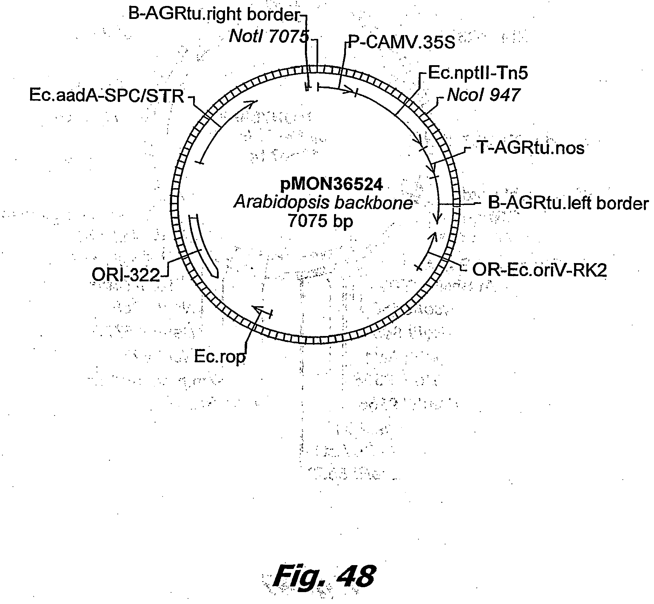

The ligated fragments were digested with NotI to remove the cassette. They were then ligated into pMON36524 (Figure 48) at the CIP treated NotI site. These clones were digested to find a cassette inserted in the same orientation as the NPTII gene creating pMON66599 (Figure 42).

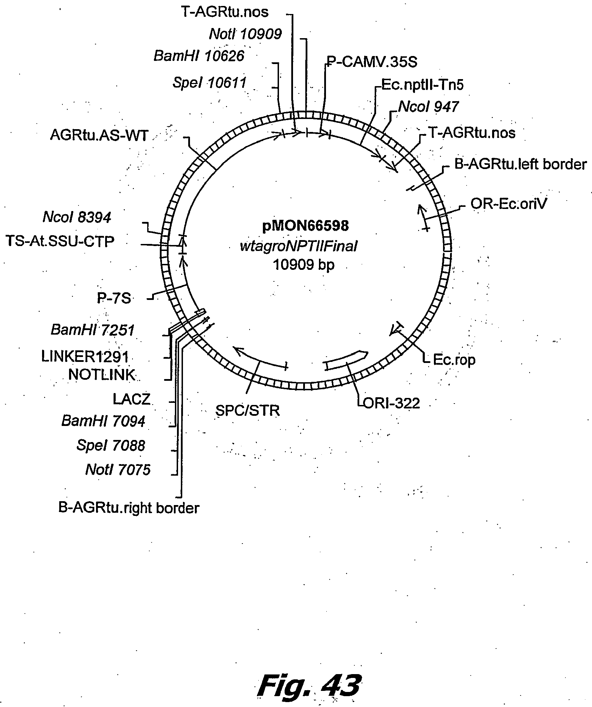

The vector pMON66599 was then transformed into *Agrobacterium* and used to *transform Arabidopsis.* The control construct in this experiment was pMON66598 (Figure 43), which is the same cassette insert as described for pMON66599, except containing the *Agrobacterium* AS wild type gene.

### Complementation Assay

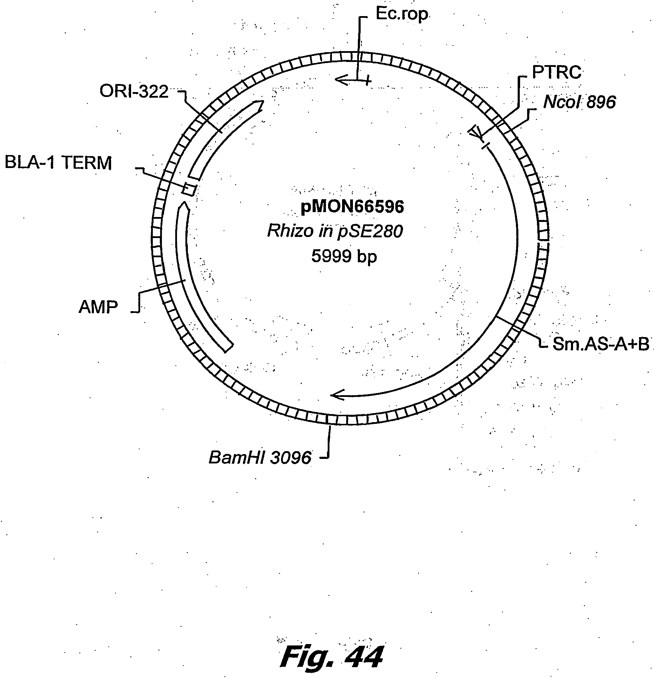

The *Hizobium* AS gene from pMON66595 was excised with BamHI and NcoI and cloned into the corresponding sites of the pSE280 vector (Invitrogen), creating pMON66596 (Figure 44). pMON66596 was then transformed into a mutant *E. coli* strain, EMG2 ΔtrpE (created from the EMG strain WT K12, F+, which was obtained from ATCC) showing that the homomeric gene complements the genome of the trp-strain.

### Activity Assay

pMON66596 was transformed into BL-21 cells and induced with IPTG to express protein. The crude cell extract was assayed by HPLC and found to have activity and an IC₅₀ around 10 µM trp (Poulson, Journal of Chromatography, 547:155-160 (1991)).

### Protein Expression and Purification

His-tagged protein was expressed by inducing pMON66595 with IPTG. Protein purification was completed using native conditions outlined in the QIAexpressionist 2002 Handbook and nickel resin (Ni-NTA Spin Handbook, 2000). Rabbit sera show recognition of purified protein.

Plants are transformed with the vector containing the *Rhizobium* anthranilate synthase gene, as in Examples 2, 3, and 6, and show elevated levels of tryptophan in the seed.

### EXAMPLE 12: Corn Transformation with a Vector Containing Maize Anthranilate Synthase α-Subunit Gene and a Maize Anthranilate Synthase β-Subunit Gene.

To create a shuttle vector containing the coding sequence for maize anthranilate synthase α-subunit, pMON65150, was digested with both EcoRI and SacII. The resulting 6195 base pair fragment was gel purified and then dephosphorylated. The plasmid pMON66604 was digested with both EcoRI and SacII, to generate a 1077 base pair fragment that was gel purified. The 1077 base pair fragment was then ligated in the sticky-ended 6195 base pair fragment containing the maize ASα coding sequence to generate pMON67149, a maize L3 (oleosin) promoter-maize hsp70 intron-maizeASα-Tr7 3' UTR expression vector. This vector, was subsequently digested with XhoI, resulting in a 4364 base pair DNA fragment containing the maize oleosin promoter, maize heat shock protein 70 intron, maize anthranilate α-subunit coding sequence, and the Tr7 3'UTR.

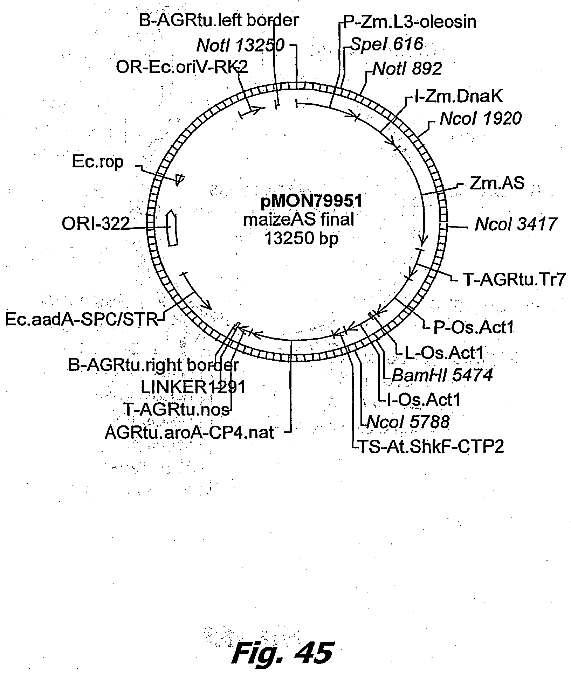

The plasmid pMON30167 was digested with XhoI resulting in an 8.89 Kb DNA fragment. This fragment was then ligated to the 4964 base pair fragment to create pMON79951 (Figure 45), a transformation vector containing L3 promotor-zmhsp70 intron-maizeASα-Tr7 3' UTR.

The coding sequence for a maize anthranilate synthase β-subunit was isolated by PCR from a Monsanto proprietary cDNA library, using the following primers: 5' primer 5'TGCTGA**CCATGG**CCTGCTCCCACATCGTCG3' (SEQ ID NO: 142), which contains the NcoI restriction site (shown in bold), and 3' primer 5'CAGT**GAATTC**CTACGAACGCTGCTTCTCCAGTTC3' (SEQ ID NO: 143), which contains the EcoRI restriction site (shown in bold).

The PCR parameters used were 2°/second to 95°; 95° for 5 minutes; 2°/second to 95°; 95° for 30 seconds; 2°/second to 55°; 55° for 45sec; 2°/second to 72°; 72° for 55 seconds; cycle 25 times starting at the third step; 2°/second to 72°; 72° for 10 minutes; 2°/second to 4° forever (all temperatures shown are in °C, unless otherwise noted). The PCR mix contained: 1 µl of miniprep (Qiagen method) DNA from pMON79952, 1.5 µl of each primer (10µM stock) 5 µl of Roche 10X PCR buffer with magnesium chloride, 2 µl 10mM dNTP mix, 1µl Hi-Fi Taq mix (Roche Expand High Fidelity PCR System # 1732650) and water to a total volume of 50µl. The resulting PCR product was ligated into the pGEM-T vector (Promega pGEM--T Vector System I #A3600). Sequencing the above-described fragment revealed that it was missing restriction sites. The PCR was performed again using the pGEM clone as a template and the product cloned into the TOPO2.1 PCR Vector (Invitrogen TOPO TA Cloning Kit pCR 2.1-TOPO vector #45-0641). This clone was confirmed by sequencing. The resulting vector, containing maize ASβ, was named pMON66592. This vector was then digested with both NcoI and EcoRI, to generate an 850 base pair (bp) fragment. After isolation, the ends of the 850 bp fragment were made blunt and dephosphorylated: The plasmid pMON79953 was digested with BamHI and SmaI, to generate a 5353 bp fragment. After isolation, the ends of the 5353 bp fragment were made blunt. The 850 bp fragment containing the ASβ gene was then ligated into the blunt-ended 5353 bp pMON79953 fragment to generate pMON79954, a maize L3 promotor-zmhsp70 intron-maizeASβ-Tr7 3' UTR vector.

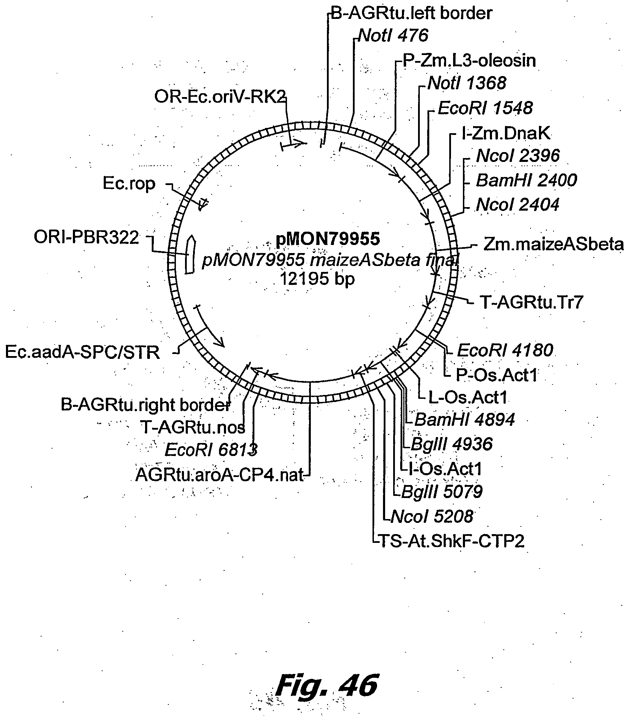

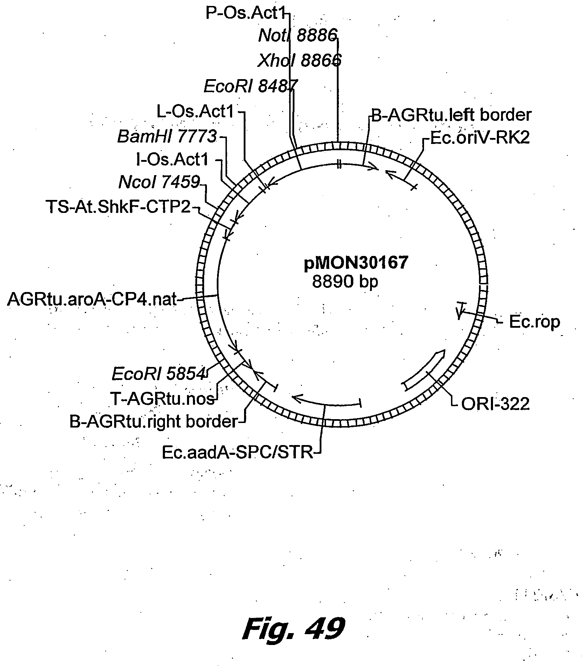

The vector pMON79954, was subsequently digested with XhoI to generate a 2907 base pair DNA fragment containing the maize oleosin promoter, maize hsp70 intron, maize ASβ coding sequence, and the Tr7 3' UTR. The plasmid pMON30167 (Figure 49) was digested with XhoI to generate an 8.89 Kb fragment. The two fragments were ligated together to generate pMON79955 (Figure 46), a transformation vector containing L3 promotor-zmhsp70 intron-maizeASβ-Tr7 3' UTR.

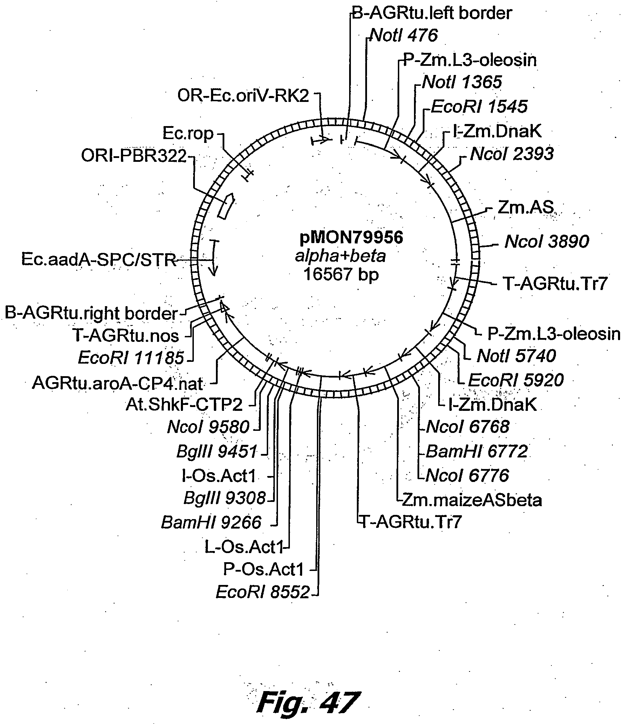

To create the maize ASα and ASP stacking vector, pMON79955 was digested with HindIII, to generate a 16.57 Kb fragment. The fragment was made blunt and dephosphorylated. The plasmid pMON67149 was digested with XhoI, to generate a 4364 base pair DNA fragment, which was subsequently blunt ended. The two fragments were ligated together to create pMON79956 (Figure 47), a final transformation vector containing maize L3 promotor-zmhsp70 intron-maizeASα-Tr7 3' UTR stacked with maize L3 promotor-zmhsp70 intron-maizeASβ-Tr7 3' UTR.

### Maize Transformation Using Agrobacterium tumefaciens

Maize plants (inbred line LH198/Hi11) are grown in a greenhouse under standard practices. The ears of the plants are harvested when the embryos are 1.5 to 2.0 mm in length, usually 10-15 days after pollination. The ears are surface sterilized by spraying or soaking in 80% ethanol.

The immature embryos are isolated from individual kernels using methods known to those of skill in the art. Immature embryos are cultured on medium 211 (N6 salts, 2% sucrose, 1 mg/L 2,4-dichlorophenyoxyacetic acid (2,4-D), 0.5 mg/L niacin, 1.0 mg/L thiamine-HCI, 0.91 g/L L-asparagine, 100 mg/L: myo-inositol, 0.5 g/L MES, 100 mg/L casein hydrolysate, 1.6 g/L MgC12, 0.69 g/L L-proline, 2 g/L GELGRO tm, pH 5.8) containing 16.9 mg/L AgNO3 (designated medium 2112V) for 3-6 days prior to transformation.

Methods of *Agrobacterium* mediated transformation of maize cells and other monocots are known (U.S. Patents 5,591,616 and 5,981,840; and EP 0 672 752). The *Agrobacterium* strain ABI, and an *Agrobacterium tumefaciens* binary vector system are used for the transformations.

Prior to co-culture with the maize embryo cells, *Agrobacterium* cells are grown at 28°C in LB (DIFCO) liquid medium containing approximately 50 µg/ml kanamycin and 100 µg/ml spectinomycin to select for maintenance of the modified Ti plasmid and binary vector. Prior to inoculation of maize cells the *Agrobacterium* cells are grown overnight at room temperature in AB medium (Chilton et al., Proc. Nat. Acad. Sci. (U.S.A.), 71:3672-3676 (1974)) comprising appropriate antibiotics for plasmid maintenance and 200 µM acetosyringone. Immediately prior to inoculation the *Agrobacterium* cells are pelleted by centrifugation, washed in ½ MSVI medium (2.2 g/L GIBCO MS (Murashige and Skoog, Physiol. Plant 15:473-497 (1962)) basal salts, 2 mg/L glycine, 0.5 g/L niacin, 0.5 g/L L-pyridoxin-HCl, 0.1 mg/L thiamine, 115 g/L L-proline, 10 g/L D-glucose, and 10 g/L sucrose, pH 5.4) containing 200 µM acetosyringone.

The immature maize embryos are excised, immersed in an *Agrobacterium* suspension in 1/₂MSPL medium and incubated at room temperature with *Agrobacterium* for approximately 5 minutes.

Following *Agrobacterium* infection and co-culture, the embryos are transferred to type II delay medium for 5 to 7 days and cultured at 27°C in the dark. The delay medium consists of MS basal salts containing 2.0 mg/L 2,4-D (GIBCO), 100 mg/L-casamino acids, 12 mM proline, 500 mg/L carbenicillin and 20 µM silver thiosulfate. All media chemicals were tissue culture grade. Once signs of type II callus initiation from immature embryos are observed, as defined by Selman et al., in The Maize Handbook, Freeling and Walbot, eds., Springer Verlag, p. 672 (1994), the coleoptiles are removed from the embryos. The embryos are then transferred to MS medium containing 2.0 mg/L 2,4-D, 12 mM proline, 20 µM silver thiosulfate, 500 mg/L carbenicillin and 0.5 mM glyphosate (Monsanto Company, St. Louis, MO) and incubated at 27°C in the dark for 2 weeks.

Embryos forming callus are transferred to the MS medium described above, but additionally containing 1.0 mM glyphosate. The cultures are then incubated for 2 weeks in the dark at 27°C. The embryos still having callus are then transferred to MS medium containing 3.0 mM glyphosate for an additional 2 weeks.

Plant regeneration is achieved by transferring the callus to MS medium containing 0.1 mg/L 2,4-D and 0.1 µM abscisic acid (ABA) for 2 weeks and then to MS medium containing 6% sucrose and no 2,4-D for another 2 weeks. Both incubations are done in the dark at 27°C to permit somatic embryo, maturation and conversion in the regeneration process.

Somatic embryos that are ready to germinate are transferred to hormone-free MS medium, and incubated in the light until shoots with attached roots are produced. After approximately 2 to 3 weeks, plantlets are produced.

Plantlets are then transferred to the greenhouse and grown under standard greenhouse conditions.

### Analysis of Amino Acid Content of R1 Seed

Several transgenic corn lines were established for each vector and propagated through the number of generations. These lines are grown and self-pollinated to generate homozygous lines. At each generation, expression of the transgenes are determined using western blot analysis on immature seed and mature R1 seed is produced and analyzed for free amino acid content using fluorescence detection as described in Agilent Technologies Technical Bulletin REV14. Maize seeds expressing ASα protein generate elevated amounts of tryptophan relative to baseline levels (corresponding to negative isolines and nontransgenic controls). Baseline free tryptophan levels for corn range from about 5 to about 25 ppm.

## Claims

1. A transgenic soybean plant comprising a DNA molecule encoding an anthranilate synthase, wherein the DNA molecule encodes the protein of SEQ ID NO: 66 and wherein the levels of free L-tryptophan in a plant cell, plant tissue, plant part or plant are levels that are about 5 to 150 times the levels found in an untransformed plant cell, plant tissue, plant part or plant.

2. The transgenic soybean plant of claim 1, wherein the DNA molecule is one of SEQ ID NOs: 67 and 68.

3. A transgenic soybean plant comprising a DNA construct comprising a first expression cassette, wherein the first expression cassette in operable linkage comprises (i) a heterologous promoter; (ii) a DNA molecule encoding an anthranilate synthase, wherein the DNA molecule encodes the protein of SEQ ID NO: 66 or wherein the DNA molecule is one of SEQ ID NOs: 67 and 68; and (iii) a transcriptional terminator and wherein the levels of free L-tryptophan in a plant cell, plant tissue, plant part or plant are levels that are about 5 to 150 times the levels found in an untransformed plant cell, plant tissue, plant part or plant.

4. The transgenic soybean plant of claim 3, further comprising a second expression cassette in operable linkage comprising (i) a heterologous promoter; (ii) a DNA molecule encoding an anthranilate synthase β-domain protein; and (iii) a transcriptional terminator.

5. The transgenic soybean plant of claim 4, wherein the DNA molecule encoding an anthranilate synthase α-domain comprises SEQ ID NO: 67.

6. The transgenic soybean plant of claim 4, wherein the β-domain protein is SEQ ID NO: 105.

7. A method for altering the tryptophan content in a soybean plant comprising:
a. introducing into regenerable cells of a soybean plant a transgene comprising a DNA molecule encoding an anthranilate synthase, wherein the DNA molecule encodes the protein of SEQ ID NO: 66 or wherein the DNA molecule is one of SEQ ID NOs: 67 and 68, wherein the DNA is operably linked to a promoter functional in a plant cell, to yield transformed plant cells; and
b. regenerating a soybean plant from the transformed plant cells wherein the cells of the soybean plant express the anthranilate synthase encoded by the DNA in an amount effective to increase the tryptophan content in the soybean plant relative to the tryptophan content in a second soybean plant of the same or similar genetic background that does not include the transgene, wherein the levels of free L-tryptophan in a plant cell, plant tissue, plant part or plant are levels that are about 5 to 150 times the levels found in an untransformed plant cell, plant tissue, plant part or plant.

8. The method of claim 7, wherein the anthranilate synthase comprises a chimeric monomeric anthranilate synthase comprising a fusion of an anthranilate synthase α-domain of a first species and an anthranilate synthase β-domain from a second species, wherein one of the species is *Zea mays.*

9. A method for making an animal feed or a human food comprising:
a. introducing into regenerable cells of a soybean plant a transgene comprising a DNA molecule encoding an anthranilate synthase, wherein the DNA molecule encodes the protein of SEQ ID NO: 66 or wherein the DNA molecule is one of SEQ ID NOs: 67 and 68, wherein the DNA molecule is operably linked to a promoter functional in a plant cell, to yield transformed plant cells; and
b. regenerating a soybean plant from the transformed plant cells wherein the cells of the soybean plant express the anthranilate synthase encoded by the DNA molecule in an amount effective to increase the tryptophan content in the soybean plant relative to the tryptophan content in a second soybean plant of the same or similar genetic background that does not include the transgene, wherein the levels of free L-tryptophan in a plant cell, plant tissue, plant part or plant are levels that are about 5 to 150 times the levels found in an untransformed plant cell, plant tissue, plant part or plant.

10. The method of claim 9, wherein the anthranilate synthase comprises a chimeric monomelic anthranilate synthase comprising a fusion of an anthranilate synthase α-domain of a first species and an anthranilate synthase β-domain from a second species, wherein one of the species is *Zea mays.*

11. The method of claim 9 or 10, wherein at least a portion of the soybean plant carrying the transgene is incorporated into the animal feed or human food.

12. A part of a transgenic soybean plant of one of claims 1 to 6, wherein the part comprises a DNA molecule encoding an anthranilate synthase, wherein the DNA molecule encodes the protein of SEQ ID NO: 66 or wherein the DNA molecule is one of SEQ ID NOs: 67 and 68.

13. Part of a transgenic soybean plant according to claim 12, wherein the part is seeds, leaves stems, roots, tubers or fruits.

## Patentansprüche

1. Transgene Sojabohnenpflanze, welche ein DNA Molekül umfasst, das für eine Anthranilat-Synthase kodiert, wobei das DNA Molekül für ein Protein mit der SEQ ID Nr.: 66 kodiert und in der die Mengen an freiem L-Tryptophan in einer Pflanzenzelle, einem Pflanzengewebe, einem Pflanzenteil oder einer Pflanze etwa das 5-150 fache der Mengen betragen, die in einer nicht-transformierten Pflanzenzelle, einem Pflanzengewebe, einem Pflanzenteil oder einer Pflanze gefunden werden.

2. Transgene Sojabohnenpflanze gemäß Anspruch 1, bei der das DNA Molekül aus den SEQ ID Nrn.: 67 und 68 ausgewählt wurde.

3. Transgene Sojabohnenpflanze, die ein DNA Konstrukt umfasst, das eine erste Expressionskassette umfasst, wobei die erste Expressionskassette in operativer Verknüpfung folgendes umfasst: (i) einen heterologen Promoter; (ii) ein DNA Molekül, das für eine Anthranilat-Synthase kodiert, wobei das DNA Molekül für ein Protein der SEQ ID Nr.: 66 kodiert oder wobei das DNA Molekül eines der SEQ ID Nrn.: 67 oder 68 ist; und (iii) einen transkriptionalen Terminator, und wobei die Mengen an freiem L-Tryptophan in einer Pflanzenzelle, einem Pflanzengewebe, einem Pflanzenteil oder einer Pflanze, etwa das 5-150 fache der Mengen sind, die in einer nicht-transformierten Pflanzenzelle, einem Pflanzengewebe, einem Pflanzenteil oder einer Pflanze gefunden werden.

4. Transgene Sojabohnenpflanze gemäß Anspruch 3, die ferner eine zweite Expressionskassette umfasst, welche in operativer Verknüpfung folgendes umfasst: (i) einen heterologen Promotor; (ii) ein DNA Molekül, das für eine β-Domänen Protein einer Anthranilat-Synthase kodiert; und (iii) einen transkriptionalen Terminator.

5. Transgene Sojabohnenpflanze gemäß Anspruch 4, bei der das DNA Molekül, das für eine α-Domäne einer Anthranilat-Synthase kodiert, SEQ ID Nr.: 67 umfasst.

6. Transgene Sojabohnenpflanze gemäß Anspruch 4, wobei die β-Domäne des Proteins SEQ ID Nr.: 105 ist.

7. Verfahren zur Veränderung des Tryptophangehalts einer Sojabohnenpflanze, Schritte umfassend, bei denen man:
a. ein Transgen in regenerierbare Zellen einer Sojabohnenpflanze einführt, das ein DNA Molekül umfasst, das für eine Anthranilat-Synthase kodiert, wobei das DNA Molekül für ein Protein mit der SEQ ID Nr.: 66 kodiert oder wobei das DNA Molekül eine der SEQ ID Nrn.: 67 oder 68 ist, wobei die DNA operativ mit einem Promotor verknüpft ist, der in einer Pflanzenzelle aktiv ist, wobei eine transformierte Pflanzenzelle erhalten wird; und
b. eine Sojabohnenpflanze aus der transformierten Pflanzenzelle regeneriert, wobei die Zellen der Sojabohnenpflanze die Anthranilat-Synthase, welche durch die DNA kodiert wird, in einer wirksamen Menge exprimieren, wodurch der Tryptophan-Gehalt der Sojabohnenpflanze im Verhältnis zum Tryptophan-Gehalt in einer zweiten Sojabohnenpflanze, welche den gleichen oder ähnlichen genetischen Hintergrund aber nicht das Transgen aufweist, zu erhöhen, wobei die Mengen des freien L-Tryptophan in einer Pflanzenzelle, in einem Pflanzengewebe, in einem Pflanzenteil oder in einer Pflanze etwa das 5-150 fache der Mengen beträgt, die in einer nicht-transformierten Pflanzenzelle, in einem Pflanzengewebe, in einem Pflanzenteil oder in einer Pflanze gefunden werden.

8. Verfahren gemäß Anspruch 7, bei dem die Anthranylat-Synthase eine chimäre monomere Anthranylat-Synthase umfasst, die eine Fusion aus einer α-Domäne einer Anthranylat-Synthase einer ersten Art und einer β-Domäne einer Anthranylat-Synthase einer zweiten Art umfasst, wobei eine der Arten *Zea mays* ist.

9. Verfahren zur Herstellung eines Futter- oder Nahrungsmittels, Schritte umfassend, bei denen man:
a. ein Transgen in regenerierbare Zellen einer Sojabohnenpflanze einführt, das ein DNA Molekül umfasst, das für eine Anthranilat-Synthase kodiert, wobei das DNA Molekül für ein Protein mit der SEQ ID Nr.: 66 kodiert oder wobei das DNA Molekül eine der SEQ ID Nrn.: 67 oder 68 ist, wobei die DNA operativ mit einem Promotor verknüpft ist, der in einer Pflanzenzelle aktiv ist, wobei eine transformierte Pflanzenzelle erhalten wird; und
b. eine Sojabohnenpflanze aus der transformierten Pflanzenzelle regeneriert, wobei die Zellen der Sojabohnenpflanze die Anthranilat-Synthase, welche durch die DNA kodiert wird, in einer wirksamen Menge exprimieren, wodurch der Tryptophan-Gehalt der Sojabohnenpflanze im Verhältnis zum Tryptophan-Gehalt in einer zweiten Sojabohnenpflanze, welche den gleichen oder ähnlichen genetischen Hintergrund aber nicht das Transgen aufweist, zu erhöhen, wobei die Mengen des freien L-Tryptophan in einer Pflanzenzelle, in einem Pflanzengewebe, in einem Pflanzenteil oder in einer Pflanze etwa das 5-150 fache der Mengen beträgt, die in einer nicht-transformierten Pflanzenzelle, in einem Pflanzengewebe, in einem Pflanzenteil oder in einer Pflanze gefunden werden.

10. Verfahren gemäß Anspruch 9, bei dem die Anthranylat-Synthase eine chimäre monomere Anthranylat-Synthase umfasst, die eine Fusion aus einer α-Domäne einer Anthranylat-Synthase einer ersten Art und einer β-Domäne einer Anthranylat-Synthase einer zweiten Art umfasst, wobei eine der Arten *Zea mays* ist.

11. Verfahren gemäß Anspruch 9 oder 10, bei dem mindestens ein Teil der Sojabohnenpflanze, die das Transgen trägt, in das Futter- oder Nahrungsmittel aufgenommen wird.

12. Teil einer transgenen Sojabohnenpflanze gemäß irgendeinem der Ansprüche 1-6, bei dem der Teil ein DNA Molekül umfasst, das für eine Anthanylat-Synthase kodiert, wobei das DNA Molekül für das Protein SEQ ID Nr.: 66 kodiert oder das DNA Molekül eine der SEQ ID Nrn.: 67 oder 68 ist.

13. Teil einer transgenen Sojabohnenpflanze gemäß Anspruch 12, wobei der Teil Samen, Blätter, Stämme, Wurzeln, Knollen oder Früchte ist.

## Revendications

1. Plant de soja transgénique comprenant une molécule d'ADN qui code une anthranilate synthétase, laquelle molécule d'ADN code la protéine présentant la Séquence N° 66, et dans lequel plant les niveaux de L-tryptophane libre dans une cellule, un tissu ou une partie du plant ou chez le plant sont des niveaux qui valent à peu près de 5 à 150 fois les niveaux observés dans une cellule, un tissu ou une partie d'un plant non-transformé ou chez un plant non-transformé.

2. Plant de soja transgénique conforme à la revendication 1, dans lequel la molécule d'ADN est l'une des molécules de Séquences N° 67 et N° 68.

3. Plant de soja transgénique comprenant une construction d'ADN qui comporte une première cassette d'expression, laquelle première cassette d'expression comprend, en raccordement opérationnel :
i) un promoteur hétérologue,
ii) une molécule d'ADN qui code une anthranilate synthétase, laquelle molécule d'ADN code la protéine présentant la Séquence N° 66, ou laquelle molécule d'ADN est l'une des molécules de Séquences N° 67 et N° 68,
iii) et un terminateur de transcription ;
et dans lequel plant les niveaux de L-tryptophane libre dans une cellule, un tissu ou une partie du plant ou chez le plant sont des niveaux qui valent à peu près de 5 à 150 fois les niveaux observés dans une cellule, un tissu ou une partie d'un plant non-transformé ou chez un plant non-transformé.

4. Plant de soja transgénique conforme à la revendication 3, qui comprend en outre une deuxième cassette d'expression comprenant, en raccordement opérationnel :
i) un promoteur hétérologue,
ii) une molécule d'ADN qui code une protéine de domaines β d'anthranilate synthétase,
iii) et un terminateur de transcription.

5. Plant de soja transgénique conforme à la revendication 4, dans lequel la molécule d'ADN qui code un domaine α d'anthranilate synthétase comprend la Séquence N° 67.

6. Plant de soja transgénique conforme à la revendication 4, dans lequel la protéine de domaine P est celle de Séquence N° 105.

7. Procédé visant à modifier la teneur en tryptophane chez un plant de soja, comportant les étapes suivantes :
a) introduire, dans des cellules d'un plant de soja capables de régénération, un transgène comprenant une molécule d'ADN qui code une anthranilate synthétase, laquelle molécule d'ADN code la protéine présentant la Séquence N° 66, ou laquelle molécule d'ADN est l'une des molécules de Séquences N° 67 et N° 68, et lequel ADN est opérationnellement raccordé à un promoteur qui peut fonctionner dans une cellule végétale, afin d'obtenir des cellules de plant transformées ;
b) et régénérer un plant de soja à partir de ces cellules de plant transformées, duquel plant de soja les cellules expriment l'anthranilate synthétase codée par l'ADN en une quantité qui a pour effet d'augmenter la teneur en tryptophane chez le plant de soja, par rapport à la teneur en tryptophane chez un deuxième plant de soja présentant un terrain génétique identique ou similaire, mais n'incluant pas le transgène, et chez lequel plant les niveaux de L-tryptophane libre dans une cellule, un tissu ou une partie du plant ou chez le plant sont des niveaux qui valent à peu près de 5 à 150 fois les niveaux observés dans une cellule, un tissu ou une partie d'un plant non-transformé ou chez un plant non-transformé.

8. Procédé conforme à la revendication 7, dans lequel l'anthranilate synthétase comprend une anthranilate synthétase monomère chimérique comprenant un produit de fusion d'un domaine α d'anthranilate synthétase d'une première espèce et d'un domaine P d'anthranilate synthétase d'une deuxième expèce, l'une de ces espèces étant *Zea mays.*

9. Procédé de fabrication d'un aliment pour animaux ou d'un produit pour l'alimentation humaine, comprenant les étapes suivantes :
a) introduire, dans des cellules d'un plant de soja capables de régénération, un transgène comprenant une molécule d'ADN qui code une anthranilate synthétase, laquelle molécule d'ADN code la protéine présentant la Séquence N° 66, ou laquelle molécule d'ADN est l'une des molécules de Séquences N° 67 et N° 68, et laquelle molécule d'ADN est opérationnellement raccordée à un promoteur qui peut fonctionner dans une cellule végétale, afin d'obtenir des cellules de plant transformées ;
b) et régénérer un plant de soja à partir de ces cellules de plant transformées, duquel plant de soja les cellules expriment l'anthranilate synthétase codée par l'ADN en une quantité qui a pour effet d'augmenter la teneur en tryptophane chez le plant de soja, par rapport à la teneur en tryptophane chez un deuxième plant de soja présentant un terrain génétique identique ou similaire, mais n'incluant pas le transgène, et chez lequel plant les niveaux de L-tryptophane libre dans une cellule, un tissu ou une partie du plant ou chez le plant sont des niveaux qui valent à peu près de 5 à 150 fois les niveaux observés dans une cellule, un tissu ou une partie d'un plant non-transformé ou chez un plant non-transformé.

10. Procédé conforme à la revendication 9, dans lequel l'anthranilate synthétase comprend une anthranilate synthétase monomère chimérique comprenant un produit de fusion d'un domaine α d'anthranilate synthétase d'une première espèce et d'un domaine P d'anthranilate synthétase d'une deuxième expèce, l'une de ces espèces étant *Zea mays.*

11. Procédé conforme à la revendication 9 ou 10, dans lequel au moins une partie du plant de soja portant le transgène est incorporé dans l'aliment pour animaux ou le produit pour alimentation humaine.

12. Partie d'un plant de soja transgénique conforme à l'une des revendications 1 à 6, laquelle partie comprend une molécule d'ADN qui code une anthranilate synthétase, laquelle molécule d'ADN code la protéine présentant la Séquence N° 66, ou laquelle molécule d'ADN est l'une des molécules de Séquences N° 67 et N° 68.

13. Partie de plant de soja transgénique, conforme à la revendication 12, laquelle partie est constituée des semences, des feuilles, des tiges, des racines, des tubercules ou des fruits.
